(19) Europäisches Patentamt
European Patent Office
Office européen des brevets

(11) **EP 4 471 128 A1**

(12) **EUROPEAN PATENT APPLICATION**
published in accordance with Art. 153(4) EPC

(43) Date of publication:
**04.12.2024 Bulletin 2024/49**

(21) Application number: **23746392.2**

(22) Date of filing: **28.01.2023**

(51) International Patent Classification (IPC):
*C12N 5/0781* (2010.01)      *C12N 5/0783* (2010.01)
*C12N 5/078* (2010.01)      *A61K 35/17* (2015.01)
*A61P 35/00* (2006.01)

(52) Cooperative Patent Classification (CPC):
**A61K 35/17; A61P 35/00; C12N 5/06**

(86) International application number:
**PCT/CN2023/073559**

(87) International publication number:
**WO 2023/143515 (03.08.2023 Gazette 2023/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.01.2022 PCT/CN2022/074930**

(71) Applicants:
• **Suzhou Grit Biotechnology Co., Ltd.
Shanghai 201315 (CN)**
• **Zhuhai Tuoyu Biotechnology Co., Ltd.
Shanghai 201315 (CN)**
• **Shanghai Grit Biotechnology Co., Ltd.
Shanghai 201315 (CN)**
• **Shenzhen Grit Biotechnology Co., Ltd.
Shanghai 201315 (CN)**

(72) Inventors:
• **LIU, Yarong
Shanghai 201315 (CN)**
• **SUN, Jingwei
Shanghai 201315 (CN)**
• **SHENG, Yao
Shanghai 201315 (CN)**

(74) Representative: **Jones Day
Rechtsanwälte, Attorneys-at-Law, Patentanwälte
Prinzregentenstrasse 11
80538 München (DE)**

Remarks:
The complete document including Reference
Table(s) and the Sequence Listing(s) can be
downloaded from the EPO website

(54) **MODIFIED TUMOR INFILTRATING LYMPHOCYTE AND USE THEREOF**

(57)     The present application relates to a modified tumor infiltrating lymphocyte and a use thereof, and specifically relates to a method for culturing a tumor infiltrating lymphocyte (TIL), which comprises causing a reduction of activity and/or expression of two or more proteins of the TIL. The present application also relates to a method for preventing and/or treating a tumor by using the tumor infiltrating lymphocyte.

EP 4 471 128 A1

**Description**

**TECHNICAL FIELD**

The present application relates to the field of biomedicine, and specifically to a modified tumor infiltrating lymphocyte and a use thereof.

**BACKGROUND OF THE INVENTION**

Treating tumors by using adoptive autologous transferred tumor infiltrating lymphocytes is an effective approach to treat patients with poor prognosis. However, treating tumors by adoptive autologous transferred tumor infiltrating lymphocytes requires a large number of tumor infiltrating lymphocytes, and the tumor infiltrating lymphocytes from patients' tumors currently have a weak ability to persist and expand in the body, a weak ability to kill target cells, and a limited function due to multiple inhibitions from the tumor microenvironment.

Therefore, how to provide a robust and reliable method for culturing tumor infiltrating lymphocytes is an urgent issue to be solved.

**SUMMARY OF THE INVENTION**

In one aspect, the present application provides a method for culturing tumor infiltrating lymphocytes (TILs), the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the TILs with feeder cells after contacting the TILs with T cell activators and/or T cell growth factors for a period of time.

In one aspect, the present application provides a method for culturing tumor infiltrating lymphocytes (TILs), the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein the TILs comprise TILs obtained by co-culturing with feeder cells after contacting the TILs with T cell activators and/or T cell growth factors for a period of time.

In one aspect, the present application provides a method for culturing tumor infiltrating lymphocytes (TILs), the method comprises co-culturing the TILs with feeder cells after contacting the TILs with T cell activators and/or T cell growth factors for a period of time, wherein the TILs comprise TILs obtained by reducing the expression and/or decreasing the activity of the two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

In one aspect, the present application provides a method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);

(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the second TIL population with feeder cells after contacting the second TIL population with T cell activators and/or T cell growth factors for a period of time, wherein a third TIL population is obtained via the step (B).

In one aspect, the present application provides a method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of the first TIL population derived from tumor tissues and not expanded *in vitro*;

(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the second TIL population with feeder cells after contacting the second TIL population with T cell activators and/or T cell growth factors for a period of time, wherein a third TIL population is obtained via the step (B). In

EP 4 471 128 A1

one aspect, the present application provides

In one aspect, the present application provides a method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the second TIL population with T cell activators and/or T cell growth factors, wherein a third TIL population is obtained via the step (B);
(C) co-culturing the third TIL population with feeder cells, wherein a fourth TIL population is obtained via the step (C).

In one aspect, the present application provides a method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of the first TIL population derived from tumor tissues and not expanded *in vitro;*
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the second TIL population with T cell activators and/or T cell growth factors, wherein a third TIL population is obtained via the step (B);
(C) co-culturing the third TIL population with feeder cells, wherein a fourth TIL population is obtained via the step (C).

In one aspect, the present application provides a method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);
(B) contacting the second TIL population with T cell activators and/or T cell growth factors, wherein a third TIL population is obtained via the step (B);
(C) reducing the expression and/or decreasing the activity of two or more proteins of the third TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein a fourth TIL population is obtained via the step (C);
(D) co-culturing the fourth TIL population with feeder cells, wherein a fifth TIL population is obtained via the step (D).

In one aspect, the present application provides a method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of the first TIL population derived from tumor tissues and not expanded *in vitro;*
(B) contacting the second TIL population with T cell activators and/or T cell growth factors, wherein a third TIL population is obtained via the step (B);
(C) reducing the expression and/or decreasing the activity of two or more proteins of the third TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein a fourth TIL population is obtained via the step (C);
(D) co-culturing the fourth TIL population with feeder cells, wherein a fifth TIL population is obtained via the step (D). In one aspect, the present application provides

In one aspect, the present application provides a method for culturing tumor infiltrating lymphocytes (TILs), the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist.

In one aspect, the present application provides a method for culturing tumor infiltrating lymphocytes (TILs), the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein the TILs comprise TILs obtained by contacting the TILs with a CD28 agonist.

In one aspect, the present application provides a method for culturing tumor infiltrating lymphocytes (TILs), the method comprises contacting the TILs with the CD28 agonist, wherein the TILs comprise TILs obtained by reducing the expression and/or decreasing the activity of the two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

In one aspect, the present application provides a method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist, wherein a third TIL population is obtained via the step (B).

In one aspect, the present application provides a method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of the first TIL population derived from tumor tissues and not expanded *in vitro*;
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist, wherein a third TIL population is obtained via the step (B).

In one aspect, the present application provides a tumor infiltrating lymphocyte (TIL) obtained by the method of the present application.

In one aspect, the present application provides a composition comprising the TIL of the present application.

In one aspect, the present application provides a pharmaceutical composition comprising the TIL of the present application and/or the composition of the present application, and optionally a pharmaceutically acceptable carrier.

In one aspect, the present application provides a method for affecting the tumor cell growth, comprising administering to a subject the TIL of the present application, the composition of the present application and/or the pharmaceutical composition of the present application.

In one aspect, the present application provides a use of the TIL of the present application, the composition of the present application and/or the pharmaceutical composition of the present application in the manufacture of drugs for preventing and/or treating a tumor.

Other aspects and advantages of the present application can be readily perceived by those skilled in the art from the following detailed description. In the following detailed description, only exemplary embodiments of the present application are shown and described. As will be recognized by those skilled in the art, the disclosure of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

## BRIEF DESCRIPTION OF THE DRAWING

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:

FIG. 1 shows the analysis results of the proliferation ability of TILs cultured with feeder cells added at different times.

FIGs. 2 and 3 show the proportion of CD45RA$^-$CCR7$^+$ central memory T cells (Tcm) in the TIL cells obtained by culturing with feeder cells added at 0, 24 or 48 hours after the addition of OKT3 and IL-2.

FIG. 4 shows the proportion of CD4$^+$CD25$^+$Foxp3$^+$ regulatory T cells (Treg) in the TIL cells obtained by culturing with feeder cells added at 0, 24 or 48 hours after the addition of OKT3 and IL-2.

FIGs. 5 and 6 show the proportion of activated T cells in the TIL cells obtained by culturing with feeder cells added at 0, 24 or 48 hours after the addition of OKT3 and IL-2.

FIG. 7 shows the proportion of CD103$^+$CD39$^+$ tumor-specific T cells in the TIL cells obtained by culturing with feeder cells added at 0, 24 or 48 hours after the addition of OKT3 and IL-2.

FIG. 8 shows the proportion of TCF1$^+$ stem cell-like T cells in the TIL cells obtained by culturing with feeder cells added at 0, 24 or 48 hours after the addition of OKT3 and IL-2.

FIG. 9 shows the analysis results of the proliferation ability of the test groups added with different forms of CD28 agonists and the control group.

FIGs. 10 and 11 show the proportion of T cell subpopulations in the TIL cells obtained from culture in the mixed antibody group and the control group, respectively, for TILs from different donor sources.

FIGs. 12 and 13 show the proportion of T cell subpopulations in the TIL cells obtained from culture in the magnetic bead group and the control group, respectively, for TILs from different donor sources.

FIG. 14 shows the proportion of T cell subpopulations in the TIL cells obtained from culture in the nanomatrix group and the control group.

FIG. 15 shows the cell killing ability of the TIL cells obtained from culture in the nanomatrix group and the control group.

FIG. 16 shows the results of intracellular factor expression assay of TIL cells obtained from culture in the mixed antibody group and the control group.

FIGs. 17, 18, 19, and 20 show the results of intracellular factor expression assay of TIL cells obtained from culture in the magnetic bead group and the control group, respectively, for TILs from different donor sources.

FIG. 21 shows the results of intracellular factor expression assay of TIL cells obtained from culture in the nanomatrix group and the control group.

FIG. 22 shows the results of cytokine secretion assay of TIL cells obtained from culture in the nanomatrix group and the control group.

FIG. 23 shows the results of cytokine secretion assay of TIL cells obtained from culture in the nanomatrix group and the control group after co-incubating with tumor cells.

FIGs. 24 and 25 show the results of gene knockout efficiency of TIL cells obtained from culture in the nanomatrix group and the control group, respectively, for TILs from different donor sources.

FIGs. 26, 27 and 28 show the analysis results of the proliferation ability of the test groups upon *in vitro* expansion in different ways in the terminal stimulation stage, respectively, for TILs from different donor sources.

FIGs. 29A to 29B show the fluorescence after expansion of each group of TIL cells edited with combined genes CB and ZC in the absence of any stimulants, for TIL cells derived from donors 504 and 713.

FIGs. 30A to 30D show the fluorescence after expansion of each group of TIL cells edited with combined genes SC and CB in the absence of any stimulants, for TIL cells derived from donors 504, 713, and 316.

FIGs. 31A to 31C show the fluorescence after expansion of each group of TIL cells edited with combined genes SC and TP in the absence of any stimulants, for TIL cells derived from donors 504, 316, and 713.

FIGs. 31D to 31E show the fluorescence after expansion of each group of TIL cells edited with combined genes SC and TP under the stimulation of a CD3 antibody, for TIL cells derived from donors 316, and 713.

FIGs. 32A to 32D show the fluorescence after expansion of each group of TIL cells edited with combined genes SC and ZC in the absence of any stimulants, for TIL cells derived from donors 504, 316, and 713.

FIG. 32E shows the fluorescence after expansion of each group of TIL cells edited with combined genes SC and ZC under the stimulation of the CD3 antibody, for TIL cells derived from donor 316.

FIGs. 33A to 33B show the fluorescence after expansion of each group of TIL cells edited with combined genes TP and CB in the absence of any stimulants, for TIL cells derived from donors 504 and 713.

FIGs. 34A to 34D show the fluorescence after expansion of each group of TIL cells edited with combined genes TP and ZC in the absence of any stimulants, for TIL cells derived from donors 504, 316, and 713.

FIGs. 34E to 34F show the fluorescence after expansion of each group of TIL cells edited with combined genes TP and ZC under the stimulation of the CD3 antibody, for TIL cells derived from donors 316 and 713.

FIGs. 35A to 35C show the test results of the killing ability of TIL cells derived from donors 504 and 713 which were co-cultured with Hey-T30 tumor cells at an effector-target ratio of 1:1 and 1:3.

FIGs. 36A to 36C show the test results of the killing ability of TIL cells derived from donors 504 and 713 which were co-cultured with Hey-T30 tumor cells at an effector-target ratio of 1:1 and 1:3. FIG. 36D shows the test results of the killing ability of TIL cells derived from donor 316 which were co-cultured with A375 tumor cells at an effector-target ratio of 1:1.

FIGs. 37A to 37E show the test results of the killing ability of TIL cells derived from donors 504, 713, and 607 which were co-cultured with Hey-T30 tumor cells at an effector-target ratio of 1:1 and 1:3. FIG. 37F shows the test results of the killing ability of TIL cells derived from donor 316 which were co-cultured with A375 tumor cells at an effector-target ratio of 1:1.

FIGs. 38A to 38D show the test results of the killing ability of TIL cells derived from donors 504 and 713 which were co-cultured with Hey-T30 tumor cells at an effector-target ratio of 1:1 and 1:3. FIG. 38E shows the test results of the killing ability of TIL cells derived from donor 316 which were co-cultured with A375 tumor cells at an effector-target ratio of 1:1.

FIGs. 39A to 39D show the test results of the killing ability of TIL cells derived from donors 504, 713, and 607 which were co-cultured with Hey-T30 tumor cells at an effector-target ratio of 1:1 and 1:3.

FIGs. 40A to 40E show the test results of the killing ability of TIL cells derived from donors 504, 713, and 607 which were co-cultured with Hey-T30 tumor cells at an effector-target ratio of 1:1 and 1:3. FIGs. 40F to 40G show the test results of the killing ability of TIL cells derived from donor 316 which were co-cultured with A375 tumor cells at an effector-target ratio of 1:1 and 1:3.

FIGs. 41A to 41B show the apoptosis assay results of TIL cells from donor 504. The results show that the gene-edited TIL cells may exhibit more significant anti-apoptotic ability.

FIGs. 42A to 42C show the proportion of CD38-positive, PD-1-positive, LAG-3-positive cells in each group of TIL cells, for TIL cells from donors 504 and 713. The results show that the gene-edited TIL cells have a lower proportion of depletion cells.

FIGs. 43A to 43C show the proportion of CD45RO-positive CD62L-positive central memory T cells (Tcm) in each group of TIL cells, for TIL cells from donors 316 and 713. The results show that the gene-edited TIL cells have a higher proportion of central memory T cells.

FIGs. 43D to 43I show the proportion of PD-1-positive, CD38-positive, CD101-positive, TIM-3-positive, and LAG-3-positive cells in each group of TIL cells, for TIL cells from donors 504 and 713. The results show that the gene-edited TIL cells have a lower proportion of depletion cells.

FIGs. 44A to 44B show the proportion of CD45RO-positive CD62L-positive central memory T cells (Tcm) in each group of TIL cells, for TIL cells from donor 713. The results show that the gene-edited TIL cells have a higher proportion of central memory T cells.

FIGs. 44C to 44H show the proportion of PD-1-positive, CD38-positive, CD101-positive, and TIM-3-positive cells in each group of TIL cells, for TIL cells from donors 504, 713, and 607. The results show that the gene-edited TIL cells have a lower proportion of depletion cells.

FIGs. 45A to 45C show the proportion of CD45RO-positive CD62L-positive central memory T cells (Tcm) in each group of TIL cells, for TIL cells from donors 316 and 713. The results show that the gene-edited TIL cells have a higher proportion of central memory T cells.

FIGs. 45D to 45I show the proportion of PD-1-positive, CD38-positive, CD101-positive, TIM-3-positive, and LAG-3-positive cells in each group of TIL cells, for TIL cells from donors 504 and 713. The results show that the gene-edited TIL cells have a lower proportion of depletion cells.

FIGs. 46A to 46D show the proportion of CD3 8-positive, CD101-positive, TIM-3-positive, PD-1-positive, and LAG-3-positive cells in each group of TIL cells, for TIL cells from donors 504, 607, and 713. The results show that the gene-edited TIL cells have a lower proportion of depletion cells.

FIG. 47A shows the proportion of CD45RO-negative CD62L-positive naive T cells in each group of TIL cells, for TIL cells from donor 504. The results show that the gene-edited TIL cells have a higher proportion of naive T cells.

FIG. 47B shows the proportion of CD45RO-positive CD62L-positive central memory T cells (Tcm) in each group of TIL cells, for TIL cells from donor 713. The results show that the gene-edited TIL cells have a higher proportion of central memory T cells.

FIGs. 47C to 47I show the proportion of CD38-positive, CD101-positive, TIM-3-positive, PD-1-positive, and LAG-3-positive cells in each group of TIL cells, for TIL cells from donors 504, 607, and 713. The results show that the gene-edited TIL cells have a lower proportion of depletion cells.

FIGs. 48A to 48B show the relative expression level of CD107a, TNF-$\alpha$, and IFN-$\gamma$ in each group of TIL cells under the stimulation of the CD3 antibody, for TIL cells from donor 713.

FIGs. 49A to 49D show the relative expression level of CD107a, TNF-$\alpha$, and GZMB in each group of TIL cells in the absence of any stimulants, for TIL cells derived from donors 713 and 316.

FIGs. 49E to 49F show the relative expression level of CD107a, TNF-$\alpha$, GZMB, and IFN-$\gamma$ in each group of TIL cells under the stimulation of the CD3 antibody, for TIL cells from donor 713.

FIGs. 50A to 50D show the relative expression level of CD107a, TNF-$\alpha$, GZMB, and IFN-$\gamma$ in each group of TIL cells in the absence of any stimulants, for TIL cells from donors 713 and 316.

FIGs. 50E to 50F show the relative expression level of CD107a, TNF-$\alpha$, GZMB, and IFN-$\gamma$ in each group of TIL cells under the stimulation of the CD3 antibody, for TIL cells from donor 713.

FIGs. 51A to 51D show the relative expression level of CD107a, TNF-$\alpha$, GZMB, and IFN-$\gamma$ in each group of TIL cells in the absence of any stimulants, for TIL cells from donors 713 and 316.

FIGs. 51E to 51F show the relative expression level of CD107a, TNF-$\alpha$, GZMB, and IFN-$\gamma$ in each group of TIL cells under the stimulation of the CD3 antibody, for TIL cells from donor 713.

FIGs. 52A to 52B show the proportion of CD107a, TNF-$\alpha$, and IFN-$\gamma$-expressing cells in each group of TIL cells in the absence of any stimulants, for TIL cells from donor 607.

FIGs. 52C to 52D show the proportion of CD107a, TNF-$\alpha$, GZMB, and IFN-$\gamma$-expressing cells in each group of TIL cells under the stimulation of the CD3 antibody, for TIL cells from donor 607.

FIGs. 53A to 53B show the proportion of CD107a and TNF-$\alpha$-expressing cells in each group of TIL cells in the absence of any stimulants, for TIL cells from donor 607.

FIGs. 53C to 53F show the relative expression level of CD107a, TNF-$\alpha$, GZMB, and IFN-$\gamma$ in each group of TIL cells in the absence of any stimulants, for TIL cells from donors 713 and 316.

FIGs. 53G to 53H show the proportion of CD107a, TNF-$\alpha$, GZMB, and IFN-$\gamma$-expressing cells in each group of TIL cells under the stimulation of the CD3 antibody, for TIL cells from donor 607.

FIG. 54A shows the cell proliferation ability of TIL cells obtained by culturing with feeder cells added at 0, 24 or 48 hours after the addition of OKT3 and IL-2.

FIG. 54B shows the proportion of CD45RA$^-$CCR7$^+$ central memory T cells (Tcm) in the TIL cells obtained by culturing with feeder cells added at 0, 24 or 48 hours after the addition of OKT3 and IL-2.

FIG. 54C shows the proportion of TCF1$^+$ stem cell-like T cells in the TIL cells obtained by culturing with feeder cells added at 0, 24 or 48 hours after the addition of OKT3 and IL-2.

FIG. 54D shows the proportion of CD4$^+$CD25$^+$Foxp3$^+$ regulatory T cells (Treg) in the TIL cells obtained by culturing with feeder cells added at 0, 24 or 48 hours after the addition of OKT3 and IL-2.

FIG. 54E shows the proportion of activated T cells (PD-1$^+$) in the TIL cells obtained by culturing with feeder cells added at 0, 24 or 48 hours after the addition of OKT3 and IL-2.

FIG. 54F shows the proportion of CD103$^+$CD39$^+$ tumor-specific T cells in the TIL cells obtained by culturing with feeder cells added at 0, 24 or 48 hours after the addition of OKT3 and IL-2.

FIG. 54G shows the proportion of activated T cells (CD28$^+$) in the TIL cells obtained by culturing with feeder cells added at 0, 24 or 48 hours after the addition of OKT3 and IL-2.

FIG. 54H shows the proportion of activated T cells (41BB$^+$) in the TIL cells obtained by culturing with feeder cells added at 0, 24 or 48 hours after the addition of OKT3 and IL-2.

FIG. 54I shows the proportion of activated T cells (CD25$^+$) in the TIL cells obtained by culturing with feeder cells added at 0, 24 or 48 hours after the addition of OKT3 and IL-2.

FIG. 54J shows the results of intracellular factor expression assay of TIL cells obtained by culturing with feeder cells added at 0, 24 or 48 hours after the addition of OKT3 and IL-2.

FIG. 54K shows the results of cytokine secretion assay of TIL cells obtained by culturing with feeder cells added at 0, 24 or 48 hours after the addition of OKT3 and IL-2.

FIG. 54L shows the proliferation ability of TIL cells obtained by culturing with feeder cells added at 0, 6, 12, 24, 48, 72 hours, or 5 days after the addition of OKT3 and IL-2.

FIG. 54M shows the proportion of CD8$^+$ T cells in the TIL cells obtained by culturing with feeder cells added at 0, 6, 12, 24, 48, 72 hours, or 5 days after the addition of OKT3 and IL-2.

FIG. 54N shows the proportion of CD45RO$^+$CD62L$^+$ T cells in the TIL cells obtained by culturing with feeder cells added at 0, 6, 12, 24, 48, 72 hours, or 5 days after the addition of OKT3 and IL-2.

FIG. 54O shows the proportion of NK T cells in the TIL cells obtained by culturing with feeder cells added at 0, 6, 12, 24, 48, 72 hours, or 5 days after the addition of OKT3 and IL-2.

FIG. 54P shows the proportion of CD4$^+$CD25$^+$Foxp3$^+$ regulatory T cells (Treg) in the TIL cells obtained by culturing with feeder cells added at 0, 6, 12, 24, 48, 72 hours, or 5 days after the addition of OKT3 and IL-2.

FIG. 54Q shows the cell killing ability of TIL cells obtained by culturing with feeder cells added at 48 hours after the addition of OKT3 and IL-2.

FIGs. 55A to 55B show that both the TP4+ZC3 double-knockout group and the SC3+TP4 double-knockout group exhibited significant tumor suppression ability, with a tumor suppression effect significantly stronger than that of the NT group.

FIG. 55C shows the changes in the body weight of Hey-T30 CDX mouse models, with no significant changes in the body weight of mice in each group.

FIG. 55D shows that, the proportion of CD3+T cells in the peripheral blood of the TP4+ZC3 double-knockout group and the SC3+TP4 double-knockout group after administration was significantly higher than that in the NT group, suggesting that both TP+ZC and SC+TP double-gene editing significantly promote the proliferation and survival of T cells *in vivo,* and may inhibit the apoptosis of TILs.

FIG. 55E shows that, the effector memory T cells (EM) in the peripheral blood of the TP4+ZC3 double-knockout group and the SC3+TP4 double-knockout group on days 8 and 15 were significantly higher than that in the NT group, suggesting that both TP+ZC and SC+TP double-gene editing may increase the proportion of effector memory T cells, that is consistent with the stronger tumor killing ability *in vivo*.

FIG. 55F shows that, the release of IFN-$\gamma$ in the TP4+ZC3 double-knockout group and the SC3+TP4 double-knockout group at various time points after administration was significantly higher than that in the NT group, further suggesting that both TP+ZC and SC+TP double-gene editing may enhance the antitumor function of TILs *in vivo*.

FIGs. 56A to 56B show that, both the TP4+ZC3 double-knockout group and TP4+CB 1 double-knockout group exhibited significant tumor suppression ability, with a tumor suppression effect significantly stronger than that of the NT group.

FIG. 56C shows the changes in the body weight of PDX mouse models, with no significant changes in the body weight of mice in each group.

FIG. 56D shows that, the proportion of CD3+T cells in the TP4+ZC3 double-knockout group was higher than that in the NT group from Day 7 after administration, suggesting that TP/ZC double-gene knockout significantly promotes the proliferation and survival of T cells *in vivo*, and may inhibit the apoptosis of TILs.

FIG. 56E shows that, the central memory T cells (CM) in the peripheral blood of the TP4+ZC3 double-knockout group and the TP4+CB 1 double-knockout group were significantly higher than that in the NT group on Day 4, Day 7, Day 14, and Day 21; meanwhile, there was a rising trend in the proportion of effector memory T cells (EM) in the T cells of peripheral blood on Day 4 to Day 14, that is consistent with the good antitumor effect of the TP4+ZC3 double-knockout group and the TP4+CB1 double-knockout group *in vivo*.

FIGs. 57A to 57C show that, in the late REP stage, TILs from multiple knockout combinations of TP+ZC, SC+TP, and SC+TP+ZC exhibited enhanced proliferation ability compared with the NT group.

FIGs. 58A to 58I show that, after withdrawal of IL-2, TILs from multiple knockout combinations of TP+ZC, SC+TP, and SC+TP+ZC exhibited significantly enhanced proliferation ability compared with the NT group.

FIGs. 59A to 59E show that, after withdrawal of IL-2, under additional stimulation of OKT3, TILs from multiple knockout combination of TP+ZC exhibited significantly enhanced proliferation ability compared with the NT group.

FIGs. 60A to 60D show that, after withdrawal of IL-2, under additional stimulation of transACT, TILs from multiple knockout combinations of TP+ZC, SC+TP, and SC+TP+ZC exhibited significantly enhanced proliferation ability compared with the NT group.

FIG. 61 shows that, TIL cells with combined gene editing (TP4+ZC3) in the present application may have significantly enhanced long-term persistence *in vitro*.

FIGs. 62A to 62K show the test results of the killing ability of TIL cells derived from donors 104, 812, 316, 107, and 309 which were co-cultured with A375 tumor cells at an effector-target ratio of 4:1, 3:1 or 1:1. TILs from multiple knockout combinations of TP+ZC, SC+TP, and SC+TP+ZC exhibited significantly enhanced tumor cell killing ability compared with the NT group.

FIG. 63 shows the test results of the killing ability of TIL cells derived from donor 107 which were co-cultured with Hcc827 tumor cells at an effector-target ratio of 4:1. TILs from multiple knockout combination of TP+ZC exhibited significantly enhanced tumor cell killing ability compared with the NT group.

FIGs. 64A to 64B show the test results of the killing ability of TIL cells derived from donor 709 which were co-cultured with Caski tumor cells at an effector-target ratio of 3:1 or 1:1. TILs from multiple knockout combination of TP+ZC exhibited significantly enhanced tumor cell killing ability compared with the NT group.

FIGs. 65A to 65Y show the relative expression level of CD107a, TNF-$\alpha$, GZMB, and IFN-$\gamma$ in each group of TIL cells in the absence of any stimulants, for TIL cells derived from donors 104, 812, 709, 107, 713, 316, 312, 504, 704, and 309. TILs from multiple knockout combinations of TP+ZC, SC+TP, and SC+TP+ZC exhibited significantly enhanced intracellular cytokine expression capacity compared with the NT group.

FIGs. 66A to 66P show the relative expression level of CD107a, TNF-$\alpha$, GZMB, and IFN-$\gamma$ in each group of TIL cells under the stimulation of OKT3, for TIL cells derived from donors 104, 812, 107, 312, 316, 504, 713, 709, and 309. TILs from multiple knockout combination of TP+ZC exhibited significantly enhanced intracellular cytokine expression capacity compared with the NT group.

FIGs. 67A to 67H show the relative expression level of CD107a, TNF-$\alpha$, GZMB, and IFN-$\gamma$ in each group of TIL cells under the stimulation of transACT, for TIL cells derived from donors 812, 107, and 309. TILs from multiple knockout combinations of SC+TP and SC+TP+ZC exhibited significantly enhanced intracellular cytokine expression capacity compared with the NT group.

FIGs. 68A to 68B show the proportion of TCF1-positive cells in each group of TIL cells, for TIL cells derived from donors 812 and 107. The results show that, TILs from the knockout combination of SC+TP+ZC exhibited significantly enhanced expression capacity of TCF1 compared with the NT group. Therefore, gene-edited TIL cells have higher sternness, such as higher expression capacity of TCF1.

FIGs. 69A to 69D show the proportion of CD101-positive, LAG-3-positive, and TIM-3-positive cells in each group of TIL cells, for TIL cells derived from donors 812 and 107. The results show that, the TILs from the knockout combinations of SC+TP and SC+TP+ZC exhibited significantly reduced expression capacity of CD101, LAG-3, and TIM-3 compared with the NT group. Therefore, gene-edited TIL cells have reduced expression of T cell depletion molecules, such as lower expression capacity of CD101, LAG-3, and TIM-3.

FIGs. 70A to 70B show the release level of cytokines for TIL cells derived from donor 709 after being co-cultured with autologous tumor cells. The results show that, TILs from multiple double-knockout combination of TP+ZC could significantly promote the release of IFN-$\gamma$ and TNF-$\alpha$ after co-culture with autologous tumors compared with the NT group.

FIG. 70C shows the release level of cytokines for TIL cells derived from donor 704 after being co-cultured with autologous tumor cells. The results show that, TILs from the double-knockout combination of TP2+ZC-13 could significantly promote the release of IFN-$\gamma$ after co-culture with autologous tumors compared with the NT group.

FIGs. 71A to 71B show the test results of the serial killing ability of TIL cells derived from donor 607 which were co-cultured with Hey-T30 tumor cells at an effector-target ratio of 4:1. TILs from the knockout combination of TP4+ZC3 exhibited significantly enhanced tumor cell serial killing ability compared with the NT group.

FIGs. 71C to 71D show the test results of the serial killing ability of TIL cells derived from donor 316 which were co-cultured with A375 tumor cells at an effector-target ratio of 4:1. TILs from multiple knockout combination of TP+ZC exhibited significantly enhanced tumor cell serial killing ability compared with the NT group.

## DETAILED DESCRIPTION

The implementation of the present application will be illustrated below by specific examples, and other advantages and effects of the present application will be easily known by those familiar with the art from the disclosure in the specification.

### Definition of terms

In the present application, the term "suppressor of Janus kinase-signal transducer and activator of transcription (JAK-STAT) pathway" generally refers to a suppressor of a signaling pathway. For example, the suppressor of JAK-STAT pathway may include a substance that inhibits the activation of the JAK-STAT pathway or inhibits the activity of the JAK-STAT pathway transducer.

In the present application, the term "Janus kinase (JAK)" generally refers to a signaling pathway factor. For example, the Janus kinase may serve as a factor for transmitting the JAK-STAT pathway signals. For example, the UniProt Accession No. of JAK may be P23458.

In the present application, the term "suppressor of cytokine signaling" generally refers to a substance that inhibits the signaling of cytokines. For example, an exemplary suppressor of cytokine signaling may be SOCS, or a variant thereof, or a substance comprising the above functionally active fragments.

In the present application, the term "suppressor of cytokine signaling 1 (SOCS1)" generally refers to a substance that inhibits the signaling of cytokines. For example, SOCS1 may inhibit the transduction of the JAK-STAT pathway. For example, the UniProt Accession No. of SOCS1 may be 015524. In the present application, SOCS1 may encompass unprocessed SOCS1, any forms of processed SOCS1, variants of SOCS1 or substances comprising functionally active fragments of SOCS1.

In the present application, the term "suppressor of NF-$\kappa$B pathway" generally refers to a suppressor of a signaling pathway. For example, the suppressor of NF-$\kappa$B pathway can achieve the inhibition of signaling pathway transduction by promoting the ubiquitination of the target protein. For example, the suppressor of NF-$\kappa$B pathway is capable of ubiquitinating a protein selected from the group consisting of: receptor-interacting protein 1 (RIP1, UniProt Accession No. Q13546), mucosa-associated lymphoid tissue lymphoma translocation protein 1 (MALT1, UniProt Accession No. Q9UDY8), receptor-interacting protein 2 (RIP2, UniProt Accession No. O43353), and tumor necrosis factor receptor-associated factor 6 (TRAF6, UniProt Accession No. Q9Y4K3).

In the present application, the term "tumor necrosis factor-$\alpha$-induced protein 3 (TNFAIP3)" generally refers to a suppressor of a signaling pathway. For example, TNFAIP3 can enable the ubiquitination of the signaling substances of the NF-$\kappa$B pathway. For example, the UniProt Accession No. of TNFAIP3 may be P21580. In the present application, TNFAIP3 may encompass unprocessed TNFAIP3, any forms of processed TNFAIP3, variants of TNFAIP3 or substances comprising functionally active fragments of TNFAIP3.

In the present application, the term "CBLB" generally refers to a gene encoding a protein or the protein. For example, CBLB may encode a protein comprising an E3 ubiquitin ligase. For example, the NCBI Gene Accession No. of CBLB may be 868. For example, CBLB can mediate downstream signals, playing a critical role in the growth, development, and differentiation of cells and tissues. In the present application, CBLB may encompass unprocessed CBLB, any forms of processed CBLB, variants of CBLB or substances comprising functionally active fragments of CBLB.

In the present application, the term "ZC3H12A" generally refers to a gene encoding a protein or the protein. For example, ZC3H12A can encode a protein of a zinc finger domain. For example, ZC3H12A can mediate downstream signals, playing a critical role in the growth, development, and differentiation of cells and tissues. For example, the NCBI Gene Accession No. of ZC3H12A may be 80149. In the present application, ZC3H12A may encompass unprocessed ZC3H12A, any forms of processed ZC3H12A, variants of ZC3H12A or substances comprising functionally active fragments of ZC3H12A.

In the present application, the term "gene regulatory system" generally refers to a system regulating the expression or activity of a target gene. For example, the gene regulatory system may comprise gene regulatory molecules. For example, the gene regulatory system may regulate the expression or activity of a gene, such as, by inactivating or activating the gene, increasing or decreasing the amount of the gene, increasing or decreasing the amount of the transcription of the gene, and/or inactivating or activating the transcription product of the gene; for example, the gene regulatory system may regulate the expression or activity of a gene, such as by increasing or decreasing the amount of the expression product of the gene in a single cell, and/or increasing or decreasing the amount of cells expressing the expression product of the gene.

In the present application, the term "guide nucleic acid molecule" generally refers to a nucleic acid molecule that can be used for gene editing. For example, the guide nucleic acid molecule may provide information on nucleotide insertion or deletion to guide the editing process. For example, the guide nucleic acid molecule may be a guide RNA or gRNA. For example, "gRNA" may refer to an RNA molecule that binds to a Cas protein and targets the Cas protein to a specific position within the target DNA. For example, the hybridization between gRNA and the DNA targeting sequence can promote the formation of a CRISPR complex without necessarily requiring complete complementarity, e.g., as long as there is sufficient complementarity to cause hybridization and promote the formation of the CRISPR complex.

In the present application, the term "zymoprotein" generally refers to a protein with enzymatic activity. For example, the zymoprotein may refer to a Cas protein. For example, a Cas protein may comprise at least one RNA recognition or binding domain which can interact with gRNA. A Cas protein may also comprise a nuclease domain (e.g., DNA enzyme or RNA enzyme domain), a DNA binding domain, a helicase domain, a protein-protein interacting domain, a dimerization domain, and/or other domains. The nuclease domain may have catalytic activity for nucleic acid cleavage. Cleavage may include the breaking of covalent bonds in nucleic acid molecules. A Cas protein may be a wild-type protein (i.e., naturally occurring protein), a modified Cas protein (i.e., variants of Cas protein) or fragments of a wild-type or modified Cas protein. A Cas protein may also be active variants or fragments of a wild-type or modified Cas protein. In the present application, a Cas protein may encompass an unprocessed Cas protein, any forms of a processed Cas protein, variants of a Cas protein or substances comprising functionally active fragments of a Cas protein.

In the present application, the term "ribonucleoprotein complex" generally refers to a complex of protein and nucleic acid. For example, the protein in the ribonucleoprotein complex may have nuclease activity. For example, the ribonucleoprotein complex may cleave the target sequence under the guidance of the nucleic acid therein. For example, the ribonucleoprotein complex may be a complex of a Cas protein and a gRNA.

In the present application, the term "exon" generally refers to a part of a gene that can be expressed as a protein. For example, an exon may refer to the ability to be expressed as a protein during the biosynthesis of the protein. For example, the exon sequence of a cleaved target gene may reduce the activity or function of the target gene.

In the present application, the term "protospacer adj acent motif (PAM)" generally refers to a short sequence following the target sequence. For example, when Cas9 performs site-specific cleavage of the target DNA, the PAM sequence can be used to determine the cleavage site. For example, once the PAM region is identified, a person of skill in the art can easily determine the suitable location for the target sequence and design a gRNA sequence for cleaving the target sequence.

In the present application, the term "reduced expression" generally refers to the reduction in the expression of a product or genes thereof, and/or the decrease in the proportion of cells capable of expressing the product. For example, it may be the reduction in the amount of the genetically expressed product, or the reduction in the proportion of cells comprising the genetically expressed product, or the reduction in the proportion of cells secreting the genetically expressed product. For example, the reduction in the expression of the gene can be indirectly indicated by detecting the knockout amount of the gene in the genome of the cell. For example, the reduction in the expression of the gene can be indirectly indicated by detecting the proportion of cells with the gene knocked out in a cell population.

In the present application, the term "activity" generally refers to a biological function of a substance. For example, the activity of a gene may refer to the transcription and/or translation state of the gene. For example, the decrease in the gene activity may refer to a reduction in the transcriptional function of the gene, the inability of the gene to be transcribed normally, or the suppression on the function of the transcription product of the gene.

In the present application, the term "CD80" generally refers to a cell stimulating molecule. For example, CD80 may be a ligand of CD28. For example, CD80 can be found in GenBank Accession No. P33681. The CD80 protein of the present application may also encompass functionally active fragments thereof, not limited to substances comprising the functionally active fragments of CD80 produced from processing and/or modification occurring in the cells. For example, the CD80 of the present application may comprise functionally active fragments of CD80 and any other domains.

In the present application, the term "CD86" generally refers to a cell stimulating molecule. For example, CD86 may be a ligand of CD28. For example, CD86 can be found in GenBank Accession No. P42081. The CD86 protein of the present application may also encompass functionally active fragments thereof, not limited to substances comprising the functionally active fragments of CD86 produced from processing and/or modification occurring in the cells. For example, the CD86 of the present application may comprise functionally active fragments of CD86 and any other domains.

In the present application, the term "secretion" generally means that a substance may be localized to the extracellular part of a cell. For example, the secreted substance may be synthesized inside the cell and then transported to the extracellular space of the cell. For example, whether a substance is a secreted substance may be tested by enzyme-linked immunosorbent assay or other detection methods.

In the present application, the term "T cell receptor" or "TCR" generally refers to a complex of membrane proteins involved in the activation of T cells in response to antigen presentation. TCRs may be responsible for recognizing antigens bound to major histocompatibility complex molecules. TCRs may be composed of heterodimers of alpha ($\alpha$) and beta ($\beta$) chains, or of gamma ($\gamma$) and delta ($\delta$) chains. TCRs may exist in $\alpha/\beta$ and $\gamma/\delta$ forms, which are structurally similar but have distinct anatomical locations and functions. For example, TCRs may be TCRs modified on any cells expressing TCRs. For example, the type of TCR can be analyzed with TCR subtype analysis reagents.

In the present application, the term "clonal diversity" generally means that a certain substance has multiple clonotypes. For example, the clonal diversity of a TCR may mean that the TCR may have different sequence structures and/or antigen recognition abilities. For example, the diversity of TCRs is often distinguished by $\beta$-chain subtypes, which can comprise V$\beta$ 23, V$\beta$ 7.2, V$\beta$ 5.2, V$\beta$ 11, V$\beta$ 16, V$\beta$ 3, etc. When one T-cell population has more $\beta$-chain subtypes, the T-cell population can be considered to have a higher clonal diversity.

In the present application, "CD4$^+$ cells" generally refers to CD4 positive cells, e.g., T cells. The terms "CD4$^+$ cells" and "CD4 positive cells" may be used synonymously. These cells can be identified by methods known in the art, for example, by staining the cells with a fluorescently labeled antibody against CD4 and by using fluorescence activated cell sorting. For example, it has demonstrated from existing data that an increase in the proportion of CD4$^+$ cells can lead to an increase in the ability of the cell population to secrete IFN-$\gamma$ and/or TNF and can improve the effect of the T cell population on promoting the tumor suppression. For example, see Tay, R. E., Richardson, E. K. et, al. (2020). Cancer Gene Therapy, 1-13. However, there is a lack of a method to increase the proportion of CD4$^+$ cells in the field, and the present application may provide a method to affect the proportion of CD4$^+$ cells.

In the present application, "CD8$^+$ cells" generally refers to CD8 positive cells, e.g., T cells. The terms "CD8$^+$ cells" and "CD8 positive cells" may be used synonymously. These cells can be identified by methods known in the art, for example, by staining the cells with a fluorescently labeled antibody against CD8 and by using fluorescence activated cell sorting.

In the present application, the term "IC$_{50}$ value" or "IC50 value" generally refers to the concentration of a target required to obtain 50% inhibition of a biological process. The IC50 value can be converted to an absolute inhibition constant (Ki) using the Cheng-Prusoff equation (Biochem. Pharmacol. (1973) 22:3099).

In the present application, the term "K$_D$ value" or "KD value" generally refers to the dissociation constant, which can be determined by surface plasmon resonance. Generally, surface plasmon resonance analysis uses the BIAcore system (Pharmacia Biosensor, Piscataway, NJ) to measure real-time binding interactions between a ligand (a substance immobilized on a biosensor substrate) and an analyte (a substance in a solution) via surface plasmon resonance (SPR). Surface plasmon analysis can also be performed by immobilizing the analyte (substances on the biosensor substrate) and presenting the ligand.

In the present application, the term "encoding" generally refers to the ability to infer, directly or indirectly, information about the structure or composition of one type of molecule from information about the structure or composition of another type of molecule related thereto, based on essentially defined rules. For example, the nucleotide sequence of an amino acid can be inferred from its sequence, e.g., based on the properties of deoxyribonucleic acid-transcribed complementary nucleic acids, including those that can be translated into polypeptides. For example, deoxyribonucleic acids can encode RNAs transcribed from the deoxyribonucleic acids. Deoxyribonucleic acids can similarly encode polypeptides translated by RNAs that are transcribed from the deoxyribonucleic acids.

In the present application, the term "small molecule compounds" generally refers to peptides, peptidomimetics, amino acids, amino acid analogues, polynucleotides, polynucleotide analogues, nucleotides, nucleotide analogues, organic or inorganic substances of molecular weight less than about 10,000 g/mol (i.e., including heterologous organic substances and organometallic compounds), organic or inorganic substances of molecular weight less than about 5,000 g/mol, organic or inorganic substances of molecular weight less than about 1,000 g/mol, organic or inorganic substances of molecular weight less than about 500 g/mol, as well as salts, esters and other pharmaceutically acceptable forms of such drugs.

In the present application, the term "NK cell", also referred to as "natural killer cell", generally refers to a cell with large granules in the cytoplasm. NK cells are developed from bone marrow lymphoid stem cells and can differentiate and develop depending on the bone marrow or thymus microenvironment. In the present application, the proportion of NK cells in TIL cells can be altered by the methods of the present application.

In the present application, the term "antibody" generally refers to an immunoglobulin or fragments or derivatives thereof, encompassing any polypeptides comprising antigen binding sites, no matter produced *in vitro* or *in vivo.* The term comprises, but not limited to, polyclonal, monoclonal, mono-specific, multi-specific, non-specific, humanized, single chain, chimeric, synthetic, recombinant, hybrid, mutant, and transplanted antibodies. Unless otherwise modified by the term "intact", such as in the "intact antibody", for the purpose of the present application, the term "antibody" also comprises antibody fragments, such as Fab, F(ab')2, Fv, scFv, Fd, dAb and other antibody fragments that maintain the antigen binding function (e.g., specifically binding to CD3). Generally, such fragments should comprise antigen binding domains. A basic 4-chain antibody unit is heterotetrameric glycoprotein consisting of two identical light (L) chains and two identical heavy (H) chains. An IgM antibody consists of 5 basic heterotetrameric units and an additional polypeptide known as J-chain and contains 10 antigen binding sites; while an IgA antibody consists of 2-5 basic 4-chain units that can combine and polymerize with the J-chain to form multivalent combinations. With regard to IgG, a 4-chain unit is generally about 150,000 Dalton. Each L chain is linked to an H chain through a covalent disulfide bond, while two H chains are linked to each other through one or more disulfide bonds depending on the isotype of the H chain. Each of H and L chains also has regularly spaced intra-chain disulfide bridges. Each H chain has a variable domain (VH) at the N-terminus, which is followed by three constant domains (CHs) for each of $\alpha$ and $\gamma$ chains or followed by four CH domains for $\mu$ and $\epsilon$ isotypes. Each L chain has a variable domain (VL) at the N-terminus and has a constant domain at the other terminus. VL corresponds to VH, and CL corresponds to the first constant domain (CH1) of the heavy chain. Specific amino acid residues are considered to form an interface between the light chain and heavy chain variable domains. VH is paired with VL to form a single antigen-binding site. L chains from any vertebrate species can be classified into one of two distinct types based on the amino acid sequences of their constant domains, called kappa and lambda. Depending on the amino acid sequences of its heavy chain (CH) constant domain, immunoglobulin can be classified into different types or isotypes. There are currently five types of immunoglobulins: IgA, IgD, IgE, IgG, and IgM, which have heavy chains named $\alpha$, $\delta$, $\epsilon$, $\gamma$ and $\mu$, respectively.

In the present application, the term "antigen binding fragments" generally refers to one or more polypeptide fragments having the ability of specifically binding to an antigen (e.g., CD3). In the present application, the antigen binding fragments may comprise Fab, Fab', F(ab)$_2$, Fv fragments, F(ab')$_2$, scFv, di-scFv, and/or dAb.

In the present application, the term "solid medium" or "medium" generally refers to a solid material having the binding function. For example, the solid medium of the present application may refer to a material that binds one or more substances within the medium and/or on the surface of the medium through covalent binding and/or non-covalent binding. For example, the solid medium of the present application may refer to a material that binds CD28 antibody or antigen-binding fragments thereof and CD3 antibody or antigen-binding fragments thereof within the medium and/or on the surface of the medium through covalent binding and/or non-covalent binding. For example, the solid medium of the present application may be a polymeric material.

In the present application, the term "expression" generally refers to the process of transcription and/or translation of gene encoding a target polypeptide that occurs within a cell. The transcription level of the gene encoding the target polypeptide in the host cell can be determined by measuring the amount of corresponding mRNA present in the cell. For example, mRNA transcribed from the gene encoding the target polypeptide can be quantitatively measured by PCR or by RNA hybridization. The translation level of the gene encoding the target polypeptide can be measured by a variety of methods, such as by ELISA, by polypeptide bioactivity assays, or by Western blotting or radioimmunoassay. In the present application, the term "expression" may also generally refer to the transcriptional and/or translational processes that occur in the product. For example, the expression of cytokine may be the processes of transcribing and/or translating the cytokine by a cell. For example, the expression of cytokine can be determined by detecting the amount of corresponding mRNA present in the cell or by detecting the amount of the cytokine produced by the cell, or both.

In the present application, the "stage" in the terms "one stage *of in vitro* expansion", "a single stage *of in vitro* expansion", or "the first stage *of in vitro* expansion", etc. generally refers to a process of expansion that TILs are subjected to *in vitro.* In one embodiment, each stage can be divided depending on the change in the number of TIL cells. In one embodiment, when the number of the TIL cells is increased by at least about 1-fold, it can be considered that the TIL cells enter the next stage *of in vitro* expansion. In some embodiments, when the number of the TIL cells is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold, it can be considered that the TIL cells enter the next stage of *in vitro* expansion. In one embodiment, each stage can also be divided by the culture conditions of the TIL cells. In one embodiment, after T cell activators and/or T cell growth factors are added or supplemented into the cell culture medium, it can be considered that the TIL cells enter the next stage *of in vitro* expansion. In one embodiment, after TIL cells have been centrifuged and/or washed, it can be considered that the TIL cells enter the next stage *of in vitro* expansion. In one embodiment, each stage can also be divided by the culture days of the TIL cells. In one embodiment, after the TIL cells have been cultured *in vitro* for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, it can be considered that the TIL cells enter the next stage of in vitro expansion.

In the present application, the term "the first stage of in vitro expansion" generally refers to a stage of expansion using T cell growth factors after primary TILs are obtained from tissues. In one embodiment, the tissues in the present application may be selected from the group consisting of: tumor tissues and pleural effusion, and the pleural effusion in the present application may be pleural effusion from patients with metastatic carcinomas. In one embodiment, the expansion in the present application may be autologous or allogeneic in vivo expansion, or it may be in vitro expansion. The first stage of in vitro expansion in the present application can also be referred to as a preREP (pre-rapid expansion protocol) stage. For example, TILs derived from tumor tissues and not expanded in vitro can be referred to as the first TIL population. For example, the TILs obtained after the first stage of in vitro expansion in the two-step culture method of the present application can be referred to as the second TIL population.

In the present application, the term "the second stage of in vitro expansion" generally refers to a stage of expansion again, after a tissue has been removed from a subject and expanded. In one embodiment, compared to the TILs subjected to the first stage of in vitro expansion, the number of the TIL cells subjected to the second stage of in vitro expansion is increased, e.g., it may be increased by at least about 10-fold (or at least about 20, 30, 40, 50, 60, 70, 80 or 90-fold), or in one embodiment, the cell number may be increased by at least about 100-fold. In one embodiment, the second and the first stages of in vitro expansion may be different in culture conditions, e.g., the culture substances added may be different. For example, the second stage of in vitro expansion in the two-step culture method of the present application can also be referred to as a REP (rapid expansion protocol) stage. For example, the TILs obtained after the second stage of in vitro expansion in the two-step culture method of the present application can be referred to as the third TIL population.

In the present application, the term "in vivo" generally refers to an event that occurs in the body of a subject.

In the present application, the term "in vitro" generally refers to an event that occurs outside the body of a subject.

In the present application, the term "ex vivo" generally refers to an event that involves a treatment or surgery on cells, tissues and/or organs which have been removed from a subject. In one embodiment, the cells, tissues and/or organs can be returned into the subject's body by a surgery or treatment method.

In the present application, the term "secretion capacity" generally refers to the ability of a cell to express a polypeptide or protein and transfer the polypeptide or protein of the present application to the extracellular environment.

In the present application, the term "irradiation" generally refers to the treatment of a substance by means of radiation. For example, in one embodiment, irradiation may refer to irradiating a substance with X-rays, alpha rays, beta rays, or gamma rays.

In the present application, the term "engineered cells" generally refers to cells which have been genetically modified by adding additional genetic material in the form of DNA or RNA to the total genetic material of the cells. In one embodiment, the engineered cells can be TILs genetically modified to express the T cell activators and/or T cell growth factors of the present application.

In the present application, the term "co-culturing" generally refers to culturing two or more different populations of cells with some degree of contact between them. The "contact" between two or more different populations of cells of the present application, in one embodiment, can be through direct contact, i.e., the cells of one population are directly physically contacted with the cells of another population. Alternatively, in one embodiment, the contact can be through indirect contact mediated by sharing a culture medium. The shared culture medium in the present application may contain metabolites produced and released from at least one population of the co-cultured cells and is used to culture the cells of another population.

In the present application, the term "contacting" generally means that two or more substances of different types are brought into contact together in any order, in any manner, and for any length of time. In one embodiment, the contact can be through direct contact, for example, one or more feeder cells, T cell activators and/or T cell growth factors can be added into the culture medium of the TIL cells; for example, a culture medium comprising one or more feeder cells, T cell activators and/or T cell growth factors can be added into and/or used to replace the culture medium of the TIL cells; for example, a culture medium comprising one or more feeder cells, T cell activators and/or T cell growth factors can be used for the culture of the TIL cells. In one embodiment, the contact can be through indirect contact, for example, metabolites produced and released from feeder cells can be used to culture the TIL cells.

In the present application, the term "mixture" generally refers to a combination of two or more different substances. For example, the CD28 antibody or antigen-binding fragments thereof and the CD3 antibody or antigen-binding fragments thereof in the present application may be added into the cell culture medium as a mixture after mixing.

In the present application, the terms "simultaneous contact", "concurrent contact", "while contacting with", "simultaneously" and "concurrently" generally refer to the administration of two or more substances to a subject or cells such that the substances are present in the subject and/or the environment in which the cells are cultured at the same time. Simultaneous contact can comprise administration of different compositions at the same time, administration of different compositions at different times, or administration of a composition in which two or more active pharmaceutical ingredients are present. For example, "simultaneous contact" in the present application generally may refer to contacting substantially simultaneously.

In the present application, the term "expansion" generally refers to a several-fold increase in the number of cells over a period of time. In one embodiment, the cell number can be increased by at least about 3-fold (or 4, 5, 6, 7, 8 or 9-fold). In one embodiment, the cell number can be increased by at least about 10-fold (or 20, 30, 40, 50, 60, 70, 80 or 90-fold). Alternatively, in one embodiment, the cell number can be increased by at least about 100-fold. In the present application, the term "expanded" generally means that the cells of the present application have been subjected to the above one or more expansions.

In the present application, the term "polymer" generally refers to a molecule composed of individual chemical moieties linked together, which moieties can be the same or different in the present application. In one embodiment, the term "polymer" may refer to individual chemical moieties linked tail to tail to form a linear molecule, as well as individual chemical moieties linked together in the form of branched (e.g., "multi-arm" or "star") structures. In one embodiment, a polymer may comprise, for example, polysaccharide, glucan, hydrogel, polyethylene glycol, or poloxamer. Poloxamer is a nonionic triblock copolymer with a central hydrophobic chain of polyoxypropylene (polypropylene oxide)) and two pendant hydrophilic chains of polyoxyethylene (poly(ethylene oxide)). The substances encompassed in the present application may be formulated with, or administered with, any polymers described herein or known in the art.

In the present application, the term "chimeric antibody" generally refers to an antibody that is formed from the fusion of the variable region of a mouse-derived antibody with the constant region of a human antibody, which can reduce the immune response induced by the mouse-derived antibody. Chimeric antibodies are established so that a hybridoma that secretes murine-derived specific monoclonal antibodies can be established and variable region genes are then cloned from the murine hybridoma cells, constant region genes of the human antibody can be cloned as needed, murine variable region genes are ligated with human constant region genes to form chimeric genes which are then inserted into expression vectors, and the chimeric antibody molecules can be expressed in eukaryotic or prokaryotic systems.

In the present application, the term "humanized antibody", also referred to as CDR-grafted antibody, generally refers to an antibody produced by grafting the murine CDR sequences into the human antibody variable region frameworks, that is, an antibody produced in different types of human germline antibody framework sequences. Humanized antibodies can overcome heterologous responses induced by chimeric antibodies which carry a large number of murine protein components. Such framework sequences can be obtained from public DNA database covering germline antibody gene sequences or published references. For example, germline DNA sequences of human heavy and light chain variable region genes can be found in "VBase" human germline sequence database.

In the present application, the term "fully humanized antibody", "fully human antibody" or "wholly humanized antibody", also referred to as "fully humanized monoclonal antibody" means that both the variable and constant regions of the antibody can be of human origin, with immunogenicity and toxicities removed. The development of monoclonal antibodies has gone through four stages, namely: murine-derived monoclonal antibodies, chimeric monoclonal antibodies, humanized monoclonal antibodies and fully humanized monoclonal antibodies. The antibodies or ligands of the present application may be fully humanized monoclonal antibodies. The relevant technologies for preparing fully human antibodies may be human hybridoma technology, EBV transformed B lymphocyte technology, phage display technology, transgenic mouse antibody preparation technology, single B cell antibody preparation technology, and the like.

In the present application, the term "CDR" generally refers to one of the six hypervariable regions within the variable domain of an antibody that are primarily responsible for promoting the antigen binding. One of the most commonly used definitions of the six CDRs may be provided by Kabat E.A. et, al., Chothia et, al., and MacCallum et, al. As used in the present application, the Kabat definition of CDRs can be applied to CDR1, CDR2 and CDR3 of the light chain variable domain (CDR L1, CDR L2, CDR L3 or L1, L2, L3) and CDR1, CDR2 and CDR3 of the heavy chain variable domain (CDR H1, CDR H2, CDR H3 or H1, H2, H3).

In the present application, the term "anti-CD3 antibody" generally refers to an antibody targeting CD3 or variants thereof, for example, monoclonal antibodies, including human, humanized, chimeric or murine antibodies, which are directed against CD3 receptors in the T cell antigen receptors of mature T cells. Anti-CD3 antibodies may comprise OKT3. Anti-CD3 antibodies may comprise SP34. Anti-CD3 antibodies may also comprise other anti-CD3 antibodies, including, e.g., in one embodiment, otelixizumab, teplizumab, and visilizumab.

In the present application, the term "IL-2" or "IL2" generally refers to T cell growth factors known as interleukin 2 and comprises all forms of IL-2, which may comprise, in one embodiment, human and mammalian forms, conservative amino acid substitutions, glycoform modifications or variants, or active fragments thereof. The GeneID of the gene encoding IL-2 may be 3558.

In the present application, the term "antigen-presenting cells" or "APCs" generally refers to immune system cells, such as helper cells (e.g., B cells, dendritic cells, etc.), that display exogenous antigens complexed with major histocompatibility complexes (MHCs) on their surface. T cells can recognize these complexes using their T cell receptors (TCRs). APCs can process antigens and present them to T cells. In one embodiment, the antigen-presenting cells may comprise cells selected from the group consisting of: peripheral mononuclear cells, dendritic cells, and artificial antigen-presenting cells.

In the present application, the term "TIL properties" generally refers to the properties of TIL cells obtained by the culture method of the present application. The changes in TIL properties may comprise increased number of TIL cells, increased proportion of viable cells, enhanced persistence, improved proportion of T cell subpopulations, enhanced cytokine secretion capacity, enhanced tumor cell killing ability, enhanced T cell receptor (TCR) clonal diversity, and increased number of TIL cells in tissues, or any combinations thereof. The changes in the present application may be improvement or decreasement.

In the present application, the term "persistence" generally refers to the existence of cells *in vitro* and/or in a subject. For example, the enhanced persistence of TIL cells may refer to an increase in the time that TIL cells are present in the body. For example, the enhanced persistence may refer to an increase in the time that the cells are present in the tissues of the subject, such as tumors, spleen, bone marrow, lung tissues, and blood. For example, the enhanced persistence may be the enhancement of the persistence of TIL cells after the withdrawal of IL-2 from the culture medium.

In the present application, the term "nanoparticles" generally refers to microscopic particles with at least one dimension less than 100 nm. Generally, nanoparticles have diameters ranging from 50 nm to 500 nm (i.e., from 0.05 $\mu$m to 0.5 $\mu$m); are structurally stable in the physiological environment; and can accommodate smaller molecules (e.g., drugs or other biologically active agents) that can then be delivered to the desired sites. For example, the nanoparticles in the present application may comprise CD28 antibodies or antigen-binding fragments thereof. For example, the nanoparticles in the present application may comprise CD28 antibodies or antigen-binding fragments thereof and CD3 antibodies or antigen-binding fragments thereof. For example, the anti-CD3 antibodies may comprise OKT3. For example, the anti-CD28 antibodies may comprise 15E8.

In the present application, the term "artificial antigen-presenting cells" generally refers to immune cells artificially constructed for the presentation of exogenous antigens, e.g., the presentation of exogenous antigens may be by means of comprising a complex of the exogenous antigen with a major histocompatibility complex (MHC) on the surface of an artificial antigen-presenting cell. In one embodiment, isolated artificial antigen-presenting cells (aAPC) can be comprised, which may comprise cells expressing HLA-A/B/C (the GeneID of the gene encoding it can be 3105, 3106 or 3107), CD64 (the GeneID of the gene encoding it can be 2209), CD80 (the GeneID of the gene encoding it can be 941), ICOS-L (the GeneID of the gene encoding it can be 23308) and CD58 (the GeneID of the gene encoding it can be 965), and can be modified to express more than one T cell activator, with the "more than" in the present application being inclusive.

In the present application, the term "fusion protein" generally refers to a polypeptide or protein comprising the amino acid sequence of a first polypeptide or protein or fragments thereof, analogues or derivatives and the amino acid sequence of a heterologous polypeptide or protein (i.e., a second polypeptide or protein or fragments, analogues or derivatives thereof different from the first polypeptide or protein or fragments, analogues or derivatives thereof, or usually not a part of the first polypeptide or protein or fragments, analogues or derivatives thereof). In some cases, fusion protein may comprise prophylactic or therapeutic drugs fused with heterologous protein, polypeptide, or peptide. Wherein, the heterologous protein, polypeptide or peptide of the present application may be or may not be different types of prophylactic or therapeutic drugs. For example, two different proteins, polypeptides or peptides with immunomodulatory activity can be fused together to form a fusion protein. In some cases, the fusion protein may maintain or have increased activity compared to the activity of the initial polypeptide or protein prior to the fusion of heterologous protein, polypeptide or protein. For example, the fusion protein of the present application may be a fusion protein incorporating a CD28 antibody or an antigen-binding fragment thereof and a CD3 antibody or an antigen-binding fragment thereof.

In the present application, the term "killing ability" generally refers to the ability to kill target cells by contacting the cells of the present application with an effective amount of substances. In one embodiment, the substances of the present application may be TIL cells. The killing in the present application may comprise killing cells by itself or by promoting CDC, apoptosis, ADCC, and/or phagocytosis of other cells or substances, or by a combination of two or more of these mechanisms.

In the present application, the term "administering" generally refers to the delivery of a substance to a subject in need thereof by any route known in the art. Pharmaceutically acceptable carriers and formulations or compositions are also well known in the art. Routes of administration may comprise intravenous, intramuscular, intradermal, subcutaneous, transdermal, mucosal, intratumoral, and/or mucosal.

In the present application, the term "kit" generally refers to two or more components packaged together in a container, a receptacle, or other containers, one of which corresponds to the substance of the present application. For example, the TIL cells of the present application are comprised.

In the present application, the term "subject" generally refers to cells or animals, which may be mammals, such as human, non-human primates (apes, gibbons, gorillas, chimpanzees, orangutans, macaques), domestic animals (dogs and cats), farm animals (poultry such as chickens and ducks, horses, cows, goats, sheep, pigs), and laboratory animals (mice, rats, rabbits, guinea pigs). Human subjects comprise fetal, neonatal, infant, adolescent, and adult subjects. Subjects comprise animal disease models, such as tumor animal models, and other animal models known to those of skill in the art.

In the present application, the term "feeder cells" generally refers to culture cells that can be used to support the growth of another type of cells of interest. For example, at least one factor can be secreted to the culture medium by *in vitro* growth. In one embodiment, feeder cells may comprise antigen-presenting cells.

In the present application, the term "specifically binding" generally refers to a binding substance that recognizes a specific target substance but does not substantially recognize or bind other molecules in the sample. For example, if a binding substance can specifically bind the specific target substance of the present application from one species, the binding substance of the present application can also specifically bind the target substance of the present application or homologous target substances thereof from one or more other species. This interspecific reactivity itself may not change the classification of the binding substance as specificity. In some cases, a binding substance that specifically binds to a target substance may also bind to a different allelic form of the target substance.

In the present application, the term "complete culture process" generally refers to the complete process of isolating cells from isolated tumor tissues in the patient, and then subjecting to one or more expansions to finally obtain cells that can be administered to the subject.

In the present application, the term "cell culture medium" generally refers to a nutrient solution in which cells, such as mammalian cells, are grown. The formulation of cell culture media is well known in the art. Typically, the cell culture media comprise buffer, salts, carbohydrates, amino acids, vitamins, and essential trace elements. The cell culture media may or may not contain sera, peptone, and/or protein. The cell culture media may be supplemented with additional components or increased concentrations of components, such as amino acids, salts, sugars, vitamins, hormones, growth factors, buffers, antibiotics, lipids, trace elements, etc., depending on the requirements of the cells to be cultured and/or the desired cell culture parameters.

In the present application, the term "pharmaceutical composition" or "pharmaceutical formulation" generally refers to a preparation that allows the biological activity of the active ingredient to be effective and may not contain additional components that are unacceptably toxic to the subject to whom the formulation is to be administered. Such formulations are sterile. "Pharmaceutically acceptable" excipients (carriers, additives) are those which can reasonably be administered to a test mammal to provide an effective dose of the active ingredient employed.

In the present application, the term "tumor infiltrating lymphocytes" or "TILs" generally refers to a population of cells initially acquired as leukocytes, which in the present application have left the bloodstream of the subject and migrated into the tumor. TILs may comprise, but not limited to, $CD8^+$ cytotoxic T cells (lymphocytes), Th1 and Th17 $CD4^+$ T cells, natural killer cells, dendritic cells and M1 macrophages. TILs may comprise primary TILs and secondary TILs. The "primary TILs" may be those TIL cells obtained from the tissue samples of the subject, while the "secondary TILs" may be any TIL populations that have been expanded or expanded in the present application. In some embodiments, the tumor infiltrating lymphocytes of the present application may have not been isolated and purified or may be infiltrated with tumor cells. In one embodiment, the TILs of the present application may refer to TIL populations.

In the present application, the term "central memory T cells" generally refers to T cells that have long-term memory and can be restimulated by antigens. Central memory T cells may have a phenotype of $CD45RO^+CD62L^+$ or $CD45RA^-CCR7^+$, for example, central memory T cells can be identified by $CD45RO^+$ and $CD62L^+$ or by $CD45RA^-$ and $CCR7^+$. Central memory T cells may have a stronger ability to resist tumor growth compared with regular T cells.

In the present application, the term "regulatory T cells" generally refers to a subpopulation of T cells that control autoimmune reactivity in the body. Regulatory T cells may have a phenotype of $CD4^+CD25^+Foxp3^+$, for example, regulatory T cells can be identified by $CD4^+$, $CD25^+$, and $Foxp3^+$. Regulatory T cells may have an ability to inhibit the anti-tumor growth of T cells.

In the present application, the term "activated T cells" generally refers to T cells that have been activated to have the ability to resist the tumor growth. Activated T cells may have a phenotype of $PD-1^+$ ($PD1^+$), $LAG-3^+$ ($LAG3^+$) or $CD28^+$, for example, activated T cells can be identified by $PD-1^+$, $LAG-3^+$ or $CD28^+$. Activated T cells may have an ability to resist the tumor growth.

In the present application, the term "tumor-specific T cells" generally refers to T cells that can specifically resist the tumor growth. Tumor-specific T cells may have a phenotype of $CD103^+CD39^+$, for example, tumor-specific T cells can be identified by $CD103^+$ and $CD39^+$. Tumor-specific T cells may have a more specific ability to resist the tumor growth compared with regular T cells.

In the present application, the term "stem cell-like T cells" generally refers to a class of T cells that may have the potential for self-proliferation and/or differentiation. For example, cells with differentiation potential and/or sustained proliferation capacity in the present application can be considered as stem cell-like cells. For example, naive T cells ($CD45RO^-CD62L^+$) can be considered as stem cell-like cells. For example, naive T cells may have a phenotype of $CD45RO^- CD62L^+$. For example, stem cell-like T cells can be identified by $CD45ROCD62L^+$. For example, stem cell-like T cells may have a phenotype of $TCF1^+$, for example, stem cell-like T cells can be identified by $TCF1^+$. Stem cell-like T cells may have a stronger and/or longer-term ability to resist the tumor growth compared with regular T cells.

In the present application, the term "tumor debris" generally refers to tumor debris that can be formed by mechanical disruption, enzymatic digestion and/or other disruption methods after the tumor tissue is removed from the subject.

In the present application, the term "composition" or "pharmaceutical composition" generally refers to a mixture of at least one cell as well as at least one and optionally more than one other pharmaceutically acceptable chemical components such as carriers, stabilizers, diluents, dispersing agents, suspending aids, thickening agents and/or excipients.

In the present application, the term "pharmaceutically acceptable carrier" generally refers to one or more nontoxic materials that do not interfere with the active ingredients. For example, the pharmaceutically acceptable carriers may not interfere with the biological activity of the active ingredients; for example, the pharmaceutically acceptable carriers may not interfere with the effectiveness of the biological activity of the active ingredients. Such formulations may conventionally contain salts, buffers, preservatives, compatible carriers, and optionally other therapeutic agents. Such pharmaceutically acceptable formulations may also contain compatible solid or liquid fillers, diluent, or encapsulating substances suitable for administration to human. Other contemplated carriers, excipients, and/or additives that can be used in the formulations described herein can comprise, for example, flavoring agents, antimicrobial agents, sweeteners, antioxidants, antistatic agents, lipids, protein excipients (such as serum albumin, gelatin, and casein), salt-forming counterions (such as sodium), and the like. These and other known pharmaceutical carriers, excipients and/or additives suitable for use in the formulations described herein are known in the art. In the present application, the "pharmaceutically acceptable carrier" may be understood as not including vectors in the form of nucleic acid used in genetic engineering.

In the present application, the term "functionally active fragment" generally refers to a fragment having a partial region of a full-length protein or nucleic acid but maintaining or partially maintaining the biological activity or function of the full-length protein or nucleic acid. For example, a functionally active fragment may maintain or partially maintain the ability of the full-length protein to bind another molecule. For example, the functionally active fragment of the growth factor IL-2 may maintain or partially maintain the biologically active function of the full-length IL-2 to cause cell proliferation.

In the present application, the term "T cell activator" generally refers to a substance that binds the corresponding binding receptor on T cells and mediates the costimulatory response of the T cells. T cell activators may be substances other than antigen receptors that are required for T cells to produce effective immune responses. T cell activators may refer to T cell costimulatory molecules. For example, the T cell activators in the present application may comprise variants, homologues thereof or any substances comprising functionally active fragments thereof. T cell activators may comprise, but not limited to, MHC type I molecules, TNF receptor protein, immunoglobulin-like protein, cytokine receptors, integrins, signaling lymphocytic activation molecules (SLAM protein), NK cell activation receptors, BTLA (the GeneID of the gene encoding it can be 151888), Toll ligand receptors, OX40 (the GeneID of the gene encoding it can be 7293), CD2 (the GeneID of the gene encoding it can be 914), CD7 (the GeneID of the gene encoding it can be 924), CD27 (the GeneID of the gene encoding it can be 939), CD28 (the GeneID of the gene encoding it can be 940), CD30 (the GeneID of the gene encoding it can be 943), CD40 (the GeneID of the gene encoding it can be 958), CDS, ICAM-1 (the GeneID of the gene encoding it can be 3383), LFA-1 (CD11a/CD18) (the GeneID of the gene encoding it can be 3689), 4-1BB (CD137) (the GeneID of the gene encoding it can be 3604), B7-H3 (the GeneID of the gene encoding it can be 80381), ICOS (CD278) (the GeneID of the gene encoding it can be 29851), GITR (the GeneID of the gene encoding it can be 8784), BAFFR (the GeneID of the gene encoding it can be 115650), LIGHT (the GeneID of the gene encoding it can be 8740), HVEM (LIGHTR) (the GeneID of the gene encoding it can be 8764), KIRDS2, SLAMF7 (the GeneID of the gene encoding it can be 57823), NKp80 (KLRF1) (the GeneID of the gene encoding it can be 51348), NKp44 (the GeneID of the gene encoding it can be 9436), NKp30 (the GeneID of the gene encoding it can be 259197), NKp46 (the GeneID of the gene encoding it can be 9437), CD19 (the GeneID of the gene encoding it can be 930), CD4 (the GeneID of the gene encoding it can be 920), CD8$\alpha$ (the GeneID of the gene encoding it can be 925), CD8$\beta$ (the GeneID of the gene encoding it can be 926), IL-2R$\beta$, IL-2R$\gamma$, IL7R$\alpha$ (the GeneID of the gene encoding it can be ), ITGA4 (the GeneID of the gene encoding it can be 3676), VLA1 (the GeneID of the gene encoding it can be 3672), CD49a (the GeneID of the gene encoding it can be 3672), IA4 (the GeneID of the gene encoding it can be 3732), CD49D (the GeneID of the gene encoding it can be 3676), ITGA6 (the GeneID of the gene encoding it can be 3655), VLA-6 (the GeneID of the gene encoding it can be 3655), CD49f (the GeneID of the gene encoding it can be 3655), ITGAD (the GeneID of the gene encoding it can be 3681), CD11d (the GeneID of the gene encoding it can be 3681), ITGAE (the GeneID of the gene encoding it can be 3682), CD103 (the GeneID of the gene encoding it can be 3682), ITGAL (the GeneID of the gene encoding it can be 3683), CD11a (the GeneID of the gene encoding it can be 3683), LFA-1 (the GeneID of the gene encoding it can be 3683), ITGAM (the GeneID of the gene encoding it can be 3684), CD11b (the GeneID of the gene encoding it can be 3684), ITGAX (the GeneID of the gene encoding it can be 3687), CD11c (the GeneID of the gene encoding it can be 3687), ITGB1 (the GeneID of the gene encoding it can be 3688), CD29 (the GeneID of the gene encoding it can be 3688), ITGB2 (the GeneID of the gene encoding it can be 3689), CD18 (the GeneID of the gene encoding it can be 3689), LFA-1 (the GeneID of the gene encoding it can be 3689), ITGB7 (the GeneID of the gene encoding it can be 3695), NKG2D (the GeneID of the gene encoding it can be 22914), NKG2C (the GeneID of the gene encoding it can be 3822), TNFR2 (the GeneID of the gene encoding it can be 7133), TRANCE/RANKL (the GeneID of the gene encoding it can be 8600), DNAM1 (CD226) (the GeneID of the gene encoding it can be 10666), SLAMF4 (CD244, 2B4) (the GeneID of the gene encoding it can be 51744), CD84 (the GeneID

of the gene encoding it can be 8832), CD96 (Tactile) (the GeneID of the gene encoding it can be 10225), CEACAM1 (the GeneID of the gene encoding it can be 634), CRTAM (the GeneID of the gene encoding it can be 56253), Ly9 (CD229) (the GeneID of the gene encoding it can be 4063), CD160 (BY55) (the GeneID of the gene encoding it can be 11126), PSGL1 (the GeneID of the gene encoding it can be 6404), CD100 (SEMA4D) (the GeneID of the gene encoding it can be 10507), CD69 (the GeneID of the gene encoding it can be 969), SLAMF6 (NTB-A, Ly108) (the GeneID of the gene encoding it can be 114836), SLAM (SLAMF1, CD150, IPO-3) (the GeneID of the gene encoding it can be 6504), BLAME (SLAMF8) (the GeneID of the gene encoding it can be 56833), SELPLG (CD162) (the GeneID of the gene encoding it can be 6404), LTBR (the GeneID of the gene encoding it can be 4055), LAT (the GeneID of the gene encoding it can be 27040), GADS (the GeneID of the gene encoding it can be 9402), SLP-76 (the GeneID of the gene encoding it can be 3937), PAG/Cbp (the GeneID of the gene encoding it can be 55824), CD19a, and ligands specifically binding to CD3, ligands specifically binding to CD28, ligands specifically binding to HVEM, ligands specifically binding to CD40L, ligands specifically binding to OX40, and ligands specifically binding to 4-1BB. A costimulatory intracellular signaling domain may refer to the intracellular portion of a T cell activator. An intracellular signaling domain may comprise the complete intracellular portion of a molecule from which it is derived or the complete natural intracellular signaling domain or a functional fragment thereof.

In the present application, the term "T cell growth factor" generally refers to a biological active polypeptide or a small molecular compound that causes cell proliferation. For example, the T cell growth factors in the present application may comprise variants, homologues thereof or any substances comprising functionally active fragments thereof. In one embodiment, the T cell growth factors may be one or more factors selected from the group consisting of: IL-2 (the GeneID of the gene encoding it can be 3558), IL-4 (the GeneID of the gene encoding it can be 3565), IL-6 (the GeneID of the gene encoding it can be 3569), IL-7 (the GeneID of the gene encoding it can be 3574), IL-10 (the GeneID of the gene encoding it can be 3586), IL-12 (the GeneID of the gene encoding it can be 3592 or 3593), IL-15 (the GeneID of the gene encoding it can be 3600), IL-21 (the GeneID of the gene encoding it can be 59067), TNF-$\alpha$ (the GeneID of the gene encoding it can be 100137091), interferon $\gamma$ (the GeneID of the gene encoding it can be 3458), GZMB (the GeneID of the gene encoding it can be 3002), CD107a (the GeneID of the gene encoding it can be 6499), etc.

In the present application, the term "substantially simultaneously" generally means that TILs can be in contact with two or more substances simultaneously within a period of time during the contact process but may not be limited to the fact that the TILs are always in contact with the two or more substances simultaneously during the entire contact process. In one embodiment, substantially simultaneously may mean that the TILs can be in contact with at least 10%, 20%, 30%, 40%, 50%, 60%, 70%, 75%, 80%, 85%, 90%, and 95% of each of the two or more substances simultaneously within a period of time.

In the present application, the term "solid medium" or "medium" generally refers to a solid phase material with a binding function. For example, the solid medium of the present application may refer to a material that binds one or more substances within the medium and/or on the surface of the medium through covalent binding and/or non-covalent binding. For example, the solid medium of the present application may bind one or more T cell activators. For example, the solid medium of the present application may refer to a material that binds CD28 antibody or an antigen-binding fragment thereof and CD3 antibody or an antigen-binding fragment thereof within the medium and/or on the surface of the medium through covalent binding and/or non-covalent binding. For example, the solid medium of the present application may be microspheres including OKT3 antibodies and 15E8 antibodies and having a diameter from about 500 nm to about 10 $\mu$m. For example, the solid medium of the present application may be polymeric materials. For example, the solid medium of the present application may be microspheres having a diameter of at least about 500 nm. For example, the solid medium of the present application may be nanomatrix. For example, the solid medium of the present application may be nanomatrix including OKT3 antibodies and 15E8 antibodies and having a diameter from about 1 nm to about 500 nm.

In the present application, the term "nanomatrix" generally refers to a material having a diameter from about 1 nm to about 500 nm. In the present application, the nanomatrix may have a binding function, for example, the nanomatrix in the present application may bind one or more T cell activators. In the present application, the nanomatrix may comprise polymers, for example, the nanomatrix in the present application may comprise degradable polymers. In the present application, the nanomatrix may comprise polysaccharide and/or glucan.

In the present application, the term "dendritic cell" generally refers to antigen-presenting cells that are present *in vivo, in vitro, ex vivo* or in the host or subject, or can be derived from hematopoietic stem cells or monocytes. Dendritic cells and precursors thereof may be isolated from various lymphoid organs such as spleen, lymphatic gland and bone marrow, and peripheral blood. The dendritic cells in the present application may have characteristic morphology, such as thin layers (lamellipodia) extending in multiple directions of the dendritic cell body. Generally, dendritic cells can express high levels of MHCs and costimulatory (e.g., B7-1 and B7-2) molecules. Dendritic cells can induce antigen-specific differentiation of T cells *in vitro* and can elicit primary T cell responses *in vitro* and *in vivo.*

In the present application, the term *"in vitro* expansion" generally refers to changes in the number of cells resulting from culture, but the expanded cells may also subject to changes in the number and/or proportion of cells, changes in secretion capacity, changes in killing ability or changes in expression capacity, or any combination thereof. The changes in the present application may be improvement or decreasement. In the present application, *in vitro* expansion may be for the purpose of expansion, or for detecting the function of TIL cells, e.g., for detecting the ability of TIL cells to release cytokines. However, the operation steps performed on TIL cells (e.g., adding one or more substances to the culture medium of TIL cells to detect the ability of TIL cells to release cytokines) may not belong to the *in vitro* expansion of the present application.

In the present application, the term "peripheral mononuclear cells" or "peripheral blood mononuclear cells" generally refers to cells with single nuclei in peripheral blood. For example, in the present application, the peripheral blood mononuclear cells of the present application may comprise lymphocytes, monocytes and/or dendritic cells.

In the present application, the term "cytokine" generally refers to a protein released by one cell population that acts as an intercellular regulator for another cell. The cytokine in the present application may be lymphokines, monokines, and polypeptide hormones. The cytokine in the present application may comprise interleukins (ILs), such as IL-1, IL-1$\alpha$, IL-2, IL-3, IL-4, IL-5, IL-6, IL-7, IL-8, IL-9, IL-10, IL-11, IL-15, IL-21, and/or IL-12. In the present application, the term cytokine may comprise proteins from natural sources or from recombinant cell cultures, biologically active equivalents of cytokines with natural sequence, and functionally active fragments thereof.

In the present application, the term "diameter" generally refers to the diameter of the cross-section of the substance of the present application. For example, when the substance of the present application is not spherical, the term "diameter" generally refers to the maximum diameter and/or average diameter of the largest cross-section of the substance of the present application. The method for determining the diameter of the substance may be a method commonly used in the art, such as transmission electron microscopy.

In the present application, the term "tumor" generally refers to any new pathological tissue proliferation. The tumor in the present application may be benign or malignant. The tumor in the present application may be solid or hematologic. The term "tumor" may be one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In the present application, the term "tumor tissue" generally refers to samples of any tissues from tumors in a subject, including any solid tumors and/or non-solid tumors in the subject.

In the present application, the term "CD28 agonist" generally refers to compounds that bind CD28 protein on the cell surface and elicit responses in the cells. For example, the CD28 agonist of the present application may be a small molecule formulation that binds CD28. For example, the CD28 agonist of the present application may be an antibody that binds CD28 or an antigen-binding fragment thereof.

In the present application, the term "proportion of T cell subpopulations" generally refers to the proportions of different T cell subpopulations in TIL cells or TIL populations. For example, the different T cell subpopulations in the present application have different immune activities and/or differentiation capacities. For example, the T cell subpopulations in the present application may be distinguished based on T cell surface markers. For example, central memory T cells may have a phenotype of CD45RO$^+$CD62L$^+$ or CD45RA$^-$CCR7$^+$. For example, naive T cells may have a phenotype of CD45RO$^-$CD62L$^+$. For example, regulatory T cells may have a phenotype of CD4$^+$CD25$^+$Foxp3$^+$. For example, activated T cells may have a phenotype of CD25$^+$, CD28$^+$, PD-1$^+$ or 41BB$^+$. For example, tumor-specific T cells may have a phenotype of CD103$^+$CD39$^+$. For example, stem cell-like T cells may have a phenotype of TCF1$^+$.

In the present application, the term "number of TIL cells" generally refers to the cell number in the TIL cells of the present application. In the present application, the number of TIL cells may refer to the cell number in the TIL population obtained in any one stage of the present application. For example, the number of TIL cells may refer to the cell number in the first TIL population derived from tumor tissues and not expanded *in vitro*. For example, the number of TIL cells may refer to the cell number of the second TIL population through the first stage *of in vitro* expansion. For example, the number of TIL cells may refer to the cell number of the third TIL population through the second stage *of in vitro* expansion. For example, the number of TIL cells may refer to the TIL cells finally obtained by any one culture method of the present application. In the present application, the number of TIL cells may be determined by methods commonly used in the art, which may comprise, for example, but not limited to, manual cell counting on a cell counting plate and/or counting with an automatic cell counter.

In the present application, the terms "about" and "approximately" generally refer to being within a statistically significant numerical range. Such a range can be within an order of magnitude of a given value or range, e.g., it can be within 50%, within 20%, within 10%, or within 5%. The permissible variation encompassed by the term "about" or "approximately" depends on the particular system under study, and can be readily understood by one of ordinary skill in the art. The terms "above", "below", "at most" and "at least" can comprise the number itself.

## Detailed description of the invention

In one aspect, the present application provides a method for culturing tumor infiltrating lymphocytes (TILs), the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the TILs with feeder cells after contacting the TILs with T cell activators and/or T cell growth factors for a period of time.

For example, the method may comprise reducing the expression and/or decreasing the activity of two or more proteins of the TILs after co-culturing the TILs with the feeder cells.

For example, the method may comprise co-culturing the TILs with the feeder cells after reducing the expression and/or decreasing the activity of two or more proteins of the TILs.

For example, the method may comprise reducing the expression and/or decreasing the activity of two or more proteins of the TILs after contacting the TILs with the T cell activators and/or the T cell growth factors and before co-culturing the TILs with the feeder cells.

For example, the method may comprise reducing the expression and/or decreasing the activity of two or more proteins of the TILs substantially simultaneously with contacting the TILs with the T cell activators and/or the T cell growth factors.

For example, the method may comprise reducing the expression and/or decreasing the activity of two or more proteins of the TILs substantially simultaneously with contacting the TILs with the feeder cells.

In one aspect, the present application provides a method for culturing tumor infiltrating lymphocytes (TILs), the method may comprise reducing the expression and/or decreasing the activity of two or more proteins of the TILs.

In another aspect, the present application provides a method for culturing tumor infiltrating lymphocytes (TILs), the method may comprise reducing the expression and/or decreasing the activity of two or more proteins of the TILs, wherein the TILs comprise TILs obtained by co-culturing the TILs with feeder cells after contacting the TILs with T cell activators and/or T cell growth factors for a period of time.

In another aspect, the present application further provides a method for culturing tumor infiltrating lymphocytes (TILs), the method may comprise co-culturing the TILs with feeder cells after contacting the TILs with T cell activators and/or T cell growth factors for a period of time, wherein the TILs comprise TILs obtained by reducing the expression and/or decreasing the activity of the two or more proteins of the TILs.

In another aspect, the present application further provides a method for culturing tumor infiltrating lymphocytes (TILs), the method may comprise reducing the expression and/or decreasing the activity of two or more proteins of the TILs and contacting the TILs with a CD28 agonist.

In another aspect, the present application further provides a method for culturing tumor infiltrating lymphocytes (TILs), the method may comprise reducing the expression and/or decreasing the activity of two or more proteins of the TILs, wherein the TILs comprise TILs obtained by contacting the TILs with a CD28 agonist.

In another aspect, the present application further provides a method for culturing tumor infiltrating lymphocytes (TILs), the method may comprise contacting the TILs with a CD28 agonist, wherein the TILs comprise TILs obtained by reducing the expression and/or decreasing the activity of the two or more proteins of the TILs.

For example, the CD28 agonist comprises an anti-CD28 antibody and/or an antigen-binding fragment thereof, CD80 and/or a functionally active fragment thereof, and/or CD86 and/or a functionally active fragment thereof, and recombinant protein of the above substances.

For example, compared to TILs with unchanged expression and/or activity of the two or more proteins, TILs obtained by reducing the expression and/or decreasing the activity of the two or more proteins of the TILs show improved TIL properties. In one embodiment, TILs with unchanged expression and/or activity of the two or more proteins may refer to TIL cells which are derived from the same donor and in which the expression of the two or more proteins of the TILs has not been reduced and/or the activity thereof has not been decreased. In one embodiment, TILs with unchanged expression and/or activity of the two or more proteins may refer to TIL cells which are derived from the same donor and in which the expression of another gene other than the two or more proteins of the TILs (e.g., basically having no effect on cell function if knocking out this another gene) has not been reduced and/or the activity thereof has not been decreased.

In one embodiment, corresponding TILs in which the expression of two or more proteins of the TILs has not been reduced and/or the activity thereof has not been decreased may refer to TIL cells which are derived from the same donor and isolated in the same way and in which the expression of the two or more proteins of the TILs has not been reduced and/or the activity thereof has not been decreased. In one embodiment, corresponding TILs in which the expression of two or more proteins of the TILs has not been reduced and/or the activity thereof has not been decreased may refer to TIL cells which are derived from the same tumor source of the same donor and in which the expression of the two or more proteins of the TILs has not been reduced and/or the activity thereof has not been decreased. In one embodiment, corresponding TILs in which the expression of two or more proteins of the TILs has not been reduced and/or the activity thereof has not been decreased may refer to TIL cells which are derived from the same tumor source of the same donor and isolated in the same way and in which the expression of the two or more proteins of the TILs has not been reduced and/or the activity thereof has not been decreased. In one embodiment, corresponding TILs in which the expression of two or more proteins of the TILs has not been reduced and/or the activity thereof has not been decreased may mean that, the TIL cells derived from the same donor are divided into two groups, wherein one group of TIL cells in which the expression of two or more proteins of the TILs has not been reduced and/or the activity thereof has not been decreased may be corresponding TILs in which the expression of two or more proteins of the TILs has not been reduced and/or the activity thereof has not been decreased. In one embodiment, corresponding TILs in which the expression of two or more proteins of the TILs has not been reduced and/or the activity thereof has not been decreased may mean that, the TIL cells derived from the same donor and isolated in the same way are divided into two groups, wherein one group of TIL cells in which the expression of two or more proteins of the TILs has not been reduced and/or the activity thereof has not been decreased may be corresponding TILs in which the expression of two or more proteins of the TILs has not been reduced and/or the activity thereof has not been decreased. In one embodiment, corresponding TILs in which the expression of two or more proteins of the TILs has not been reduced and/or the activity thereof has not been decreased may mean that, the TIL cells derived from the same tumor source of the same donor are divided into two groups, wherein one group of TIL cells in which the expression of two or more proteins of the TILs has not been reduced and/or the activity thereof has not been decreased may be corresponding TILs in which the expression of two or more proteins of the TILs has not been reduced and/or the activity thereof has not been decreased. In one embodiment, corresponding TILs in which the expression of two or more proteins of the TILs has not been reduced and/or the activity thereof has not been decreased may mean that, TIL cells derived from the same tumor source of the same donor and isolated in the same way are divided into two groups, wherein one group of TIL cells in which the expression of two or more proteins of the TILs has not been reduced and/or the activity thereof has not been decreased may be corresponding TILs in which the expression of two or more proteins of the TILs has not been reduced and/or the activity thereof has not been decreased. For example, the reduction in the expression and/or the decrease in the activity of the two or more proteins may mean that, the two or more proteins of a natural cell may be expressed to a certain extent, and through the treatment of the present application, the expression level of the two or more proteins of the cell is reduced. In other words, the reduction in the expression level of the two or more proteins can involve the natural cell transitioning from expressing the two or more proteins to substantially not expressing the two or more proteins or expressing the two or more proteins in a reduced amount.

In one embodiment, the improved TIL properties in the present application comprise one or more properties selected from the group consisting of: increased number and expansion capacity of TIL cells, increased proportion of viable cells, enhanced persistence, improved proportion of T cell subpopulations, enhanced cytokine secretion capacity, enhanced tumor cell killing ability, enhanced anti-apoptotic ability, and enhanced T cell receptor (TCR) clonal diversity. For example, TILs prepared by the method of the present application may have enhanced tumor suppression capacity, enhanced expansion capacity, enhanced *in vivo* persistence, enhanced cytokine release capacity and/or improved proportion of T cell subpopulations under a culture condition of IL-2 at low concentration. For example, the supporting concentration of IL-2 for TILs prepared by the method of the present application may be about 90% or less, about 80% or less, about 70% or less, about 60% or less, about 50% or less, about 40% or less, about 30% or less, about 20% or less, about 10% or less, about 5% or less, about 2% or less, about 1% or less, or about 0.1% or less of the supporting concentration for TILs commonly used in the art. For example, TILs prepared by the method of the present application may have enhanced tumor cell serial killing ability. For example, TILs prepared by the method of the present application may have enhanced persistence. For example, TILs obtained in the present application may have enhanced long-term persistence under culture after withdrawal of IL-2. For example, compared to the culture conditions for TILs not obtained by the culture method of the present application, when the concentration of IL-2 in the culture conditions for TILs obtained in the present application can be reduced by 100%, at least 90%, at least 80%, at least 70%, at least 60%, at least 50%, at least 40%, at least 30%, at least 20%, at least 10%, at least 5%, or at least 1%, the TILs obtained in the present application can be continuously expanded.

In one embodiment, the improved number and expansion capacity of TIL cells and the enhanced anti-apoptotic ability in the present application may mean that, compared to corresponding TILs in which the expression of two or more proteins of the TILs has not been reduced and/or the activity thereof has not been decreased during the stage of *in vitro* expansion, the cell number of the TILs of the present application in which the expression of the two or more proteins of the TILs is reduced and/or the activity thereof is decreased during at least one stage of *in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the enhanced expansion capacity of TILs can be manifested as an increase in the proliferation rate of TILs. For example, the improved TIL cell number can be manifested as an increase in the cell viability of TILs. In one embodiment, the increased TIL cell number in the present application may mean that, compared to corresponding TILs in which the expression of two or more proteins of the TILs has not been reduced and/or the activity thereof has not been decreased during the stage of *in vitro* expansion, the cell number of the TILs in the present application in which the expression of the two or more proteins of the TILs is reduced and/or the activity thereof is decreased during at least one stage *of in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the enhanced cytokine secretion capacity in the present application may refer to the enhanced secretion capacity of cytokine of TIL cells selected from the group consisting of: CD107a, GZMB, IL-2, TNF-$\alpha$, and IFN-$\gamma$. In one embodiment, the enhanced cytokine secretion capacity in the present application may mean that, compared to corresponding TILs in which the expression of two or more proteins of the TILs has not been reduced and/or the activity thereof has not been decreased during the stage of *in vitro* expansion, the cytokine secretion capacity of the TILs in the present application in which the expression of the two or more proteins of the TILs is reduced and/or the activity thereof is decreased during at least one stage of *in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion capacity in the present application may mean that, compared to corresponding TILs in which the expression of the two or more proteins of the TILs has not been reduced and/or the activity thereof has not been decreased during the stage of *in vitro* expansion, the cytokine secretion capacity of the TILs in the present application in which the expression of the two or more proteins of the TILs is reduced and/or the activity thereof is decreased during at least one stage of *in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the cytokine secretion capacity of the TILs in the present application may be determined by measuring the cytokine expression capacity of the TIL cells. In one embodiment, the cytokine secretion capacity of the TILs in the present application may be determined by measuring the cytokine release capacity of the TIL cells. In one embodiment, the cytokine secretion capacity of the TILs in the present application is determined by flow cytometry or CBA (Cytometric Bead Array).

In one embodiment, the increased NK cell proportion in the present application may be an increase in the proportion of NK cells in the TIL cells. For example, the proportion of NK cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the enhanced tumor cell killing ability in the present application may mean that, compared to corresponding TILs in which the expression of two or more proteins of the TILs has not been reduced and/or the activity thereof has not been decreased during the stage of *in vitro* expansion, the tumor cell killing rate of the TILs in the present application in which the expression of the two or more proteins of the TILs is reduced and/or the activity thereof is decreased during at least one stage *of in vitro* expansion may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced tumor cell killing ability in the present application may mean that, compared to corresponding TILs in which the expression of the two or more proteins of the TILs has not been reduced and/or the activity thereof has not been decreased during the stage of *in vitro* expansion, the tumor cell killing rate of the TILs in the present application in which the expression of the two or more proteins of the TILs is reduced and/or the activity thereof is decreased during at least one stage *of in vitro* expansion may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the tumor cell killing rate of the TILs in the present application may be measured by CFSE and DAPI staining methods. In one embodiment, the tumor cell killing rate of the TILs in the present application may be determined by measuring the activity of Caspase-3/7 using an IncuCyte system. In one embodiment, the tumor cell killing of the TILs in the present application may refer to the ability of TILs to kill solid tumor cells. In one embodiment, the tumor cell killing of the TILs in the present application may refer to the ability of TILs to kill melanoma cells. In one embodiment, the tumor cell killing of the TILs in the present application may refer to the ability of TILs to kill ovarian cancer cells.

In one embodiment, the enhanced T cell receptor (TCR) clonal diversity in the present application may comprise that, during the long-term culture, compared to corresponding TILs in which the expression of two or more proteins of the TILs has not been reduced and/or the activity thereof has not been decreased during the stage *of in vitro* expansion, there are more kinds of TCRs being expressed in the TIL cell populations of the present application in which the expression of the two or more proteins of the TILs has been reduced and/or the activity thereof has been decreased during at least one stage *of in vitro* expansion, e.g., it may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the improved proportion of T cell subpopulations in the present application may comprise one or more selected from the group consisting of: increased proportion of CD4$^+$ cells, decreased proportion of CD8$^+$ cells, increased proportion of central memory T cells and/or naive T cells, decreased proportion of regulatory T cells, increased proportion of activated T cells, increased proportion of tumor-specific T cells, and increased proportion of stem cell-like T cells.

In one embodiment, the increased proportion of CD4$^+$ cells in the present application may be an increase in the proportion of CD4 positive cells in TIL cells. For example, the proportion of CD4$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the decreased proportion of CD8$^+$ cells in the present application may be a decrease in the proportion of CD8 positive cells in TIL cells. For example, the proportion of CD8$^+$ cells in TIL cells may decrease by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the increased proportion of central memory T cells in the present application may be an increase in the proportion of CD45RA$^-$CCR7$^+$ cells or CD45RO$^+$CD62L$^+$ cells in TIL cells. For example, the proportion of central memory T cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the decreased proportion of regulatory T cells in the present application may be a decrease in the proportion of CD4$^+$CD25$^+$Foxp3$^+$ cells in TIL cells. For example, the proportion of regulatory T cells in TIL cells may decrease by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the increased proportion of activated T cells in the present application may be an increase in the proportion of CD25$^+$, CD28$^+$, PD-1$^+$ or 41BB$^+$ cells in TIL cells. For example, the proportion of activated T cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of CD25$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of CD28$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of PD-1$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of 41BB$^+$ cells in TIL cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or may increase by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

For example, the increased proportion of naive T cells in the present application may be an increase in the proportion of CD45RO$^-$CD62L$^+$ cells in immune cells. For example, the proportion of naive cells in immune cells may increase by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

For example, the step of reducing the expression and/or decreasing the activity of the two or more proteins of the TILs in the method of the present application may comprise introducing a gene regulatory system into the TIL cells.

For example, the gene regulatory system is capable of destroying the target gene encoding the two or more proteins at the DNA level. For example, the gene regulatory system can destroy a region or a fragment thereof containing the target gene in the genome of TIL cells. For example, after using the gene regulatory system, the DNA region or a fragment thereof containing the target gene in TIL cells is cleaved, leading to a reduction in the expression capacity of the target gene or the suppression of the activity of the target gene. For example, the editing effect of the gene regulatory system on the target gene can be long-lasting and sustained.

For example, the gene regulatory system may comprise a guide nucleic acid molecule and a zymoprotein. For example, the zymoprotein may have nucleic acid cleavage activity, and the guide nucleic acid molecule can guide the zymoprotein to specifically cleave the region or a fragment thereof wherein the target gene encoding the two or more proteins is located. For example, the guide nucleic acid molecule and the zymoprotein can exist in the form of a ribonucleoprotein complex (RNP) or independently of each other. For example, the zymoprotein may comprise a Cas protein.

For example, in the method of the present application, the step of reducing the expression and/or decreasing the activity of the two or more proteins of the TILs may comprise introducing a ribonucleoprotein complex (RNP) comprising the guide nucleic acid molecule and the zymoprotein into the TILs. For example, the zymoprotein may comprise a Cas protein, a Cas protein homolog, or functionally active fragments thereof. For example, the guide nucleic acid molecule may comprise a guide RNA (gRNA). For example, the two or more ribonucleoprotein complexes (RNP) comprising the guide nucleic acid molecule and the zymoprotein can be introduced into the TILs at the same time. For example, the two or more ribonucleoprotein complexes (RNP) comprising the guide nucleic acid molecule and the zymoprotein can be introduced into the TILs in succession.

For example, lipid nanoparticles (LNPs) are a method well known for delivering nucleotide and protein cargos, which can be used to deliver gRNA, mRNA encoding Cas9, Cas9 protein, and RNP in the present application. In some embodiments, LNPs are used to deliver nucleic acid, protein, or nucleic acid and protein. In some embodiments, an electroporator known in the art is used to deliver nucleic acid, protein, or nucleic acid and protein. In some embodiments, the present application comprises a method for delivering one or more gRNAs of the present application to an individual, wherein the gRNAs are bound to the LNPs. In some embodiments, gRNA/LNP are also combined with Cas9 protein or mRNA encoding the Cas9.

For example, the gRNA can be used for binding the sequence of the target gene encoding the two or more proteins. For example, the binding of the gRNA to the sequence of the target gene may be completely complementary, partially complementary, or may involve hybridization with the sequence of the target gene under moderate to stringent conditions. For example, the binding of the gRNA to the sequence of the target gene allows for the specific cleavage of the target gene by the CRISPR system associated with the gRNA.

For example, the target gene of the present application may include a gene of a protein encoding the suppressor of Janus kinase-signal transducer and activator of transcription (JAK-STAT) pathway, e.g., SOCS1. In one embodiment, the guide nucleic acid molecule is capable of binding to a region or a fragment thereof defined by any one group of genomic coordinates shown in Table 1C.

For example, the target gene of the present application may comprise a gene of a protein encoding an NF-κB pathway suppressor, e.g., TNFAIP3. In one embodiment, the guide nucleic acid molecule is capable of binding to a region or a fragment thereof defined by any one group of genomic coordinates shown in Table 1D.

For example, the target gene of the present application may comprise a gene of a protein encoding an E3 ubiquitin ligase, e.g., CBLB. In one embodiment, the guide nucleic acid molecule is capable of binding to a region or a fragment thereof defined by any one group of genomic coordinates shown in Table 1A.

For example, the target gene of the present application may comprise a gene of a protein encoding a zinc finger domain, e.g., ZC3H12A. In one embodiment, the guide nucleic acid molecule is capable of binding to a region or a fragment thereof defined by any one group of genomic coordinates shown in Table 1B.

For example, the guide nucleic acid molecule of the present application is capable of binding to a region or a fragment thereof selected from a group as shown below: SEQ ID NOs: 34 to 35, 38 to 39, 42 to 44, and 48 to 50. For example, the guide nucleic acid molecule of the present application is capable of binding to a region or a fragment thereof selected from a group as shown below: SEQ ID NOs: 59 to 63. For example, the target sequence of the present application may be derived from human chr16: 11255238- 11255256, human chr6: 137871501- 137871520, human chr6: 137876102-137876121, human chr6: 137871475- 137871494, human chr1: 37481709-37481728, and human chr1: 37481641-37481660. For example, the guide nucleic acid molecule of the present application is capable of binding to a region or a fragment thereof selected from a group as shown below: SEQ ID NO: 64 to 68. For example, the guide nucleic acid molecule of the present application is capable of binding to SOCS1, and the guide nucleic acid molecule comprises GACGCCTGCGGATTCTACT (SEQ ID NO: 54), referred to as SC-4 in the present application. For example, the guide nucleic acid molecule of the present application is capable of binding to TNFAIP3, and the guide nucleic acid molecule comprises CTTGTGGCGCTGAAAACGAA (SEQ ID NO: 40), referred to as TP2 in the present application. For example, the guide nucleic acid molecule of the present application is capable of binding to TNFAIP3, and the guide nucleic acid molecule comprises TGTCATAGCCGAGAACAATG (SEQ ID NO: 55), referred to as TP-N8 in the present application. For example, the guide nucleic acid molecule of the present application is capable of binding to TNFAIP3, and the guide nucleic acid molecule comprises TCAACTGGTGTCGAGAAGTC (SEQ ID NO:56), referred to as TP-N20 in the present application. For example, the guide nucleic acid molecule of the present application is capable of binding to ZC3H12A, and the guide nucleic acid molecule comprises AGCTGGCCTACGAGTCTGAC (SEQ ID NO: 57), referred to as ZC-11 in the present application. For example, the guide nucleic acid molecule of the present application is capable of binding to ZC3H12A, and the guide nucleic acid molecule comprises GTCGTGATGGTGTGAACACC (SEQ ID NO: 58), referred to as ZC-13 in the present application. For example, there may be a protospacer adjacent motif (PAM) downstream of the region targeted by the guide nucleic acid molecule of the present application, which may be GGG, TGG, or AGG. For example, once the PAM region of the target gene is identified, a person of skill in the art can easily determine the target sequence consisting of about 15 to about 25 nucleotides upstream of the 5' end of the PAM of the target gene, and at the same time, a suitable gRNA can be designed for that target gene. For example, the guide nucleic acid molecule is capable of binding to a sequence consisting of about 15 to about 25 nucleotides upstream of the 5' end of the protospacer adjacent motif (PAM) selected from a group as shown below: AGG, TGG, GGG, and CGG.

For example, the guide nucleic acid molecule may comprise a target sequence consisting of about 15 to about 25 nucleotides preceding the PAM region as indicated by CGG in the DNA wherein the SOCS1 gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by CGG in the DNA wherein the SOCS1 gene is located. For example, the target sequence may be derived from human chr16:11255292-11255311.

For example, the guide nucleic acid molecule may comprise a target sequence consisting of about 10 to about 30 nucleotides preceding the PAM region as indicated by AGG in the DNA wherein the SOCS1 gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by AGG in the DNA wherein the SOCS1 gene is located. For example, the target sequence may be derived from human chr16:11255230-11255249.

For example, the guide nucleic acid molecule may comprise a target sequence consisting of about 10 to about 30 nucleotides preceding the PAM region as indicated by CGG in the DNA wherein the TNFAIP3 gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by CGG in the DNA wherein the TNFAIP3 gene is located. For example, the target sequence may be derived from human chr6: 137871501-137871520.

For example, the guide nucleic acid molecule may comprise a target sequence consisting of about 10 to about 30 nucleotides preceding the PAM region as indicated by AGG in the DNA wherein the TNFAIP3 gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by AGG in the DNA wherein the TNFAIP3 gene is located. For example, the target sequence may be derived from human chr6: 137878653-137878672.

For example, the guide nucleic acid molecule may comprise a target sequence consisting of about 10 to about 30 nucleotides preceding the PAM region as indicated by AGG in the DNA wherein the CBLB gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by TGG in the DNA wherein the CBLB gene is located. For example, the target sequence may be derived from human chr3: 105746016-105746035.

For example, the guide nucleic acid molecule may comprise a target sequence consisting of about 10 to about 30 nucleotides preceding the PAM region as indicated by TGG in the DNA wherein the CBLB gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by AGG in the DNA wherein the CBLB gene is located. For example, the target sequence may be derived from human chr3:

105751606-105751625.

For example, the guide nucleic acid molecule may comprise a target sequence consisting of about 10 to about 30 nucleotides preceding the PAM region as indicated by GGG in the DNA wherein the CBLB gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by AGG in the DNA wherein the CBLB gene is located. For example, the target sequence may be derived from human chr3: 105702222-105702241.

For example, the guide nucleic acid molecule may comprise a target sequence consisting of about 10 to about 30 nucleotides preceding the PAM region as indicated by GGG in the DNA wherein the ZC3H12A gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by GGG in the DNA wherein the ZC3H12A gene is located. For example, the target sequence may be derived from human chr1: 37475809-37475828.

For example, the guide nucleic acid molecule may comprise a target sequence consisting of about 10 to about 30 nucleotides preceding the PAM region as indicated by TGG in the DNA wherein the ZC3H12A gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by TGG in the DNA wherein the ZC3H12A gene is located. For example, the target sequence may be derived from human chr1: 37481716-37481735.

For example, the guide nucleic acid molecule may comprise a target sequence consisting of about 10 to about 30 nucleotides preceding the PAM region as indicated by AGG in the DNA wherein the ZC3H12A gene is located. For example, the guide nucleic acid molecule may comprise a target sequence consisting of about 15 to about 25, about 17 to about 25, about 19 to about 25, about 20 to about 25, about 21 to about 25, about 23 to about 25, about 15 to about 23, about 17 to about 23, about 19 to about 23, about 20 to about 23, about 21 to about 23, about 15 to about 21, about 17 to about 21, about 19 to about 21, about 20 to about 21, about 15 to about 20, about 17 to about 20, about 19 to about 21, about 15 to about 19, about 17 to about 19, or about 15 to about 17 nucleotides preceding the PAM region as indicated by AGG in the DNA wherein the ZC3H12A gene is located. For example, the target sequence may be derived from human chr1: 37483345-37483364.

For example, the guide nucleic acid molecule may comprise a sequence as shown in any one of SEQ ID NOs: 36 to 37, 40 to 41, 45 to 47, and 51 to 53.

For example, the present application provides a combination of guide nucleic acid molecules, and the combination of guide nucleic acid molecules can reduce the expression of CBLB and ZC3H12A, SOCS1 and CBLB, SOCS1 and TNFAIP3, SOCS1 and ZC3H12A, TNFAIP3 and CBLB, and/or TNFAIP3 and ZC3H12A. For example, the guide nucleic acid molecule targeting CBLB in the present application can be represented by CB, such as CB1 and CB2; for example, the guide nucleic acid molecule targeting SOCS1 in the present application can be represented by SC, such as SC3 and SC4; for example, the guide nucleic acid molecule targeting TNFAIP3 in the present application can be represented by TP, such as TP4, TP2, TP-N8, and TP-N20; for example, the guide nucleic acid molecule targeting ZC3H12A in the present application can be represented by ZC, such as ZC3, ZC7, ZC-11, and ZC13.

For example, the combined administration of CB1 and ZC3, wherein the sgRNA as shown in SEQ ID NO: 45 (TTCCGCAAAATAGAGCCCCA) is CB1, the sgRNA as shown in SEQ ID NO: 51 (CAGCTCCCTCTAGTCCCGCG) is ZC3.

The combined administration of SC3 and CB1, wherein the sgRNA as shown in SEQ ID NO: 37 (GGGCCCCCAGTAGAATCCGC) is SC3, the sgRNA as shown in SEQ ID NO: 45 (TTCCGCAAAATAGAGCCCCA) is CB1.

The combined administration of SC3 and CB2, wherein the sgRNA as shown in SEQ ID NO: 37 (GGGCCCCCAGTAGAATCCGC) is SC3, the sgRNA as shown in SEQ ID NO: 47 (TGTGGGATGTCGACTCCTAG) is CB2.

The combined administration of SC3 and TP4, wherein the sgRNA as shown in SEQ ID NO: 37 (GGGCCCCCAGTAGAATCCGC) is SC3, the sgRNA as shown in SEQ ID NO: 41 (TATGCCATGAGTGCTCAGAG) is TP4.

The combined administration of SC3 and ZC3, wherein the sgRNA as shown in SEQ ID NO: 37 (GGGCCCCCAGTAGAATCCGC) is SC3, the sgRNA as shown in SEQ ID NO: 51 (CAGCTCCCTCTAGTCCCGCG) is ZC3.

The combined administration of SC3 and ZC7, wherein the sgRNA as shown in SEQ ID NO: 37 (GGGCCCCCAGTAGAATCCGC) is SC3, the sgRNA as shown in SEQ ID NO: 53 (GGTTCAGACCAGTACTCTCG) is ZC7.

The combined administration of TP4 and CB1, wherein the sgRNA as shown in SEQ ID NO: 41 (TATGCCATGAGTGCTCAGAG) is TP4, the sgRNA as shown in SEQ ID NO: 45 (TTCCGCAAAATAGAGCCCCA) is CB1.

The combined administration of TP4 and ZC3, wherein the sgRNA as shown in SEQ ID NO: 41 (TATGCCATGAGTGCTCAGAG) is TP4, the sgRNA as shown in SEQ ID NO: 51 (CAGCTCCCTCTAGTCCCGCG) is ZC3.

The combined administration of TP4 and ZC7, wherein the sgRNA as shown in SEQ ID NO: 41 (TATGCCATGAGTGCTCAGAG) is TP4, the sgRNA as shown in SEQ ID NO: 53 (GGTTCAGACCAGTACTCTCG) is ZC7.

For example, the proportion of cells expressing two or more proteins is reduced and/or the expression of the two or more proteins in an individual cell is decreased in the TILs obtained by reducing the expression and/or decreasing the activity of the two or more proteins of the TILs, compared to that in TILs with unchanged expression and/or activity of the two or more proteins.

For example, in the method of the present application, the proportion of cells expressing the two or more proteins is reduced by at least about 5% in the TILs obtained by reducing the expression and/or decreasing the activity of the two or more proteins of the TILs, compared to that in TILs with unchanged expression and/or activity of the two or more proteins. For example, the proportion of cells expressing the two or more proteins is reduced by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the proportion of cells expressing the two or more proteins may be from an observable cell proportion to 0%. For example, the proportion of cells expressing the two or more proteins may be reduced to at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%. For example, the proportion of cells expressing the two or more proteins can be tested by flow cytometry.

For example, in the method of the present application, the proportion of cells expressing the two or more proteins can be at most about 95% in the TILs obtained by reducing the expression and/or decreasing the activity of the two or more proteins of the TILs. For example, the proportion of cells expressing the two or more proteins can be at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5%. For example, the proportion of cells expressing the two or more proteins can be tested by flow cytometry.

For example, in the method of the present application, the expression of the two or more proteins in an individual cell can be reduced by at least about 5% in the TILs obtained by reducing the expression and/or decreasing the activity of the two or more proteins of the TILs, compared to that in TILs with unchanged expression and/or activity of the two or more proteins. For example, the expression of the two or more proteins in an individual cell may be reduced by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, or at least about 5%. For example, the expression of the two or more proteins in an individual cell may be from an observable level to 0%. For example, the expression of the two or more proteins in an individual cell may be reduced to at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, or at least about 1%.

For example, in the method of the present application, the expression of the two or more proteins in an individual cell in the TILs obtained by reducing the expression and/or decreasing the activity of the two or more proteins of the TILs can be at most about 95% that in the TILs with unchanged expression and/or activity of the two or more proteins. For example, the expression of the two or more proteins in an individual cell of TILs can be at most about 95%, at most about 90%, at most about 80%, at most about 70%, at most about 60%, at most about 50%, at most about 40%, at most about 30%, at most about 20%, at most about 19%, at most about 18%, at most about 17%, at most about 16%, at most about 15%, at most about 14%, at most about 13%, at most about 12%, at most about 11%, at most about 10%, at most about 9%, at most about 8%, at most about 7%, at most about 6%, or at most about 5% that in the TILs with unchanged expression and/or activity of the two or more proteins.

In one embodiment, the method of the present application may further comprise subjecting the TILs derived from tumor tissues and not expanded *in vitro* to at least one stage of *in vitro* expansion, wherein the TILs of the present application can be co-cultured with feeder cells during the at least one stage *of in vitro* expansion in the present application.

In one embodiment, during a single stage *of in vitro* expansion in the present application, the expression of two or more proteins of the TILs may be reduced and/or the activity thereof may be decreased, and the TILs may be co-cultured with the feeder cells of the present application. In one embodiment, the single stage of *in vitro* expansion in the present application may refer to the same one stage of *in vitro* expansion in the present application. For example, it may be all in the first stage of *in vitro* expansion in the present application, all in the second stage of *in vitro* expansion in the present application, or all in the third stage of *in vitro* expansion in the present application, etc.

In one embodiment, during the first stage *of in vitro* expansion in the present application, the expression of two or more proteins of the TILs may be reduced and/or the activity thereof may be decreased and the TILs may be co-cultured with the feeder cells of the present application. In one embodiment, during the second stage of *in vitro* expansion in the present application, the expression of two or more proteins of the TILs may be reduced and/or the activity thereof may be decreased and the TILs may be co-cultured with the feeder cells of the present application. In one embodiment, during the third stage *of in vitro* expansion in the present application, the expression of two or more proteins of the TILs may be reduced and/or the activity thereof may be decreased and the TILs may be co-cultured with the feeder cells of the present application.

In one embodiment, each stage of *in vitro* expansion may be divided depending on the change in the number of TIL cells. In one embodiment, when the number of TIL cells increases by at least about 1-fold, it can be considered that the TIL cells enter the next stage of *in vitro* expansion. In some embodiments, when the number of TIL cells increases by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, it can be considered that the TIL cells enter the next stage of *in vitro* expansion. In one embodiment, each stage of *in vitro* expansion may also be divided depending on the change in the culture conditions of the TIL cells. In one embodiment, after T cell activators and/or T cell growth factors are added or supplemented into the cell culture medium, it can be considered that the TIL cells enter the next stage of *in vitro* expansion. For example, after IL-2 is added or supplemented into the cell culture medium, it can be considered that the TIL cells enter the next stage of *in vitro* expansion. For example, when the expression of two or more proteins of the TILs is reduced and/or the activity thereof is decreased, it can be considered that the TIL cells enter the next stage of *in vitro* expansion. For example, after feeder cells are added or supplemented into the cell culture medium, it can be considered that the TIL cells enter the next stage of *in vitro* expansion. In one embodiment, after TIL cells have subjected to centrifugation and/or washing, it can be considered that the TIL cells enter the next stage of *in vitro* expansion. In one embodiment, each stage may also be divided depending on the culture days of the TIL cells. In one embodiment, after the TIL cells have been cultured *in vitro* for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days, or about 100 days, it can be considered that the TIL cells enter the next stage of *in vitro* expansion.

For example, the second stage of *in vitro* expansion can be carried out for at least about 7 days. For example, the second stage of *in vitro* expansion can be carried out for at least about 9 days. For example, the second stage of *in vitro* expansion can be carried out for at most about 14 days. For example, the second stage of *in vitro* expansion can be carried out for at most about 13 days. For example, the second stage of *in vitro* expansion can be carried out for about 7 days to about 14 days, about 9 days to about 14 days, about 7 days to about 13 days or about 9 days to about 13 days. For example, the second stage of *in vitro* expansion in the present application can be carried out for at least about 9 days, at least about 10 days, at least about 11 days, at least about 12 days, at least about 13 days, or at least about 14 days. For example, the second stage of *in vitro* expansion in the present application can be carried out for about 9 days to about 14 days. For example, the second stage of *in vitro* expansion in the present application can be carried out for about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the second stage of *in vitro* expansion in the present application can be considered as a REP (rapid expansion protocol) stage.

For example, the first stage of *in vitro* expansion can be carried out for at least about 7 days. For example, the first stage of *in vitro* expansion can be carried out for about 7 days to about 14 days. For example, the first stage *of in vitro* expansion in the present application can be carried out for at least about 7 days, at least about 8 days, at least about 9 days, at least about 10 days, at least about 11 days, at least about 12 days, at least about 13 days, or at least about 14 days. For example, the first stage of *in vitro* expansion in the present application can be carried out for about 7 days to about 14 days, about 8 days to about 14 days, about 9 days to about 14 days, about 10 days to about 14 days, about 11 days to about 14 days, about 12 days to about 14 days, about 13 days to about 14 days, about 9 days to about 13 days, about 10 days to about 13 days, about 11 days to about 13 days, about 12 days to about 13 days, about 9 days to about 12 days, about 10 days to about 12 days, about 11 days to about 12 days, or about 10 days to about 11 days. For example, the first stage of *in vitro* expansion in the present application can be considered as a preREP stage.

In one embodiment, the days for the second stage of *in vitro* expansion in the present application may be calculated from the time when the second stage of *in vitro* expansion starts. For example, just when the second stage of *in vitro* expansion starts, it can be considered that the second stage of *in vitro* expansion has been carried out for about 0 days. For example, after the second stage of *in vitro* expansion has been carried out for about 24 hours, it can be considered that the second stage *of in vitro* expansion has been carried out for about 1 day. For example, on the day when the second stage of *in vitro* expansion starts, it can be considered that the second stage *of in vitro* expansion has been carried out for about 0 days. In one embodiment, the days for the second stage of *in vitro* expansion in the present application may be calculated from the days the second stage of *in vitro* expansion has been carried out. For example, on the second day after the second stage of *in vitro* expansion starts, it can be considered that the second stage of *in vitro* expansion has been carried out for about 1 day.

In one embodiment, the culture method of the present application may be divided into two steps. For example, (A) a first TIL population derived from tumor tissues and not expanded *in vitro* can be contacted with T cell growth factors, wherein a second TIL population is obtained via the step (A); (B) the expression of two or more proteins of the second TIL population can be reduced and/or the activity thereof can be decreased, and the second TIL population is co-cultured with feeder cells after contacting the second TIL population with T cell activators and/or T cell growth factors for a period of time, wherein a third TIL population is obtained via the step (B). For example, the step (A) can be carried out for about 7 days to about 14 days. For example, the step (B) can be carried out for about 7 days to about 14 days.

In one embodiment, the culture method of the present application may be divided into three steps. For example, (A) a first TIL population derived from tumor tissues and not expanded *in vitro* can be contacted with T cell growth factors, wherein a second TIL population is obtained via the step (A); (B) the expression of two or more proteins of the second TIL population can be reduced and/or the activity thereof can be decreased, and the second TIL population can be contacted with T cell activators and/or T cell growth factors, wherein a third TIL population is obtained via the step (B); (C) the third TIL population can be co-cultured with feeder cells, wherein a fourth TIL population is obtained via the step (C). For example, the step (A) can be carried out for about 7 days to about 14 days. For example, the step (B) can be carried out for about 0 days to about 8 days. For example, the step (C) can be carried out for about 5 days to about 14 days.

In one embodiment, the culture method of the present application may be divided into four steps. For example, (A) a first TIL population derived from tumor tissues and not expanded *in vitro* can be contacted with T cell growth factors, wherein a second TIL population is obtained via the step (A); (B) the second TIL population can be contacted with T cell activators and/or T cell growth factors, wherein a third TIL population is obtained via the step (B); (C) the expression of two or more proteins of the third TIL population can be reduced and/or the activity thereof can be decreased, wherein a fourth TIL population is obtained via the step (C); (D) the fourth TIL population can be co-cultured with feeder cells, wherein a fifth TIL population is obtained via the step (D). For example, the step (A) can be carried out for about 7 days to about 14 days. For example, the step (B) can be carried out for about 0 days to about 4 days. For example, the step (C) can be carried out for about 0 days to about 4 days. For example, the step (D) can be carried out for about 5 days to about 14 days.

In one embodiment, the step (A) in the culture method of the present application is to resuscitate and/or continue culturing an *in vitro* TIL population to obtain a second TIL population. For example, the *in vitro* TIL population may comprise a TIL population obtained by *in vitro* expansion of a first TIL population derived from tumor tissues and not expanded *in vitro*. For example, the *in vitro* TIL population may comprise a TIL population obtained by contacting the first TIL population with T cell growth factors. For example, the *in vitro* TIL population may comprise a TIL population obtained by cryopreserving the first TIL population. For example, the *in vitro* TIL population may comprise a TIL population obtained by contacting the first TIL population with T cell growth factors and cryopreservation. For example, when the step (A) in the present application is to resuscitate and/or continue culturing an *in vitro* TIL population to obtain the second TIL population, the step (A) can be carried out for about 2 hours to about 4 days.

In one embodiment, during a single stage of *in vitro* expansion in the present application, the TILs of the present application can be contacted with one or more T cell activators and/or one or more T cell growth factors of the present application for a period of time and then co-cultured with feeder cells of the present application. In one embodiment, the period of time in the present application may be at least about 2 hours. In one embodiment, the period of time in the present application may be at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours, or at least about 72 hours. In one embodiment, the period of time in the present application may be about 6 hours to about 72 hours. In one embodiment, the period of time in the present application may be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. In one embodiment, the period of time in the present application may be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. In one embodiment, the period of time in the present application may be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

In one embodiment, the feeder cells of the present application may comprise antigen-presenting cells. In one embodiment, the feeder cells of the present application may comprise one or more cells selected from the group consisting of: peripheral mononuclear cells, dendritic cells, and artificial antigen-presenting cells. In one embodiment, the feeder cells of the present application may be peripheral mononuclear cells. In one embodiment, the feeder cells of the present application may be irradiated feeder cells. For example, the feeder cells of the present application may be isolated artificial antigen-presenting cells (aAPC), and the artificial antigen-presenting cells of the present application may comprise cells expressing HLA-A/B/C, CD64, CD80, ICOS-L, and/or CD58, can be modified to express more than one T cell activators of the present application. In one embodiment, the feeder cells of the present application may be irradiated, e.g., irradiated with gamma rays or irradiated with X-rays.

In one embodiment, the step of co-culturing the TILs of the present application with the feeder cells of the present application may comprise contacting the surface of the feeder cells of the present application with the surface of the TILs of the present application. In one embodiment, the step of co-culturing the TILs of the present application with the feeder cells of the present application comprises adding the feeder cells of the present application into the cell culture medium of the TILs of the present application.

In one embodiment, the feeder cells of the present application may be added into the cell culture medium of the TILs of the present application at a ratio of the feeder cells of the present application to the TILs of the present application from about 40:1 to about 400:1 in the present application. In one embodiment, the feeder cells of the present application may be added into the cell culture medium of the TILs of the present application at a ratio of the feeder cells of the present application to the TILs of the present application from about 40:1 to about 400:1, about 40:1 to about 300:1, about 40:1 to about 200:1, about 40:1 to about 100:1, about 40:1 to about 90:1, about 40:1 to about 80:1, about 40:1 to about 70:1, about 40:1 to about 60:1, about 40:1 to about 50:1, about 50:1 to about 400:1, about 60:1 to about 400:1, about 70:1 to about 400:1, about 80:1 to about 400:1, about 90:1 to about 400:1, about 100:1 to about 400:1, about 200:1 to about 400:1, or about 300:1 to about 400:1.

In one embodiment, the method of the present application may also comprise: subjecting TILs derived from tumor tissues and not expanded *in vitro* to at least one stage of *in vitro* expansion, wherein the TILs of the present application are contacted with one or more T cell activators during the at least one stage of *in vitro* expansion in the present application.

In one embodiment, during a single stage of i*n vitro* expansion in the present application, the TILs are contacted with the one or more T cell activators. For example, T cell activators may comprise agonists of one or more targets selected from the group consisting of: CD3, CD28, HVEM, CD40L, OX40, and 4-1BB. In one embodiment, during the single stage of *in vitro* expansion, the expression of two or more proteins of the TILs is reduced and/or the activity thereof is decreased, and the TILs are contacted with one or more T cell activators of the present application. In one embodiment, during the first stage of *in vitro* expansion in the present application, the expression of two or more proteins of the TILs may be reduced and/or the activity thereof may be decreased and the TILs may be contacted with one or more T cell activators of the present application. In one embodiment, during the second stage of *in vitro* expansion in the present application, the expression of two or more proteins of the TILs may be reduced and/or the activity thereof may be decreased and the TILs may be contacted with one or more T cell activators of the present application. In one embodiment, during the third stage of *in vitro* expansion in the present application, the expression of two or more proteins of the TILs may be reduced and/or the activity thereof may be decreased and the TILs may be contacted with one or more T cell activators of the present application.

In one embodiment, during a single stage of *in vitro* expansion in the present application, the expression of two or more proteins of the TILs may be reduced and/or the activity thereof may be decreased substantially simultaneously with contacting the TILs with one or more T cell activators of the present application. In one embodiment, during the single stage of *in vitro* expansion in the present application, the expression of two or more proteins of the TILs may be reduced and/or the activity thereof may be decreased first, e.g., 2 hours, 4 hours, 8 hours, 12 hours, 24 hours, or 48 hours etc., before contacting the TILs with one or more T cell activators of the present application. In one embodiment, during the single stage of *in vitro* expansion in the present application, the TILs of the present application may be contacted with one or more T cell activators of the present application first, e.g., 2 hours, 4 hours, 8 hours, 12 hours, 24 hours, or 48 hours etc., before reducing the expression and/or decreasing the activity of two or more proteins of the TILs.

In one embodiment, during the first stage of *in vitro* expansion in the present application, the expression of two or more proteins of the TILs may be reduced and/or the activity thereof may be decreased substantially simultaneously with contacting the TILs with one or more T cell activators of the present application. In one embodiment, during the second stage of *in vitro* expansion in the present application, the expression of two or more proteins of the TILs may be reduced and/or the activity thereof may be decreased substantially simultaneously with contacting the TILs with one or more T cell activators of the present application. In one embodiment, during the third stage of *in vitro* expansion in the present application, the expression of two or more proteins of the TILs may be reduced and/or the activity thereof may be decreased substantially simultaneously with contacting the TILs with one or more T cell activators of the present application.

In one embodiment, the T cell activators of the present application may comprise one or more target genes selected from the group consisting of: CD80, CD86, B7-H3, 4-1BBL, CD27, CD30, CD134, B7h, CD40, LIGHT, and functionally active fragments thereof. In one embodiment, the T cell activators of the present application may comprise agonists of one or more targets selected from the group consisting of: CD3, CD28, HVEM, CD40L, OX40, and 4-1BB. In one embodiment, the T cell activators of the present application may comprise antibodies selected from the group consisting of: CD3, CD28, HVEM, CD40L, OX40, and 4-1BB, and antigen binding fragments thereof. In one embodiment, the T cell activators of the present application may comprise a CD3 agonist. In one embodiment, the T cell activators of the present application may comprise an anti-CD3 antibody and/or an antigen-binding fragment thereof, e.g., it may be OKT3 from Miltenyi Biotech, or SP34 from BD. In one embodiment, the T cell activators of the present application may comprise a CD28 agonist. In one embodiment, the T cell activators of the present application may comprise an anti-CD28 antibody and/or an antigen-binding fragment thereof, e.g., it may be 15E8 from Merck. The T cell activators of the present application may comprise CD80 and/or a functionally active fragment thereof and/or CD86 and/or a functionally active fragment thereof, and recombinant protein of the above substances.

In one embodiment, the T cell activators of the present application may comprise an anti-CD3 antibody and/or an antigen-binding fragment thereof, e.g., they may comprise a light chain VL and a heavy chain VH of OKT3 from Miltenyi Biotech, or a light chain VL and a heavy chain VH of SP34 from BD. In one embodiment, the T cell activators of the present application may comprise a CD28 agonist. In one embodiment, the T cell activators of the present application may comprise an anti-CD28 antibody and/or an antigen-binding fragment thereof, e.g., they may comprise a light chain VL and a heavy chain VH of 15E8 from Merck. In one embodiment, the T cell activators of the present application may comprise an anti-CD3 antibody and/or an antigen-binding fragment thereof, e.g., they may comprise light chain LCDRs 1-3 and heavy chain HCDRs 1-3 of OKT3 from Miltenyi Biotech, or light chain LCDRs 1-3 and heavy chain HCDRs 1-3 of SP34 from BD, and the anti-CD3 antibody and/or the antigen-binding fragment thereof in the present application may have the ability of binding CD3. In one embodiment, the T cell activators of the present application may comprise a CD28 agonist. In one embodiment, the T cell activators of the present application may comprise an anti-CD28 antibody and/or an antigen-binding fragment thereof, e.g., they may comprise light chain LCDRs 1-3 and heavy chain HCDRs 1-3 of 15E8 from Merck, and the anti-CD28 antibody and/or the antigen-binding fragment thereof in the present application may have the ability of binding CD28. In the present application, the antibodies or the antigen binding proteins thereof in the present application comprise at least one CDR in the heavy chain variable region VH of the antibodies. The CDRs in the present application may be defined according to the nomenclature of IMGT, the CDRs in the present application may be defined according to Chothia, or the CDRs in the present application may be defined according to Kabat.

In one embodiment, the CD3 agonist of the present application may be a CD3 antibody or an antigen binding protein thereof.

In the present application, the antibodies or antigen binding proteins thereof in the present application comprise at least one CDR in the heavy chain variable region VH of the antibodies. The CDRs in the present application may be defined according to the nomenclature of IMGT, the CDRs in the present application may be defined according to Chothia, or the CDRs in the present application may be defined according to Kabat.

For example, the antibodies or antigen binding proteins thereof in the present application may comprise an HCDR1, and the HCDR1 in the present application may comprise an amino acid sequence as shown in any one of SEQ ID NOs: 2 and 12; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an HCDR2, and the HCDR2 in the present application may comprise an amino acid sequence as shown in any one of SEQ ID NOs: 3 and 13; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an HCDR3, and the HCDR3 in the present application may comprise an amino acid sequence as shown in any one of SEQ ID NOs: 4 and 14; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise HCDRs 1-3, wherein the HCDR1 in the present application may comprise an amino acid sequence as shown in any one of SEQ ID NOs: 2 and 12, the HCDR2 in the present application may comprise an amino acid sequence as shown in any one of SEQ ID NOs: 3 and 13, and the HCDR3 in the present application may comprise an amino acid sequence as shown in any one of SEQ ID NOs: 4 and 14; the CDRs in the present application may be defined according to the nomenclature of IMGT; the CDRs in the present application may be defined according to Kabat; for example, the antigen binding proteins in the present application may have the ability of binding CD3 .

For example, the antibodies or antigen binding proteins thereof in the present application may comprise the same HCDRs 1-3 as those of OKT3, wherein the HCDR1 in the present application may comprise an amino acid sequence as shown in SEQ ID NO: 2, the HCDR2 in the present application may comprise an amino acid sequence as shown in SEQ ID NO: 3, and the HCDR3 in the present application may comprise an amino acid sequence as shown in SEQ ID NO: 4; the CDRs in the present application may be defined according to the nomenclature of IMGT; for example, the antigen binding proteins in the present application may have the ability of binding CD3 .

For example, the antibodies or antigen binding proteins thereof in the present application may comprise the same HCDRs 1-3 as those of SP34, wherein the HCDR1 in the present application may comprise an amino acid sequence as shown in SEQ ID NO: 12, the HCDR2 in the present application may comprise an amino acid sequence as shown in SEQ ID NO: 13, and the HCDR3 in the present application may comprise an amino acid sequence as shown in SEQ ID NO: 14; the CDRs in the present application may be defined according to Kabat; for example, the antigen binding proteins in the present application may have the ability of binding CD3.

In the present application, the antibodies or antigen binding proteins thereof in the present application comprise at least one CDR in the light chain variable region VL of the antibodies. The CDRs in the present application may be defined according to the nomenclature of IMGT, or the CDRs in the present application may be defined according to Kabat.

For example, the antibodies or antigen binding proteins thereof in the present application may comprise an LCDR1, and the LCDR1 in the present application may comprise an amino acid sequence as shown in any one of SEQ ID NOs: 5 and 15; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an LCDR2, and the LCDR2 in the present application may comprise an amino acid sequence as shown in any one of SEQ ID NOs: 6 and 16; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise an LCDR3, and the LCDR3 in the present application may comprise an amino acid sequence as shown in any one of SEQ ID NOs: 7 and 17; the CDRs in the present application may be defined according to the nomenclature of IMGT; the CDRs in the present application may be defined according to Kabat; for example, the antigen binding proteins in the present application may have the ability of binding CD3.

For example, the antibodies or antigen binding proteins thereof in the present application may comprise LCDRs 1-3, wherein the LCDR1 in the present application may comprise an amino acid sequence as shown in any one of SEQ ID NOs: 5 and 15, the LCDR2 in the present application may comprise an amino acid sequence as shown in any one of SEQ ID NOs: 6 and 16, and the LCDR3 in the present application may comprise an amino acid sequence as shown in any one of SEQ ID NOs: 7 and 17; the CDRs in the present application may be defined according to the nomenclature of IMGT; the CDRs in the present application may be defined according to Kabat; for example, the antigen binding proteins in the present application may have the ability of binding CD3 .

For example, the antibodies or antigen binding proteins thereof in the present application may comprise the same

EP 4 471 128 A1

LCDRs 1-3 as those of OKT3, wherein the LCDR1 in the present application may comprise an amino acid sequence as shown in SEQ ID NO: 5, the LCDR2 in the present application may comprise an amino acid sequence as shown in SEQ ID NO: 6, and the LCDR3 in the present application may comprise an amino acid sequence as shown in SEQ ID NO: 7; the CDRs in the present application may be defined according to the nomenclature of IMGT; for example, the antigen binding proteins in the present application may have the ability of binding CD3 .

For example, the antibodies or antigen binding proteins thereof in the present application may comprise the same LCDRs 1-3 as those of SP34, wherein the LCDR1 in the present application may comprise an amino acid sequence as shown in SEQ ID NO: 15, the LCDR2 in the present application may comprise an amino acid sequence as shown in SEQ ID NO: 16, and the LCDR3 in the present application may comprise an amino acid sequence as shown in SEQ ID NO: 17; the CDRs in the present application may be defined according to Kabat; for example, the antigen binding proteins in the present application may have the ability of binding CD3.

For example, the antibodies or antigen binding proteins thereof in the present application may comprise HCDRs 1-3 and LCDRs 1-3, wherein the HCDR1 in the present application may comprise an amino acid sequence as shown in any one of SEQ ID NOs: 2 and 12, the HCDR2 in the present application may comprise an amino acid sequence as shown in any one of SEQ ID NOs: 3 and 13, the HCDR3 in the present application may comprise an amino acid sequence as shown in any one of SEQ ID NOs: 4 and 14, the LCDR1 in the present application may comprise an amino acid sequence as shown in any one of SEQ ID NOs: 5 and 15, the LCDR2 in the present application may comprise an amino acid sequence as shown in any one of SEQ ID NOs: 6 and 16, and the LCDR3 in the present application may comprise an amino acid sequence as shown in any one of SEQ ID NOs: 7 and 17; the CDRs in the present application may be defined according to the nomenclature of IMGT; the CDRs in the present application may be defined according to Kabat; for example, the antigen binding proteins in the present application may have the ability of binding CD3 .

For example, the antibodies or antigen binding proteins thereof in the present application may comprise the same HCDRs 1-3 and LCDRs 1-3 as those of OKT3, wherein the HCDR1 in the present application may comprise an amino acid sequence as shown in SEQ ID NO: 2, the HCDR2 in the present application may comprise an amino acid sequence as shown in SEQ ID NO: 3, the HCDR3 in the present application may comprise an amino acid sequence as shown in SEQ ID NO: 4, the LCDR1 in the present application may comprise an amino acid sequence as shown in SEQ ID NO: 5, the LCDR2 in the present application may comprise an amino acid sequence as shown in SEQ ID NO: 6, and the LCDR3 in the present application may comprise an amino acid sequence as shown in SEQ ID NO: 7; the CDRs in the present application may be defined according to the nomenclature of IMGT; for example, the antigen binding proteins in the present application may have the ability of binding CD3.

For example, the antibodies or antigen binding proteins thereof in the present application may comprise the same HCDRs 1-3 as those of SP34, wherein the HCDR1 in the present application may comprise an amino acid sequence as shown in SEQ ID NO: 12, the HCDR2 in the present application may comprise an amino acid sequence as shown in SEQ ID NO: 13, the HCDR3 in the present application may comprise an amino acid sequence as shown in SEQ ID NO: 14, the LCDR1 in the present application may comprise an amino acid sequence as shown in SEQ ID NO: 15, the LCDR2 in the present application may comprise an amino acid sequence as shown in SEQ ID NO: 16, and the LCDR3 in the present application may comprise an amino acid sequence as shown in SEQ ID NO: 17; the CDRs in the present application may be defined according to Kabat; for example, the antigen binding proteins in the present application may have the ability of binding CD3.

In one embodiment, the antibodies or antigen binding proteins thereof in the present application may comprise a heavy chain variable region VH, and the VH in the present application may comprise an amino acid sequence as shown in any one of SEQ ID NOs: 8 and 18; for example, the antigen binding proteins in the present application may have the ability of binding CD3.

For example, the antibodies or antigen binding proteins thereof in the present application may comprise the same VH as that of OKT3, and the VH in the present application may comprise an amino acid sequence as shown in SEQ ID NO: 8; for example, the antigen binding proteins in the present application may have the ability of binding CD3.

For example, the antibodies or antigen binding proteins thereof in the present application may comprise the same VH as that of SP34, and the VH in the present application may comprise an amino acid sequence as shown in SEQ ID NO: 18; for example, the antigen binding proteins in the present application may have the ability of binding CD3.

In one embodiment, the antibodies or antigen binding proteins thereof in the present application may comprise a light chain variable region VL, and the VL in the present application may comprise an amino acid sequence as shown in any one of SEQ ID NOs: 9 and 19; for example, the antigen binding proteins in the present application may have the ability of binding CD3.

For example, the antibodies or antigen binding proteins thereof in the present application may comprise the same VL as that of OKT3, and the VL in the present application may comprise an amino acid sequence as shown in SEQ ID NO: 9; for example, the antigen binding proteins in the present application may have the ability of binding CD3.

For example, the antibodies or antigen binding proteins thereof in the present application may comprise the same VL as that of SP34, and the VL in the present application may comprise an amino acid sequence as shown in SEQ ID NO:

19; for example, the antigen binding proteins in the present application may have the ability of binding CD3.

In one embodiment, the antibodies or antigen binding proteins thereof in the present application may comprise a heavy chain variable region VH and a light chain variable region VL, and the VH in the present application may comprise an amino acid sequence as shown in any one of SEQ ID NOs: 8 and 18, and the VL in the present application may comprise an amino acid sequence as shown in any one of SEQ ID NOs: 9 and 19; for example, the antigen binding proteins in the present application may have the ability of binding CD3.

For example, the antibodies or antigen binding proteins thereof in the present application may comprise the same VH and VL as those of OKT3, and the VH in the present application may comprise an amino acid sequence as shown in SEQ ID NO: 8, the VL in the present application may comprise an amino acid sequence as shown in SEQ ID NO: 9; for example, the antigen binding proteins in the present application may have the ability of binding CD3.

For example, the antibodies or antigen binding proteins thereof in the present application may comprise the same VH and VL as those of SP34, and the VH in the present application may comprise an amino acid sequence as shown in SEQ ID NO: 18, the VL in the present application may comprise an amino acid sequence as shown in SEQ ID NO: 19; for example, the antigen binding proteins in the present application may have the ability of binding CD3.

In one embodiment, the antibodies or antigen binding proteins thereof in the present application may comprise a heavy chain, and the heavy chain in the present application may comprise an amino acid sequence as shown in any one of SEQ ID NOs: 10 and 20; for example, the antigen binding proteins in the present application may have the ability of binding CD3.

For example, the antibodies or antigen binding proteins thereof in the present application may comprise the same heavy chain as that of OKT3, and the heavy chain in the present application may comprise an amino acid sequence as shown in SEQ ID NO: 10; for example, the antigen binding proteins in the present application may have the ability of binding CD3.

For example, the antibodies or antigen binding proteins thereof in the present application may comprise the same heavy chain as that of SP34, and the heavy chain in the present application may comprise an amino acid sequence as shown in SEQ ID NO: 20; for example, the antigen binding proteins in the present application may have the ability of binding CD3.

In one embodiment, the antibodies or antigen binding proteins thereof in the present application may comprise a light chain, and the light chain in the present application may comprise an amino acid sequence as shown in any one of SEQ ID NOs: 11 and 21; for example, the antigen binding proteins in the present application may have the ability of binding CD3.

For example, the antibodies or antigen binding proteins thereof in the present application may comprise the same light chain as that of OKT3, and the light chain in the present application may comprise an amino acid sequence as shown in SEQ ID NO: 11; for example, the antigen binding proteins in the present application may have the ability of binding CD3.

For example, the antibodies or antigen binding proteins thereof in the present application may comprise the same light chain as that of SP34, and the light chain in the present application may comprise an amino acid sequence as shown in SEQ ID NO: 21; for example, the antigen binding proteins in the present application may have the ability of binding CD3.

In one embodiment, the antibodies or antigen binding proteins thereof in the present application may comprise a heavy chain and a light chain, and the heavy chain in the present application may comprise an amino acid sequence as shown in any one of SEQ ID NOs: 10 and 20, the light chain in the present application may comprise an amino acid sequence as shown in any one of SEQ ID NOs: 11 and 21; for example, the antigen binding proteins in the present application may have the ability of binding CD3.

For example, the antibodies or antigen binding proteins thereof in the present application may comprise the same heavy chain and light chain as those of OKT3, and the heavy chain in the present application may comprise an amino acid sequence as shown in SEQ ID NO: 10, the light chain in the present application may comprise an amino acid sequence as shown in SEQ ID NO: 11; for example, the antigen binding proteins in the present application may have the ability of binding CD3.

For example, the antibodies or antigen binding proteins thereof in the present application may comprise the same heavy chain and light chain as those of SP34, and the heavy chain in the present application may comprise an amino acid sequence as shown in SEQ ID NO: 20, the light chain in the present application may comprise an amino acid sequence as shown in SEQ ID NO: 21; for example, the antigen binding proteins in the present application may have the ability of binding CD3.

In one embodiment, the CD28 agonist of the present application may be a CD28 antibody or an antigen binding protein thereof.

In the present application, the antibodies or antigen binding proteins thereof in the present application comprise at least one CDR in the heavy chain variable region VH of the antibodies. The CDRs in the present application may be defined according to the nomenclature of IMGT, or the CDRs in the present application may be defined according to Kabat.

For example, the antibodies or antigen binding proteins thereof in the present application may comprise an HCDR1, and the HCDR1 in the present application may comprise an amino acid sequence as shown in SEQ ID NO: 22; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an HCDR2, and the HCDR2 in the present application may comprise an amino acid sequence as shown in SEQ ID NO: 23; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an HCDR3, and the HCDR3 in the present application may comprise an amino acid sequence as shown in SEQ ID NO: 24; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise HCDRs 1-3, wherein the HCDR1 in the present application may comprise an amino acid sequence as shown in SEQ ID NO: 22, the HCDR2 in the present application may comprise an amino acid sequence as shown in SEQ ID NO: 23, and the HCDR3 in the present application may comprise an amino acid sequence as shown in SEQ ID NO: 24; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same HCDRs 1-3 as those of 15E8, wherein the HCDR1 in the present application may comprise an amino acid sequence as shown in SEQ ID NO: 22, the HCDR2 in the present application may comprise an amino acid sequence as shown in SEQ ID NO: 23, and the HCDR3 in the present application may comprise an amino acid sequence as shown in SEQ ID NO: 24; the CDRs in the present application may be defined according to the nomenclature of IMGT; for example, the antigen binding proteins in the present application may have the ability of binding CD28.

In the present application, the antibodies or antigen binding proteins thereof in the present application comprises at least one CDR in the light chain variable region VL of the antibodies. The CDRs in the present application may be defined according to the nomenclature of IMGT, or the CDRs in the present application may be defined according to Kabat.

For example, the antibodies or antigen binding proteins thereof in the present application may comprise an LCDR1, and the LCDR1 in the present application may comprise an amino acid sequence as shown in SEQ ID NO: 25; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an LCDR2, and the LCDR2 in the present application may comprise an amino acid sequence as shown in SEQ ID NO: 26; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise an LCDR3, and the LCDR3 in the present application may comprise an amino acid sequence as shown in SEQ ID NO: 27; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise LCDRs 1-3, wherein the LCDR1 in the present application may comprise an amino acid sequence as shown in SEQ ID NO: 25, the LCDR2 in the present application may comprise an amino acid sequence as shown in SEQ ID NO: 26, and the LCDR3 in the present application may comprise an amino acid sequence as shown in SEQ ID NO: 27; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same LCDRs 1-3 as those of 15E8, wherein the LCDR1 in the present application may comprise an amino acid sequence as shown in SEQ ID NO: 25, the LCDR2 in the present application may comprise an amino acid sequence as shown in SEQ ID NO: 26, and the LCDR3 in the present application may comprise an amino acid sequence as shown in SEQ ID NO: 27; the CDRs in the present application may be defined according to the nomenclature of IMGT; for example, the antigen binding proteins in the present application may have the ability of binding CD28.

For example, the antibodies or antigen binding proteins thereof in the present application may comprise HCDRs 1-3 and LCDRs 1-3, wherein the HCDR1 in the present application may comprise an amino acid sequence as shown in SEQ ID NO: 22, the HCDR2 in the present application may comprise an amino acid sequence as shown in SEQ ID NO: 23, the HCDR3 in the present application may comprise an amino acid sequence as shown in SEQ ID NO: 24, the LCDR1 in the present application may comprise an amino acid sequence as shown in SEQ ID NO: 25, the LCDR2 in the present application may comprise an amino acid sequence as shown in SEQ ID NO: 30, and the LCDR3 in the present application may comprise an amino acid sequence as shown in SEQ ID NO: 26; the CDRs in the present application may be defined according to the nomenclature of IMGT; the CDRs in the present application may be defined according to Kabat; for example, the antigen binding proteins in the present application may have the ability of binding CD28.

For example, the antibodies or antigen binding proteins thereof in the present application may comprise the same HCDRs 1-3 and LCDRs 1-3 as those of 15E8, wherein the HCDR1 in the present application may comprise an amino acid sequence as shown in SEQ ID NO: 22, the HCDR2 in the present application may comprise an amino acid sequence as shown in SEQ ID NO: 23, the HCDR3 in the present application may comprise an amino acid sequence as shown in SEQ ID NO: 24, the LCDR1 in the present application may comprise an amino acid sequence as shown in SEQ ID NO: 25, the LCDR2 in the present application may comprise an amino acid sequence as shown in SEQ ID NO: 26, and the LCDR3 in the present application may comprise an amino acid sequence as shown in SEQ ID NO: 27; the CDRs in the present application may be defined according to the nomenclature of IMGT; for example, the antigen binding proteins in the present application may have the ability of binding CD28.

In one embodiment, the antibodies or antigen binding proteins thereof in the present application may comprise a heavy chain variable region VH, and the VH in the present application may comprise an amino acid sequence as shown in any one of SEQ ID NOs: 28 and 29; for example, the antigen binding proteins in the present application may have the ability of binding CD28.

For example, the antibodies or antigen binding proteins thereof in the present application may comprise the same VH as that of one 15E8, and the VH in the present application may comprise an amino acid sequence as shown in SEQ ID NO: 28; for example, the antigen binding proteins in the present application may have the ability of binding CD28.

For example, the antibodies or antigen binding proteins thereof in the present application may comprise the same VH as that of another 15E8, and the VH in the present application may comprise an amino acid sequence as shown in SEQ ID NO: 29; for example, the antigen binding proteins in the present application may have the ability of binding CD28.

In one embodiment, the antibodies or antigen binding proteins thereof in the present application may comprise a light chain variable region VL, and the VL in the present application may comprise an amino acid sequence as shown in SEQ ID NO: 30; for example, the antigen binding proteins in the present application may have the ability of binding CD28.

For example, the antibodies or antigen binding proteins thereof in the present application may comprise the same VL as that of 15E8, and the VL in the present application may comprise an amino acid sequence as shown in SEQ ID NO: 30; for example, the antigen binding proteins in the present application may have the ability of binding CD28.

In one embodiment, the antibodies or antigen binding proteins thereof in the present application may comprise a heavy chain variable region VH and a light chain variable region VL, and the VH in the present application may comprise an amino acid sequence as shown in any one of SEQ ID NOs: 28 and 29, the VL in the present application may comprise an amino acid sequence as shown in SEQ ID NO: 30; for example, the antigen binding proteins in the present application may have the ability of binding CD28.

For example, the antibodies or antigen binding proteins thereof in the present application may comprise the same VH and VL as those of one 15E8, and the VH in the present application may comprise an amino acid sequence as shown in SEQ ID NO: 28, the VL in the present application may comprise an amino acid sequence as shown in SEQ ID NO: 30; for example, the antigen binding proteins in the present application may have the ability of binding CD28.

For example, the antibodies or antigen binding proteins thereof in the present application may comprise the same VH and VL as those of another 15E8, and the VH in the present application may comprise an amino acid sequence as shown in SEQ ID NO: 29, the VL in the present application may comprise an amino acid sequence as shown in SEQ ID NO: 30; for example, the antigen binding proteins in the present application may have the ability of binding CD28.

In one embodiment, the antibodies or antigen binding proteins thereof in the present application may comprise a heavy chain, and the heavy chain in the present application may comprise an amino acid sequence as shown in any one of SEQ ID NOs: 31 and 32; for example, the antigen binding proteins in the present application may have the ability of binding CD28.

For example, the antibodies or antigen binding proteins thereof in the present application may comprise the same heavy chain as that of one 15E8, and the heavy chain in the present application may comprise an amino acid sequence as shown in SEQ ID NO: 31; for example, the antigen binding proteins in the present application may have the ability of binding CD28.

For example, the antibodies or antigen binding proteins thereof in the present application may comprise the same heavy chain as that of another 15E8, and the heavy chain in the present application may comprise an amino acid sequence as shown in SEQ ID NO: 32; for example, the antigen binding proteins in the present application may have the ability of binding CD28.

In one embodiment, the antibodies or antigen binding proteins thereof in the present application may comprise a light chain, and the light chain in the present application may comprise an amino acid sequence as shown in SEQ ID NO: 33; for example, the antigen binding proteins in the present application may have the ability of binding CD28.

For example, the antibodies or antigen binding proteins thereof in the present application may comprise the same light chain as that of 15E8, and the light chain in the present application may comprise an amino acid sequence as shown in SEQ ID NO: 33; for example, the antigen binding proteins in the present application may have the ability of binding CD28.

In one embodiment, the antibodies or antigen binding proteins thereof in the present application may comprise a heavy chain and a light chain, and the heavy chain in the present application may comprise an amino acid sequence as shown in any one of SEQ ID NOs: 31 and 32, the light chain in the present application may comprise an amino acid sequence as shown in SEQ ID NO: 33; for example, the antigen binding proteins in the present application may have the ability of binding CD28.

For example, the antibodies or antigen binding proteins thereof in the present application may comprise the same heavy chain and light chain as those of one 15E8, and the heavy chain in the present application may comprise an amino acid sequence as shown in SEQ ID NO: 31, the light chain in the present application may comprise an amino acid sequence as shown in SEQ ID NO: 33; for example, the antigen binding proteins in the present application may have the ability of binding CD28.

For example, the antibodies or antigen binding proteins thereof in the present application may comprise the same heavy chain and light chain as those of another 15E8, and the heavy chain in the present application may comprise an amino acid sequence as shown in SEQ ID NO: 32, the light chain in the present application may comprise an amino acid sequence as shown in SEQ ID NO: 33; for example, the antigen binding proteins in the present application may have the ability of binding CD28.

In one embodiment, the step of contacting the TILs of the present application with one or more T cell activators of the present application may comprise one or more ways selected from the group consisting of: (1) adding the T cell activators of the present application into the cell culture medium of the TILs of the present application; (2) adding engineered cells expressing the T cell activators of the present application into the cell culture medium of the TILs of the present application; (3) adding a solid medium comprising the T cell activators of the present application into the cell culture medium of the TILs of the present application. In one embodiment, the step of contacting the TILs of the present application with one or more T cell activators of the present application may comprise adding a solid medium comprising the T cell activators of the present application into the cell culture medium of the TILs of the present application. In one embodiment, the step of contacting the TILs of the present application with one or more T cell activators of the present application may comprise adding a solid medium comprising the CD28 antibodies and CD3 antibodies of the present application into the cell culture medium of the TILs of the present application.

In one embodiment, the initial concentration of the T cell activators in the cell culture medium of the TILs of the present application may be at least about 30 ng/mL. For example, the initial concentration of the CD28 antibodies of the present application in the cell culture medium of the TILs of the present application may be at least about 30 ng/mL; for example, the initial concentration of the CD3 antibodies of the present application in the cell culture medium of the TILs of the present application may be at least about 30 ng/mL. For example, the choice of the initial concentration of the CD28 antibodies of the present application may be independent of the choice of the initial concentration of the CD3 antibodies of the present application; for example, the initial concentrations of the CD28 antibodies of the present application and the CD3 antibodies of the present application in the cell culture medium of the TILs of the present application may be in any combination. For example, the initial concentration of the CD28 antibodies of the present application in the cell culture medium of the TILs of the present application may be optionally selected from about 30 ng/mL to about 300 ng/mL. For example, the initial concentration of the CD3 antibodies of the present application in the cell culture medium of the TILs of the present application may be optionally selected from about 30 ng/mL to about 300 ng/mL. For example, the initial concentration of the CD28 antibodies of the present application in the cell culture medium of the TILs of the present application may be optionally selected from about 30 ng/mL to about 300 ng/mL, and the initial concentration of the CD3 antibodies of the present application in the cell culture medium of the TILs of the present application may be optionally selected from about 30 ng/mL to about 300 ng/mL, the choice of the initial concentration of the CD28 antibodies of the present application may be independent of the choice of the initial concentration of the CD3 antibodies of the present application. In one embodiment, the diameter of the solid medium of the present application can be about 500 nm to about 10 μm. In one embodiment, the diameter of the solid medium of the present application can be measured by transmission electron microscopy. In one embodiment, the diameter of the solid medium of the present application can be about 1 nm to about 500 nm. In one embodiment, the diameter of the solid medium of the present application can be about 100 nm to about 500 nm. In one embodiment, the diameter of the solid medium of the present application can be about 200 nm to about 500 nm. In one embodiment, the diameter of the solid medium of the present application can be measured by transmission electron microscopy.

In one embodiment, the solid medium of the present application may comprise a polymer. In one embodiment, the solid medium of the present application may comprise glucan.

In one embodiment, each mg of the solid medium of the present application comprises at least about 25 μg of the T cell activators of the present application.

In one embodiment, the solid medium comprising the T cell activators of the present application is added into the cell culture medium of the TILs of the present application at a ratio of the solid medium of the present application to the TILs of the present application from about 1:100 to about 1:2000. In one embodiment, the solid medium comprising the T cell activators of the present application is added into the cell culture medium of the TILs of the present application at a ratio of the solid medium of the present application to the TILs of the present application from about 2:1 to about 1:2.

For example, when the diameter of the solid medium of the present application is about 100 nm to about 500 nm, the solid medium comprising the T cell activators of the present application may be added into the cell culture medium of the TILs of the present application at a ratio of the solid medium of the present application to the TILs of the present application from about 2:1 to about 1:2. For example, when the diameter of the solid medium of the present application is about 100 nm to about 500 nm, the solid medium comprising the T cell activators of the present application, e.g., the CD3 agonist and/or the CD28 agonist, may be added into the cell culture medium of the TILs of the present application at a ratio of the solid medium of the present application to the TILs of the present application from about 2:1 to about 1:2, from about 2:1 to about 1:1, or from about 1:1 to about 1:2.

For example, when the diameter of the solid medium of the present application is about 100 nm to about 500 nm, the solid medium comprising the T cell activators of the present application may be added into the cell culture medium of the TILs of the present application at a ratio of the solid medium of the present application to the TILs of the present application from about 1:100 to about 1:2000. For example, when the diameter of the solid medium of the present application is about 100 nm to about 500 nm, the solid medium comprising the CD28 agonist and CD3 agonist of the present application may be added into the cell culture medium of the TILs of the present application at a ratio of the solid medium of the present application to the TILs of the present application from about 1:100 to about 1:2000, from about 1:200 to about 1:2000, from about 1:300 to about 1:2000, from about 1:400 to about 1:2000, from about 1:500 to about 1:2000, from about 1:600 to about 1:2000, from about 1:700 to about 1:2000, from about 1:800 to about 1:2000, from about 1:900 to about 1:2000, from about 1:1000 to about 1:2000, from about 1:1200 to about 1:2000, from about 1:1400 to about 1:2000, from about 1:1600 to about 1:2000, or from about 1:1800 to about 1:2000.

In one embodiment, the method of the present application may also comprise: subjecting TILs derived from tumor tissues and not expanded *in vitro* to at least one stage *of in vitro* expansion, wherein the TILs of the present application are contacted with one or more T cell growth factors during the at least one stage *of in vitro* expansion in the present application.

In one embodiment, during a single stage *of in vitro* expansion in the present application, the TILs of the present application may be contacted with the T cell activators of the present application and contacted with one or more T cell growth factors of the present application. For example, during the first stage of *in vitro* expansion in the present application, the TILs of the present application may be contacted with the T cell activators of the present application and contacted with one or more T cell growth factors of the present application. For example, during the second stage *of in vitro* expansion in the present application, the TILs of the present application may be contacted with the T cell activators of the present application and contacted with one or more T cell growth factors of the present application. For example, during the third stage of *in vitro* expansion in the present application, the TILs of the present application may be contacted with the T cell activators of the present application and contacted with one or more T cell growth factors of the present application.

In one embodiment, during a single stage *of in vitro* expansion in the present application, the expression of two or more proteins of the TILs may be reduced and/or the activity thereof may be decreased substantially simultaneously with contacting the TILs with the T cell growth factors. In one embodiment, during a single stage of *in vitro* expansion in the present application, the expression of two or more proteins of the TILs may be reduced and/or the activity thereof may be decreased substantially simultaneously with contacting the TILs with the T cell growth factors. In one embodiment, during a single stage of *in vitro* expansion in the present application, the expression of two or more proteins of the TILs may be reduced and/or the activity thereof may be decreased first, for example, 2 hours, 4 hours, 8 hours, 12 hours, 24 hours, or 48 hours before contacting the TILs of the present application with one or more T cell growth factors of the present application. In one embodiment, during a single stage of *in vitro* expansion in the present application, the TILs of the present application may be contacted with one or more T cell growth factors of the present application first, for example, 2 hours, 4 hours, 8 hours, 12 hours, 24 hours, or 48 hours before reducing the expression and/or decreasing the activity of the two or more proteins of the TILs.

For example, during the first stage of *in vitro* expansion in the present application, the expression of two or more proteins of the TILs may be reduced and/or the activity thereof may be decreased substantially simultaneously with contacting the TILs with the T cell growth factors. For example, during the second stage of *in vitro* expansion in the present application, the expression of two or more proteins of the TILs may be reduced and/or the activity thereof may be decreased substantially simultaneously with contacting the TILs with the T cell growth factors. For example, during the third stage *of in vitro* expansion in the present application, the expression of the two or more proteins of the TILs may be reduced and/or the activity thereof may be decreased substantially simultaneously with contacting the TILs with the T cell growth factors.

In one embodiment, the T cell growth factors of the present application may be one or more T cell growth factors selected from the group consisting of: IL-2, IL-7, IL-12, IL-15, IL-21, interferon γ, and functionally active fragments thereof. In one embodiment, the T cell growth factors of the present application may comprise IL-2 and/or functionally active fragments thereof. For example, the functionally active fragments of IL-2 may comprise fragments of IL-2 capable of binding to IL-2 receptors of T cells that are known in the art.

In one embodiment, the step of contacting the TILs of the present application with one or more T cell growth factors of the present application may comprise adding the T cell growth factors of the present application into the cell culture medium of the TILs of the present application. In one embodiment, the initial concentration of the T cell growth factors of the present application in the cell culture medium of the TILs of the present application may be at least about 300 IU/mL. In one embodiment, the initial concentration of the IL-2 of the present application in the cell culture medium of the TILs of the present application may be at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

In one embodiment, the TILs of the present application may be TILs derived from the tumor tissue debris of the present application. In one embodiment, the TILs of the present application may be obtained by processing the tumor tissues into tumor debris. In one embodiment, the tumor debris of the present application has a volume of about 1-27 mm$^3$. In one embodiment, the tumor debris of the present application has a volume of about 1 mm$^3$, about 2 mm$^3$, about 3 mm$^3$, about 4 mm$^3$, about 5 mm$^3$, about 6 mm$^3$, about 7 mm$^3$, about 8 mm$^3$, about 9 mm$^3$, about 10 mm$^3$, about 11 mm$^3$, about 12 mm$^3$, about 13 mm$^3$, about 15 mm$^3$, about 17 mm$^3$, about 19 mm$^3$, about 20 mm$^3$, about 21 mm$^3$, about 23 mm$^3$, about 24 mm$^3$, about 25 mm$^3$, about 26 mm$^3$, or about 27 mm$^3$. For example, the TILs may be selected from the group consisting of: TILs derived from tumor tissue debris, TILs derived from lymphatic metastasis debris, TILs derived from pleural effusion, TILs derived from peritoneal effusion, and TILs resuscitated after cryopreservation.

In another aspect, the present application provides a method for culturing tumor infiltrating lymphocytes (TILs), which may comprise: (A) contacting a first TIL population derived from tumor tissues and not expanded *in vitro* with T cell growth factors, wherein a second TIL population is obtained via the step (A); (B) reducing the expression and/or decreasing the activity of two or more proteins of the TILs, and co-culturing the second TIL population with feeder cells after contacting the second TIL population with T cell activators and/or T cell growth factors for a period of time, wherein a third TIL population is obtained via the step (B).

In another aspect, the present application provides a method for culturing tumor infiltrating lymphocytes (TILs), which may comprise: (A) resuscitating and/or continuing culturing an *in vitro* TIL population to obtain a second TIL population, wherein the *in vitro* TIL population comprises a TIL population obtained by the *in vitro* expansion of the first TIL population, the first TIL population is a TIL population derived from tumor tissues and not expanded *in vitro;* (B) reducing the expression and/or decreasing the activity of the two or more proteins of the TILs, and co-culturing the second TIL population with feeder cells after contacting the second TIL population with T cell activators and/or T cell growth factors for a period of time, wherein a third TIL population is obtained via the step (B).

For example, a TIL population derived from tumor tissues at a certain time and/or at a certain location and not expanded *in vitro* may be contacted with T cell growth factors first to obtain an *in vitro* TIL population. The *in vitro* TIL population may be, in one aspect, cultured continually to subject to the step (B), or, in another aspect, may be cryopreserved first and resuscitated when needed, and then subjected to the step (B).

In another aspect, the present application provides a method for culturing tumor infiltrating lymphocytes (TILs), which may comprise: (A) contacting a first TIL population derived from tumor tissues and not expanded *in vitro* with T cell growth factors, wherein a second TIL population is obtained via the step (A); (B) reducing the expression and/or decreasing the activity of two or more proteins of the TILs, and co-culturing the second TIL population with feeder cells after contacting the second TIL population with T cell activators and/or T cell growth factors for a period of time, wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with T cell growth factors, wherein a second TIL population is obtained via the step (A); (B) making the proportion of cells expressing the two or more proteins in the TILs be about 95% or less, and co-culturing the second TIL population with feeder cells after contacting the second TIL population with T cell activators and/or T cell growth factors for a period of time, wherein a third TIL population is obtained via the step (B).

In the terms used in one embodiment, the first stage of *in vitro* expansion in the present application and the step (A) in the methods of the above aspects can be optionally used interchangeably. In the terms used in one embodiment, the second stage of *in vitro* expansion in the present application and the step (B) in the methods of the above aspects can be optionally used interchangeably. In the terms used in one embodiment, the TILs upon the first stage of *in vitro* expansion in the present application and the second TIL population obtained from the step (A) in the methods of the above aspects can be optionally used interchangeably. In the terms used in one embodiment, the TILs upon the second stage of *in vitro* expansion in the present application and the third TIL population obtained from the step (B) in the methods of the above aspects can be optionally used interchangeably. In the terms used in one embodiment, if needed, the third stage of *in vitro* expansion in the present application and the optionally added step (C) in the methods of the above aspects can be optionally used interchangeably. In the terms used in one embodiment, if needed, the TILs upon the third stage of *in vitro* expansion in the present application and the fourth TIL population obtained from the optionally added step (C) in the methods of the above aspects can be optionally used interchangeably.

In another aspect, the present application provides a method for culturing tumor infiltrating lymphocytes (TILs), which may comprise: (A) contacting the first TIL population derived from tumor tissues and not expanded *in vitro* with a variety of T cell growth factors; wherein a second TIL population is obtained via the step (A); (B) contacting the second TIL population and a variety of T cell growth factors with a variety of T cell activators, reducing the expression and/or decreasing the activity of two or more proteins of the TILs, and co-culturing the TILs with feeder cells; wherein a third TIL population is obtained via the step (B).

In another aspect, the present application provides a method for culturing tumor infiltrating lymphocytes (TILs), which may comprise: (A) contacting the first TIL population derived from tumor tissues and not expanded *in vitro* with a variety of T cell growth factors; wherein a second TIL population is obtained via the step (A); (B) contacting the second TIL population and a variety of T cell growth factor with a variety of T cell activators, reducing the expression and/or decreasing the activity of two or more proteins of the TILs, and co-culturing the TILs with feeder cells; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with a variety of T cell growth factors; wherein a second TIL population is obtained via the step (A); (B) contacting the second TIL population and a variety of T cell growth factors with a variety of T cell activators, making the proportion of cells expressing the two or more proteins in the TILs be about 95% or less, and co-culturing the TILs with feeder cells; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with a variety of T cell growth factors; wherein a second TIL population is obtained via the step (A); (B) contacting the second TIL population and a variety of T cell growth factors with a variety of T cell activators, making the proportion of cells expressing the two or more proteins in the TILs be about 95% or less, and co-culturing the TILs with feeder cells at least 2 hours later; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with a variety of T cell growth factors; wherein a second TIL population is obtained via the step (A); (B) contacting the second TIL population and a variety of T cell growth factors with a variety of T cell activators, making the proportion of cells expressing the two or more proteins in the TILs be about 95% or less, and co-culturing the TILs with feeder cells at least 2 hours later, the feeder cells may comprise peripheral mononuclear cells, the feeder cells are added into the cell culture medium of the TILs; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with a variety of T cell growth factors; wherein a second TIL population is obtained via the step (A); (B) contacting the second TIL population and a variety of T cell growth factors with a variety of T cell activators, making the proportion of cells expressing the two or more proteins in the TILs be about 95% or less, and co-culturing the TILs with feeder cells at least 2 hours later, the feeder cells may comprise peripheral mononuclear cells, and the feeder cells may be added into the cell culture medium of the TILs at a ratio of the feeder cells to the TILs from about 40:1 to about 400:1; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with IL-2; wherein a second TIL population is obtained via the step (A); (B) contacting the second TIL population and IL-2 with a variety of T cell activators, making the proportion of cells expressing the two or more proteins in the TILs be about 95% or less, and co-culturing the TILs with feeder cells at least 2 hours later, the feeder cells may comprise peripheral mononuclear cells and may be added into the cell culture medium of the TILs at a ratio of the feeder cells to the TILs from about 40:1 to about 400:1; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with IL-2 of which the initial concentration in the cell culture medium of the TILs may be at least about 300 IU/mL; wherein a second TIL population is obtained via the step (A); (B) contacting the second TIL population and IL-2 of which the initial concentration in the cell culture medium of the TILs may be at least about 300 IU/mL, with CD3 antibodies of which the initial concentration in the cell culture medium of the TILs is at least about 30 ng/mL, making the proportion of cells expressing the two or more proteins in the TILs be about 95% or less, and co-culturing the TILs with feeder cells at least 2 hours later, the feeder cells may comprise peripheral mononuclear cells and may be added into the cell culture medium of the TILs at a ratio of the feeder cells to the TILs from about 40:1 to about 400:1; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with IL-2; wherein a second TIL population is obtained via the step (A); (B) contacting the second TIL population and IL-2 with a nanomatrix comprising CD3 antibodies and CD28 antibodies, making the proportion of cells expressing the two or more proteins in the TILs be about 95% or less, and co-culturing the TILs with feeder cells; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with IL-2; wherein a second TIL population is obtained via the step (A); (B) contacting the second TIL population and IL-2 with a nanomatrix comprising CD3 antibodies and CD28 antibodies, the diameter of the nanomatrix can be about 1 nm to about 500 nm, and each mg of the nanomatrix may comprise each about 25 $\mu$g of CD3 antibodies and CD28 antibodies, respectively; making the proportion of cells expressing the two or more proteins in the TILs be about 95% or less, and co-culturing the TILs with feeder cells; wherein a third TIL population is obtained via the step (B).

In another aspect, the present application provides a method for culturing tumor infiltrating lymphocytes (TILs), which may comprise: (A) contacting a first TIL population derived from tumor tissues and not expanded *in vitro* with IL-2; wherein a second TIL population is obtained via the step (A); (B) contacting the second TIL population and IL-2 with a nanomatrix comprising CD3 antibodies and CD28 antibodies, the diameter of the nanomatrix can be about 1 nm to about 500 nm, each mg of the nanomatrix may comprise each about 25 $\mu$g of CD3 antibodies and CD28 antibodies, respectively, and the nanomatrix may be added into the cell culture medium of the TILs at a ratio of the nanomatrix to the TILs from about 1: 100 to about 1 :2000; making the proportion of cells expressing the two or more proteins in the TILs be about 95% or less, and co-culturing the TILs with feeder cells; wherein a third TIL population is obtained via the step (B). The two or more proteins are selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

In another aspect, the present application provides a method for culturing tumor infiltrating lymphocytes (TILs), which may comprise: (A) contacting a first TIL population derived from tumor tissues and not expanded *in vitro* with IL-2 of which the initial concentration in the cell culture medium of the TILs may be at least about 300 IU/mL; wherein a second TIL population is obtained via the step (A); (B) contacting the second TIL population and IL-2 of which the initial concentration in the cell culture medium of the TILs may be at least about 300 IU/mL, with a nanomatrix comprising CD3 antibodies and CD28 antibodies of which the diameter can be about 1 nm to about 500 nm, each mg of the nanomatrix may comprise each about 25 $\mu$g of CD3 antibodies and CD28 antibodies, respectively, and the nanomatrix may be added into the cell culture medium of the TILs at a ratio of the nanomatrix to the TILs from about 1: 100 to about 1:2000; making the proportion of cells expressing the two or more proteins in the TILs be about 95% or less, and co-culturing the TILs with feeder cells, the feeder cells may comprise peripheral mononuclear cells and may be added into the cell culture medium of the TILs at a ratio of the feeder cells to the TILs from about 40:1 to about 400:1; wherein a third TIL population is obtained via the step (B). The two or more proteins are selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

In another aspect, the present application provides a method for culturing tumor infiltrating lymphocytes (TILs). The TIL cells obtained from the tissue samples of a subject can be orthotopic tumor samples or metastatic tumor samples surgically obtained from the patient, and the weight can be at least about 1 g, or multiple pieces of tissues can be combined. The tumor tissues are transported at about 2-8 degrees in the sample transport solution, for example, a commonly used commercial tumor tissue transport solution, tumor tissue preservation solution or tumor tissue transfer solution, and processed within 48 hours. Tissue pieces can be broken mechanically to a size of about 1-27 mm$^3$ each, transferred into a gas-permeable culture bag or Grex, added with T cell serum-free culture medium and IL-2 at a concentration of 300-9000 IU/mL (e.g., the concentration may be 1000-9000 IU/mL, e.g., 6000 IU/mL) and cultured for about 3-14 days. The harvested TIL cells can be cryopreserved and then resuscitated, or can be directly collected in the cell culture medium, and transferred into gas-permeable culture bag, or Grex, or Xuri unit. The T cell serum-free culture medium can be added with CD28 antibodies, CD3 antibodies and CD28 antibodies, magnetic beads (e.g., Dynabeads) comprising CD3 antibodies and CD28 antibodies, and/or nanomatrix (e.g., transACT) comprising CD3 antibodies and CD28 antibodies of the present application, IL-2 at a concentration of 300-9000 IU/mL (e.g., the concentration may be 1000-9000 IU/mL, e.g., 6000 IU/mL), and a ribonucleoprotein complex (RNP) formed of a gRNA as shown in any one of SEQ ID NOs: 37 to 39 and a Cas protein is used for transduction such that the proportion of cells expressing the two or more proteins in the TILs is about 95% or less. After the TILs of the present application are activated for a period of time, irradiated PBMCs are added (at a ratio of TILs to PBMCs of about 1:40 to about 1:400) and cultured by expansion for about 3-14 days. The cells in the culture medium are collected using a cell processing system, washed and cryopreserved, and detected. In the final products, the proportion of CD3 can be greater than 80%, the viability of the cells can be greater than 50%, and more than 80% of the T cells may be memory effector T cells and effector T cells. Upon stimulation, IFN-$\gamma$ can be secreted, and/or the final products may have a characteristic of up-regulated proportion of activated T cells. The two or more proteins are selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

In one aspect, the present application provides tumor infiltrating lymphocytes (TILs) which can be obtained by the culture method of the present application. In one embodiment, the TILs provided in the present application may comprise one kind or one batch of TILs obtained by the culture method of the present application. In one embodiment, the TILs provided in the present application may comprise multiple kinds or multiple batches of TILs obtained by the culture method of the present application and combined in any proportion.

In some embodiments, the TILs expanded by the method of the present application can be administered to patients as a pharmaceutical composition. In some embodiments, pharmaceutical composition may be a suspension of TILs in sterile buffer. The TILs expanded with the PBMCs of the present application can be administered in any suitable routes known in the art. In some embodiments, T cells can be administered in a single fusion intraarterially or intravenously, and the infusion may last about 30 to 60 minutes. Other suitable administration routes may comprise intraperitoneal, intrathecal, and intralymphatic administration.

In some embodiments, any suitable dose of TILs can be administered. In some embodiments, for example, when the tumor is melanoma, about $2.3 \times 10^9$ to about $13.7 \times 10^{10}$ TILs can be administered. In some embodiments, about $1 \times 10^9$ to about $12 \times 10^{10}$ TILs can be administered. In some embodiments, about $1.2 \times 10^{10}$ to about $4.3 \times 10^{10}$ TILs can be administered. In some embodiments, about $3 \times 10^{10}$ to about $12 \times 10^{10}$ TILs can be administered. In some embodiments, about $4 \times 10^{10}$ to about $10 \times 10^{10}$ TILs can be administered. In some embodiments, about $5 \times 10^{10}$ to about $8 \times 10^{10}$ TILs can be administered. In some embodiments, about $6 \times 10^{10}$ to about $8 \times 10^{10}$ TILs can be administered. In some embodiments, about $7 \times 10^{10}$ to about $8 \times 10^{10}$ TILs can be administered. In some embodiments, the therapeutically effective dose can be about $2.3 \times 10^9$ to about $13.7 \times 10^{10}$. In some embodiments, the therapeutically effective dose can be about $1 \times 10^9$ to about $12 \times 10^{10}$ TILs. In some embodiments, the therapeutically effective dose can be about $1.2 \times 10^{10}$ to about $4.3 \times 10^{10}$ TILs. In some embodiments, the therapeutically effective dose can be about $3 \times 10^{10}$ to about $12 \times 10^{10}$ TILs. In some embodiments, the therapeutically effective dose can be about $4 \times 10^{10}$ to about $10 \times 10^{10}$ TILs. In some embodiments, the therapeutically effective dose can be about $5 \times 10^{10}$ to about $8 \times 10^{10}$ TILs. In some embodiments, the therapeutically effective dose can be about $6 \times 10^{10}$ to about $8 \times 10^{10}$ TILs. In some embodiments, the therapeutically effective dose can be about $7 \times 10^{10}$ to about $8 \times 10^{10}$ TILs.

In some embodiments, the number of TILs provided in the composition of the present application can be about $1 \times 10^6$, about $2 \times 10^6$, about $3 \times 10^6$, about $4 \times 10^6$, about $5 \times 10^6$, about $6 \times 10^6$, about $7 \times 10^6$, about $8 \times 10^6$, about $9 \times 10^6$, about $1 \times 10^7$, about $2 \times 10^7$, about $3 \times 10^7$, about $4 \times 10^7$, about $5 \times 10^7$, about $6 \times 10^7$, about $7 \times 10^7$, about $8 \times 10^7$, about $9 \times 10^7$, about $1 \times 10^8$, about $2 \times 10^8$, about $3 \times 10^8$, about $4 \times 10^8$, about $5 \times 10^8$, about $6 \times 10^8$, about $7 \times 10^8$, about $8 \times 10^8$, about $9 \times 10^8$, about $1 \times 10^9$, about $2 \times 10^9$, about $3 \times 10^9$, about $4 \times 10^9$, about $5 \times 10^9$, about $6 \times 10^9$, about $7 \times 10^9$, about $8 \times 10^9$, about $9 \times 10^9$, about $1 \times 10^{10}$, about $2 \times 10^{10}$, about $3 \times 10^{10}$, about $4 \times 10^{10}$, about $5 \times 10^{10}$, about $6 \times 10^{10}$, about $7 \times 10^{10}$, about $8 \times 10^{10}$, about $9 \times 10^{10}$, about $1 \times 10^{11}$, about $2 \times 10^{11}$, about $3 \times 10^{11}$, about $4 \times 10^{11}$, about $5 \times 10^{11}$, about $6 \times 10^{11}$, about $7 \times 10^{11}$, about $8 \times 10^{11}$, about $9 \times 10^{11}$, about $1 \times 10^{12}$, about $2 \times 10^{12}$, about $3 \times 10^{12}$, about $4 \times 10^{12}$, about $5 \times 10^{12}$, about $6 \times 10^{12}$, about $7 \times 10^{12}$ about $8 \times 10^{12}$, about $9 \times 10^{12}$, about $1 \times 10^{13}$, about $2 \times 10^{13}$, about $3 \times 10^{13}$, about $4 \times 10^{13}$, about $5 \times 10^{13}$, about $6 \times 10^{13}$, about $7 \times 10^{13}$, about $8 \times 10^{13}$, or about $9 \times 10^{13}$. In some embodiments, the number of the TILs provided in the composition of the present application can range from about $1 \times 10^6$ to $5 \times 10^6$, about $5 \times 10^6$ to $1 \times 10^7$, about $1 \times 10^7$ to $5 \times 10^7$, about $5 \times 10^7$ to $1 \times 10^8$, about $1 \times 10^8$ to $5 \times 10^8$, about $5 \times 10^8$ to $1 \times 10^9$, about $1 \times 10^9$ to $5 \times 10^9$, about $5 \times 10^9$ to $1 \times 10^{10}$, about $1 \times 10^{10}$ to $5 \times 10^{10}$, about $5 \times 10^{10}$ to $1 \times 10^{11}$, about $5 \times 10^{11}$ to $1 \times 10^{12}$, about $1 \times 10^{12}$ to $5 \times 10^{12}$, or about $5 \times 10^{12}$ to $1 \times 10^{13}$.

In some embodiments, the concentration of the TILs provided in the composition of the present application can be less than, e.g., about 100%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001% w/w, w/v, or v/v of the composition.

In some embodiments, the concentration of the TILs provided in the composition of the present application can be greater than about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50%, about 4.25%, about 4%, about 3.75%, about 3.50%, about 3.25%, about 3%, about 2.75%, about 2.50%, about 2.25%, about 2%, about 1.75%, about 1.50%, about 1.25%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about or 0.0002%, or about 0.0001% w/w, w/v, or v/v of the composition.

In some embodiments, the concentration of the TILs provided in the composition of the present application can range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12%, or about 1% to about 10% w/w, w/v, or v/v of the composition.

In some embodiments, the concentration of the TILs provided in the composition of the present application can range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, or about 0.1% to about 0.9% w/w, w/v or v/v of the composition.

In some embodiments, the amount of the TILs provided in the composition of the present application can be equal to or less than about 10 g, about 9.5 g, about 9.0 g, about 8.5 g, about 8.0 g, about 7.5 g, about 7.0 g, about 6.5 g, about 6.0 g, about 5.5 g, about 5.0 g, about 4.5 g, about 4.0 g, about 3.5 g, about 3.0 g, about 2.5 g, about 2.0 g, about 1.5 g, about 1.0 g, about 0.95 g, about 0.9 g, about 0.85 g, about 0.8 g, about 0.75 g, about 0.7 g, about 0.65 g, about 0.6 g, about 0.55 g, about 0.5 g, about 0.45 g, about 0.4 g, about 0.35 g, about 0.3 g, about 0.25 g, about 0.2 g, about 0.15 g, about 0.1 g, about 0.09 g, about 0.08 g, about 0.07 g, about 0.06 g, about 0.05 g, about 0.04 g, about 0.03 g, about 0.02 g, about 0.01 g, about 0.009 g, about 0.008 g, about 0.007 g, about 0.006 g, about 0.005 g, about 0.004 g, about 0.003 g, about 0.002 g, about 0.001 g, about 0.0009 g, about 0.0008 g, about 0.0007 g, about 0.0006 g, about 0.0005 g, about 0.0004 g, about 0.0003 g, about 0.0002 g, or about 0.0001 g.

In some embodiments, the amount of the TILs provided in the composition of the present application can be greater than about 0.0001 g, about 0.0002 g, about 0.0003 g, about 0.0004 g, about 0.0005 g, about 0.0006 g, about 0.0007 g, about 0.0008 g, about 0.0009 g, about 0.001 g, about 0.0015 g, about 0.002 g, about 0.0025 g, about 0.003 g, about 0.0035 g, about 0.004 g, about 0.0045 g, about 0.005 g, about 0.0055 g, about 0.006 g, about 0.0065 g, about 0.007 g, about 0.0075 g, about 0.008 g, about 0.0085 g, about 0.009 g, about 0.0095 g, about 0.01 g, about 0.015 g, about 0.02 g, about 0.025 g, about 0.03 g, about 0.035 g, about 0.04 g, about 0.045 g, about 0.05 g, about 0.055 g, about 0.06 g, about 0.065 g, about 0.07 g, about 0.075 g, about 0.08 g, about 0.085 g, about 0.09 g, about 0.095 g, about 0.1 g, about 0.15 g, about 0.2 g, about 0.25 g, about 0.3 g, about 0.35 g, about 0.4 g, about 0.45 g, about 0.5 g, about 0.55 g, about 0.6 g, about 0.65 g, about 0.7 g, about 0.75 g, about 0.8 g, about 0.85 g, about 0.9 g, about 0.95 g, about 1 g, about 1.5 g, about 2 g, about 2.5 g, about 3 g, about 3.5 g, about 4 g, about 4.5 g, about 5 g, about 5.5 g, about 6 g, about 6.5 g, about 7 g, about 7.5 g, about 8 g, about 8.5 g, about 9 g, about 9.5 g, or about 10 g.

In some embodiments, the TILs can be administered in a single dose. Such administration can be by injection, e.g., intravenous injection. In some embodiments, the TILs can be administered in multiple doses. The doses can be once, twice, three times, four times, five times, six times or more than six times per year. The doses can be once a month, once every two weeks, once a week or once every 2 days. In some embodiments, the administration of the TILs can be continuous.

In one aspect, the present application provides a pharmaceutical composition. In some embodiments, the pharmaceutical composition may comprise the TILs of the present application and/or the composition of the present application, as well as a pharmaceutically acceptable carrier.

In one aspect, the present application provides a kit, which may comprise T cell activators, T cell growth factors and/or feeder cells used in the method for culturing tumor infiltrating lymphocytes (TILs) in the present application, and instructions describing the steps of the method for culturing tumor infiltrating lymphocytes (TILs) in the present application.

In one aspect, the present application provides a kit, which may comprise the TILs of the present application and/or the pharmaceutical composition of the present application.

In one aspect, the present application provides a method for affecting the tumor cell growth, which may comprise administering to a subject the TILs of the present application and/or the pharmaceutical composition of the present application. In some embodiments, the step of affecting the tumor growth may comprise reducing the volume of the tumor to, e.g., about 99%, about 95%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2% or about 0.1% of its volume before the administration.

In one aspect, the present application provides use of the TILs of the present application and/or the pharmaceutical composition of the present application for the manufacture of drugs which can be used for preventing and/or treating a tumor. In some embodiments, the tumor in the present application is selected from solid tumors. In some embodiments, the tumor in the present application can be one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In one aspect, the present application provides a method for preventing and/or treating a tumor, which may comprise administering to a subject the TILs of the present application and/or the pharmaceutical composition of the present application. In some embodiments, the tumor in the present application is selected from solid tumors. In some embodiments, the tumor in the present application can be one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

In one aspect, the present application provides the TILs of the present application and/or the pharmaceutical composition of the present application, which can be used for preventing and/or treating a tumor. In some embodiments, the tumor in the present application is selected from solid tumors. In some embodiments, the tumor in the present application can be one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

Without intending to be limited by any theory, the examples below are intended only to illustrate the method and use of the present application and are not intended to limit the inventive scope of the present application.

## Examples

### Example 1

1.1 Reception and preparation of feeder cells

1.1.1 Reception of apheresis blood

The information of the apheresis blood, batch numbers and volumes were recorded, and the blood sample was rewarmed to room temperature.

1.1.2 Manual isolation and cryopreservation of PBMCs (peripheral blood mononuclear cells)

A blood bag was sterilized with 75% alcohol and transferred into a biosafety cabinet. After the blood bag being cut with a sterile scissor, the apheresis blood was transferred into 50 mL centrifuge tubes. The blood bag was washed with 20 mL of PBS or normal saline injected by a 20 mL syringe. The washing solution was also transferred into the 50 mL centrifuge tubes. The liquid volume in each 50 mL centrifuge tube should not exceed 30 mL. The apheresis blood was centrifuged at 3000 g for 10 minutes. During the centrifugation, 6-8 centrifuge tubes of 50 mL were prepared, into each of which was added 20 mL of rewarmed lymphocyte isolation solution (Ficoll, Tianjin Haoyang). At the end of centrifugation, the upper layer of plasma was discarded, and the cell pellets were diluted with PBS or normal saline. The diluted blood cell mixed solution was slowly added dropwise onto the upper layer of the lymphocyte isolation solution without destroying the interface at about 25 mL of samples per tube, and the final volume within each tube should be no more than 28 mL.

Centrifugation was performed at temperature of 18-22°C and 500-600 g for 15-30 minutes using a horizontal rotor. After centrifugation, the resulting buffy coat will be at the interface between normal saline and the lymphocyte isolation solution Ficoll. The upper layer of plasma and normal saline were aspirated off, and the middle buffy coat was transferred to another 50 mL clean sterile centrifuge tube with a pipette. The collected buffy coat was diluted with PBS or normal saline and centrifuged at room temperature and 600 g for 10 minutes. At the end of centrifugation, the supernatant was discarded. The cells were washed once with PBS or normal saline and centrifuged at room temperature and 500 g for 5 minutes.

If there were lots of red blood cells, the red blood cells could be lysed after the centrifugation. A red blood cell lysis solution was added at a volume ratio of 1:2 to 1:3 of the cell pellets to the red blood cell lysis solution, and mixed well. The red blood cells were lysed at room temperature for 10 minutes, during which the centrifuge tubes were gently mixed 2-3 times to ensure the lysis effect. After the lysis was completed, PBS or normal saline were added to wash the cells. After the lysis, the cells were washed twice, centrifuged at 400 g for 6 minutes, and sampled and counted before the last centrifugation.

The supernatant was discarded, the cells were resuspended in the basic medium at a cell density adjusted to about $2\text{-}3 \times 10^7$/mL, wherein the liquid level should be not higher than 1 cm, and the volume in each T225 culture flask should be less than 200 mL. The suspension was irradiated with X-rays at 50 Gy in the tiled state. The supernatant was discarded after centrifugation, and the cells were cryopreserved according to the counting results at about $1\text{-}2\times10^8$ cells/mL and 1-2 mL/tube. The cells were placed in a programmed cooling box and transferred to a -80°C freezer for cryopreservation.

1.1.3 Automatic isolation and cryopreservation of PBMCs

The tubing of the blood bag was aseptically connected to the input end of a cpro isolation kit (Cytiva). If the blood volume was more than 120 mL, a pre-concentration step might be performed to concentrate the blood volume to less than 120 mL. A neatcell procedure might be used to isolate and wash PBMCs, wherein the washing solution was normal saline, and the intermediate volume was 20 mL; the resuspending solution was the basic medium and was added at 80 mL for each batch. After isolation, the PBMCs for each donor were one bag of 100 mL. When in the tiled state, the liquid level might be no more than 1 cm, and the suspension was irradiated with X-rays at 50 Gy. Sampling and counting were carried out after irradiation. Cells were collected and washed three times using a culture wash procedure, wherein the washing solution was normal saline; the intermediate volume and the final volume were set so that the volume per $1\times10^9$ cells was not less than 2 mL; an equal amount to 2-fold amount of cryopreservation solution was added and mixed well. The cell density was adjusted to about $1\times10^7$ cells/mL to $2\times10^8$ cells/mL with a 1-fold cryopreservation solution. The suspension was subpackaged at 20 mL per bag, cryopreserved in a programmed cooler, and stored in liquid nitrogen.

1.2 Reception and processing of tumor tissues

1.2.1 Reception of tissues

Tumor tissues and blood samples were received from donors. The sample information was checked and recorded, and corresponding sample labels were printed.

1.2.2 Processing and culture of tissues

The sample tubes and blood collection tubes were sterilized with 75% alcohol and transferred into a biosafety cabinet. The PBMC cells in the blood samples were isolated and cryopreserved according to the above procedures for manual isolation and cryopreservation of PBMCs. A kind of culture flasks and bags with gas permeable surfaces, e.g., a culture bag (Origen), were taken, into which were added 300 mL of rewarmed complete medium that could be optionally selected from X-vivo 15 culture medium or other commercially available T cell culture media, e.g., T cell culture media of Stem Cell, Lonza, Thermo, Miltenyi etc., as well as essential amino acids and antibiotics, and IL-2 at an addition concentration of 300 to 9000 IU/mL (e.g., the concentration may be 1000 to 9000 IU/mL, e.g., it may be 6000 IU/mL). Several culture dishes of 10 cm were taken, into which was added an appropriate amount of culture medium. The tumor tissues were taken out from the sample tubes into the 10 cm culture dishes using sterile ophthalmic forceps. The amount of the culture medium should be just above the tumor tissues, and the tissue morphology was observed and recorded. The tissues were washed and the culture dishes were replaced. The tissues were cut preliminarily using ophthalmic scissors and ophthalmic forceps to remove adipose tissues and necrotic tissues. Each tissue piece was further cut to a size of about 27 mm$^3$. Non-suspended tumor tissue pieces were taken. A 20 mL syringe, after removing the inner piston, was connected to the culture bag, through which about 1 g of tissue pieces were transferred into the culture bag using a pipette. The culture bag was placed in a carbon dioxide incubator for culture. The scissors and forceps were cleaned and preliminarily disinfected with 75% alcohol, and then sterilized after ultrasonic cleaning to obtain the first TIL population.

1.3 Step (A): *In vitro* expansion and harvest

1.3.1 *In vitro* expansion in step (A)

According to the cell growth status, the culture medium was replenished or half-changed every 3-7 days to ensure cell nutrition. A complete culture medium was used, which may be optionally selected from X-vivo 15 culture medium or other commercially available T cell culture media, e.g. T cell culture media of Stem Cell, Lonza, Thermo, Miltenyi etc., and into which were added essential amino acids and antibiotics as well as IL-2 (SL Pharm) at an addition concentration of 300 to 9000 IU/mL (e.g., the concentration may be 1000 to 9000 IU/mL, e.g., it may be 6000 IU/mL). Sampling and counting were carried out on days 3-14, e.g., on day 13 or 14, of the step (A). If the cell number was between $5 \times 10^5$ and $5 \times 10^8$, the cells entered the harvest step of the step (A).

1.3.2 Harvest in step (A)

The cells at the end of *in vitro* expansion of step (A) were collected and centrifuged. The culture medium was discarded. The cells were washed once with PBS or normal saline to obtain the TILs that have been subjected to the *in vitro* expansion of step (A) (the second TIL population). Sampling and counting were carried out to leave an amount of about $5 \times 10^5$ to $2 \times 10^8$ cells to enter the subsequent step *of in vitro* expansion; an amount of about $5 \times 10^5$ cells could be taken for quality control detection; and the rest of the cells were cryopreserved in a cryopreservation solution as the cryopreserved preREP TIL *in vitro* cells.

1.4 Step (B): Activation of TILs

The TILs (the second TIL population) expanded *in vitro* in step (A) continued to be cultured, or the cryopreserved preREP TIL *in vitro* cells are resuscitated for TIL activation in step (B).
A complete culture medium was used, which may be optionally selected from X-vivo 15 culture medium or other commercially available T cell culture media, e.g. T cell culture media of Stem Cell, Lonza, Thermo, Miltenyi, etc. Moreover, essential amino acids and antibiotics could be added to adjust the cell density to $5 \times 10^5$ to $2 \times 10^6$ cells/mL, and IL-2 was also added into a suspension 24-well culture plate at 1 mL/well at a concentration of 300 to 9000 IU/mL (e.g., the concentration may be 1000 to 9000 IU/mL, e.g., it may be 6000 IU/mL or 6000 IU/mL). Into the culture medium of each TIL cell population could also be added T cell activators, e.g., a CD3 agonist and/or a CD28 agonist, e.g., about 30 ng/mL of CD3 antibody (Miltenyi Biotech, OKT3), about 30 ng/mL of CD28 antibody (Merck, 15E8), magnetic beads (diameter of about 1 to 10 $\mu$m, Dynabeads, Thermo Fisher) at a ratio of magnetic beads to TILs of about 1:2 to 2:1, and/or transACT at a ratio of transACT (diameter of about 100 to 500 nm, Miltenyi) to TILs of about 1:100 to 1:2000. After culture for about 0 to 4 days, a third TIL population was obtained.

1.5 Step (C): Gene-editing of TIL cells

The gRNA provided in the present application was thawed and formulated with nuclease-free water to a concentration of about 100 $\mu$M. About 2 $\mu$L of gRNA (50 $\mu$M) was incubated at 95°C for 2 minutes for annealing and then added into P3 buffer, into which was further added 0.3 to 1 $\mu$L of Cas9 (Cure Genetics, 10 mg/mL) and incubated at 25°C for 10 minutes to form a ribonucleoprotein complex (RNP). Each RNP for double knockout or more knockout combinations was prepared separately and mixed prior to electroporation. The above mixed RNP was electroporated with approximately $1 \times 10^6$ cells of the third TIL population in P3 buffer (Lonza) using the Lonza electroporator. For example, the electroporation procedure may be human T cell stim (EO115). The electroporated gene was cultured for about 0 to 4 days after editing to obtain a fourth TIL population.

1.6 Step (D): Culture of TIL cells after gene-editing

Feeder cells were added into the fourth TIL cell population for culture. The time of TILs contacting with feeder cells needs to be at a certain time $T_n$ later after the contact of TILs with IL-2 and T cell activators in step (B) ($T_n$ for each test group may be 0 hours to 12 days, e.g., 24 hours or 48 hours). First, mixed feeder cells from 1-5 donors were resuscitated; the activated TIL cells were mixed with the feeder cells at a ratio of TIL cells to feeder cells of about 1:200 and transferred into G-Rex100 culture flasks or gas-permeable bags which was supplemented with a complete medium. Sampling and counting were carried out every 1-3 days, and the medium was replenished or half-changed according to the cell status until the total cell number was greater than $1 \times 10^9$, or the culture time *of in vitro* expansion in step (D) reached about 5 days to 14 days, then the *in vitro* expansion culture in step (D) was terminated.

1.7 Harvest of tumor infiltrating lymphocytes

The cells expanded in step (D) were centrifuged, and after discarding the supernatant of the culture medium, the cells were washed three times with PBS or normal saline or a compound electrolyte solution to obtain TILs subjected to expansion in step (D) (the fifth TIL population). Sampling and counting were carried out during the third washing. According to the counting results, the supernatant was discarded after the last centrifugation, and $3\times10^6$ cells were taken for quality control detection; all the remaining cells were added into a cryopreservation solution, and the cell density was adjusted to $1\text{-}3\times10^8$ cells/mL for cryopreservation.

**Example 2 Comparison of proliferation ability of TILs cultured with feeder cells added at different times**

After some time $T_n$ ($T_n$ can range from 0 hours to 14 days) after the addition of IL-2 and different forms of T cell activators in Example 1, feeder cells were co-cultured with tumor infiltrating lymphocytes. In this Example, $T_n$ was taken as 0 hours, 6 hours, 12 hours, 24 hours, 48 hours, 72 hours, 5 days, 7 days, and 9 days to obtain TILs cultured with the feeder cells added at different times, and a comparison experiment on the cell counts was performed.

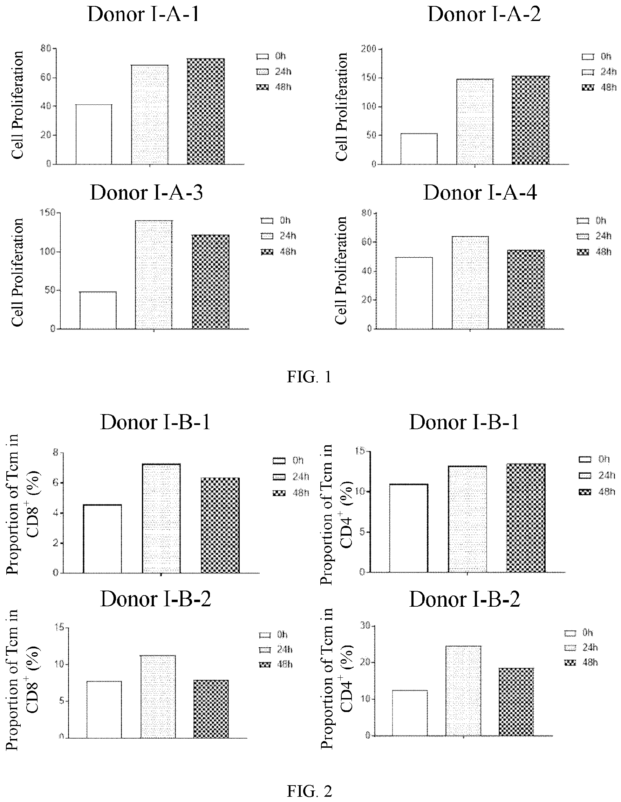

FIG. 1 shows the analysis results of the proliferation ability of TILs cultured with feeder cells added at different times. The values on the vertical coordinates in each group of graphs for TILs which were cultured with feeder cells added at different times indicate the expansion multiple of the number of TIL cells after the end *of in vitro* expansion compared with that before the start *of in vitro* expansion. The proliferation results of TILs from 4 donors show that the TILs cultured with feeder cells added at 0 hours after the addition of OKT3 and IL-2 (that is, at the same time) have weaker proliferation ability than that of TILs cultured with feeder cells added at 24 or 48 hours after the addition of OKT3 and IL-2.

**Example 3 Comparison of flow cytometry on TILs cultured with feeder cells added at different times**

After some time $T_n$ ($T_n$ can range from 0 hours to 14 days) after the addition of IL-2 and different forms of T cell activators in Example 1, feeder cells were co-cultured with tumor infiltrating lymphocytes. In this Example, $T_n$ was taken as 0 hours, 6 hours, 12 hours, 24 hours, 48 hours, 72 hours, 5 days, 7 days, and 9 days to obtain TILs cultured with the feeder cells added at different times, and a comparison experiment on the flow cytometry was performed.

Sources of test materials for TIL flow cytometry

Transcription Factor Buffer Set, manufacturer BD, product number 562574; V-bottom 96-well plate, manufacturer Corning, product number 3894; flow tube, manufacturer Corning, product number 352052.
The flow antibodies used in this example were purchased from BD or Biolegend. $1\times10^5$ to $5\times10^5$ cell samples from each group were added into flow tubes or V-bottom 96-well plates. Centrifugation was performed at 600 g for 3 minutes and the supernatant was discarded. Washing was carried out once using PBS, with 1 mL/tube for flow tubes, and 200 μL/well for 96-well plates, and the supernatant was discarded. A prepared antibody working solution was added for cell surface staining. The concentration of the antibodies (BD or Biolegend) was 1:100 to 1:200, and the active detection dye was comprised at 1:10000. Staining was carried out with 100 μL/tube for flow tubes, and 50 μL/well for 96-well plates, and incubation was performed at 2-8°C in dark for 30 minutes. The reagents required for the staining of transcription factors was formulated during the staining process: Transcription Factor Buffer Set (BD) was used to dilute a $4\times$ Fixation/Permeabilization Solution (BD) to produce $1\times$ working solution A; double distilled water was used to dilute a $5\times$ Perm/Wash Buffer (BD) to produce $1\times$ working solution B. The working solutions were pre-cooled at 4 °C for use. After staining, an appropriate amount of PBS was added to wash the cells twice (200 μL each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100 μL/well for 96-well plates, and 1 mL/tube for flow tubes) of $1\times$ working solution A was added to fix and permeabilize. Incubation was performed at 2-8°C in dark for 40-50 minutes. At the end of fixation and permeabilization, $1\times$ working solution B was added to wash the cells (200 μL each time for 96-well plates, and 2 mL each time for flow tubes). Centrifugation was performed at 2-8°C and at 350 g for 6 minutes, and washing was carried out twice. $1\times$ working solution B was used to formulate intracellular antibodies with 50 μL/well for 96-well plates, and 100 μL/tube for flow tubes, and the antibody concentration was 1:100 to 1:200. Staining was performed at 2-8°C in dark for 30 minutes. At the end of staining, $1\times$ working solution B was added to wash the cells (200 μL each time for 96-well plates, and 2 mL each time for flow tubes). Centrifugation was performed at 2-8°C and at 350 g for 6 minutes, and washing was carried out twice. The cells were resuspended in 100-500 μL of PBS for flow cytometry test on machine.
The analyses on flow cytometry results of TILs cultured with feeder cells added at different times are shown in FIGs. 2 to 8.

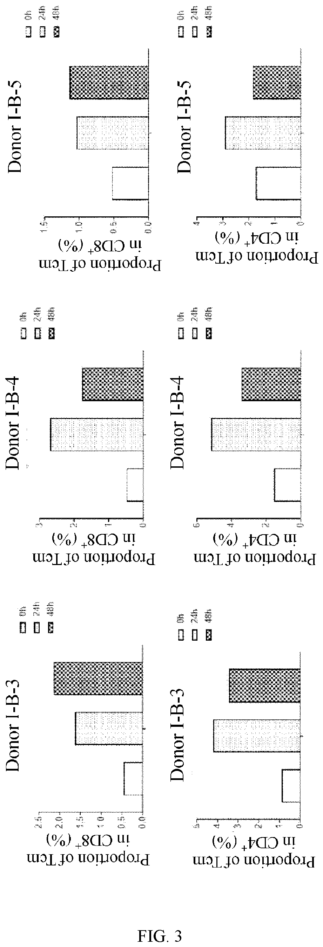

FIGs. 2 and 3 show the proportion of CD45RA⁻CCR7⁺ central memory T cells (Tcm) in the TIL cells obtained by culturing with feeder cells added at 0, 24 or 48 hours after the addition of OKT3 and IL-2. The results show that the TILs cultured with feeder cells added after 24 hours or 48 hours had a higher proportion of central memory T cells compared to that in the TILs cultured with the feeder cells added at the same time.

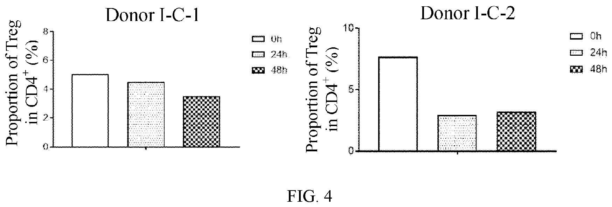

FIG. 4 shows the proportion of CD4$^+$CD25$^+$Foxp3$^+$ regulatory T cells (Treg) in the TIL cells obtained by culturing with feeder cells added at 0, 24 or 48 hours after the addition of OKT3 and IL-2. The results show that the TILs cultured with feeder cells added after 24 hours or 48 hours had a lower proportion of regulatory T cells compared to that in the TILs cultured with the feeder cells added at the same time.

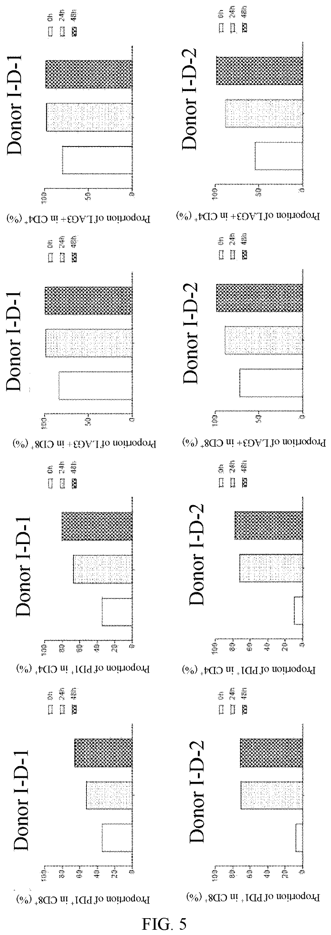

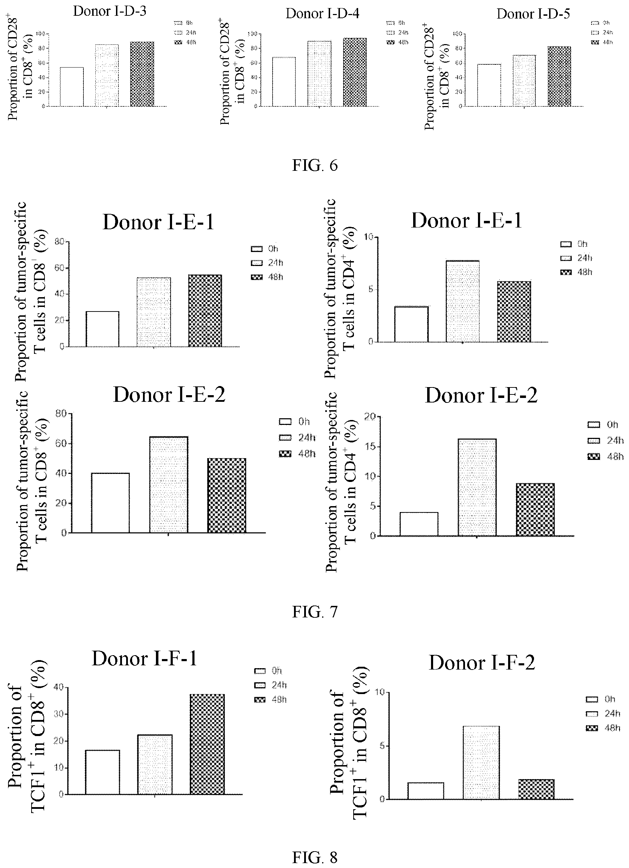

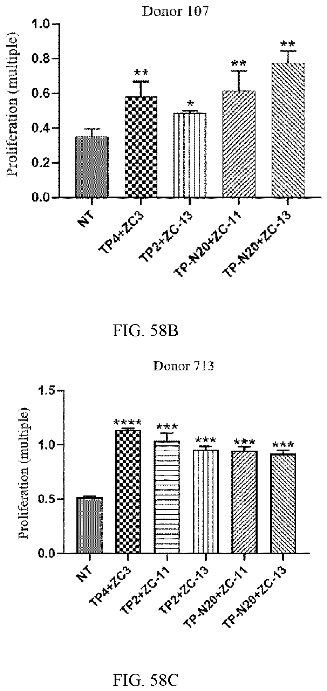

FIGs. 5 and 6 show the proportion of activated T cells in the TIL cells obtained by culturing with feeder cells added at 0, 24 or 48 hours after the addition of OKT3 and IL-2. The results show that the TILs cultured with feeder cells added after 24 hours or 48 hours had a higher proportion of activated T cells compared to that in the TILs cultured with the feeder cells added at the same time, for example, a higher proportion of PD-1$^+$, LAG-3$^+$ and/or CD28$^+$ cells.

FIG. 7 shows the proportion of CD103$^+$CD39$^+$ tumor-specific T cells in the TIL cells obtained by culturing with feeder cells added at 0, 24 or 48 hours after the addition of OKT3 and IL-2. The results show that the TILs cultured with the feeder cells added after 24 hours or 48 hours had a higher proportion of tumor-specific T cells compared to that in the TILs cultured with the feeder cells added at the same time.

FIG. 8 shows the proportion of TCF1$^+$ stem cell-like T cells in the TIL cells obtained by culturing with feeder cells added at 0, 24 or 48 hours after the addition of OKT3 and IL-2. The results show that the TILs cultured with the feeder cells added after 24 hours or 48 hours had a higher proportion of stem cell-like T cells compared to that in the TILs cultured with the feeder cells added at the same time.

**Example 4 Proliferation ability assay of TILs stimulated by adding a CD28 agonist**

Cell counts were performed on TIL populations obtained from culture of each test group in Example 1.

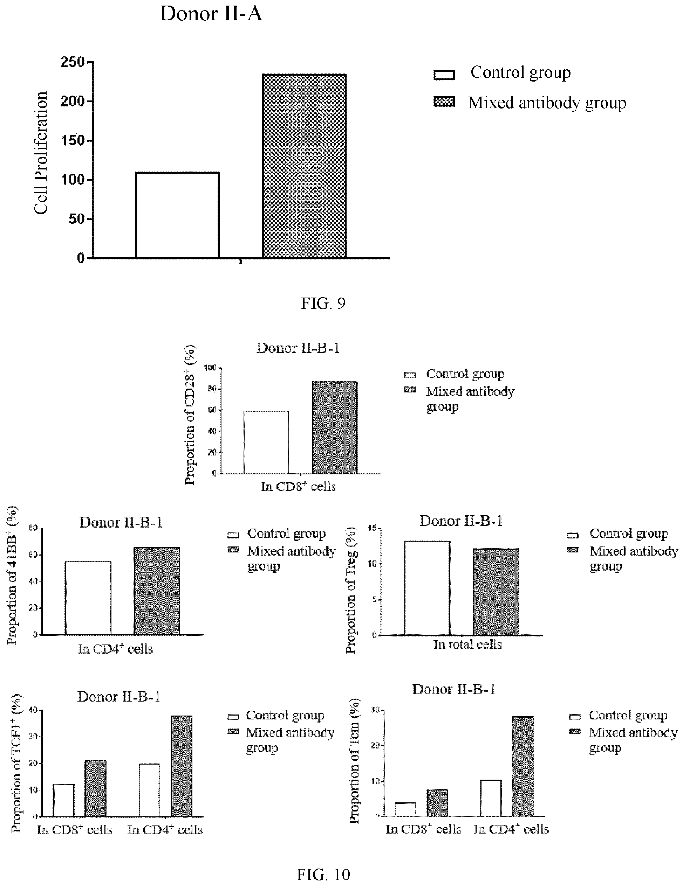

FIG. 9 shows the analysis results of the proliferation ability of the test groups added with different forms of CD28 agonists and the control group. The values on the vertical coordinates in the graphs indicate the expansion multiple of the number of TIL cells in the TIL populations obtained in each test group of *in vitro* expansion compared with the TIL populations before the start of *in vitro* expansion. The results show that, the proliferation ability of TILs obtained from the *in vitro* expansion of step (B) in the four-step method with the addition of CD28 antibodies is stronger than that of TILs cultured in the control group (without the addition of CD28 antibodies).

**Example 5 Flow cytometry of TILs stimulated by adding a CD28 agonist**

TIL populations obtained from *in vitro* expansion culture of each test group in Example 1 were tested by flow cytometry.

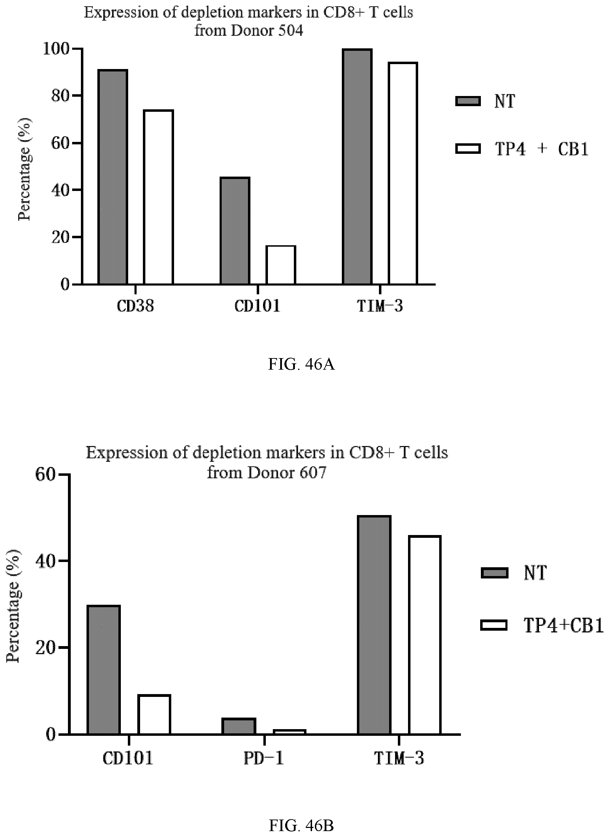

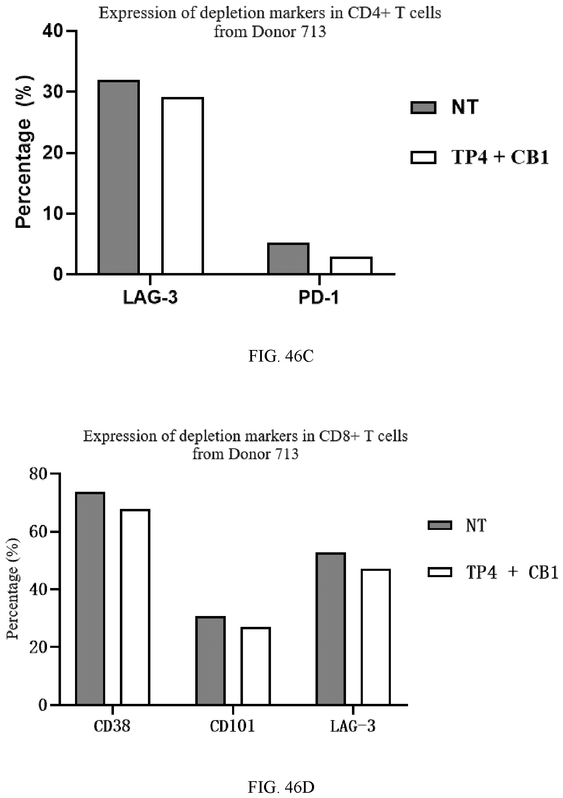

The analyses on flow cytometry results of TILs cultured by adding different forms of CD28 agonists are shown in FIGs. 46 to 50.

FIG. 10 shows the proportion of T cell subpopulations in the TIL cells obtained from culture in the mixed antibody group and the control group. The results show that, the TILs obtained from the *in vitro* expansion of step (B) in the four-step method with the addition of CD28 antibodies have an improved proportion of T cell subpopulations compared with that in the control group (without the addition of CD28 antibodies). For example, a higher proportion of activated T cells (CD28$^+$ or 41BB$^+$), a lower proportion of regulatory T cells (Treg, e.g., CD4$^+$CD25$^+$Foxp3$^+$), a higher proportion of stem cell-like T cells (TCF1$^+$), and/or a higher proportion of central memory T cells (Tcm, e.g., CD45RA CCR7$^+$).

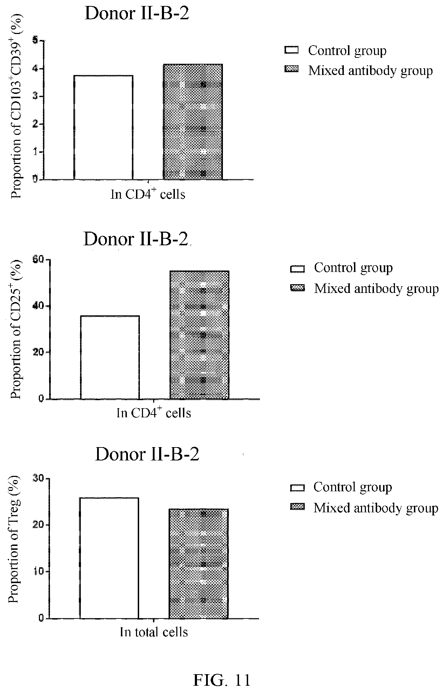

FIG. 11 shows the proportion of T cell subpopulations in the TIL cells obtained from culture in the mixed antibody group and the control group. The results show that, the TILs obtained from the *in vitro* expansion of step (B) in the four-step method with the addition of CD28 antibodies have an improved proportion of T cell subpopulations compared with that in the control group (without the addition of CD28 antibodies). For example, a higher proportion of tumor-specific T cells (CD103$^+$CD39$^+$), a higher proportion of activated T cells (CD25$^+$), and/or a lower proportion of regulatory T cells (Treg, e.g., CD4$^+$CD25$^+$Foxp3$^+$).

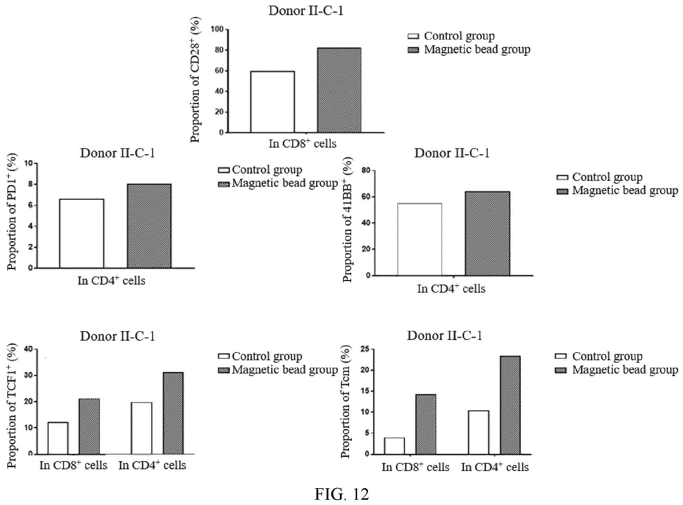

FIG. 12 shows the proportion of T cell subpopulations in TIL cells obtained from culture in the magnetic bead group and the control group. The results show that, the TILs obtained from the *in vitro* expansion of step (B) in the four-step method with the addition of CD28 antibodies (for example, adding magnetic beads comprising CD3 antibodies and CD28 antibodies) have an improved proportion of T cell subpopulations compared with that in the control group (without the addition of CD28 antibodies). For example, a higher proportion of activated T cells (CD28$^+$, PD-1$^+$ or 41BB$^+$), a higher proportion of stem cell-like T cells (TCF1$^+$), and/or a higher proportion of central memory T cells (Tcm, e.g., CD45RA$^-$ CCR7$^+$).

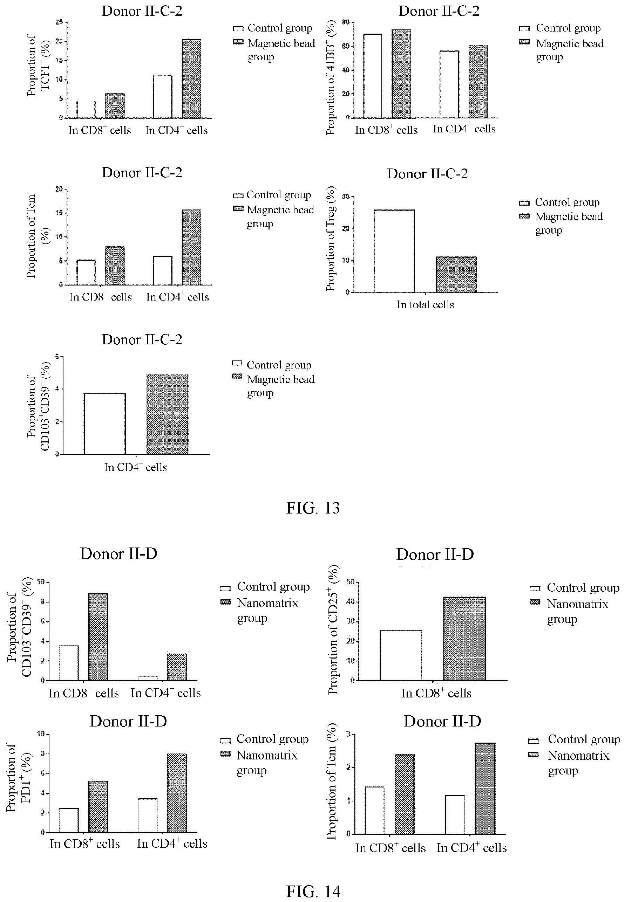

FIG. 13 shows the proportion of T cell subpopulations in TIL cells obtained from culture in the magnetic bead group and the control group. The results show that, the TILs obtained from the *in vitro* expansion of step (B) in the four-step method with the addition of CD28 antibodies (for example, adding magnetic beads comprising CD3 antibodies and CD28 antibodies) have an improved proportion of T cell subpopulations compared with that in the control group (without the addition of CD28 antibodies). For example, a higher proportion of stem cell-like T cells (TCF1$^+$), a higher proportion of activated T cells (41BB$^+$), a higher proportion of central memory T cells (Tcm, e.g., CD45RA$^-$CCR7$^+$), a lower proportion of regulatory T cells (Treg, e.g., CD4$^+$CD25$^+$Foxp3$^+$), and/or a higher proportion of tumor-specific T cells (CD103$^+$CD39$^+$).

FIG. 14 shows the proportion of T cell subpopulations in the TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved proportion of T cell subpopulations compared with that in the control group (without the addition of CD28 antibodies). For example, a higher proportion of tumor-specific T cells (CD103$^+$CD39$^+$), a higher proportion of activated T cells (CD25$^+$ or PD-1$^+$), and/or a higher proportion of central memory T cells (Tcm, e.g., CD45RA$^-$CCR7$^+$).

**Example 6 Cell killing ability assay of TILs stimulated by adding a CD28 agonist**

TIL populations from each test group in Example 1 obtained from the *in vitro* expansion of step (B) in the four-step method were tested for their cell killing ability.

Cell preparation

TILs obtained from each test group for test and target cells (e.g., Hela tumor cells) for co-culture were prepared.

Assay steps

Labeling the tumor cells with CFSE (5(6)-Carboxyfluorescein diacetate N-succinimidyl ester, Sigma, 21888-25MG-F): The tumor cells were washed with PBS, and resuspended in 500 $\mu$L of PBS; CFSE was added into 500 $\mu$L of PBS, and mixed with 500 $\mu$L of resuspension of the tumor cells in PBS to a final concentration of CFSE of 0.5 $\mu$mol/L. After incubation at 37°C for 6 minutes, a medium containing 10% FBS was added to wash. Centrifugation was performed at 600 g for 5 minutes. X-vivo 15 medium or other commercially available T cell culture media, e.g., T cell culture media of Stem Cell, Lonza, Thermo, Miltenyi brands etc., were used to resuspend the tumor cells to a concentration of $5 \times 10^6$ cells/mL. The TIL cells of each test group were centrifuged at 600 g for 5 minutes, and the TIL cells were resuspended at an effector-target ratio (the ratio of TIL cells to tumor cells) of 3:1 (i.e., the concentration of the resuspended TIL cells was $1.5 \times 10^6$ cells/mL). Each 100 $\mu$L of tumor cells and TIL cells were added to a U-bottom 96-well plate (Corning), and three replicate wells were set for each group. At the same time, a control group comprising only the tumor cells was set, and different reagents were added for different groups according to the experiment. The well plates were centrifuged at 200 g for 1 minute and incubated at 37°C for 4 hours to overnight.

At the end of the incubation, centrifugation was performed at 600 g for 3 minutes and the supernatant was discarded. Trypsin was added at 20 $\mu$L/well. Incubation was performed in an incubator at 37°C for 3-5 minutes to digest the tumor cells. After the digestion was completed, 180 $\mu$L of culture medium containing 10% FBS was added to terminate the digestion. Dapi (Beyotime, C0060) was diluted at 1:100, and then the diluted Dapi was added at 20 $\mu$L/well. Flow cytometry was performed on machine.

$$\text{Killing rate\%} = \text{Number of Dapi}^+\text{CFSE}^+ \text{cells/Total CFSE}^+ \times 100\%.$$

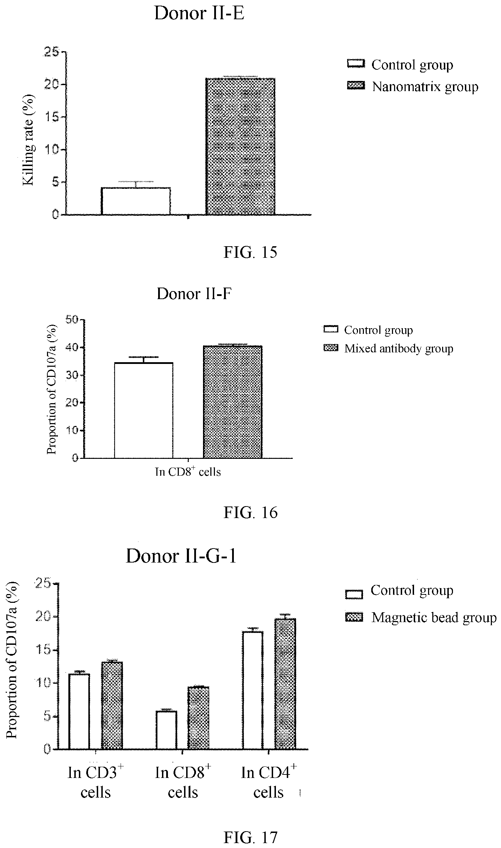

FIG. 15 shows the cell killing ability of the TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have higher cell killing ability compared with that in the control group (without the addition of CD28 antibodies).

**Example 7 Intracellular factor expression assay of TILs stimulated by adding a CD28 agonist**

TIL populations from each test group in Example 1 obtained from the *in vitro* expansion culture of step (B) in the four-step method were tested for intracellular factor expression.

Test preparation

Formulation of the culture medium required for the intracellular factor expression assay: the culture medium of T cells was taken, into which were added CD107a antibodies (BD) at a volume ratio of 1:500.

Assay steps

TILs from each test group were centrifuged, and then resuspended to $1\times10^6$ cells/mL using 600 μL of the culture medium required for the above intracellular factor expression assay, added to a 96-well plate at 100 μL/well, and incubated overnight in an incubator at 37°C.

At the end of the incubation, the cells were washed once with PBS at 200 μL/well and centrifuged at 600 g for 3 minutes. The supernatant was discarded. An antibody mixed working solution of CD3/CD4/CD8 (BD) was formulated for cell surface staining, with an antibody concentration of 1:100, a viability of 1:10000, and a staining volume of 50 μL for each group. Incubation was performed at 2-8°C in dark for 30 minutes. At the end of staining, the cells were washed and resuspended in PBS for flow cytometry on machine.

FIG. 16 shows the results of intracellular factor expression assay of TIL cells obtained from culture in the mixed antibody group and the control group. The results show that, the TILs obtained from the *in vitro* expansion of step (B) in the four-step method with the addition of CD28 antibodies have a higher intracellular factor expression capacity compared with that in the control group (without the addition of CD28 antibodies). For example, a higher CD107a expression capacity.

FIG. 17 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

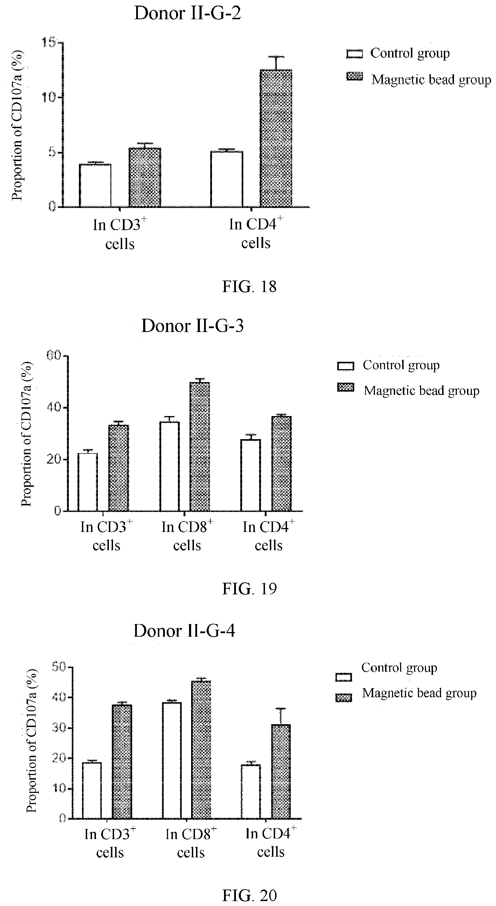

FIG. 18 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

FIG. 19 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

FIG. 20 shows the results of intracellular factor expression assay of TIL cells obtained from culture in the magnetic bead group and the control group. The results show that, the TILs obtained from the *in vitro* expansion of step (B) in the four-step method with the addition of CD28 antibodies (e.g., adding magnetic beads comprising CD3 antibodies and CD28 antibodies) have a higher intracellular factor expression capacity compared with that in the control group (without the addition of CD28 antibodies). For example, a higher CD107a expression capacity.

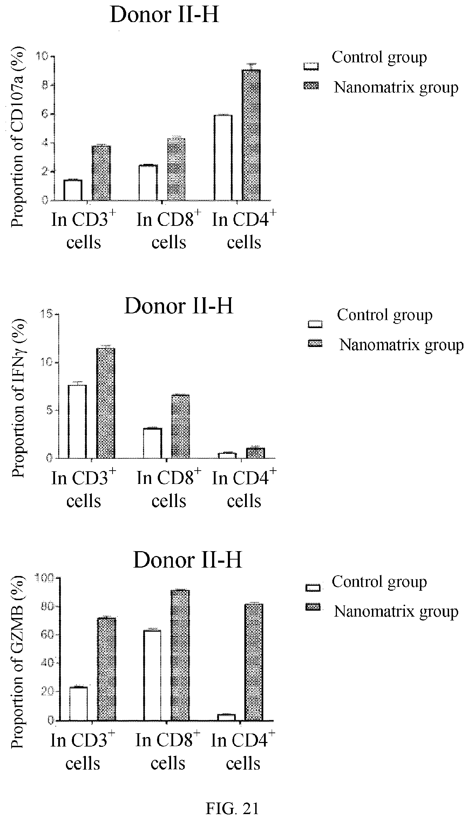

FIG. 21 the results of intracellular factor expression assay of TIL cells obtained from culture in the nanomatrix group and the control group. The results show that, the TILs obtained from the *in vitro* expansion of step (B) in the four-step method with the addition of CD28 antibodies (e.g., adding transACT comprising CD3 antibodies and CD28 antibodies) have a higher intracellular factor expression capacity compared with that in the control group (without the addition of CD28 antibodies). For example, a higher CD107a expression capacity, a higher IFN-γ expression capacity or a higher GZMB expression capacity.

**Example 8 Cytokine secretion assay of TILs stimulated by adding a CD28 agonist**

TIL populations from each test group in Example 1 obtained from the *in vitro* expansion culture of step (B) in the four-step method were tested for cytokine secretion.

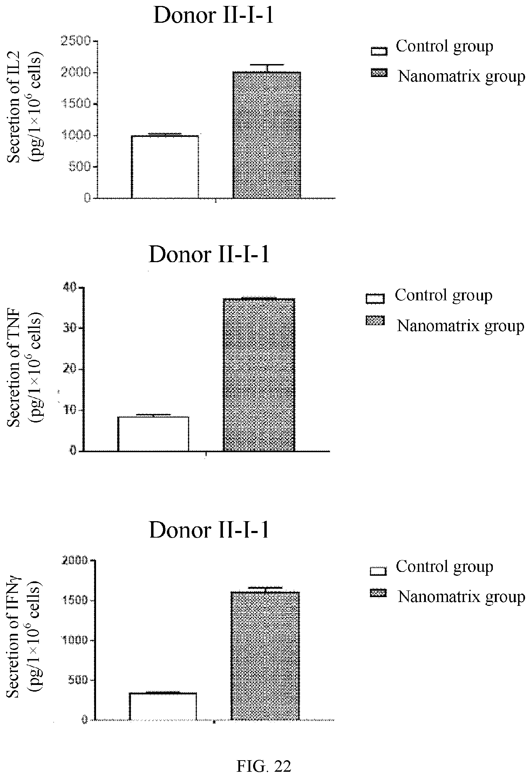

FIG. 22 shows the results of cytokine secretion assay of TIL cells obtained from culture in the nanomatrix group and the control group. The results show that, the TILs obtained from the *in vitro* expansion of step (B) in the four-step method with the addition of CD28 antibodies (e.g., adding transACT comprising CD3 antibodies and CD28 antibodies) have a higher cytokine secretion capacity compared with that in the control group (without the addition of CD28 antibodies). For example, a higher IL-2 secretion capacity, a higher TNF secretion capacity, or a higher IFN-γ secretion capacity.

The TILs obtained from each test group were incubated with tumor cells overnight, and the supernatant was taken after the incubation for cytokine secretion assay according to the assay steps of this example.

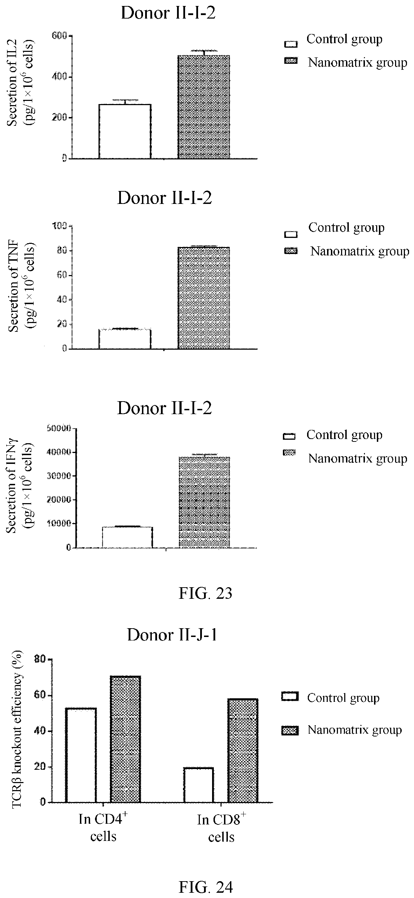

FIG. 23 shows the results of cytokine secretion assay of TIL cells obtained from culture in the nanomatrix group and the control group after being co-cultured with tumor cells. The results show that, the TILs obtained from the *in vitro* expansion of step (B) in the four-step method with the addition of CD28 antibodies (e.g., adding transACT comprising CD3 antibodies and CD28 antibodies) have a higher cytokine secretion capacity compared with that in the control group (without the addition of CD28 antibodies). For example, a higher IL-2 secretion capacity, a higher TNF secretion capacity, or a higher IFN-γ secretion capacity.

**Example 9 Gene knockout efficiency assay of TILs stimulated by adding a CD28 agonist**

TIL cells from each test group in Example 1 obtained after 48 hours *of in vitro* expansion culture of step (B) in the four-step method were tested for gene knockout efficiency.

Nuclease-free water (commercial source: Shanghai Youfan Biotech Co., Ltd; RT121-02) was used to formulate sgRNA (with its sequence as shown in SEQ ID NO: 1, GGAGAATGACGAGTGGACCC), and the concentration was adjusted to 50 μmol/L. 2 μL of gRNA was added to a PCR tube and incubated in a PCR instrument at 95°C for 2 minutes with nuclease-free water as the negative control, and then cooled at room temperature for 10 minutes.

According to a volume ratio of sgRNA:P3 Buffer:Cas9 nuclease = 2:2:1, P3 Buffer (commercial source: Lonza; V4XP-3032) and 61.7 μmol/L of Cas9 nuclease (commercial source: Suzhou Cure Genetics Co., Ltd.; C01-2019-11-001) were successively added into the PCR tube containing the sgRNA. The PCR tube was placed into the PCR instrument and incubated at 25°C for 10 minutes to form RNP, which was placed at 4°C for later use.

T cell culture medium was added at 1 mL per well and preheated in a $CO_2$ incubator. TIL cells from each test group in Example 1 obtained after 48 hours *of in vitro* expansion culture of step (B) in the four-step method were taken, mixed well and counted. $5\times10^5$ cells were taken from each test group and added into P3 Buffer (20 μL), and mixed well; the cells were added into a new PCR tube wherein they were mixed with 5 μL of RNP; the mixture of the cells and RNP was added into an electroporation strip plate, and electroporated in an electroporator (Lonza) (human T cell stimulated (E0115)). Immediately after the electroporation procedure, 180 μL of preheated T cell culture medium was added, and the entire volume was transferred to a 24-well suspension plate, and placed in a $CO_2$ incubator for culture. 24 hours later, the cells were counted, and feeder cells (irradiated PBMC cells) were added at a ratio of TILs to feeder cells = 1 :200, and incubated in a $CO_2$ incubator for further 72 hours. The TIL cells from each test group at the end of culture were taken for cell counting. $2\times10^5$ cells were taken from each test group, centrifuged at 500 g for 3 minutes, and the supernatant was aspirated and discarded after the centrifugation.

Formulation of mixed antibodies for flow cytometry test: Fixable Viability Dye eFluor 780 (commercial source: eBioscience; 65-0865-18) was diluted 10,000 times in PBS; into 100 μL of the diluted Fixable Viability Dye eFluor 780 in PBS were respectively added 1 μL of TCR-αβ-APC (commercial source: eBioscience; 17-9986-42), 1 μL of BB515 Mouse Anti-Hu CD8 (commercial source: BD Pharmingen; 564526), and 1 μL of PE-Cy7 Mouse Anti-Hu CD4 (Commercial source: BD Pharmingen; 557852), and mixed well.

Into the TIL cell samples from each test group was added 100 μL of the above mixed antibodies for flow cytometry test, mixed well and incubated on ice for 30 minutes. After the incubation, centrifugation was performed at 500 g for 3 minutes, and the supernatant was aspirated and discarded after the centrifugation. Then 200 μL of PBS was added for resuspension. A flow cytometer was used for test, and the knockdown efficiency of TCRβ was analyzed by using Flowjo software.

FIG. 24 shows the results of gene knockout efficiency of TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved gene knockout efficiency compared with that in the control group (without the addition of CD28 antibodies). For example, an enhanced TCRβ gene knockout efficiency.

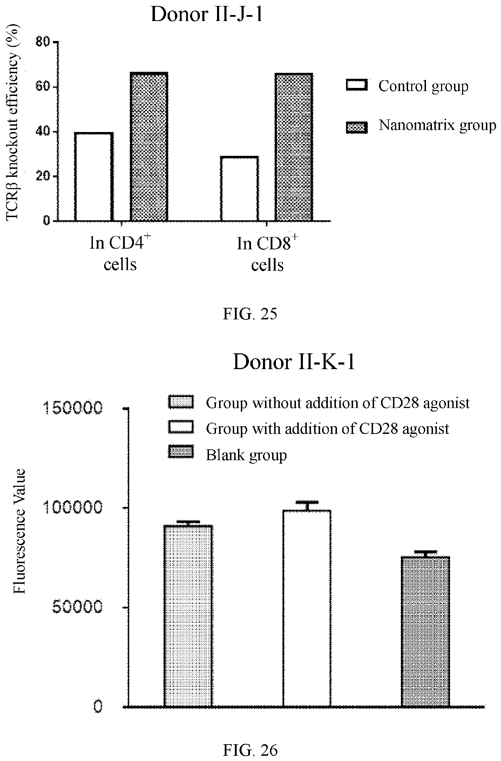

FIG. 25 shows the results of gene knockout efficiency of TIL cells obtained from culture in the nanomatrix group and the control group. The results show that, the TILs obtained from the *in vitro* expansion of step (B) in the four-step method with the addition of CD28 antibodies (for example, adding transACT comprising CD3 antibodies and CD28 antibodies) have an improved gene knockout efficiency compared with that in the control group (without the addition of CD28 antibodies). For example, an enhanced TCRβ gene knockout efficiency.

**Example 10 Proliferation ability assay of TILs with the CD28 agonist added after the end of REP stage**

A first TIL population derived from tumor tissues and not expanded *in vitro* was obtained by reference to Example 1. The first TIL population was subjected to steps (A), (B), (C), and (D) of the four-step method in the same way to obtain a fifth TIL population. The fifth TIL population was randomly divided into 3 groups, and IL-2 was added into the T cell culture medium of each test group, while the blank group was not added any T cell activators, and the group without a CD28 agonist added was added a CD3 antibody (Miltenyi Biotech, OKT3) at about 30 ng/ mL, and the group with a CD28 agonist added was added a CD3 agonist and a CD28 agonist, for example, transACT was added at a ratio of transACT to TILs of about 1:100 to 1:2000. The TILs (terminal stimulated cell population) obtained after 3 days of culture were tested for the proliferation ability of TIL cells by a cell viability test method using the CellTiter-Glo kit (commercial source: Promega).

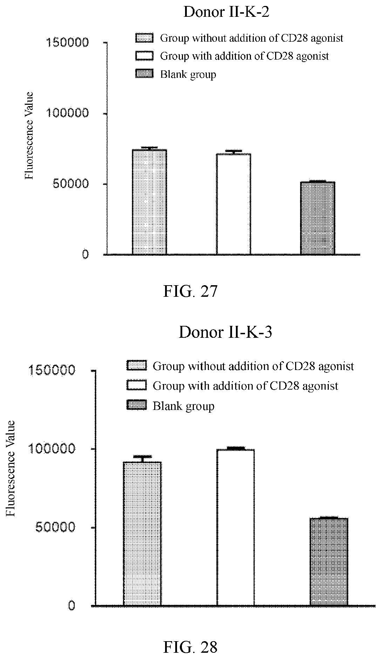

FIGs. 26, 27 and 28 show the analysis results of the proliferation ability of the test groups upon *in vitro* expansion in different ways in the terminal stimulation stage, respectively, for TILs from different donor sources. The fluorescence values on the vertical coordinates of the graphs reflect the proliferation ability of TIL cells subjected to terminal stimulation in different ways in each test group. The results show that the addition of a CD28 agonist during the terminal stimulation resulted in a similar proliferation ability of TILs compared to that resulted from the terminal stimulation without the addition of a CD28 agonist.

**Example 11 Knockout efficiency assay of TIL cells**

Reagents and materials: DNA extraction solution (QuickExtract DNA extraction solution, Lucigen, QE09050), nuclease-free water (RNase/DNase free water, Tiangen), EDTA (Sangon, 0.5M), Recombinant DNase I (RNase-free, TAKARA).

Extraction of genome DNA: After approximately 4 days following TIL cell knockout, about $1 \times 10^5$ to $2 \times 10^5$ cells were washed once with PBS, and TIL cells were resuspended with 44 $\mu$L of PBS and incubated in 6 $\mu$L of the formulated mixture of nucleases (containing 1 $\mu$L of DNase I and 5 $\mu$L of 10$\times$ DNase I Buffer) at 37°C for 5 minutes. 2.5 $\mu$L of 0.5M EDTA was added into the samples and incubated at 80°C for 10 minutes. After centrifugation, the supernatant was discarded, 50 $\mu$L of DNA extraction solution was added into the cell pellet and centrifuged briefly, then the following program was run: at 75°C for 10 min; at 95°C for 5 min; maintainance at 4°C. A spectrophotometer (NanoDrop™) was used to detect the concentration of DNA samples.

Sequencing: PCR primers can be designed in the region of about 100 to about 200 nucleotides upstream and downstream of the PAM site. A PCR reaction system was designed as below:

| Reagents | Volume |
|---|---|
| 2$\times$PCR Premix Buffer | 25 $\mu$L |
| DNA template | About 100-500 ng |
| Forward primer (10 $\mu$M) | 1 $\mu$L |
| Reverse primer (10 $\mu$M) | 1 $\mu$L |
| Double distilled water | to 50 $\mu$L |
| DMSO | 1.5 $\mu$L |

and amplification was performed according to the following PCR program:

| Temperature | Time | |
|---|---|---|
| 95°C | 4 min | |
| 95°C | 15 sec | |
| 58°C | 30 sec | |
| 72°C | 30 sec | Repeat the 3 steps for 35 cycles |
| 72°C | 7 min | |
| 4°C | maintenance | |

and the PCR products were analyzed by Sanger sequencing.

Analysis of Crispr Cas9 knockout efficiency

By using the Tracking of Indels by Decomposition (Tide) method, the Crispr Cas9 knockout efficiency was analyzed based on the Sager sequencing data. For specific methods, see (Brinkman et al, Nucl. Acids Res. (2014) or shinyapps.datacurators.nl/tide/). Knockout efficiency analysis was performed by inputting the corresponding sgRNA sequence of the present application, the pre-knockout control sequence, and the test sequence after Crispr Cas9 knockout, with the P-value threshold set to 0.001.

Different TILs may be derived from different tumor patients, wherein donors 504 and 607 are patients with ovarian cancer, donor 713 is a patient with cervical cancer, and donor 316 is a patient with cutaneous malignant melanoma.

The sgRNA as set forth in SEQ ID NO: 45 (TTCCGCAAAATAGAGCCCCA) is CB1, the sgRNA as set forth in SEQ ID NO: 51 (CAGCTCCCTCTAGTCCCGCG) is ZC3, the sgRNA as set forth in SEQ ID NO: 37 (GGGCCCCCAGTAGAATCCGC) is SC3, the sgRNA as set forth in SEQ ID NO: 47 (TGTGGGATGTCGACTCCTAG) is CB2, the sgRNA as set forth in SEQ ID NO: 41 (TATGCCATGAGTGCTCAGAG) is TP4, and the sgRNA as set forth in SEQ ID NO: 53 (GGTTCAGACCAGTACTCTCG) is ZC7.

The results show that, when CB1 and ZC3 are administered in combination, the knockout efficiencies of CB1 and ZC3 for donor 504 are about 21.8% and 29.2%, respectively, and the knockout efficiencies of CB1 and ZC3 for donor 713 are about 96.6% and 95.4%, respectively.

The results show that, when SC3 and CB1 are administered in combination, the knockout efficiencies of SC3 and CB1 for donor 504 are about 15.7% and 19.5%, respectively, and the knockout efficiencies of SC3 and CB1 for donor 713 are about 89.8% and 97.8%, respectively.

The results show that, when SC3 and CB2 are administered in combination, the knockout efficiencies of SC3 and CB2 for donor 713 are about 81.8% and 92.6%, respectively, and the knockout efficiencies of SC3 and CB2 for donor 316 are about 35% and 42.4%, respectively.

The results show that, when SC3 and TP4 are administered in combination, the knockout efficiencies of SC3 and TP4 for donor 607 are about 97.9% and 98.2%, respectively, the knockout efficiencies of SC3 and TP4 for donor 504 are about 17.5% and 24.5%, respectively, the knockout efficiencies of SC3 and TP4 for donor 713 are about 88.1% and 97.6%, respectively, and the knockout efficiencies of SC3 and TP4 for donor 316 are about 29.3% and 72.7%, respectively.

The results show that, when SC3 and ZC3 are administered in combination, the knockout efficiencies of SC3 and ZC3 for donor 504 are about 20.2% and 24.9%, respectively, and the knockout efficiencies of SC3 and ZC3 for donor 713 are about 86.3% and 93.5%, respectively.

The results show that, when SC3 and ZC7 are administered in combination, the knockout efficiencies of SC3 and ZC7 for donor 713 are about 83.1% and 88.2%, respectively, and the knockout efficiencies of SC3 and ZC7 for donor 316 are about 4.8% and 54.7%, respectively.

The results show that, when TP4 and CB1 are administered in combination, the knockout efficiencies of TP4 and CB1 for donor 607 are about 98.7% and 98.7%, respectively, the knockout efficiencies of TP4 and CB1 for donor 504 are about 17.3% and 16.2%, respectively, and the knockout efficiencies of TP4 and CB1 for donor 713 are about 95.3% and 97.2%, respectively.

The results show that, when TP4 and ZC3 are administered in combination, the knockout efficiencies of TP4 and ZC3 for donor 607 are about 98.4% and 98.1%, respectively, the knockout efficiencies of TP4 and ZC3 for donor 504 are about 23.4% and 16.2%, respectively, and the knockout efficiencies of TP4 and ZC3 for donor 713 are about 95.6% and 97.2%, respectively.

The results show that, when TP4 and ZC7 are administered in combination, the knockout efficiencies of TP4 and ZC7 for donor 713 are about 97.1% and 88.7%, respectively, and the knockout efficiencies of TP4 and ZC7 for donor 316 are about 85.4% and 54.8%, respectively.

The various combinations of gene editing modes in the present application can achieve a certain level of knockout efficiency.

**Example 12 Expansion assay of TIL cells**

Starting from day 7 after gene editing, each group of TIL cells were re-plated with the same total number of cells. 3 days later, the expansion efficiency of TIL cells was analyzed using the CTG kit (CellTiter-Glo Luminescent Cell Viability Assay, Promega).

Alternatively, starting from day 6 after gene editing, a 96-well plate was pretreated with 30 ng/mL of CD3 antibody (Miltenyi Biotech, OKT3) and incubated at 4°C overnight. The next day, each group of TIL cells were re-plated with the same total number of cells. 3 days later, the expansion efficiency of TIL cells was analyzed using the CTG kit (CellTiter-Glo Luminescent Cell Viability Assay, Promega).

CB and ZC-combined gene editing

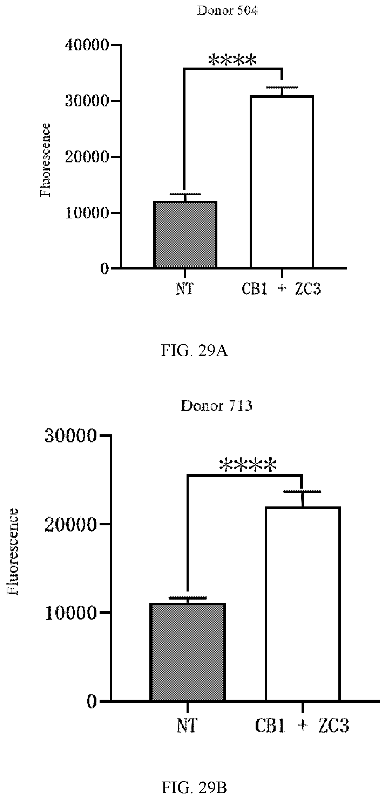

FIGs. 29A to 29B show the fluorescence after expansion of each group of TIL cells edited with combined genes CB and ZC in the absence of any stimulants, for TIL cells derived from donors 504 and 713.

SC and CB-combined gene editing

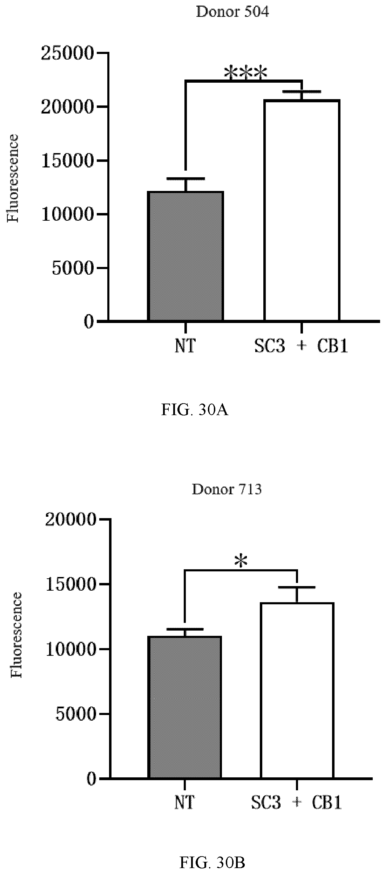

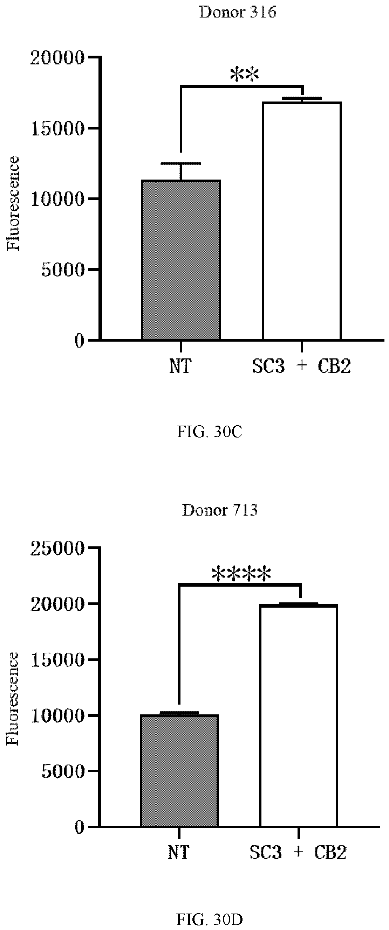

FIGs. 30A to 30D show the fluorescence after expansion of each group of TIL cells edited with combined genes SC and CB in the absence of any stimulants, for TIL cells derived from donors 504, 713, and 316.

SC and TP-combined gene editing

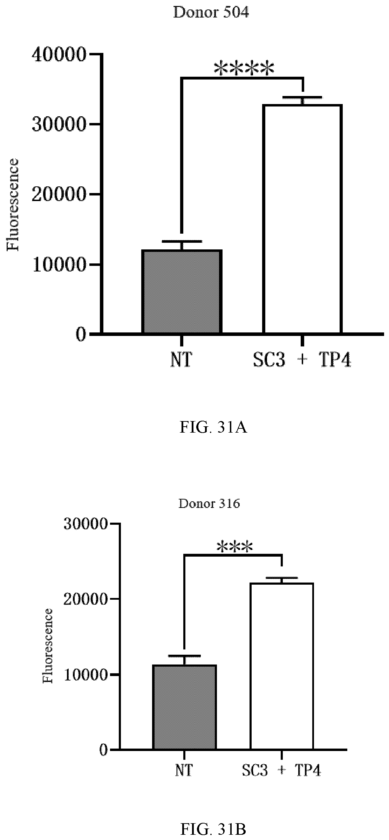

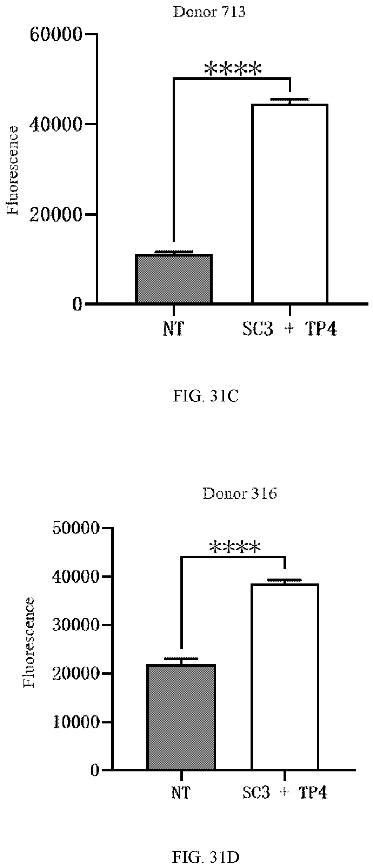

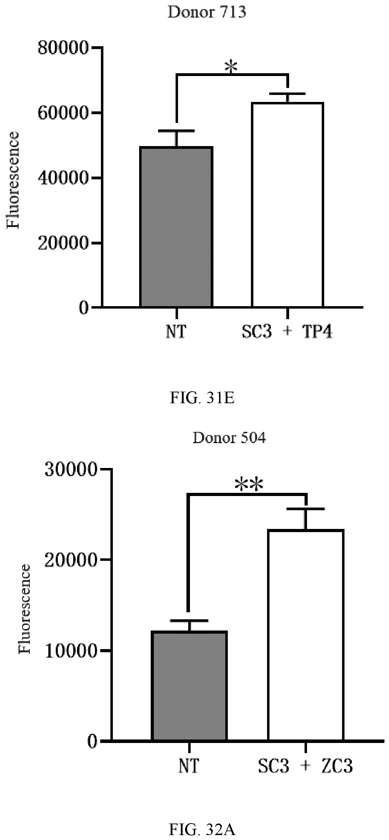

FIGs. 31A to 31C show the fluorescence after expansion of each group of TIL cells edited with combined genes SC and TP in the absence of any stimulants, for TIL cells derived from donors 504, 316, and 713.

FIGs. 31D to 31E show the fluorescence after expansion of each group of TIL cells edited with combined genes SC and TP under the stimulation of a CD3 antibody, for TIL cells derived from donors 316, and 713.

SC and ZC-combined gene editing

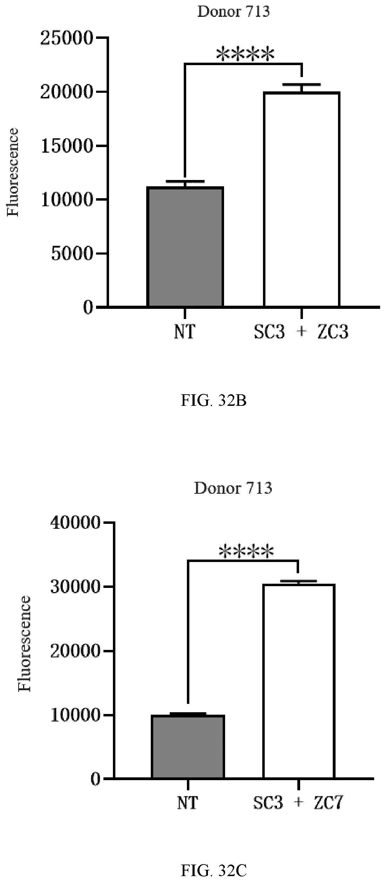

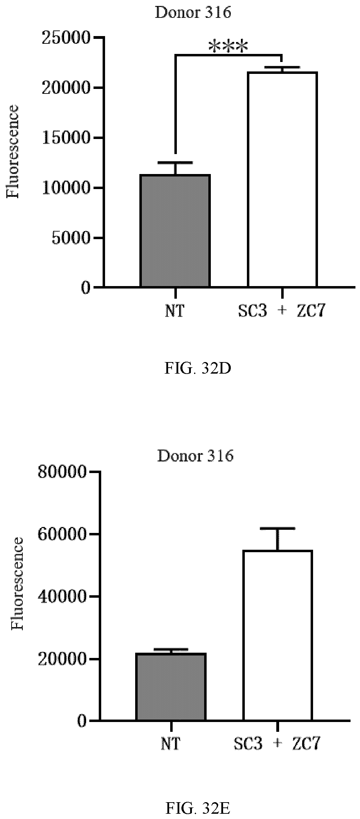

FIGs. 32A to 32D show the fluorescence after expansion of each group of TIL cells edited with combined genes SC and ZC in the absence of any stimulants, for TIL cells derived from donors 504, 316, and 713.

FIG. 32E shows the fluorescence after expansion of each group of TIL cells edited with combined genes SC and ZC under the stimulation of the CD3 antibody, for TIL cells derived from donor 316.

TP and CB-combined gene editing

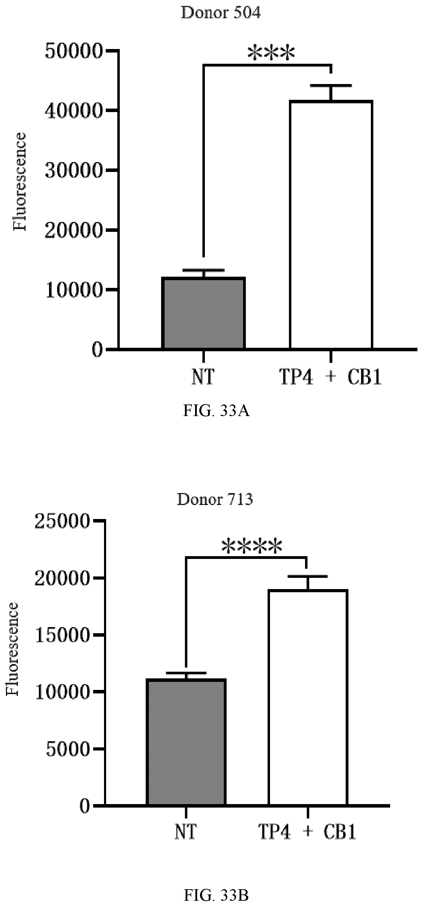

FIGs. 33A to 33B show the fluorescence after expansion of each group of TIL cells edited with combined genes TP and CB in the absence of any stimulants, for TIL cells derived from donors 504 and 713.

TP and ZC-combined gene editing

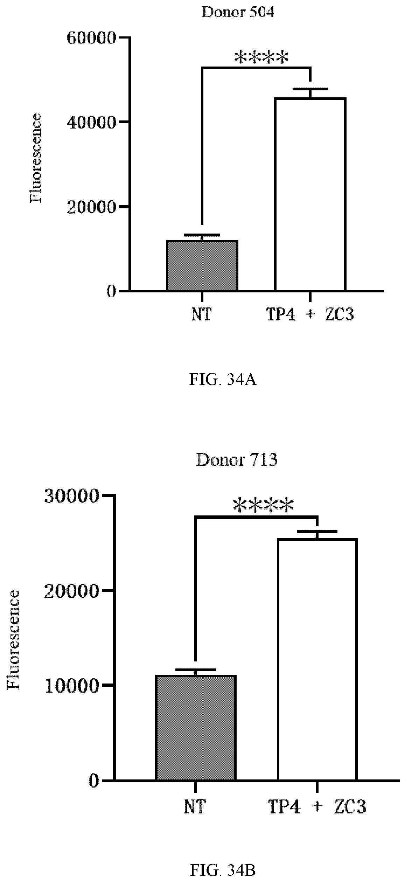

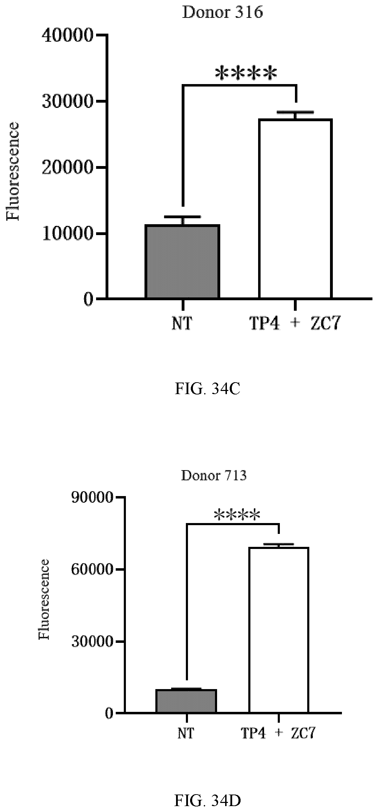

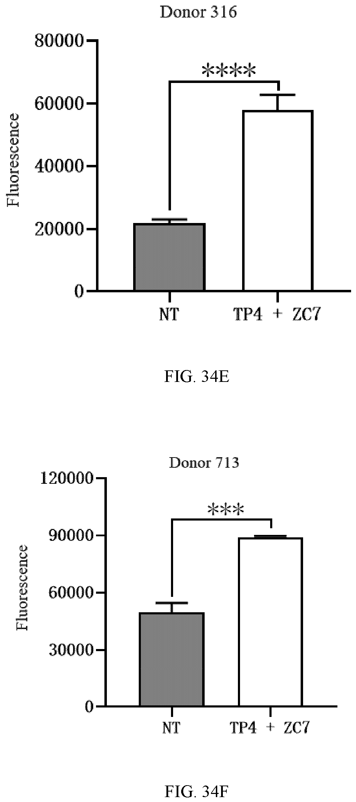

FIGs. 34A to 34D show the fluorescence after expansion of each group of TIL cells edited with combined genes TP and ZC in the absence of any stimulants, for TIL cells derived from donors 504, 316, and 713.

FIGs. 34E to 34F show the fluorescence after expansion of each group of TIL cells edited with combined genes TP and ZC under the stimulation of the CD3 antibody, for TIL cells derived from donors 316 and 713.

The results show that, the combined gene-edited TIL cells in the present application may exhibit significant expansion capacity.

**Example 13 Assay of killing ability of TIL cells**

Starting from day 6 after gene editing, Hey-T30 or A375 tumor cell lines were plated in a 96-well plate. The next day, each group of TIL cells were co-cultured with the above Hey-T30 or A375 cells at an effector-target ratio (TIL cells: tumor cells, E:T) of 1:1 or 1:3. Each 100 $\mu$L of tumor cells and TIL cells were added, and three replicate wells were set for each group. At the same time, a control group comprising only the tumor cells was set.

According to the instruction of apoptosis detection reagent (Incucyte Caspase-3/7 Green Dye for Apoptosis, Sartorius), the apoptosis detection reagent was added at 0.2 $\mu$L/well, and the culture medium was added at 25 $\mu$L/well to dilute the Caspase 3/7 Green Dye. The activity of Caspase 3/7 was recorded using an Incucyte recorder (Sartorius) to analyze the killing ability of TILs against tumor cells, once every 3 hours and for a total record period of about 5 days.

CB and ZC-combined gene editing

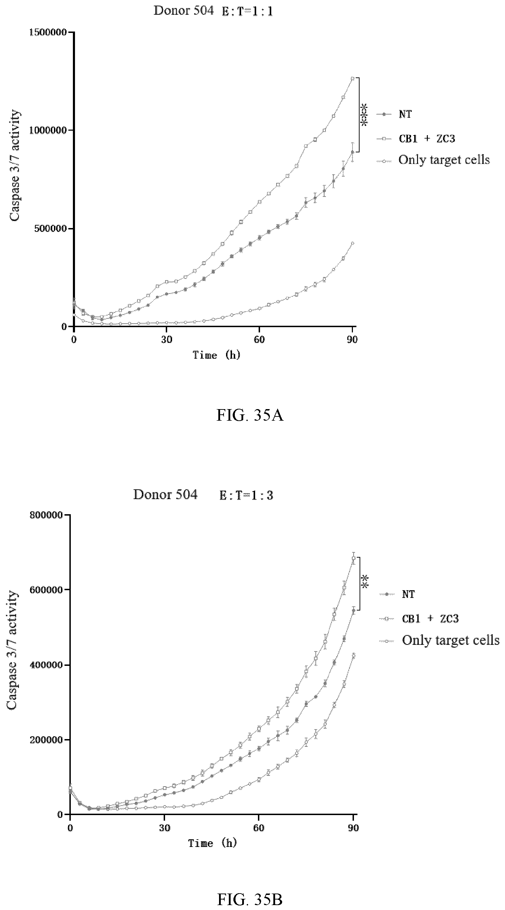

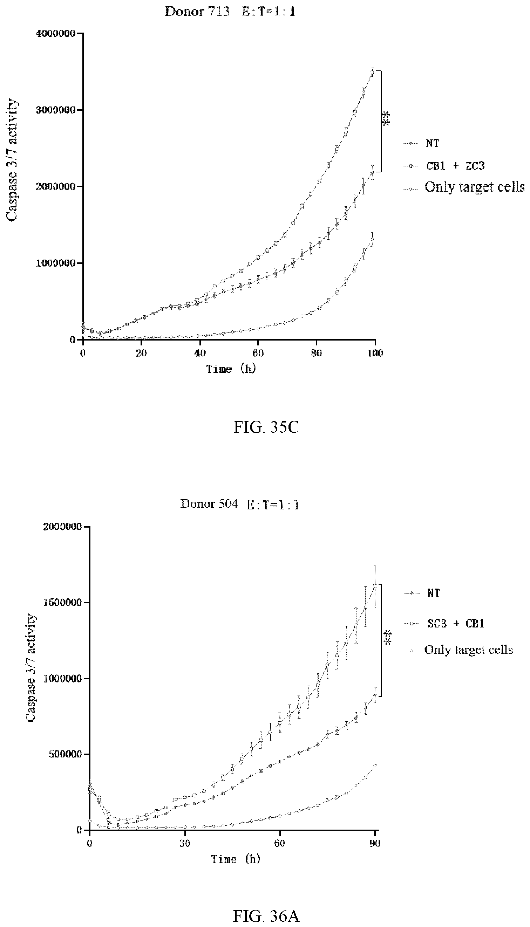

FIGs. 35A to 35C show the test results of the killing ability of TIL cells derived from donors 504 and 713 which were co-cultured with Hey-T30 tumor cells at an effector-target ratio of 1:1 and 1:3.

SC and CB-combined gene editing

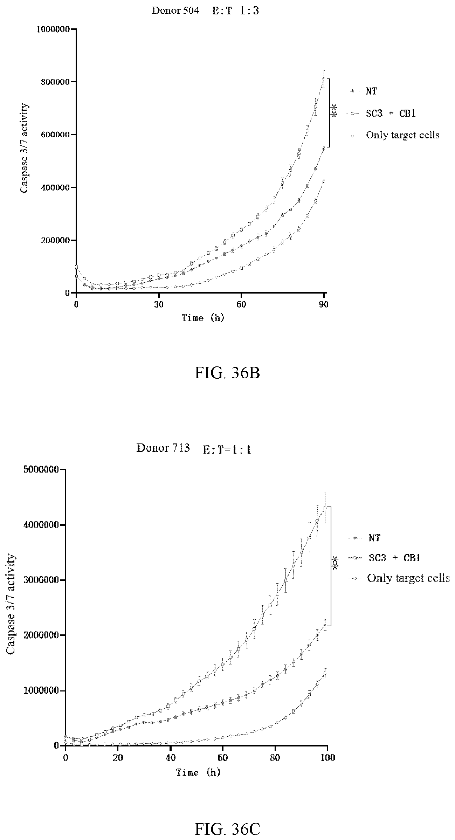

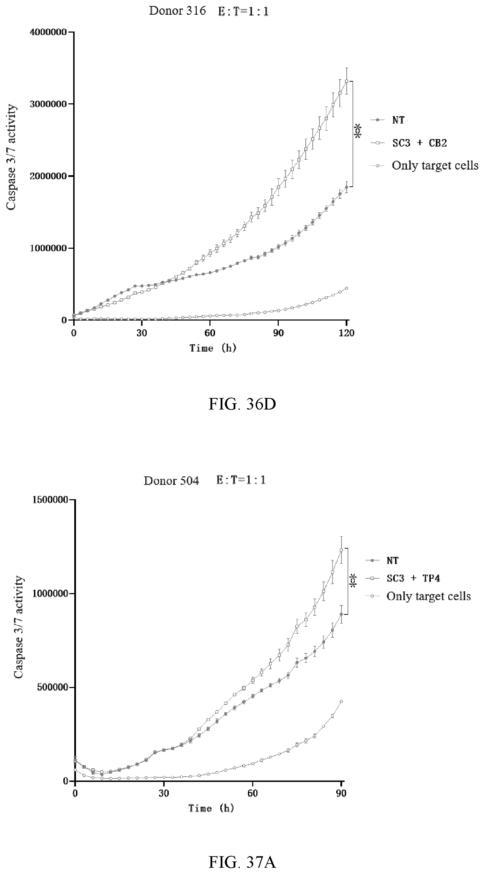

FIGs. 36A to 36C show the test results of the killing ability of TIL cells derived from donors 504 and 713 which were co-cultured with Hey-T30 tumor cells at an effector-target ratio of 1:1 and 1:3. FIG. 36D shows the test results of the killing ability of TIL cells derived from donor 316 which were co-cultured with A375 tumor cells at an effector-target ratio of 1:1.

SC and TP-combined gene editing

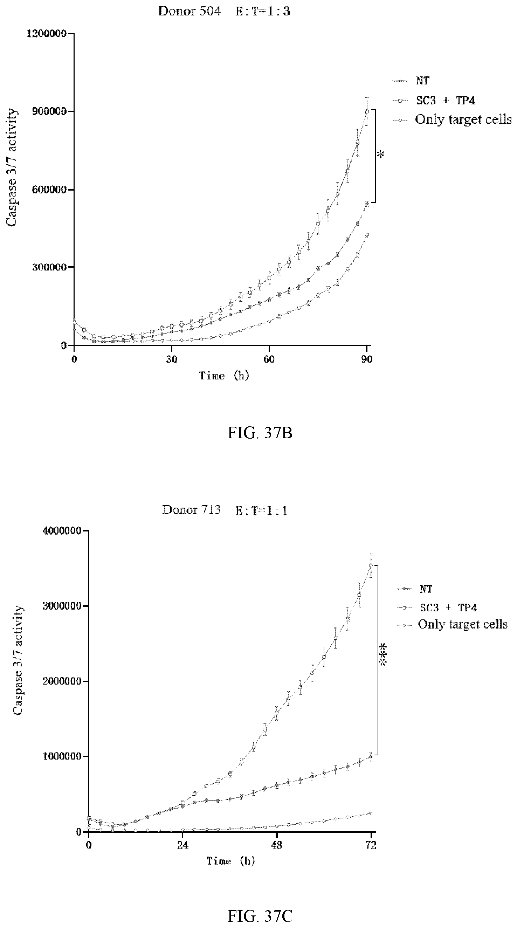

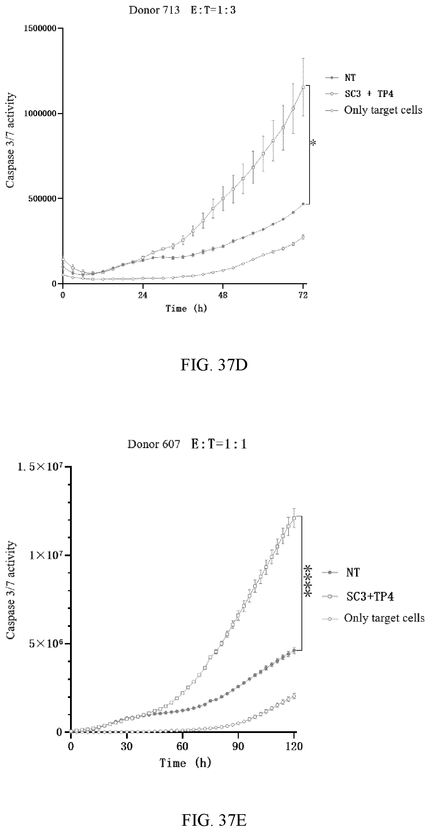

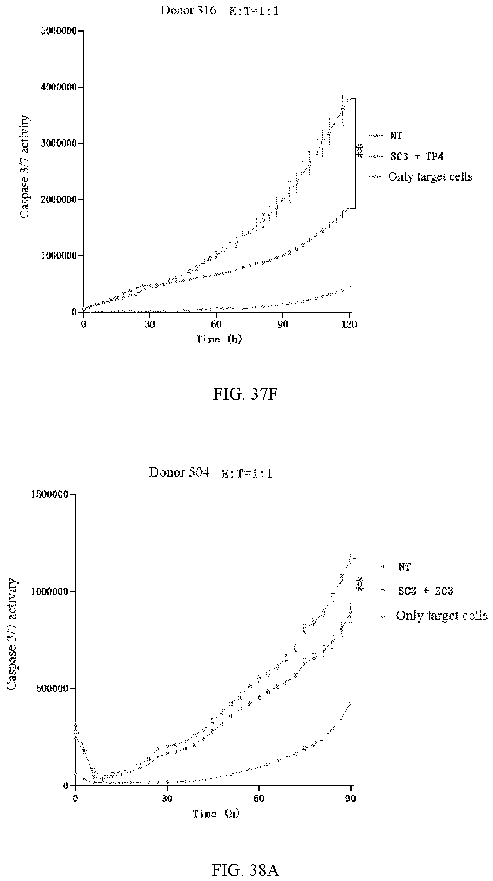

FIGs. 37A to 37E show the test results of the killing ability of TIL cells derived from donors 504, 713, and 607 which were co-cultured with Hey-T30 tumor cells at an effector-target ratio of 1:1 and 1:3. FIG. 37F shows the test results of the killing ability of TIL cells derived from donor 316 which were co-cultured with A375 tumor cells at an effector-target ratio of 1:1.

SC and ZC-combined gene editing

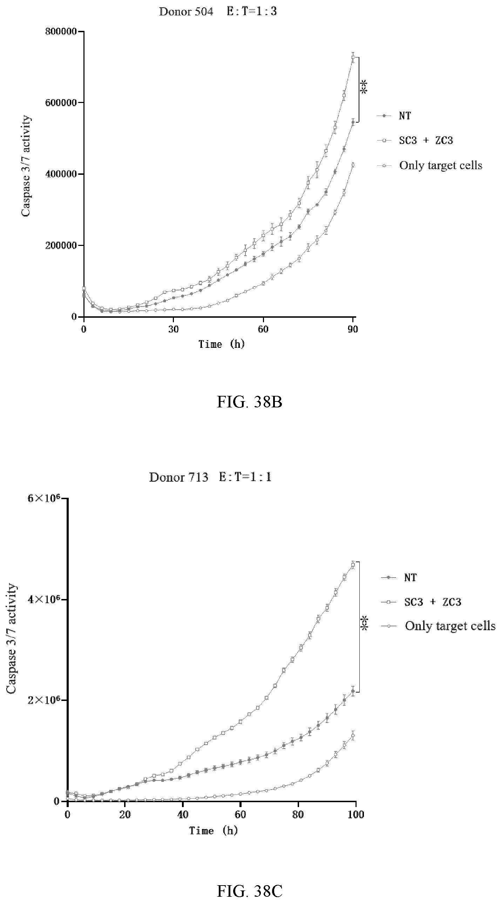

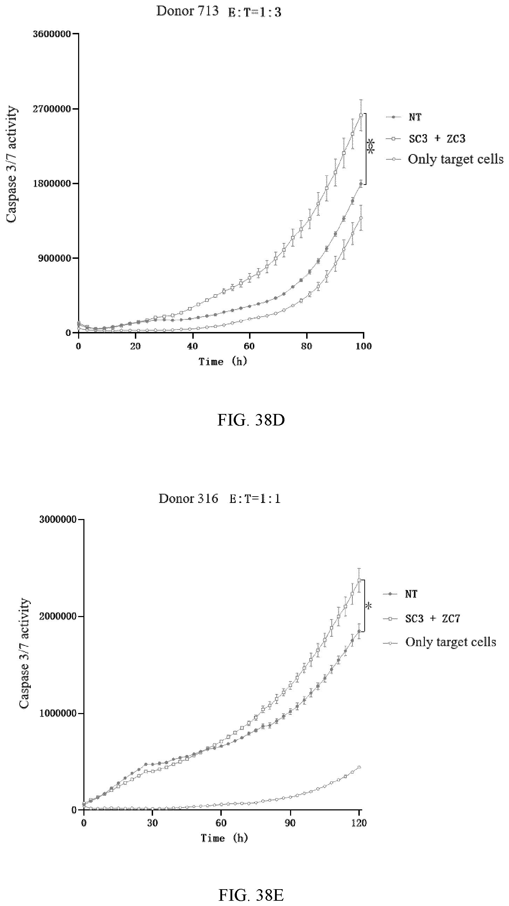

FIGs. 38A to 38D show the test results of the killing ability of TIL cells derived from donors 504 and 713 which were co-cultured with Hey-T30 tumor cells at an effector-target ratio of 1:1 and 1:3. FIG. 38E shows the test results of the killing ability of TIL cells derived from donor 316 which were co-cultured with A375 tumor cells at an effector-target ratio of 1:1.

TP and CB-combined gene editing

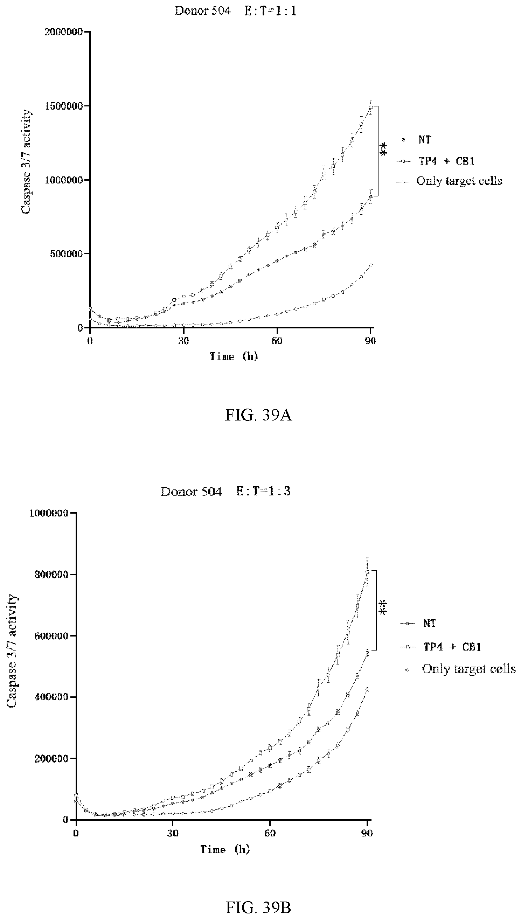

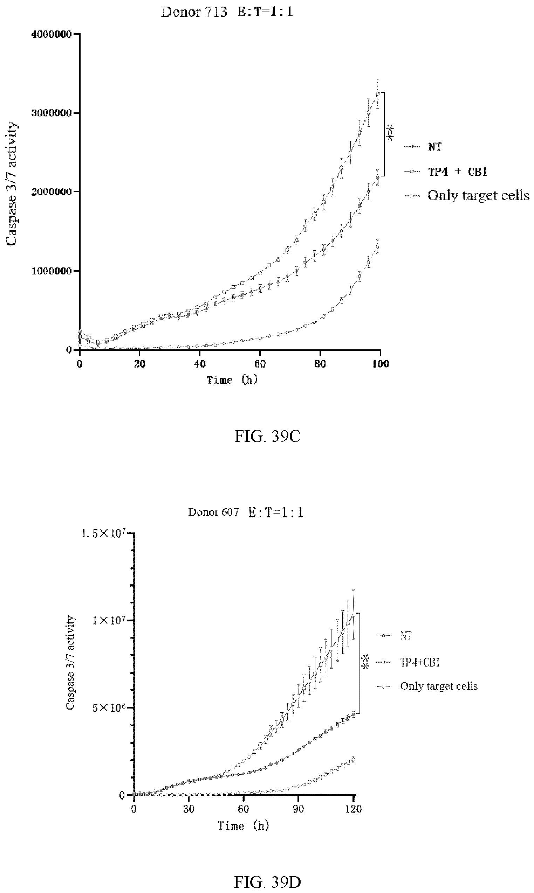

FIGs. 39A to 39D show the test results of the killing ability of TIL cells derived from donors 504, 713, and 607 which were co-cultured with Hey-T30 tumor cells at an effector-target ratio of 1:1 and 1:3.

TP and ZC-combined gene editing

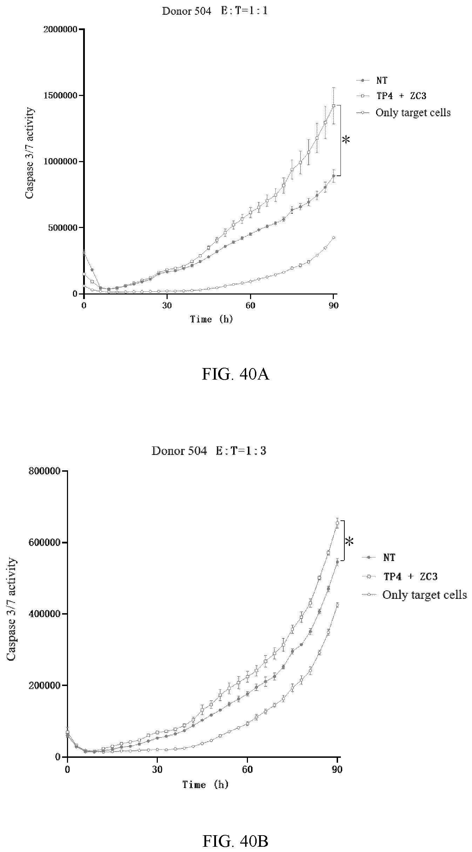

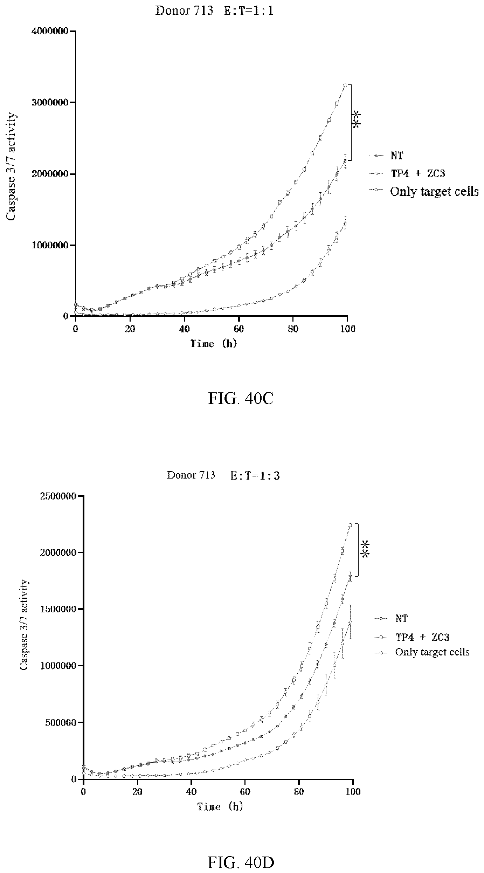

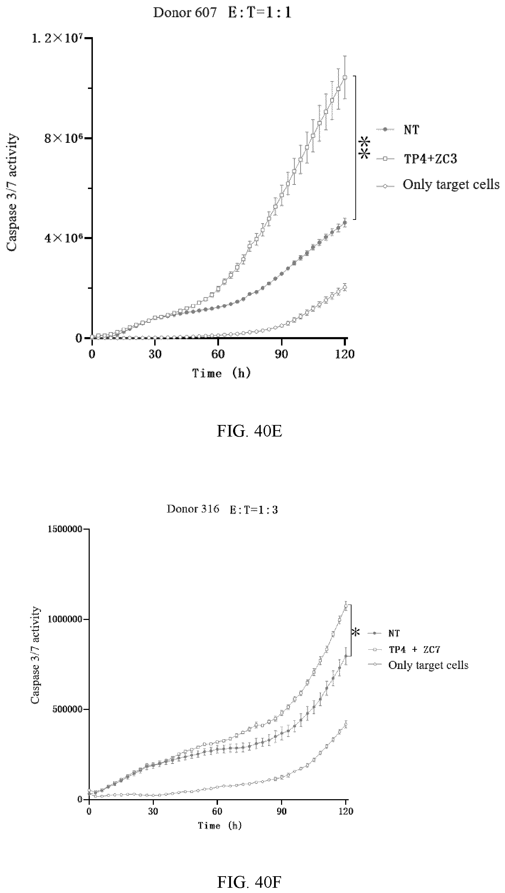

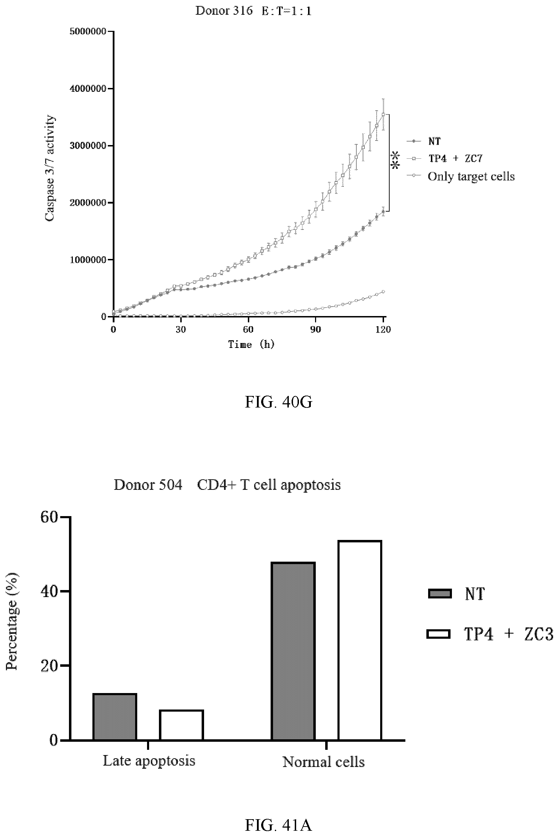

FIGs. 40A to 40E show the test results of the killing ability of TIL cells derived from donors 504, 713, and 607 which were co-cultured with Hey-T30 tumor cells at an effector-target ratio of 1:1 and 1:3. FIGs. 40F to 40G show the test results of the killing ability of TIL cells derived from donor 316 which were co-cultured with A375 tumor cells at an effector-target ratio of 1:1 and 1:3.

The results show that, the combined gene-edited TIL cells in the present application may exhibit more significant tumor cell killing ability.

## Example 14 Apoptosis assay of TIL cells

The TIL populations obtained on day 8 after gene editing for each test group in Example 1 were tested for apoptosis. The TILs obtained from the gene-knockout group or the control group (NT-no treatment) were tested for the T cell apoptosis level using an apoptosis kit (BD 559763 Annexin V PE Apoptosis kit).

TP and ZC-combined gene editing

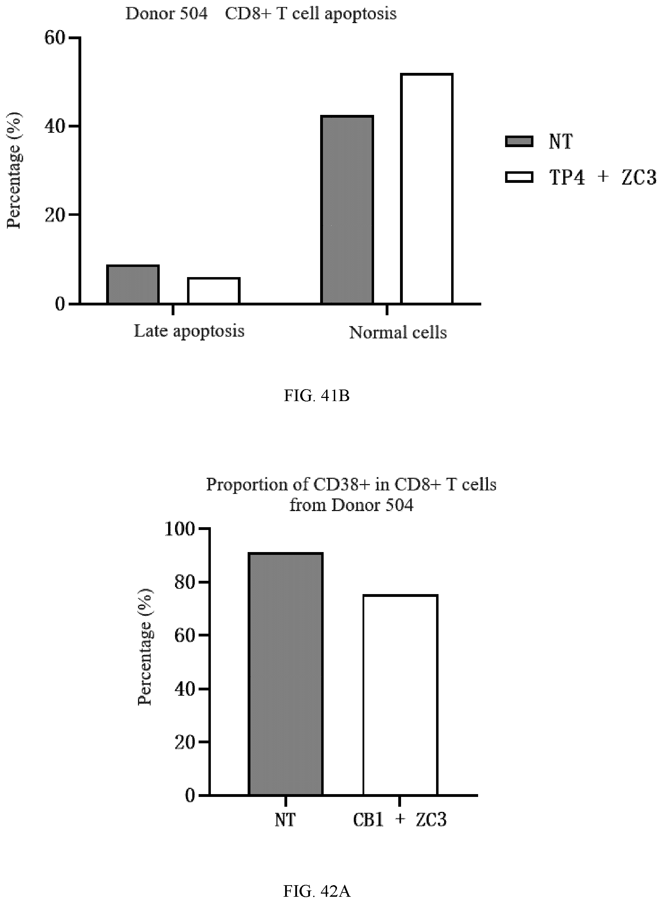

FIGs. 41A to 41B show the apoptosis assay results of TIL cells from donor 504. The results show that the gene-edited TIL cells may exhibit more significant anti-apoptotic ability.

## Example 15 Flow cytometry of TILs

The TIL populations obtained on day 8 after gene editing for each test group in Example 1 were tested by a flow cytometer.

Sources of test materials by TIL flow cytometry

V-bottom 96-well plate, manufacturer Corning, product number 3894; flow tube, manufacturer Corning, product number 352052.

The flow antibodies used in this example were purchased from BD or Biolegend. $1 \times 10^5$ to $5 \times 10^5$ cell samples from each group were added into flow tubes or V-bottom 96-well plates, and centrifuged at 600 g for 3 minutes, the supernatant was discarded. Washing was carried out once using PBS, at 1 mL/tube for flow tubes, and 200 $\mu$L/well for 96-well plates, and the supernatant was discarded. A prepared antibody working solution was added for cell surface staining. The concentration of the antibodies (BD or Biolegend) was 1: 100 to 1:200, and the active detection dye was comprised at 1: 10000. Staining was carried out at 100 $\mu$L/tube for flow tubes, and 50 $\mu$L/well for 96-well plates, and incubation was performed at 2-8°C in dark for 30 minutes. After surface staining, the cells were washed once with PBS (200 $\mu$L each time for 96-well plates, 1 mL each time for flow tubes), and centrifuged at 600 g at room temperature for 3 minutes. After the centrifugation, the supernatant was discarded. The cells were resuspended in 100-500 $\mu$L of PBS for flow cytometry test on machine.

For flow cytometry of intracellular factors, the reagents required for fixation and permeabilization was formulated: Transcription Factor Buffer Set (BD) was used to dilute a $4 \times$ Fixation/Permeabilization Solution (BD) to produce $1 \times$ working solution A; double distilled water was used to dilute a $5 \times$ Perm/Wash Buffer (BD) to produce $1 \times$ working solution B. The working solutions were pre-cooled at 4 °C for later use. After surface staining, an appropriate amount of PBS was added to wash the cells twice (200 $\mu$L each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100 $\mu$L/well for 96-well plates, and 1 mL/tube for flow tubes) of $1 \times$ working solution A was added for fixation and permeabilization. Incubation was performed at 2-8°C in dark for 40-50 minutes. At the end of fixation and permeabilization, $1 \times$ working solution B was added to wash the cells (200 $\mu$L each time for 96-well plates, and 2 mL each time for flow tubes). Centrifugation was performed at 2-8°C and at 600 g for 3 minutes, and washing was carried out twice. 1× working solution B was used to formulate intracellular antibodies with 50 μL/well for 96-well plates, and 100 μL/tube for flow tubes, and the antibody concentration was 1:100 to 1:200. Staining was performed at 2-8°C in dark for 30 minutes. At the end of staining, 1× working solution B was added to wash the cells (200 μL each time for 96-well plates, and 2 mL each time for flow tubes). Centrifugation was performed at 2-8°C and at 600 g for 3 minutes, and washing was carried out twice. The cells were resuspended in 100-500 μL of PBS for flow cytometry test on machine.

CB and ZC-combined gene editing

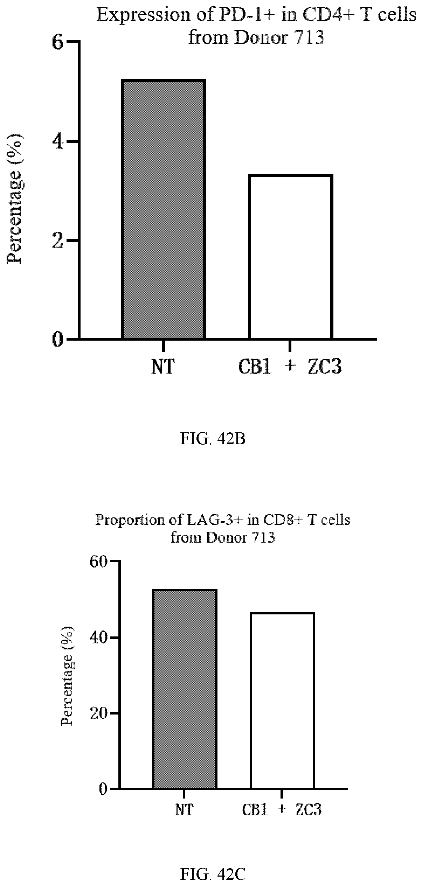

FIGs. 42A to 42C show the proportion of CD38-positive, PD-1-positive, LAG-3-positive cells in each group of TIL cells, for TIL cells from donors 504 and 713. The results show that the gene-edited TIL cells have a lower proportion of depletion cells.

SC and CB-combined gene editing

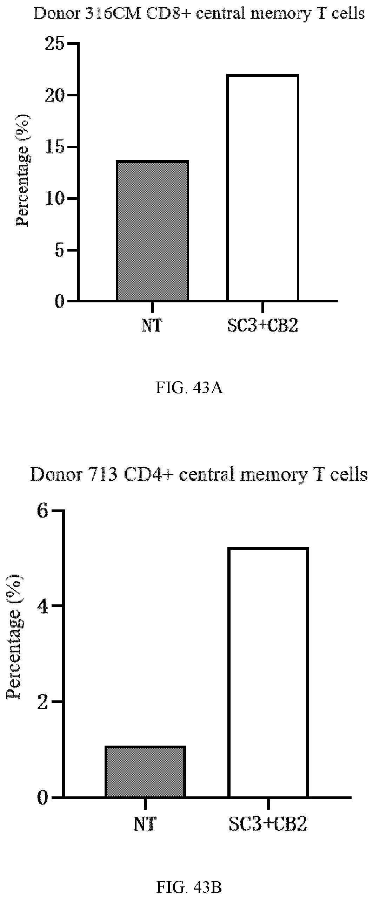

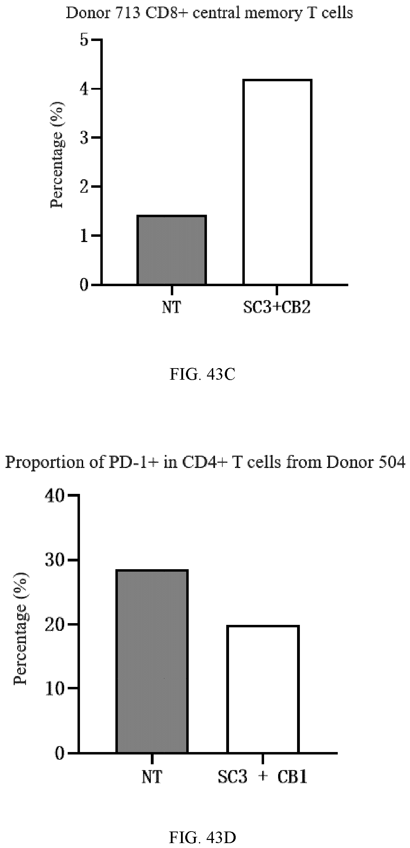

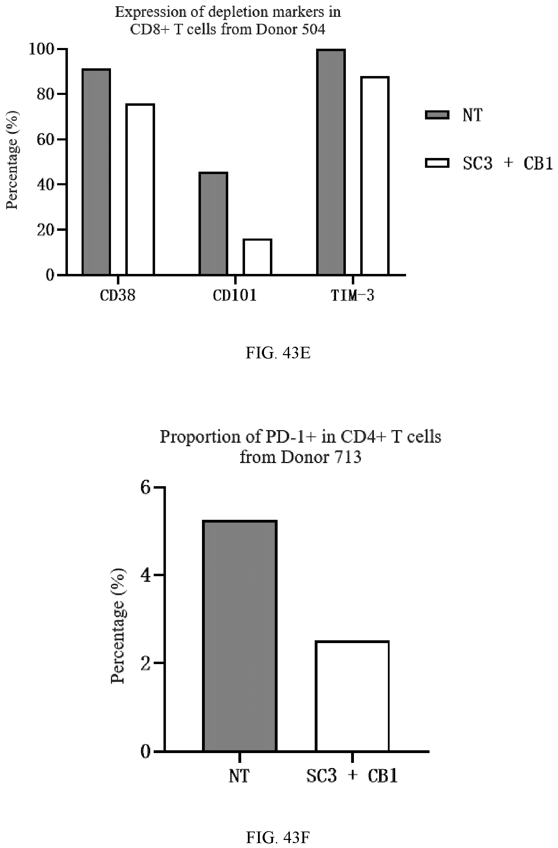

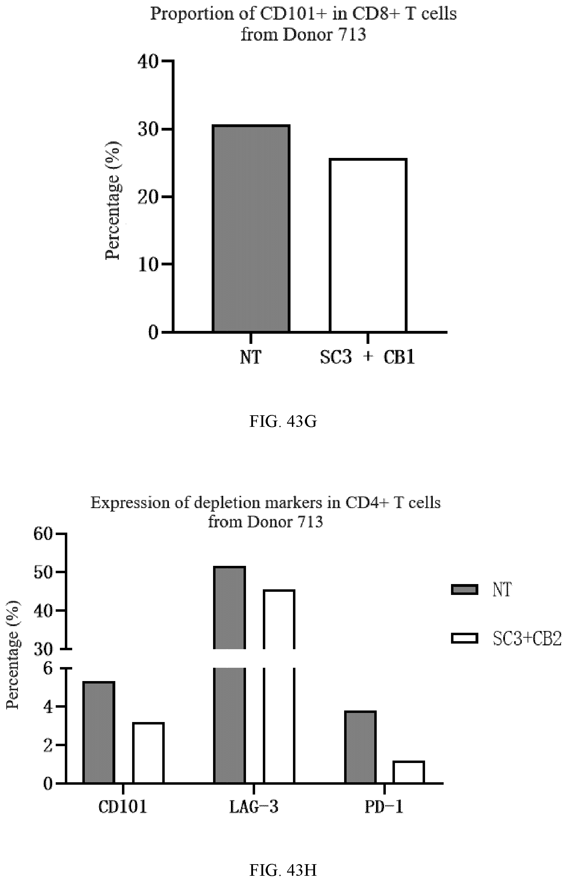

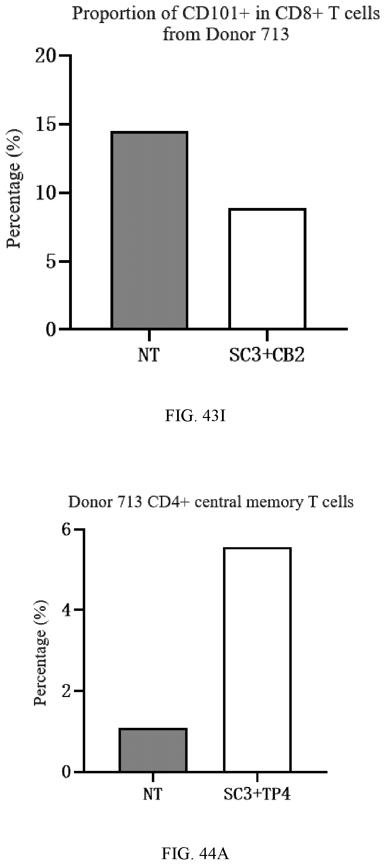

FIGs. 43A to 43C show the proportion of CD45RO-positive CD62L-positive central memory T cells (Tcm) in each group of TIL cells, for TIL cells from donors 316 and 713. The results show that the gene-edited TIL cells have a higher proportion of central memory T cells.
FIGs. 43D to 43I show the proportion of PD-1-positive, CD38-positive, CD101-positive, TIM-3-positive, and LAG-3-positive cells in each group of TIL cells, for TIL cells from donors 504 and 713. The results show that the gene-edited TIL cells have a lower proportion of depletion cells.

SC and TP-combined gene editing

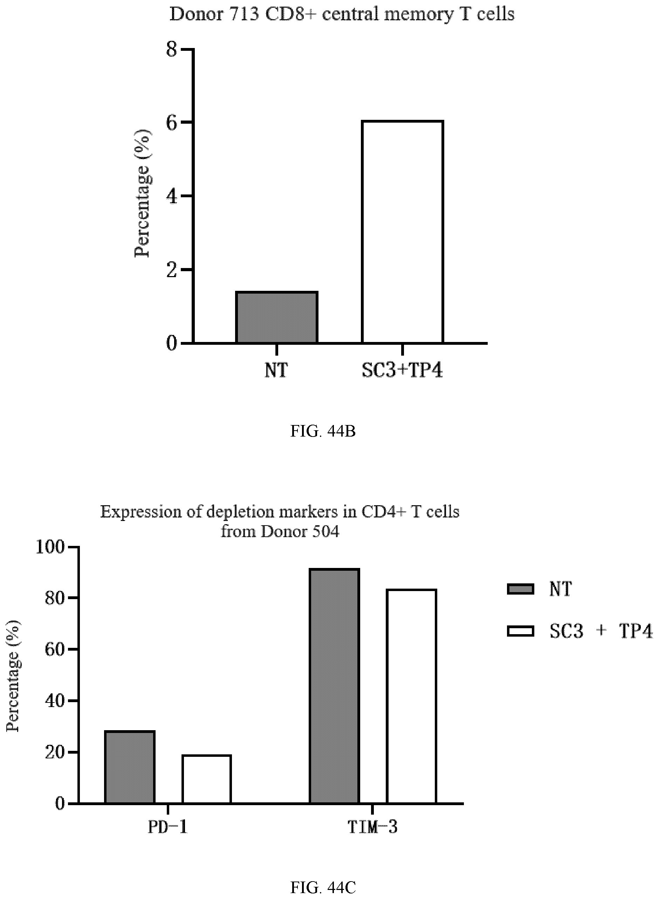

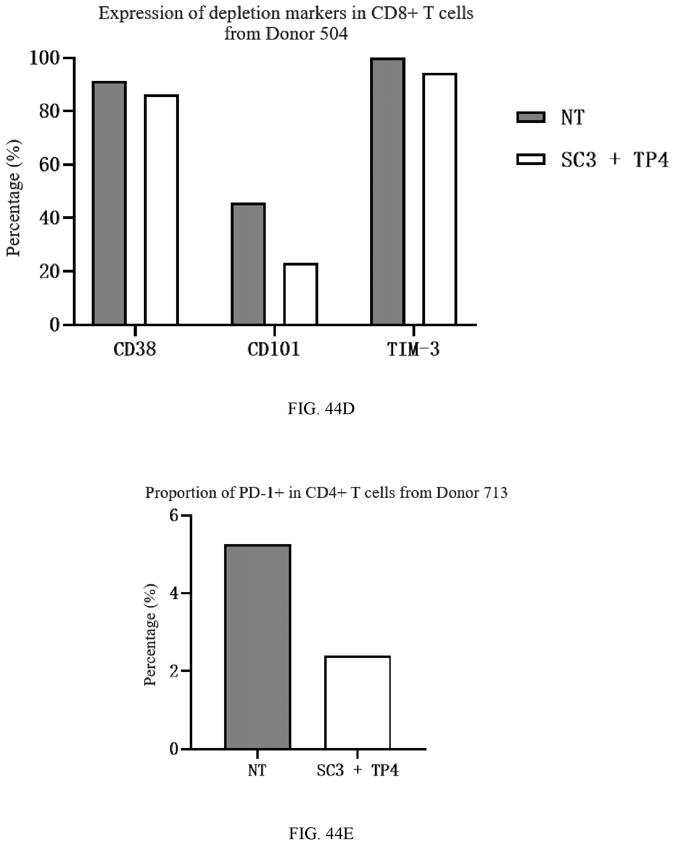

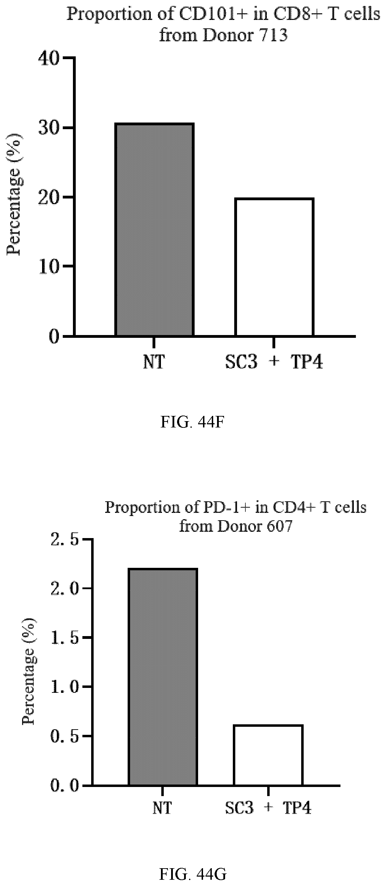

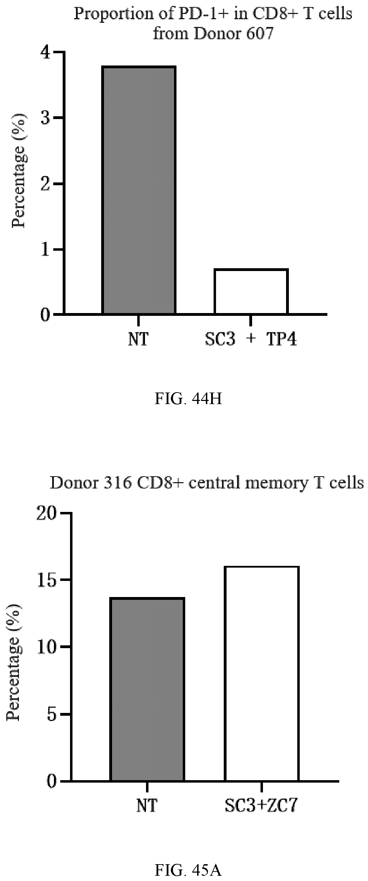

FIGs. 44A to 44B show the proportion of CD45RO-positive CD62L-positive central memory T cells (Tcm) in each group of TIL cells, for TIL cells from donor 713. The results show that the gene-edited TIL cells have a higher proportion of central memory T cells.
FIGs. 44C to 44H show the proportion of PD-1-positive, CD38-positive, CD101-positive, and TIM-3-positive cells in each group of TIL cells, for TIL cells from donors 504, 713, and 607. The results show that the gene-edited TIL cells have a lower proportion of depletion cells.

SC and ZC-combined gene editing

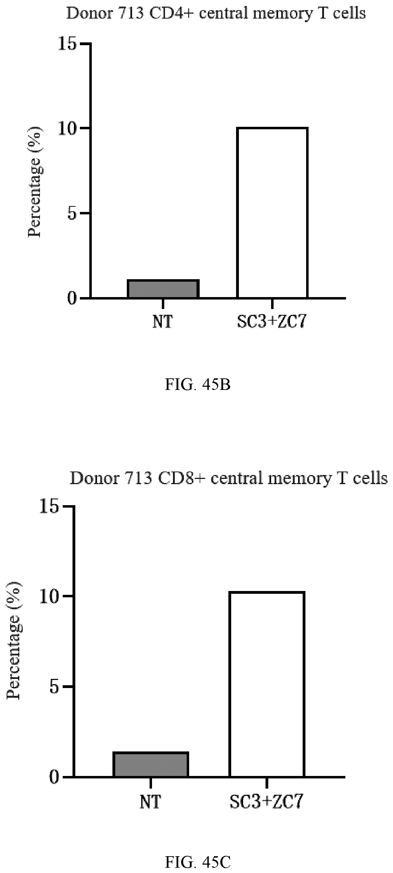

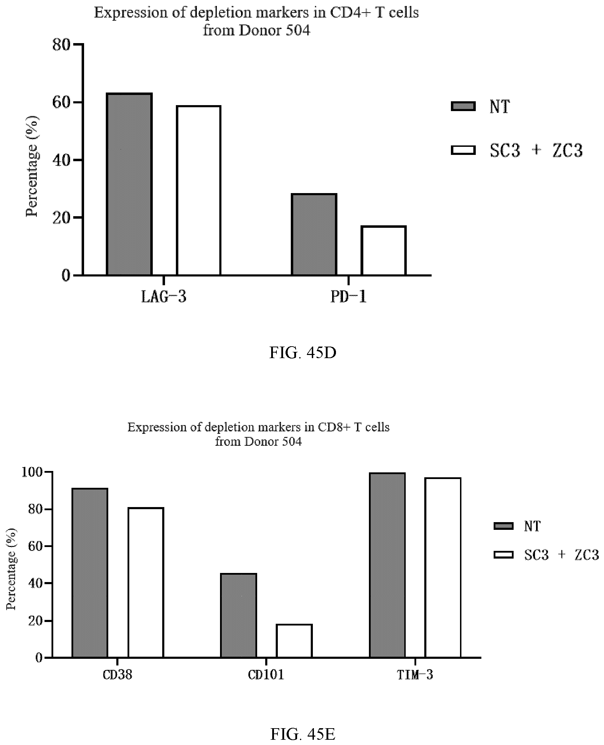

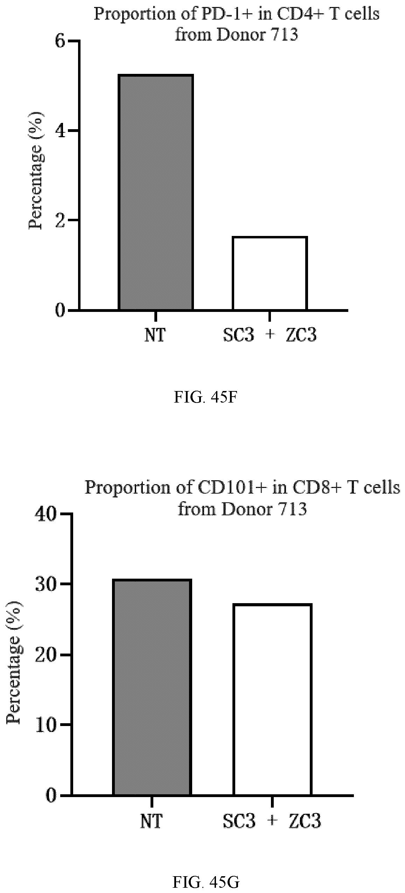

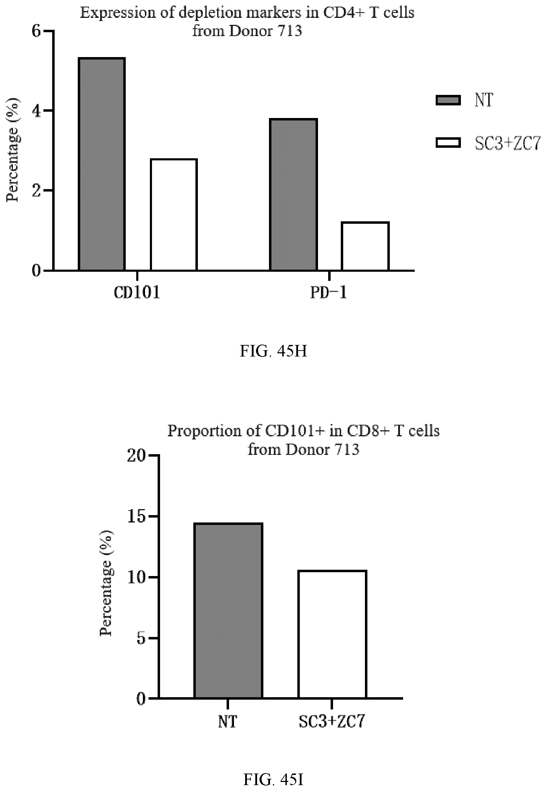

FIGs. 45A to 45C show the proportion of CD45RO-positive CD62L-positive central memory T cells (Tcm) in each group of TIL cells, for TIL cells from donors 316 and 713. The results show that the gene-edited TIL cells have a higher proportion of central memory T cells.
FIGs. 45D to 45I show the proportion of PD-1-positive, CD38-positive, CD101-positive, TIM-3-positive, and LAG-3-positive cells in each group of TIL cells, for TIL cells from donors 504 and 713. The results show that the gene-edited TIL cells have a lower proportion of depletion cells.

TP and CB-combined gene editing

FIGs. 46A to 46D show the proportion of CD38-positive, CD101-positive, TIM-3-positive, PD-1-positive, and LAG-3-positive cells in each group of TIL cells, for TIL cells from donors 504, 607, and 713. The results show that the gene-edited TIL cells have a lower proportion of depletion cells.

TP and ZC-combined gene editing

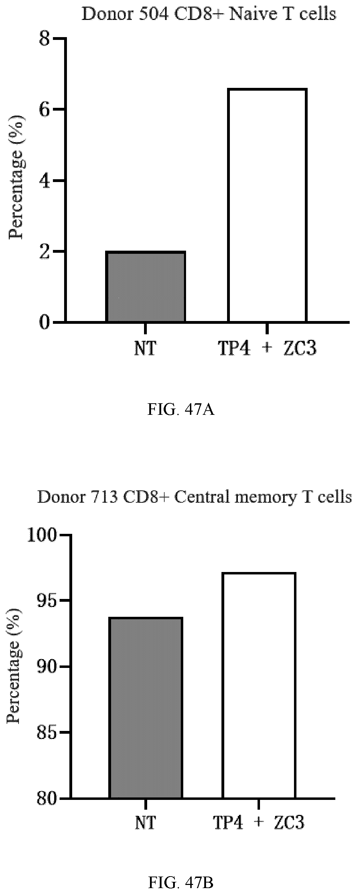

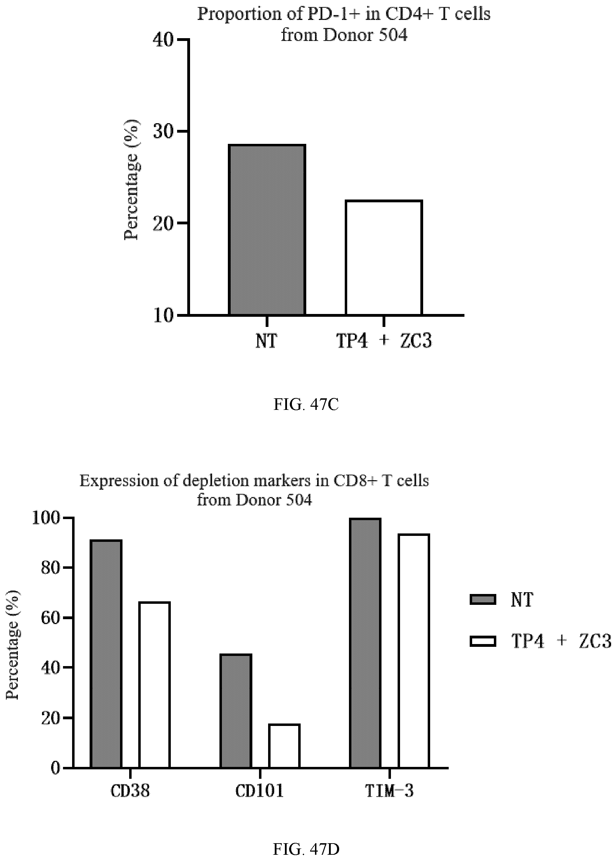

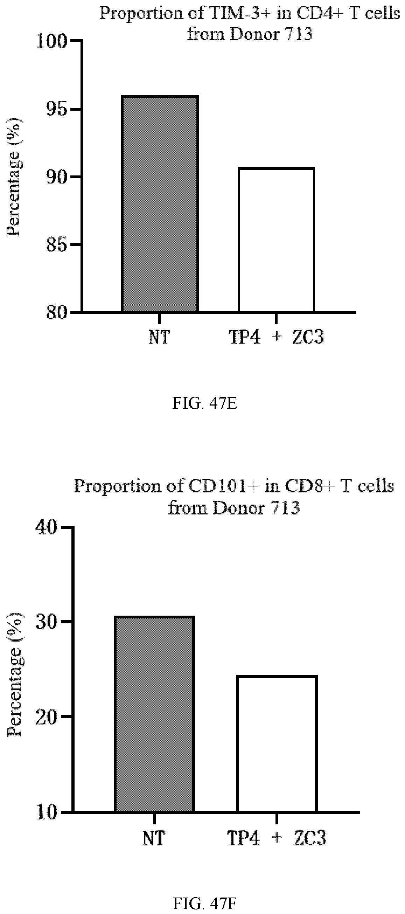

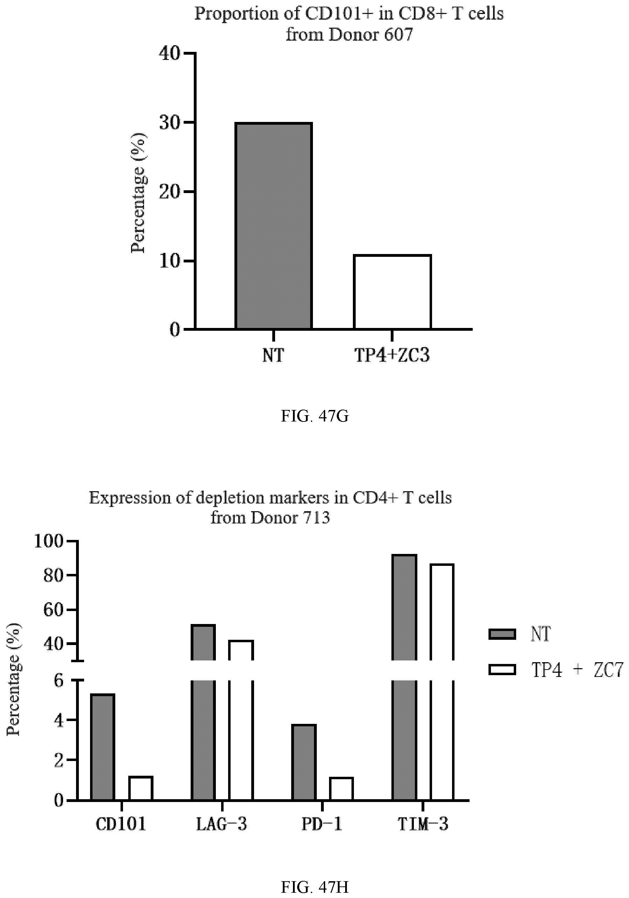

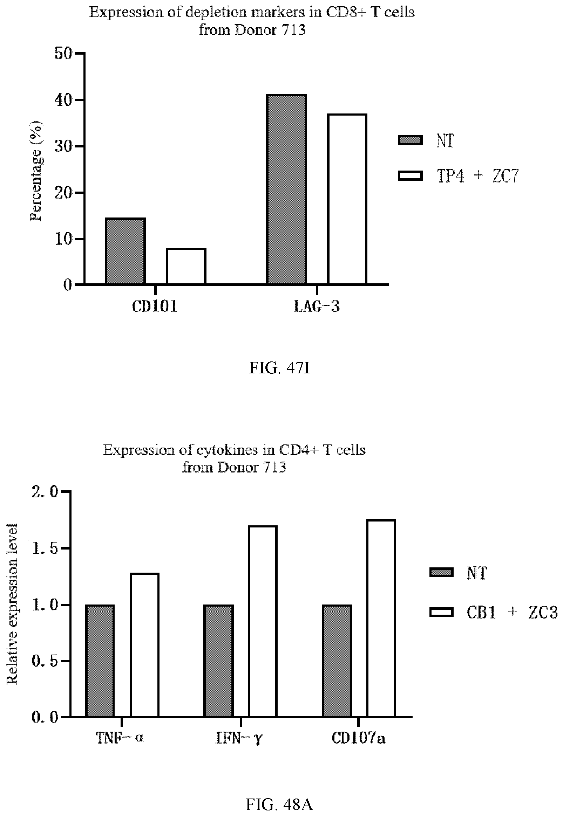

FIG. 47A shows the proportion of CD45RO-negative CD62L-positive naive T cells in each group of TIL cells, for TIL cells from donor 504. The results show that the gene-edited TIL cells have a higher proportion of naive T cells.
FIG. 47B shows the proportion of CD45RO-positive CD62L-positive central memory T cells (Tcm) in each group of TIL cells, for TIL cells from donor 713. The results show that the gene-edited TIL cells have a higher proportion of central memory T cells.
FIGs. 47C to 47I show the proportion of CD38-positive, CD101-positive, TIM-3-positive, PD-1-positive, and LAG-3-positive cells in each group of TIL cells, for TIL cells from donors 504, 607, and 713. The results show that the gene-edited TIL cells have a lower proportion of depletion cells.

**Example 16 Flow cytometry for the expression of TIL cytokine**

The TIL populations obtained on day 7 or 8 after gene editing for each test group in Example 1 were tested by a flow cytometer for the expression of cytokine.

Test preparation

For the CD3 antibody group, a flat-bottom 96-well plate was coated with 30 ng/ml of CD3 antibody (Miltenyi Biotech, OKT3) 1 day in advance and overnight at 4°C. For the transACT group, addition was performed at the time of the inoculation of T cells.

Formulation of the culture medium required for the intracellular factor expression assay: the culture medium of T cells was taken, into which were added Golgistop at 0.7:1000, Golgiplug at 1:1000, and CD107a antibody at 1:500 (i.e., 2 $\mu$L/mL) by volume ratio. No interleukin was added.

Assay steps

TILs from each test group were centrifuged, and then resuspended to $1 \times 10^6$ cells/mL using 600 $\mu$L of the culture medium required for the above intracellular factor expression assay, added to a 96-well plate at 200 $\mu$L/well, and incubated overnight in an incubator at 37°C.

At the end of the incubation, the cells were washed once with PBS at 200 $\mu$L/well, and centrifuged at 600 g for 3 minutes. The supernatant was discarded. An antibody mixed working solution of CD3/CD4/CD8 (BD) was formulated for cell surface staining, with an antibody concentration of 1:100, a viability of 1:10000, and a staining volume of 50 $\mu$L/well for 96-well plates and 100 $\mu$L/tube for flow tubes. Incubation was performed at 2-8°C in dark for 30 minutes. The reagents required for the staining of transcription factors was formulated during the staining process: Transcription Factor Buffer Set (BD) was used to dilute a 4$\times$ Fixation/Permeabilization Solution (BD) to produce 1$\times$ working solution A; double distilled water was used to dilute a 5$\times$ Perm/Wash Buffer (BD) to produce 1$\times$ working solution B. The working solutions were pre-cooled at 4 °C for later use. After staining, an appropriate amount of PBS was added to wash the cells twice (200 $\mu$L each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100 $\mu$L/well for 96-well plates, and 1 mL/tube for flow tubes) of 1$\times$ working solution A was added for fixation and permeabilization. Incubation was performed at 2-8°C in dark for 40-50 minutes. At the end of fixation and permeabilization, 1$\times$ working solution B was added to wash the cells (200 $\mu$L each time for 96-well plates, and 2 mL each time for flow tubes). Centrifugation was performed at 2-8°C and at 350 g for 6 minutes, and washing was carried out twice. 1$\times$ working solution B was used to formulate intracellular antibodies (CD107a, GZMB, TNF-$\alpha$, and IFN-$\gamma$, BD/BioLegend) at an antibody concentration of 1:100 to 1:200, with 50 $\mu$L/well for 96-well plates, and 100 $\mu$L/tube for flow tubes. Staining was performed at 2-8°C in dark for 30 minutes. At the end of staining, 1$\times$ working solution B was added to wash the cells (200 $\mu$L each time for 96-well plates, and 2 mL each time for flow tubes). Centrifugation was performed at 2-8°C and at 350 g for 6 minutes, and washing was carried out twice. The cells were resuspended in 100-500 $\mu$L of PBS for flow cytometry test on machine.

CB and ZC-combined gene editing

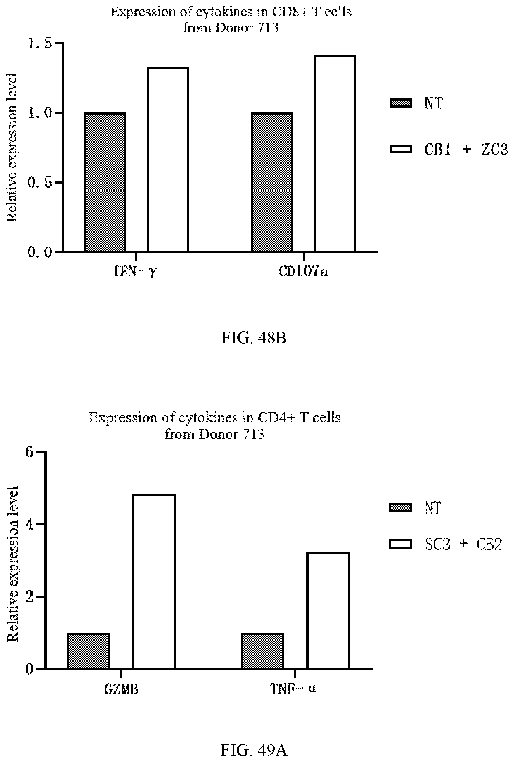

FIGs. 48A to 48B show the relative expression level of CD107a, TNF-$\alpha$, and IFN-$\gamma$ in each group of TIL cells under the stimulation of the CD3 antibody, for TIL cells from donor 713.

SC and CB-combined gene editing

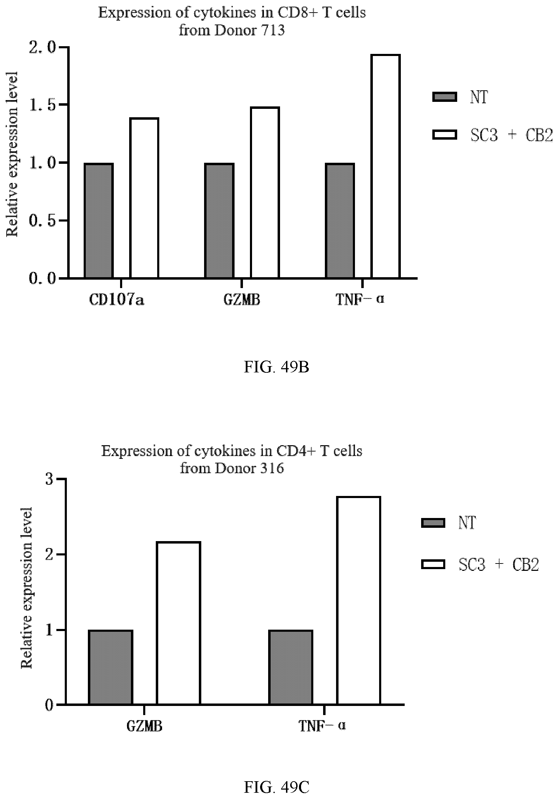

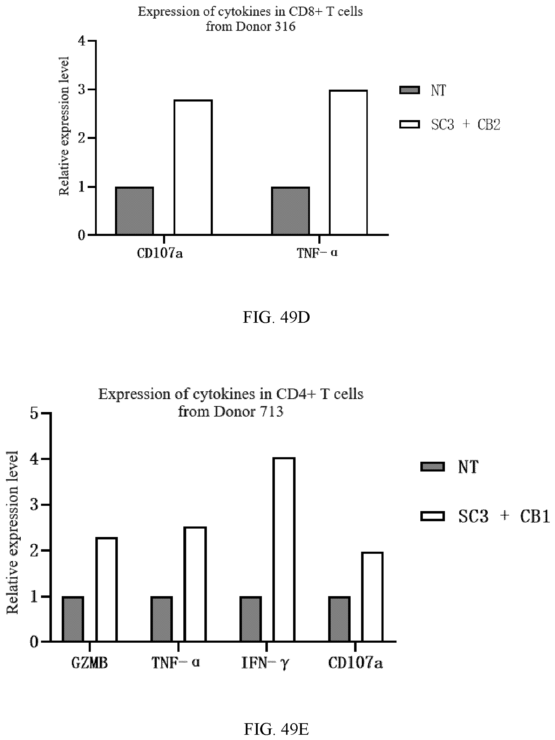

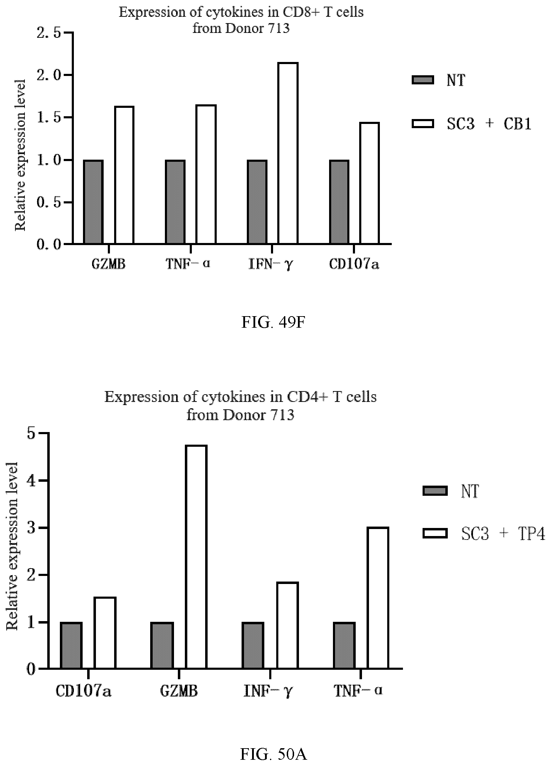

FIGs. 49A to 49D show the relative expression level of CD107a, TNF-$\alpha$, and GZMB in each group of TIL cells in the absence of any stimulants, for TIL cells derived from donors 713 and 316.

FIGs. 49E to 49F show the relative expression level of CD107a, TNF-$\alpha$, GZMB, and IFN-$\gamma$ in each group of TIL cells under the stimulation of the CD3 antibody, for TIL cells from donor 713.

SC and TP-combined gene editing

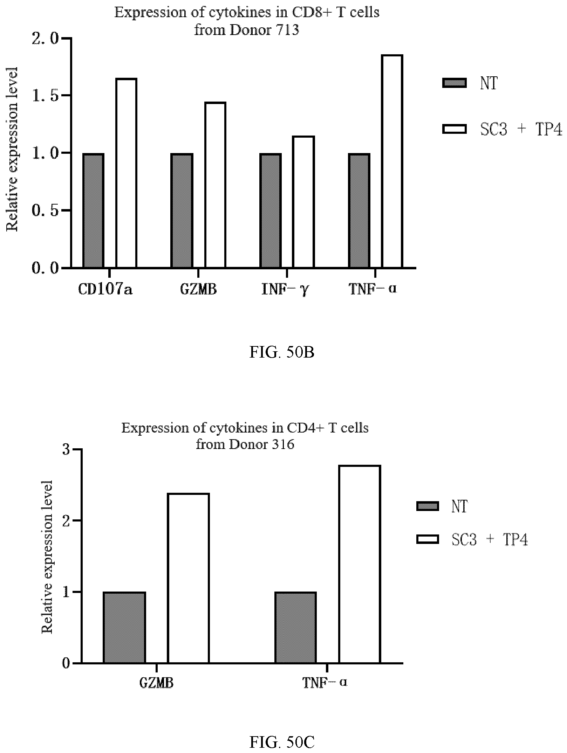

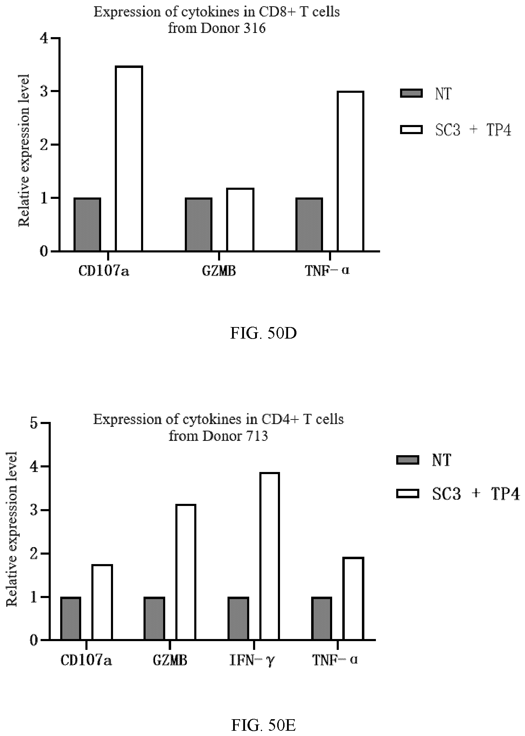

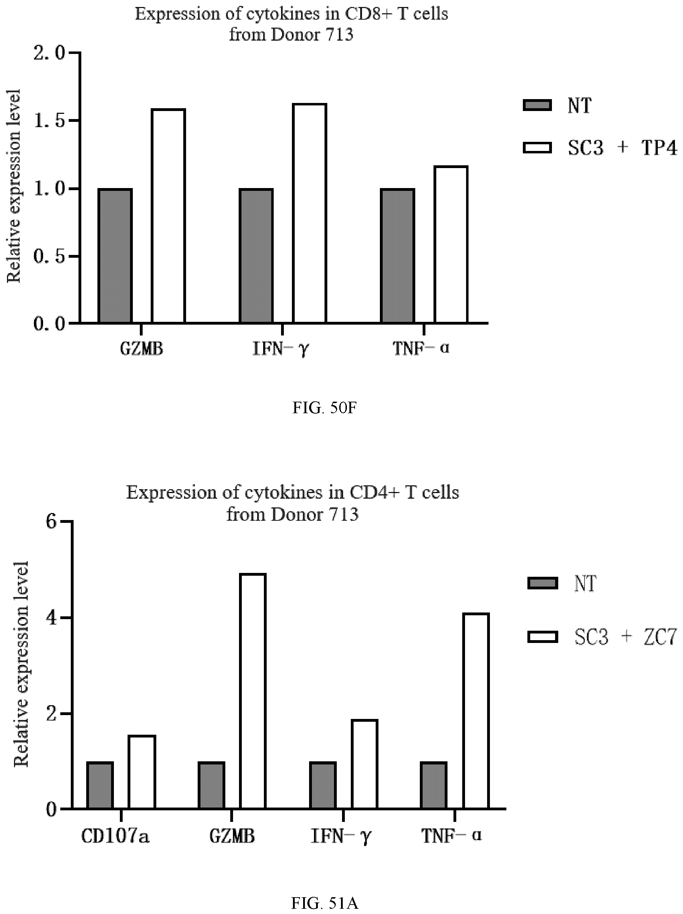

FIGs. 50A to 50D show the relative expression level of CD107a, TNF-$\alpha$, GZMB, and IFN-$\gamma$ in each group of TIL cells in the absence of any stimulants, for TIL cells from donors 713 and 316.

FIGs. 50E to 50F show the relative expression level of CD107a, TNF-$\alpha$, GZMB, and IFN-$\gamma$ in each group of TIL cells under the stimulation of the CD3 antibody, for TIL cells from donor 713.

SC and ZC-combined gene editing

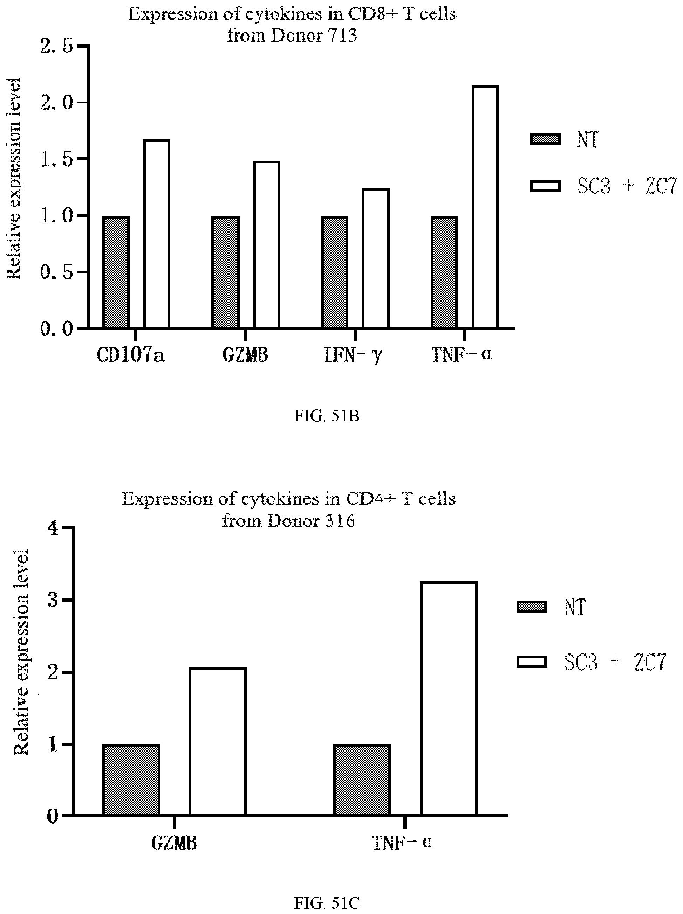

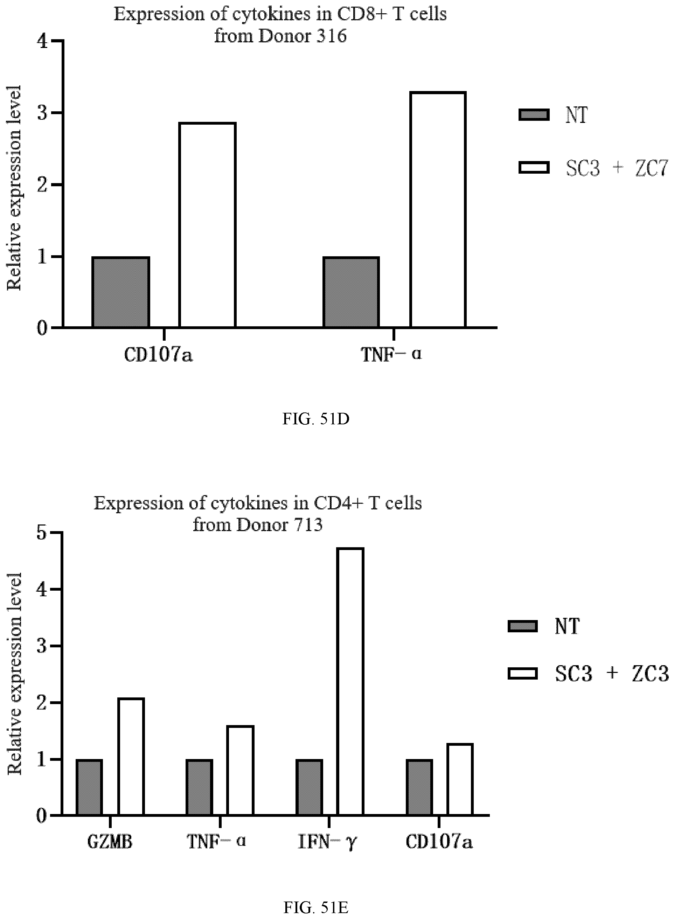

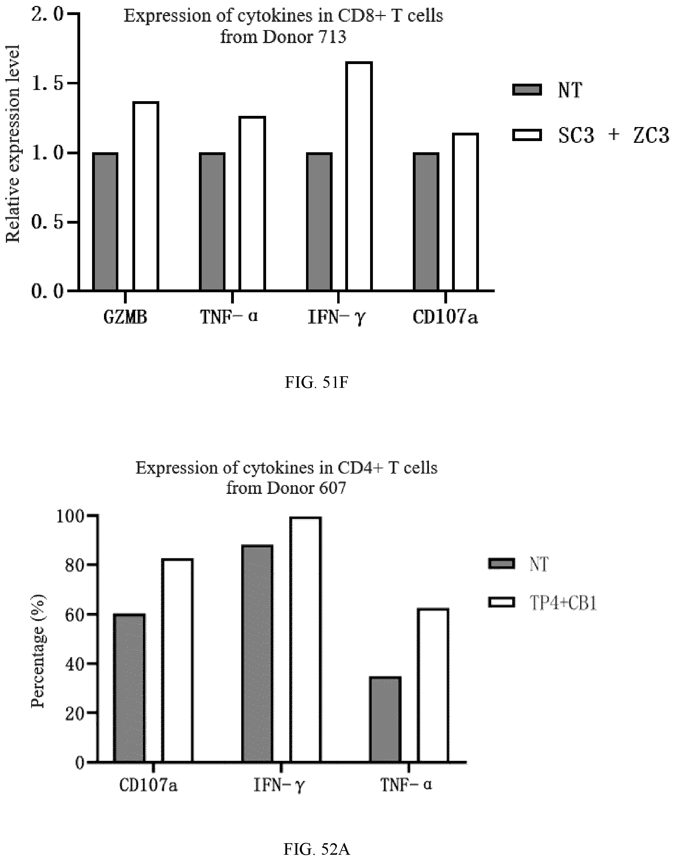

FIGs. 51A to 51D show the relative expression level of CD107a, TNF-$\alpha$, GZMB, and IFN-$\gamma$ in each group of TIL cells in the absence of any stimulants, for TIL cells from donors 713 and 316.

FIGs. 51E to 51F show the relative expression level of CD107a, TNF-$\alpha$, GZMB, and IFN-$\gamma$ in each group of TIL cells under the stimulation of the CD3 antibody, for TIL cells from donor 713.

TP and CB-combined gene editing

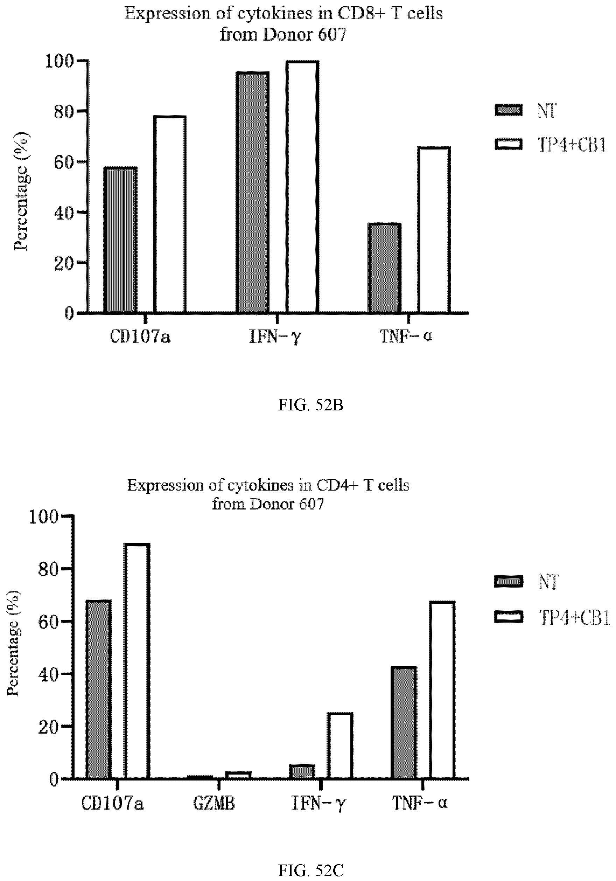

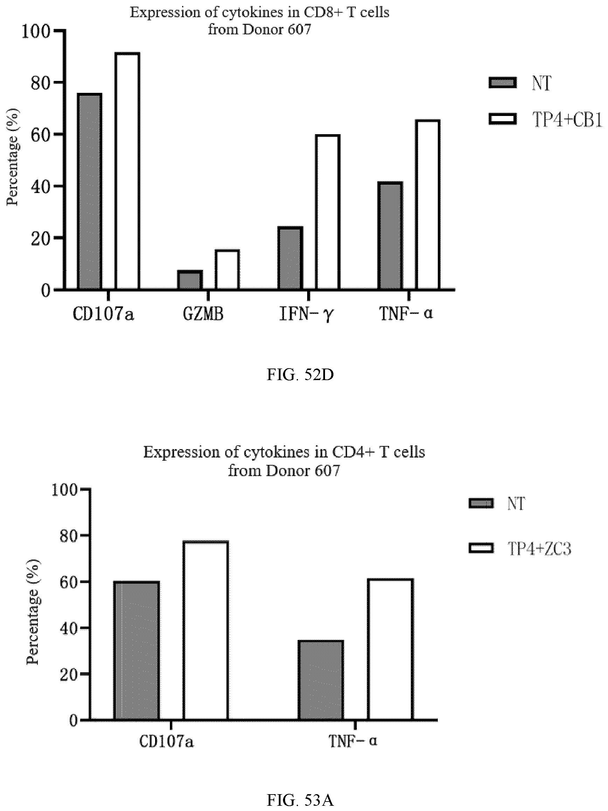

FIGs. 52A to 52B show the proportion of CD107a, TNF-$\alpha$, and IFN-$\gamma$-expressing cells in each group of TIL cells in the absence of any stimulants, for TIL cells from donor 607.

FIGs. 52C to 52D show the proportion of CD107a, TNF-$\alpha$, GZMB, and IFN-$\gamma$-expressing cells in each group of TIL cells under the stimulation of the CD3 antibody, for TIL cells from donor 607.

TP and ZC-combined gene editing

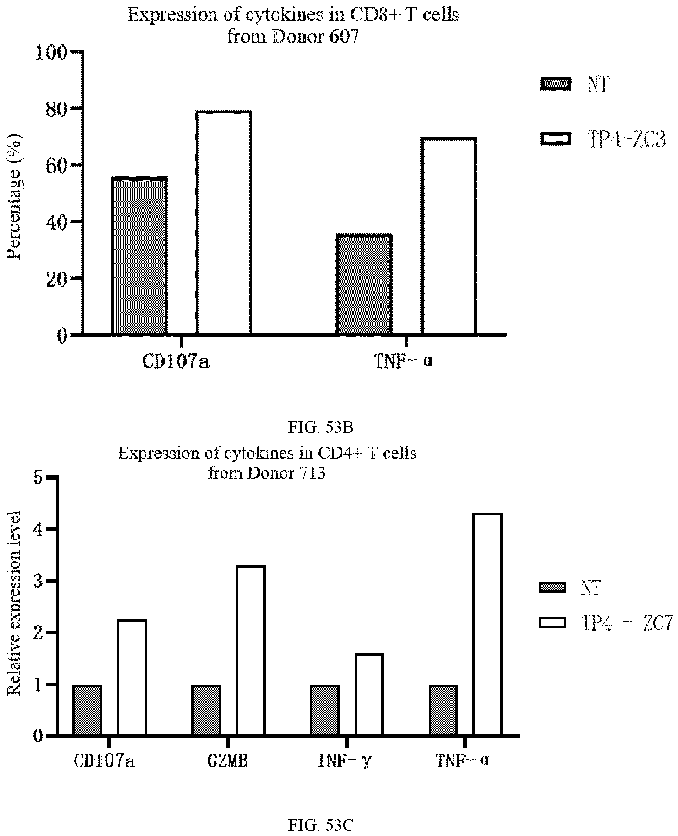

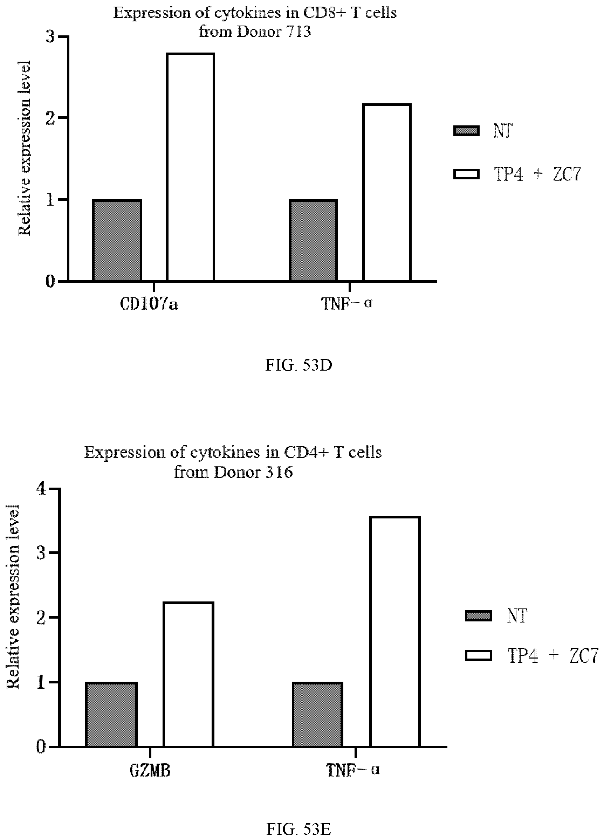

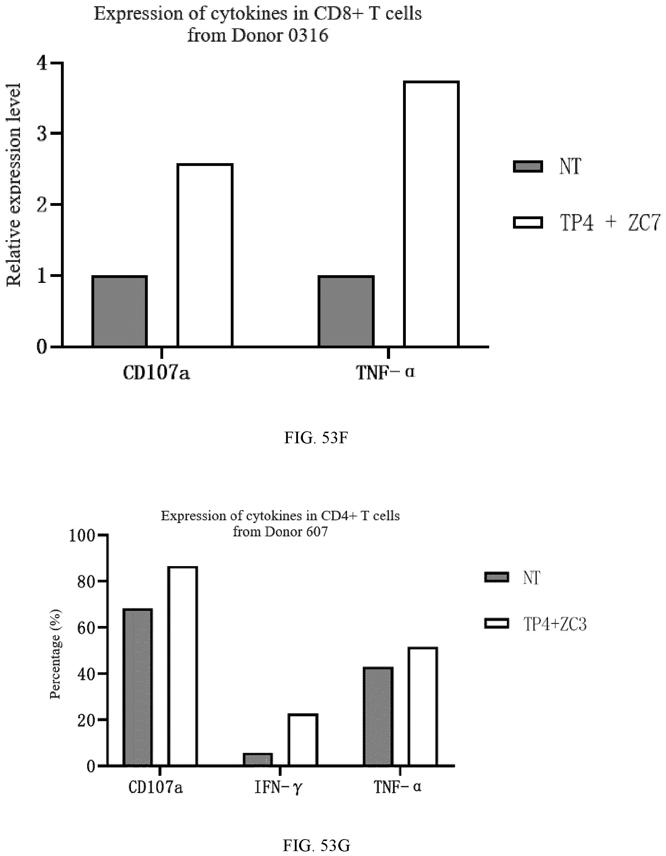

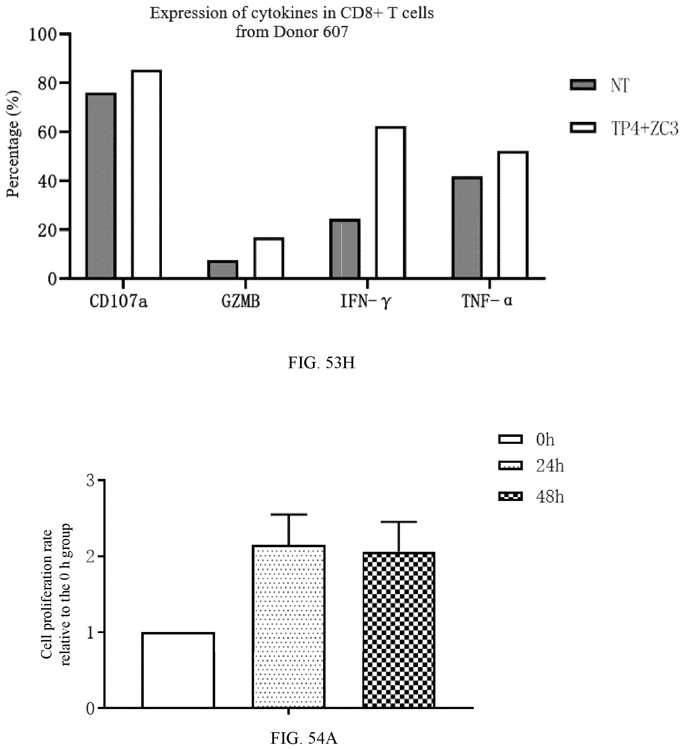

FIGs. 53A to 53B show the proportion of CD107a and TNF-$\alpha$-expressing cells in each group of TIL cells in the absence of any stimulants, for TIL cells from donor 607.

FIGs. 53C to 53F show the relative expression level of CD107a, TNF-$\alpha$, GZMB, and IFN-$\gamma$ in each group of TIL cells in the absence of any stimulants, for TIL cells from donors 713 and 316.

FIGs. 53G to 53H show the proportion of CD107a, TNF-$\alpha$, GZMB, and IFN-$\gamma$-expressing cells in each group of TIL cells under the stimulation of the CD3 antibody, for TIL cells from donor 607.

The results show that the gene-edited TIL cells have a higher intracellular factor expression capacity. For example, a higher CD107a expression capacity, a higher IFN-$\gamma$ expression capacity, a higher TNF-$\alpha$ expression capacity, or a higher GZMB expression capacity.

**Example 17**

Statistics of the results of TILs cultured with feeder cells added at different times

During the activation of TILs in the second stage of expansion in 1.4 of Example 1, an amount of cells expanded in the first stage was taken and the cell density was adjusted to $5\times10^5$ to $2\times10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 0, 24, and 48 hours after the addition of OKT3 and IL-2 above, feeder cells were added into the culture environment of tumor infiltrating lymphocytes. Where, TILs and feeder cells may be added at a ratio of 1:40 to 1:400, and all the cells were collected after about 9 to 14 days of culture by the second stage of expansion, and the results of TILs obtained from the culture was tested and counted.

Proliferation ability assay

The TIL cells obtained above by culturing with feeder cells added at different times were counted.

TILs from different donor tumor sources were used as their respective different batches; the data from each batch of the test groups in which feeder cells were added with OKT3 and IL-2 at the same time (0 h group) was used as the benchmark 1, and the data from the test groups at other time points in the same batch were normalized to count the relative proliferation ability of each test group in the second stage of expansion relative to the 0 h group.

FIG. 54A shows the cell proliferation ability of TIL cells obtained by culturing with feeder cells added at 0, 24 or 48 hours after the addition of OKT3 and IL-2. Compared to TILs cultured with feeder cells added at 0 hours after the addition of OKT3 and IL-2 (i.e., at the same time), the proliferation ability of TILs cultured with feeder cells added at 24 or 48 hours after the addition of OKT3 and IL-2 was significantly enhanced.

Detection of composition of TIL cells by flow cytometry

TIL populations obtained above by culturing with feeder cells added at different addition times were tested by flow cytometry.

TILs from different donor tumor sources were used as their respective different batches; the data from each batch of the test groups in which feeder cells were added with OKT3 and IL-2 at the same time (0 h group) was used as the benchmark 1, and the data from the test groups at other time points in the same batch were normalized to count the cell composition proportion of each test group in the second stage of expansion relative to the 0 h group.

The test procedure of flow cytometry can be carried out by referring to the disclosure in Example 3 of the present application.

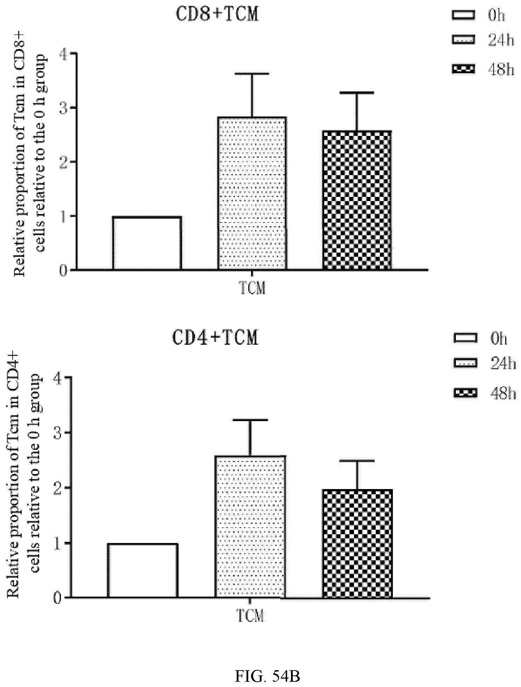

FIG. 54B shows the proportion of CD45RA$^-$CCR7$^+$ central memory T cells (Tcm) in the TIL cells obtained by culturing with feeder cells added at 0, 24 or 48 hours after the addition of OKT3 and IL-2. The results show that, TILs cultured with feeder cells added after 24 hours or 48 hours have a higher proportion of central memory T cells in CD8$^+$ and/or CD4$^+$ compared to that in the TILs cultured with feeder cells added at the same time.

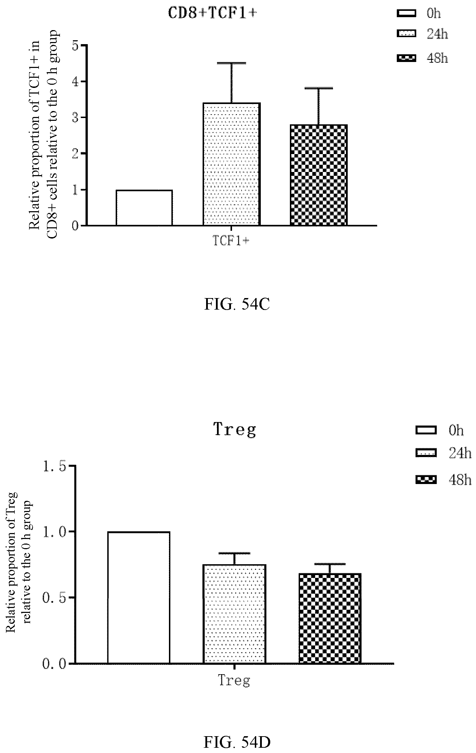

FIG. 54C shows the proportion of TCF1$^+$ stem cell-like T cells in the TIL cells obtained by culturing with feeder cells added at 0, 24 or 48 hours after the addition of OKT3 and IL-2. The results show that, TILs cultured with feeder cells added after 24 hours or 48 hours have a higher proportion of stem cell-like T cells in CD8$^+$ compared to that in the TILs cultured with the feeder cells added at the same time.

FIG. 54D shows the proportion of CD4$^+$CD25$^+$Foxp3$^+$ regulatory T cells (Treg) in the TIL cells obtained by culturing with feeder cells added at 0, 24 or 48 hours after the addition of OKT3 and IL-2. The results show that, TILs cultured with feeder cells added after 24 hours or 48 hours have a lower proportion of regulatory T cells compared to that in the TILs cultured with the feeder cells added at the same time.

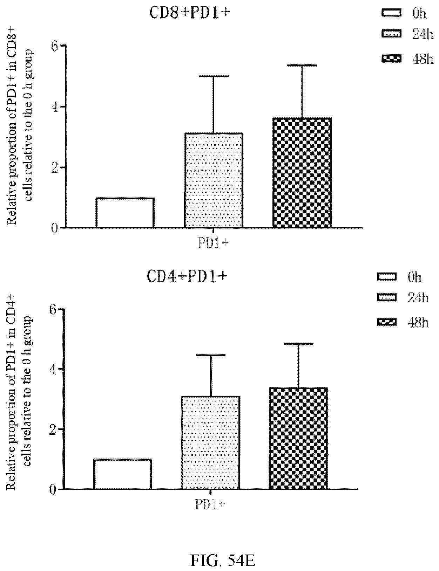

FIG 54E shows the proportion of activated T cells (PD-1$^+$) in the TIL cells obtained by culturing with feeder cells added at 0, 24 or 48 hours after the addition of OKT3 and IL-2. The results show that, TILs cultured with feeder cells added after 24 hours or 48 hours have a higher proportion of activated T cells compared to that in the TILs cultured with feeder cells added at the same time, e.g., a higher proportion of PD-1$^+$ cells in CD8$^+$ and/or CD4$^+$.

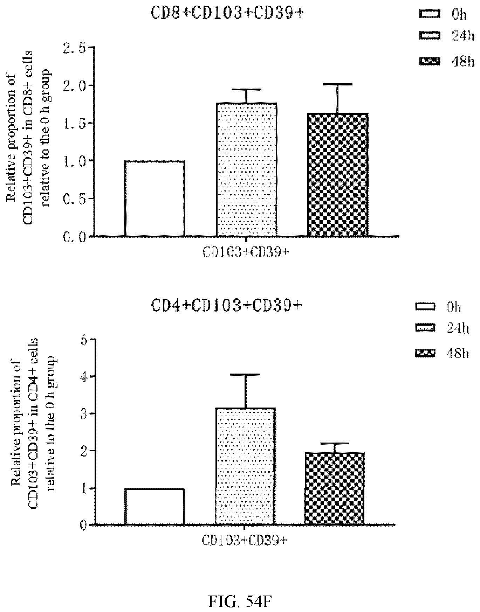

FIG. 54F shows the proportion of CD103$^+$CD39$^+$ tumor-specific T cells in the TIL cells obtained by culturing with feeder cells added at 0, 24 or 48 hours after the addition of OKT3 and IL-2. The results show that, TILs cultured with feeder cells added after 24 hours or 48 hours have a higher proportion of tumor-specific T cells in CD8$^+$ and/or CD4$^+$ compared to that in the TILs cultured with the feeder cells added at the same time.

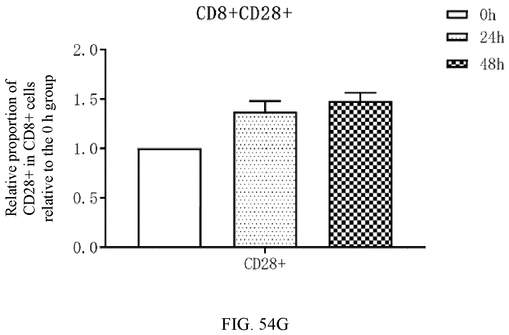

FIG. 54G shows the proportion of activated T cells (CD28$^+$) in the TIL cells obtained by culturing with feeder cells added at 0, 24 or 48 hours after the addition of OKT3 and IL-2. The results show that, TILs cultured with feeder cells added after 24 hours or 48 hours have a higher proportion of activated T cells compared to that in the TILs cultured with feeder cells added at the same time, e.g., a higher proportion of CD8$^+$CD28$^+$ cells.

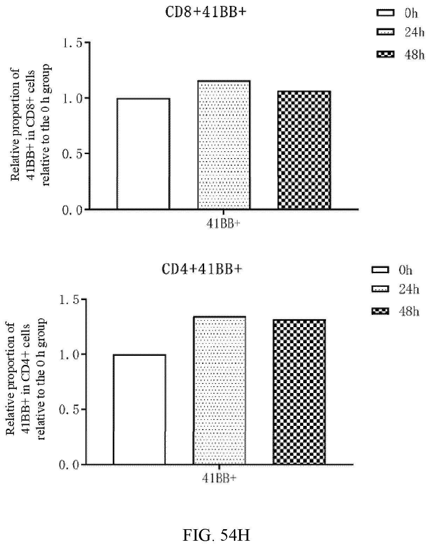

FIG. 54H shows the proportion of activated T cells (41BB$^+$) in the TIL cells obtained by culturing with feeder cells added at 0, 24 or 48 hours after the addition of OKT3 and IL-2. The results show that, TILs cultured with feeder cells added after 24 hours or 48 hours have a higher proportion of activated T cells compared to that in the TILs cultured with feeder cells added at the same time, e.g., a higher proportion of 41BB$^+$ cells in CD8$^+$ and/or CD4$^+$.

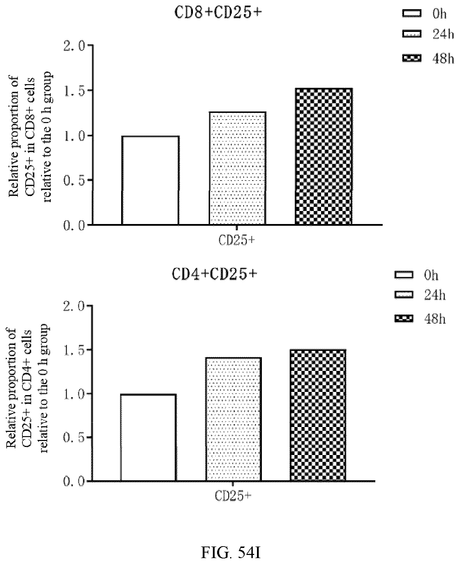

FIG. 54I shows the proportion of activated T cells (CD25$^+$) in the TIL cells obtained by culturing with feeder cells added at 0, 24 or 48 hours after the addition of OKT3 and IL-2. The results show that, TILs cultured with feeder cells added after 24 hours or 48 hours have a higher proportion of activated T cells compared to that in the TILs cultured with feeder cells added at the same time, e.g., a higher proportion of CD25$^+$ cells in CD8$^+$ and/or CD4$^+$.

Intracellular factor expression assay

Test preparation

Formulation of the culture medium required for the intracellular factor expression assay: the culture medium of T cells was taken, into which were added CD107a antibodies (BD) at a volume ratio of 1:500.

Assay steps

TILs from each test group were centrifuged, and then resuspended to $1 \times 10^6$ cells/mL using 600 $\mu$L of the culture medium required for the above intracellular factor expression assay, added to a 96-well plate at 100 $\mu$L/well, and incubated overnight in an incubator at 37°C.

At the end of the incubation, the cells were washed once with PBS at 200 $\mu$L/well and centrifuged at 600 g for 3 minutes. The supernatant was discarded. An antibody mixed working solution of CD3/CD4/CD8 (BD) was formulated for cell surface staining, with an antibody concentration of 1:100, a viability of 1:10000, and a staining volume of 50 $\mu$L for each group. Incubation was performed at 2-8°C in dark for 30 minutes. At the end of staining, the cells were washed and resuspended in PBS for flow cytometry on machine.

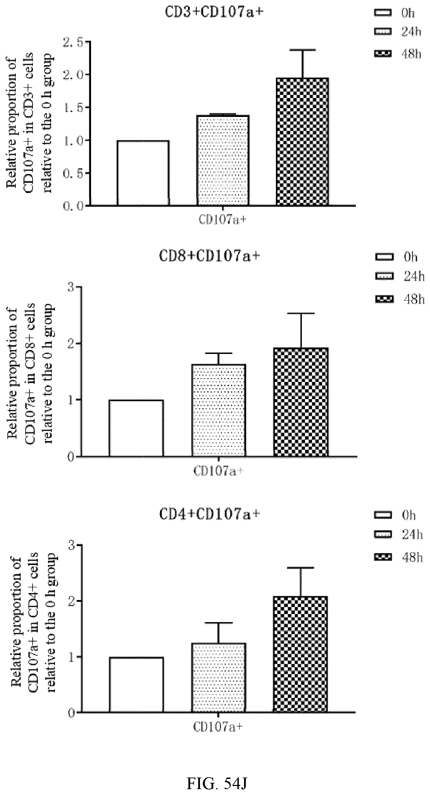

FIG. 54J shows the results of intracellular factor expression assay of TIL cells obtained by culturing with feeder cells added at 0, 24 or 48 hours after the addition of OKT3 and IL-2. The results show that TILs cultured with feeder cells added after 24 hours or 48 hours have a higher intracellular factor expression capacity compared with that of TILs cultured with feeder cells added at the same time. For example, a higher CD107a expression capacity in CD3$^+$, CD8$^+$ and/or CD4$^+$.

Cytokine secretion assay

The cytokine secretion assay method can be carried out by referring to the instruction of the cytokine assay kit (BD). Human Th1/Th2/Th17 cytokine standard freeze-dried powder (BD) was reconstituted with 2 mL of Assay Diluent (BD) (the concentration of each cytokine in the standard stock solution was 5000 pg/mL) and diluted in the following order of gradient dilutions: 1:2, 1:4, 1:8, 1:16, 1:32, 1:64, 1:128, 1:256, 1: 512, 1:1024, labeled as "standard tube". One tube comprising only the Assay Diluent was taken as a reference. Each kind of Capture Beads (BD) were added at 2 μL/Beads/well, then PE Detection Reagent (BD) was added at 10 μL/well and mixed to prepare a mixture (mix), which was added into a V-bottom 96-well plate at 22 μL/well. Into the plate were then added each standard and the supernatant of the test group at 10 μL/well and mixed, then incubated at room temperature in dark for 3 hours.

At the end of the incubation, 200 μL of Wash Buffer (BD) was added to each well and centrifuged at 500 g for 3 minutes. At the end of the centrifugation, 100 μL of Wash Buffer (BD) was added to each well for resuspension and flow cytometry.

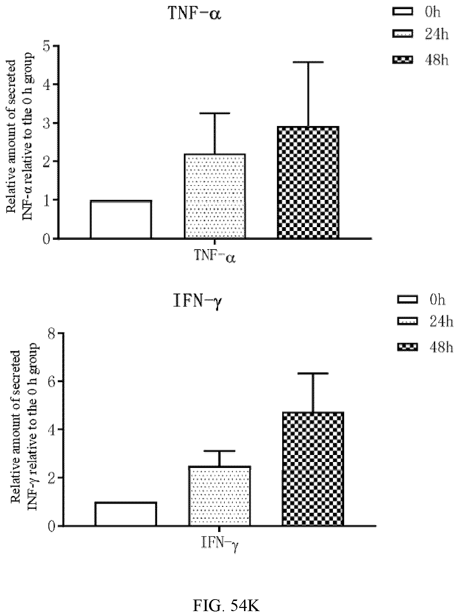

FIG. 54K shows the results of cytokine secretion assay of TIL cells obtained by culturing with feeder cells added at 0, 24 or 48 hours after the addition of OKT3 and IL-2. The results show that, TILs cultured with feeder cells added after 24 hours or 48 hours have a higher cytokine secretion capacity than that of the TILs cultured with feeder cells added at the same time. For example, a higher TNF-α secretion capacity or a higher IFN-γ secretion capacity.

Statistics of the results of TILs cultured with feeder cells added at different time

During the activation of TILs in the second stage of expansion in 1.4 of Example 1, an amount of cells expanded in the first stage was taken and the cell density was adjusted to $5 \times 10^5$ to $2 \times 10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 0, 6, 12, 24, 48, 72 hours, or 5 days after the addition of OKT3 and IL-2 above, feeder cells were added into the culture environment of tumor infiltrating lymphocytes. Where, TILs and feeder cells may be added at a ratio of 1:40 to 1:400, e.g., 1:200. All the cells were collected after about 9 to 14 days of culture by the second stage of expansion, and the results of TILs obtained from the culture were tested and counted.

Proliferation ability assay

The TIL cells obtained above by culturing with feeder cells added at different times were counted.

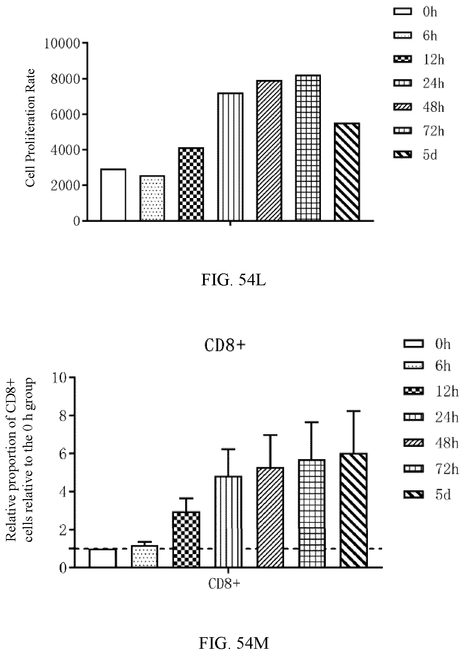

FIG. 54L shows the proliferation ability of TIL cells obtained by culturing with feeder cells added at 0, 6, 12, 24, 48, 72 hours, or 5 days after the addition of OKT3 and IL-2. Compared to TILs cultured with feeder cells added at 0 hours after the addition of OKT3 and IL-2 (i.e., at the same time), the proliferation ability of TILs cultured with feeder cells added at 12 hours or longer time after the addition of OKT3 and IL-2 was significantly enhanced.

Detection of composition of TIL cells by flow cytometry

TIL populations obtained above by culturing with feeder cells added at different addition times were tested by flow cytometry.

TILs from different donor tumor sources were used as their respective different batches; the data from each batch of the test groups in which feeder cells were added with OKT3 and IL-2 at the same time (0 h group) was used as the benchmark 1, and the data from the test groups at other time points in the same batch were normalized to count the cell composition proportion of each test group in the second stage of expansion relative to the 0 h group.

The test procedure of flow cytometry can refer to the disclosure in Example 3 of the present application.

FIG. 54M shows the proportion of CD8+ T cells in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have a higher proportion of CD8+ T cells compared to that in the TILs cultured with feeder cells added at the same time.

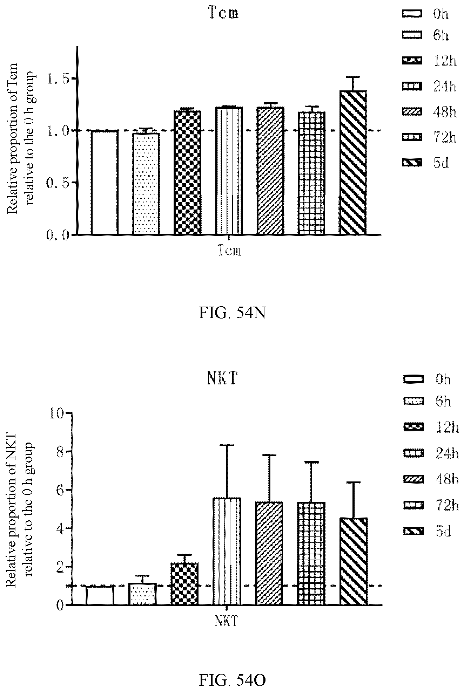

FIG. 54N shows the proportion of CD45RO+CD62L+ T cells in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have a higher proportion of memory T cells (Tcm, CD45RO+CD62L+) compared to that in the TILs cultured with feeder cells added at the same time.

FIG. 54O shows the proportion of NK T cells in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have a higher proportion of NK T cells compared to that in the TILs cultured with feeder cells added at the same time.

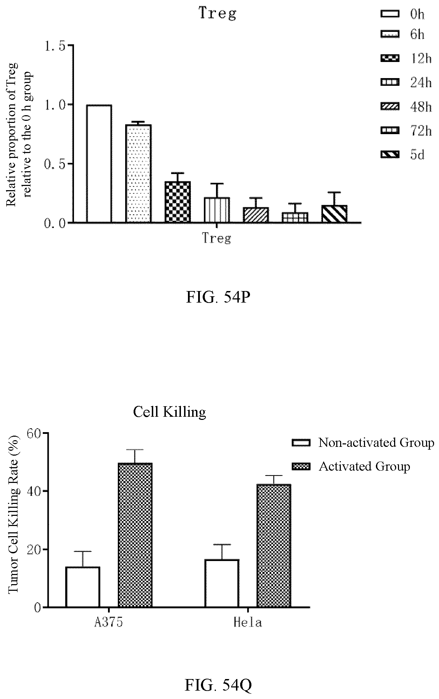

FIG. 54P shows the proportion of CD4+CD25+Foxp3+ regulatory T cells (Treg) in the TIL cells obtained by culturing with feeder cells added at 0, 6, 12, 24, 48, 72 hours, or 5 days after the addition of OKT3 and IL-2. The results show that, TILs cultured with feeder cells added at 12 hours or longer time after the addition of OKT3 and IL-2 have a lower proportion of regulatory T cells compared to that in the TILs cultured with feeder cells added at the same time.

Killing ability assay of TILs cultured in the present application

During the activation of TILs in the second stage of expansion in 1.4 of Example 1, an amount of cells expanded in the first stage was taken and the cell density was adjusted to $5 \times 10^5$ to $2 \times 10^6$/mL. In a suspension 24-well culture plate were added CD3 antibodies at 1 mL/well, e.g., about 30 ng/mL of OKT3, and IL-2 at a concentration of about 1000 to 9000 IU/mL, e.g., 3000 or 6000 IU/mL of IL-2. At 12 hours to 14 days (e.g., 48 hours) after the addition of OKT3 and IL-2 above, feeder cells were added into the culture environment of tumor infiltrating lymphocytes. Where, TILs and feeder cells may be added at a ratio of 1:40 to 1:400, and all the cells were collected after about 9 to 14 days of culture by the second stage of expansion, and the cell killing ability of TILs obtained from the culture was tested and counted.

Cell preparation

TILs obtained from each test group for assay and target cells (e.g., A375 melanoma cells and/or Hela cervical cancer cells) for co-culture were prepared.

Assay steps

Labeling the tumor cells with CFSE (5(6)-Carboxyfluorescein diacetate N-succinimidyl ester, Sigma, 21888-25MG-F): The tumor cells were washed with PBS, and resuspended in 500 $\mu$L of PBS; CFSE was added into 500 $\mu$L of PBS, and mixed with 500 $\mu$L of resuspension of the tumor cells in PBS to a final concentration of CFSE of 0.5 $\mu$mol/L. After incubation at 37°C for 6 minutes, a medium containing 10% FBS was added to wash. Centrifugation was performed at 600 g for 5 minutes. X-vivo 15 medium or other commercially available T cell culture media, e.g., T cell culture media of Stem Cell, Lonza, Thermo, Miltenyi brands etc., were used to resuspend the tumor cells to a concentration of $1\times10^6$ cells/mL. The TIL population of each test group was centrifuged at 600 g for 5 minutes, and the TIL cells were resuspended at an effector-target ratio (the ratio of TIL cells to tumor cells) of 3:1 (i.e., the concentration of the resuspended TIL cells was $3\times10^6$ cells/mL). Each 100 $\mu$L of tumor cells and TIL cells were added to a U-bottom 96-well plate (Corning), and three replicate wells were set for each group. At the same time, a control group comprising only the tumor cells was set. The well plates were centrifuged at 200 g for 1 minute and incubated at 37°C for 4 hours to overnight. Where, TILs were co-cultured with tumor cells, either without the addition of substances that activate TIL cells as a non-activated group or with the addition of transACT (Miltenyi, a nano-matrix material comprising CD3 antibodies and CD28 antibodies) as an activated group.

At the end of the incubation, centrifugation was performed at 600 g for 3 minutes and the supernatant was discarded. Trypsin was added at 20 $\mu$L/well. Incubation was performed in an incubator at 37°C for 3-5 minutes to digest the tumor cells. After the digestion was completed, 180 $\mu$L of culture medium containing 10% FBS was added to terminate the digestion. Dapi (Beyotime, C0060) was diluted at 1:100, and then the diluted Dapi was added at 20 $\mu$L/well. Flow cytometry was performed on machine.

Killing rate% = Number of Dapi$^+$CFSE$^+$ cells/Total CFSE$^+$ $\times$ 100%, or the killing rate can be expressed by the ratio of the number of Dapi$^+$ cells to the total number of tumor cells.

FIG. 54Q shows the cell killing ability of TIL cells obtained by culturing with feeder cells added at 48 hours after the addition of OKT3 and IL-2. The results show that, the TILs cultured with feeder cells added at 48 hours after the addition of OKT3 and IL-2 all have significant tumor cell killing ability, e.g., melanoma and/or cervical tumor.

The results show that the gene-edited TIL cells have a higher intracellular factor expression capacity. For example, a higher CD107a expression capacity, a higher IFN-$\gamma$ expression capacity, a higher TNF-$\alpha$ expression capacity, or a higher GZMB expression capacity.

**Example 18**

Immunodeficient mice (NOG mice, Vital River, Strain code 408) were subcutaneously inoculated with Hey-T30 cells at $1\times10^6$ cells per mouse. Nine days after inoculation, the mice were randomly grouped as shown in the table below.

| Groups | Number of TIL cells | IL-2 (IU) | Number of mice |
|---|---|---|---|
| PBS | 0 | 0 | 6 |
| NT | $2.5\times10^7$ | $3\times10^5\times6$ | 6 |
| TP4+ZC3 | $2.5\times10^7$ | $0.6\times10^5\times6$ | 6 |
| SC3+TP4 | $2.5\times10^7$ | $0.6\times10^5\times6$ | 6 |

After the mice in each group were infused with $2.5\times10^7$ therapeutic cells via the tail vein of mice, the NT control group was given $3\times10^5$ IU for each dose of IL-2 combined treatment, and the double knockout group was given $0.6\times10^5$ IU for each dose of IL-2 combined treatment twice a day for 3 consecutive days. The tumor volume of mice in each group was measured twice a week. Both double-knockout groups exhibited significant tumor suppression ability, which was significantly stronger than that of the NT group. At the same time, peripheral blood was collected from each group of mice at multiple time points, and the number of CD45+CD3+ TIL cells, T-cell memory phenotype, and cytokine release levels in the peripheral blood were detected.

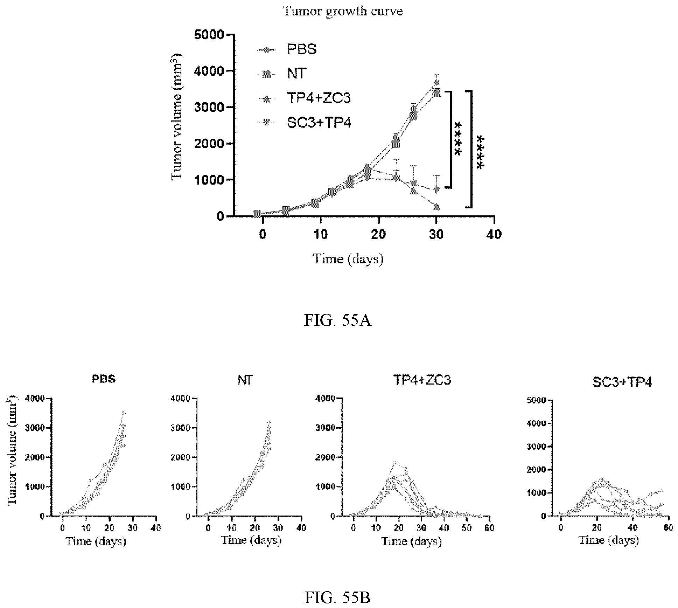

FIGs. 55A to 55B show that both the TP4+ZC3 double-knockout group and the SC3+TP4 double-knockout group exhibited significant tumor suppression ability, with a tumor suppression effect significantly stronger than that of the NT group.

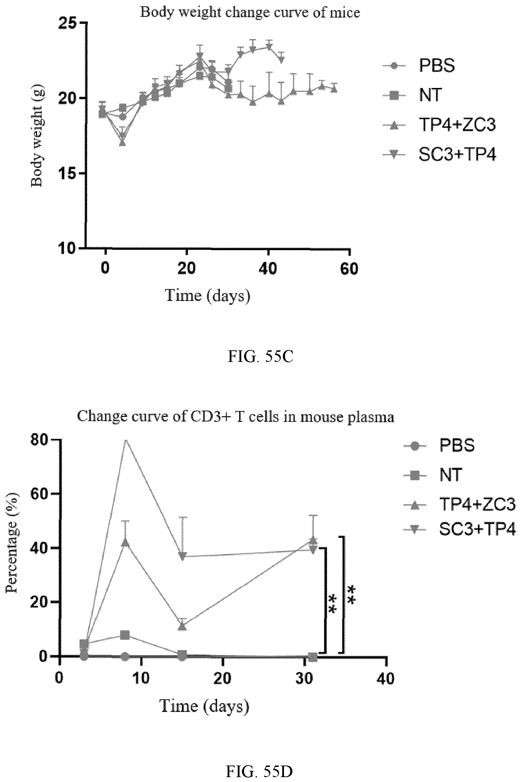

FIG. 55C shows that there were no significant changes in the body weight of mice in each group.

FIG. 55D shows that, the proportion of CD3+T cells in the peripheral blood of the TP4+ZC3 double-knockout group and the SC3+TP4 double-knockout group after administration was significantly higher than that in the NT group, suggesting that both TP+ZC and SC+TP double-gene editing significantly promote the proliferation and survival of T cells *in vivo,* and may inhibit the apoptosis of TILs.

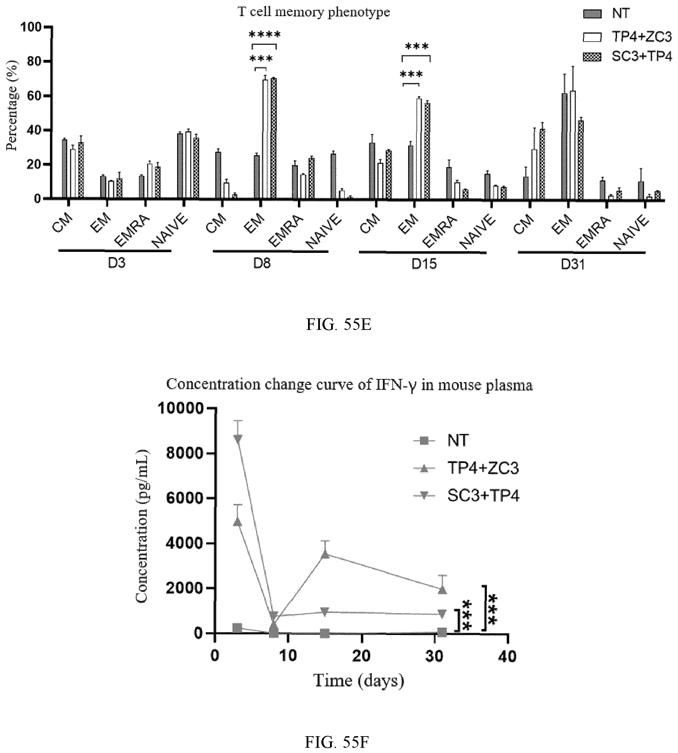

FIG. 55E shows that, the effector memory T cells (EM) in the peripheral blood of the TP4+ZC3 double-knockout group and the SC3+TP4 double-knockout group on days 8 and 15 were significantly higher than that in the NT group, suggesting that both TP+ZC and SC+TP double-gene editing may increase the proportion of effector memory T cells, that is consistent with the stronger tumor killing ability *in vivo.*

FIG. 55F shows that, the release of IFN-γ in the TP4+ZC3 double-knockout group and the SC3+TP4 double-knockout group at various time points after administration was significantly higher than that in the NT group, further suggesting that both TP+ZC and SC+TP double-gene editing may enhance the antitumor function of TILs *in vivo.*

**Example 19**

Cervical cancer tissue samples (Sample No.: Donor 709) were obtained surgically and transferred to the laboratory under sterile conditions. The necrotic tumor tissue was removed in a sterile flow cabinet, and the remaining tumor tissue was cut into pieces of 1-2 mm$^3$. The cut tumor tissues were then mixed with a small amount of Matrigel and subcutaneously inoculated into NOG mice (Vital River, Strain code 408) in an SPF-grade animal facility. Each mouse was implanted with 4-5 pieces of tumor tissue. This batch of tumor tissues were referred to as the F0 generation. When the F0 generation tumor tissue in mice grew subcutaneously to 800 mm$^3$, the F0 tumor tissue was removed surgically, and the tumor tissue was divided into 1-2 mm$^3$ again, part of which was frozen for later use, and part of which was subcutaneously inoculated into the second batch of NOG mice, wherein it continued to be passaged and amplified, and this batch of tumor tissue was referred to as the F1 generation. This process was repeated until the tumor tissue was passaged to the F3 generation in mice. The size of the tumor tissue was measured regularly until the tumor volume reached approximately 100 mm$^3$. The mice were randomly grouped as shown in the table below.

| Groups | Number of TIL cells | IL-2 (IU) | Number of mice |
|---|---|---|---|
| PBS | 0 | 0 | 5 |
| NT | $3 \times 10^7$ | $3 \times 10^5 \times 6$ | 5 |
| TP4+ZC3 | $3 \times 10^7$ | $0.6 \times 10^5 \times 6$ | 5 |
| TP4+CB1 | $3 \times 10^7$ | $0.6 \times 10^5 \times 6$ | 5 |
| Note: NT represents TILs in the control group without electroporation and gene editing. | | | |

After the mice in each group were infused with $3 \times 10^7$ therapeutic cells via the tail vein of mice, the NT control group was given $3 \times 10^5$ IU for each dose of IL-2 combined treatment, and both the TP4+ZC3 double knockout group and the TP4+CB1 double knockout group were given $0.6 \times 10^5$ IU for each dose of IL-2 combined treatment twice a day for 3 consecutive days. The tumor volume of mice in each group was measured twice a week. Peripheral blood was collected from each group of mice at days 4, 7, 14, 21, and 33 after treatment, and the number and proportion of CD3+T, CD4+T and CD8+T cells in the peripheral blood were detected.

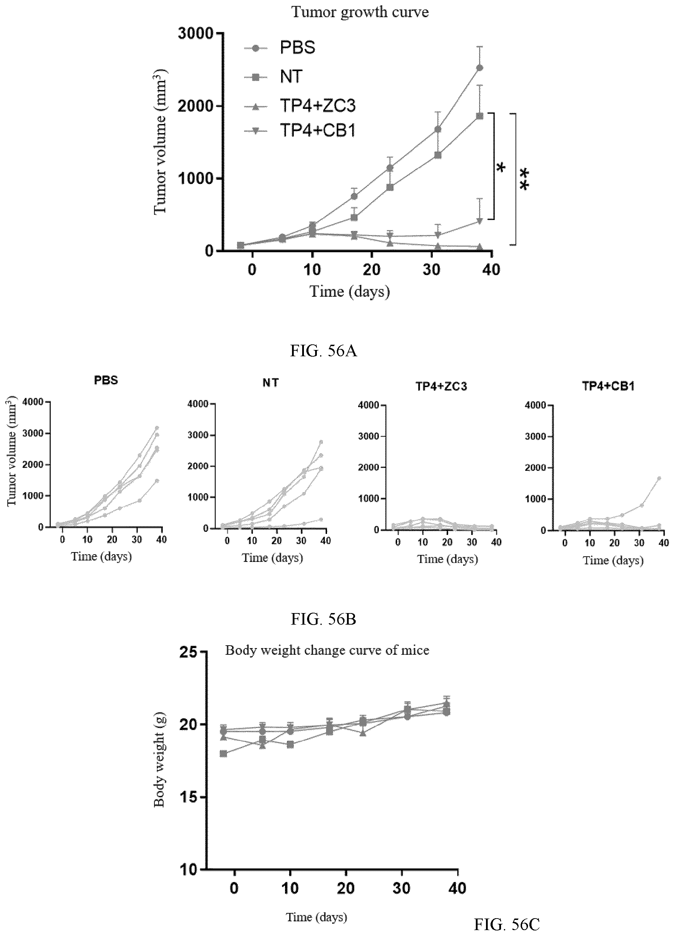

FIGs. 56A to 56B show that, both the TP4+ZC3 double-knockout group and TP4+CB 1 double-knockout group exhibited significant tumor suppression ability, with a tumor suppression effect significantly stronger than that of the NT group.

FIG. 56C shows that there were no significant changes in the body weight of mice in each group.

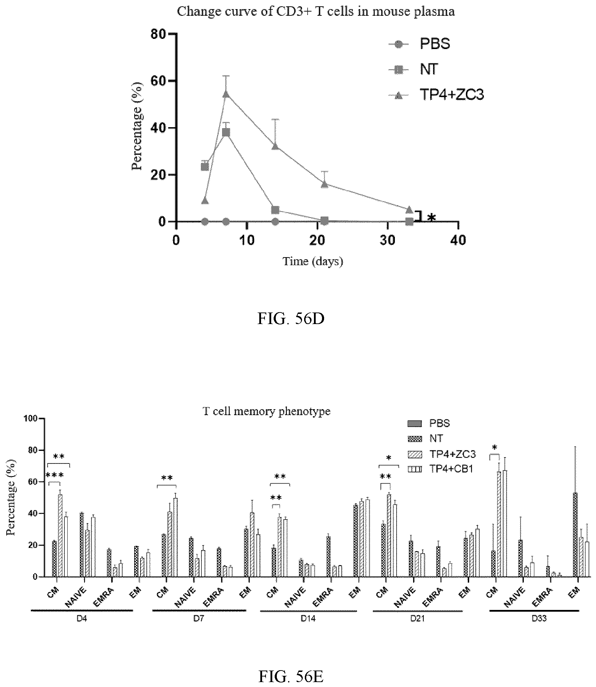

FIG. 56D shows that, the proportion of CD3+T cells in both the NT group and the TP4+ZC3 double-knockout group reached the peak on Day 7 after administration. From Day 7 on, the proportion of CD3+T cells in the TP4+ZC3 double-knockout group was higher than that in the NT group, suggesting that TP/ZC double-gene knockout significantly promotes the proliferation and survival of T cells *in vivo,* and may inhibit the apoptosis of TILs.

FIG. 56E shows that, the central memory T cells (CM) in the peripheral blood of the TP4+ZC3 double-knockout group and the TP4+CB 1 double-knockout group were significantly higher than that in the NT group on Day 4, Day 7, Day 14, and Day 21; meanwhile, there was a rising trend in the proportion of effector memory T cells (EM) in the T cells of peripheral blood on Day 4 to Day 14, that is consistent with the good antitumor effect of the TP4+ZC3 double-knockout group and the TP4+CB 1 double-knockout group *in vivo.*

**Example 20 Gene knockout efficiency assay of TIL cells**

Reagents and materials: DNA extraction solution (QuickExtract DNA extraction solution, Lucigen, QE09050), nuclease-free water (RNase/DNase free water, Tiangen), EDTA (Sangon, 0.5M), Recombinant DNase I (RNase-free, TAKARA).

Extraction of genome DNA: After approximately 7-10 days following TIL cell knockout, about $1 \times 10^5$ to $2 \times 10^5$ cells were washed once with PBS, and TIL cells were resuspended with 44 $\mu$L of PBS and incubated in 6 $\mu$L of the formulated mixture of nucleases (containing 1 $\mu$L of DNase I and 5 $\mu$L of $10\times$ DNase I Buffer) at 37°C for 5 minutes. 2.5 $\mu$L of 0.5M EDTA was added into the samples and incubated at 80°C for 10 minutes. After centrifugation, the supernatant was discarded, 50 $\mu$L of DNA extraction solution was added into the cell pellet and centrifuged briefly, then the following program was run: at 75°C for 10 min; at 95°C for 5 min; maintainance at 4°C. A spectrophotometer (NanoDrop™) was used to detect the concentration of DNA samples.

Sequencing: PCR primers can be designed in the region of about 100 to about 200 nucleotides upstream and downstream of the PAM site. A PCR reaction system was designed as below:

| Reagents | Volume |
|---|---|
| 2×PCR Premix Buffer | 25 $\mu$L |
| DNA template | About 100-500 ng |
| Forward primer (10 $\mu$M) | 1 $\mu$L |
| Reverse primer (10 $\mu$M) | 1 $\mu$L |
| Double distilled water | to 50 $\mu$L |
| DMSO | 1.5 $\mu$L |

and amplification was performed according to the following PCR program:

| Temperature | Time | |
|---|---|---|
| 95°C | 4 min | |
| 95°C | 15 sec | |
| 58°C | 30 sec | |
| 72°C | 30 sec | Repeat the 3 steps for 35 cycles |
| 72°C | 7 min | |
| 4°C | maintenance | |

and the PCR products were analyzed by Sanger sequencing.

Analysis of Crispr Cas9 knockout efficiency

By using the Tracking of Indels by Decomposition (Tide) method, the Crispr Cas9 knockout efficiency was analyzed based on the Sager sequencing data. For specific methods, see (Brinkman et al, Nucl. Acids Res. (2014) or shinyapps.datacurators.nl/tide/). Knockout efficiency analysis was performed by inputting the corresponding sgRNA sequence of the present application, the pre-knockout control sequence, and the test sequence after Crispr Cas9 knockout, with the P-value threshold set to 0.001.

Different TILs may be derived from different tumor patients, wherein donors 812, 316, and 312 are patients with cutaneous malignant melanoma, donor 504 and 704 are patients with ovarian cancer, donor 713, 709, 104, and 309 are patients with cervical cancer, and donor 107 is a patient with non-small cell lung cancer.

The table below lists the knockout efficiencies of TILs derived from different tumor patients after gene editing ("-" indicates not detected:

| Knockout combination | sgRNA | Donor 812 | Donor 107 | Donor 316 | Donor 713 | Donor 709 | Donor 104 | Donor 312 | Donor 504 | Donor 704 | Donor 309 |
|---|---|---|---|---|---|---|---|---|---|---|---|
| TP4+ZC3 | TP4 | 98.7 | 96.5 | 89.2 | 95.8 | 98.4 | 98.7 | - | - | - | - |
| | ZC3 | 97.7 | 97.7 | 90.9 | 97.8 | 98.1 | 95.9 | - | - | - | - |
| TP2+ZC-11 | TP2 | 90.0 | 87.7 | 81.6 | 89.4 | - | - | - | - | - | - |
| | ZC-11 | 98.1 | 96.0 | 93.6 | 96.9 | - | - | - | - | - | - |
| TP2+ZC-13 | TP2 | 91.6 | 88.8 | 75.4 | 87.8 | 90.7 | - | 92.7 | 91.9 | 94.1 | - |
| | ZC-13 | 95.2 | 92.5 | 83.2 | 95.8 | 95.6 | - | 97.3 | 97.1 | 96.1 | - |
| TP-N20+ZC-11 | TP-N20 | 94.8 | 94.6 | 81.4 | 88.6 | - | - | - | - | - | - |
| | ZC-11 | 98.0 | 98.1 | 89.7 | 96.9 | - | - | - | - | - | - |
| TP-N20+ZC-13 | TP-N20 | 85.9 | 92.7 | 81.4 | 90.7 | 92.7 | - | - | - | - | - |
| | ZC-13 | 85.4 | 90.3 | 87.2 | 95.2 | 92.2 | - | - | - | - | - |
| SC-4+TP-N20 | SC-4 | 82.2 | 87.1 | - | - | - | - | - | - | - | - |
| | TP-N20 | 90.2 | 91.1 | - | - | - | - | - | - | - | - |
| TP4+CB 1 | TP4 | - | - | - | - | 98.7 | - | - | - | - | - |
| | CB1 | - | - | - | - | 98.7 | - | - | - | - | - |
| SC3+TP-N8 | SC3 | 90.5 | - | - | - | - | - | - | - | - | 89.1 |
| | TP-N8 | - | - | - | - | - | - | - | - | - | 95.9 |
| SC-4+TP-N20 +ZC-11 | SC-4 | 87.6 | 88.4 | - | - | - | - | - | - | - | - |
| | TP-N20 | 90.5 | 90.5 | - | - | - | - | - | - | - | - |
| | ZC-11 | 94.3 | 96.4 | - | - | - | - | - | - | - | - |
| SC3+TP-N8 +ZC-13 | SC3 | 95.2 | - | - | - | - | - | - | - | - | 89.5 |
| | TP-N8 | - | - | - | - | - | - | - | - | - | 96.2 |
| | ZC-13 | 96.1 | - | - | - | - | - | - | - | - | 92.6 |

The various combinations of gene editing modes in the present application can achieve a certain level of knockout efficiency.

**Example 21 Proliferation ability assay of TILs during the REP stage**

The TIL cells of each knockout combination in the present application were counted on days 10-16 after gene editing.

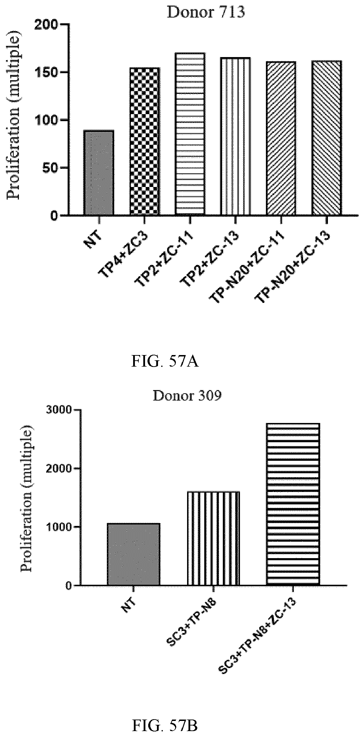

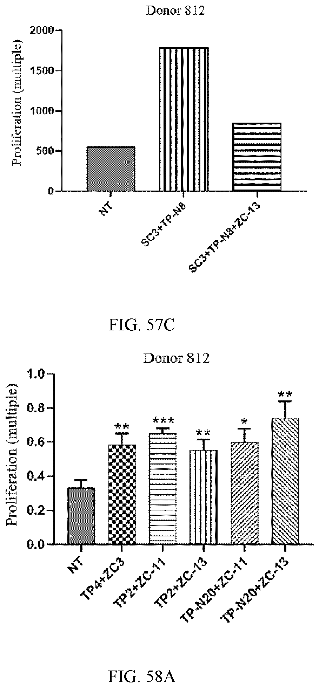

FIGs. 57A to 57C show that, in the late REP stage, TILs from multiple knockout combinations of TP+ZC, SC+TP, and SC+TP+ZC exhibited enhanced proliferation ability compared with the NT group.

**Example 22 Expansion capacity assay of TIL cells after withdrawl of IL-2**

Starting from day 8 after gene editing, each group of TIL cells were re-plated with the same total number of cells (withdrawl of IL-2), and the fluorescence amount of TIL cells at the time of plating was analyzed using the CTG kit. 3 days later, the fluorescence amount of TIL cells was analyzed again using the CTG kit. The proliferation multiple of TIL cells is characterized by the fluorescence amount on day 3/the fluorescence amount at the time of plating.

Starting from day 7 after gene editing, a 96-well plate was pretreated with 30 ng/mL of CD3 antibody (Miltenyi Biotech, OKT3) and incubated at 4°C overnight. The next day, each group of TIL cells were re-plated with the same total number of cells (withdrawl of IL-2), and the fluorescence amount of TIL cells at the time of plating was analyzed using the CTG kit (CellTiter-Glo Luminescent Cell Viability Assay, Promega). 3 days later, the fluorescence amount of TIL cells was analyzed again using the CTG kit. The proliferation multiple of TIL cells is characterized by the fluorescence amount on day 3/the fluorescence amount at the time of plating.

Starting from day 8 after gene editing, each group of TIL cells were re-plated with the same total number of cells (withdrawl of IL-2), the TILs were stimulated with CD28 antibodies (e.g., adding transACT comprising CD3 antibodies and CD28 antibodies), and the fluorescence amount of TIL cells at the time of plating was analyzed using the CTG kit. 3 days later, the fluorescence amount of TIL cells was analyzed again using the CTG kit. The proliferation multiple of TIL cells is characterized by the fluorescence amount on day 3/the fluorescence amount at the time of plating.

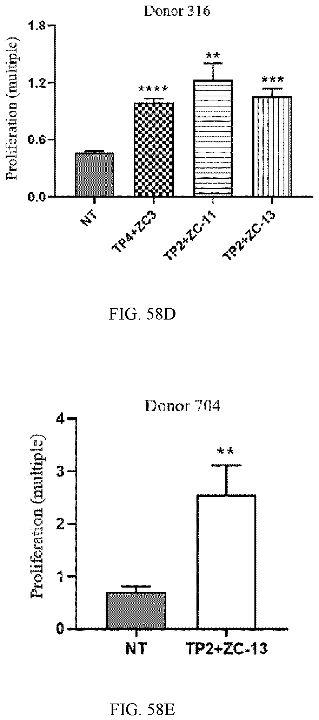

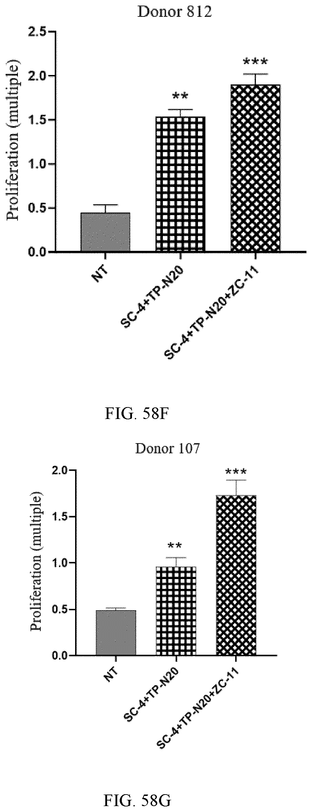

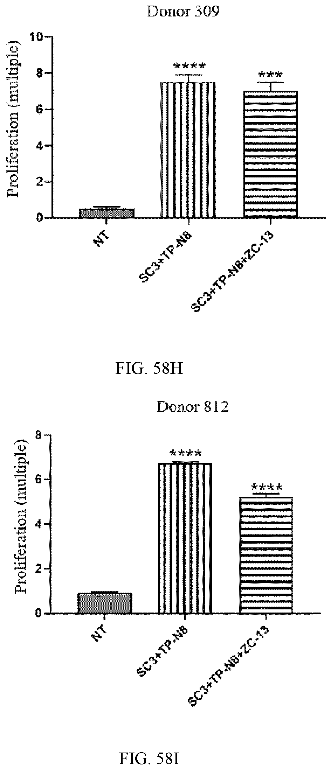

FIGs. 58A to 58I show that, after withdrawal of IL-2, TILs from multiple knockout combinations of TP+ZC, SC+TP, and SC+TP+ZC exhibited significantly enhanced proliferation ability compared with the NT group.

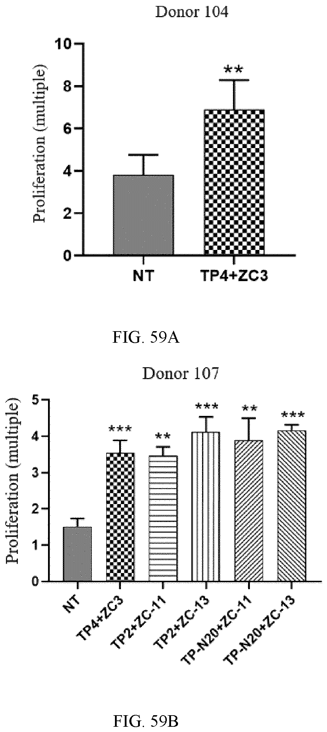

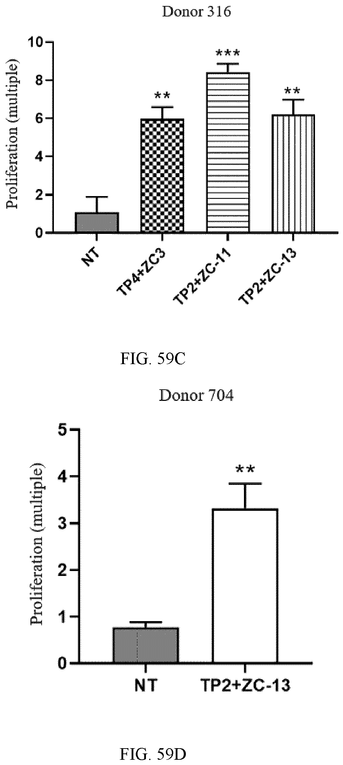

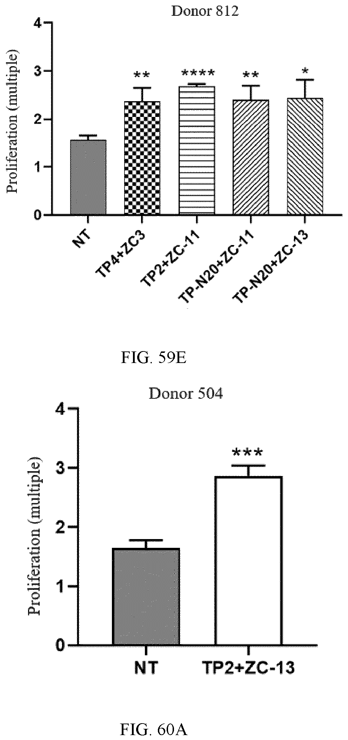

FIGs. 59A to 59E show that, after withdrawal of IL-2, under additional stimulation of OKT3, TILs from multiple knockout combination of TP+ZC exhibited significantly enhanced proliferation ability compared with the NT group.

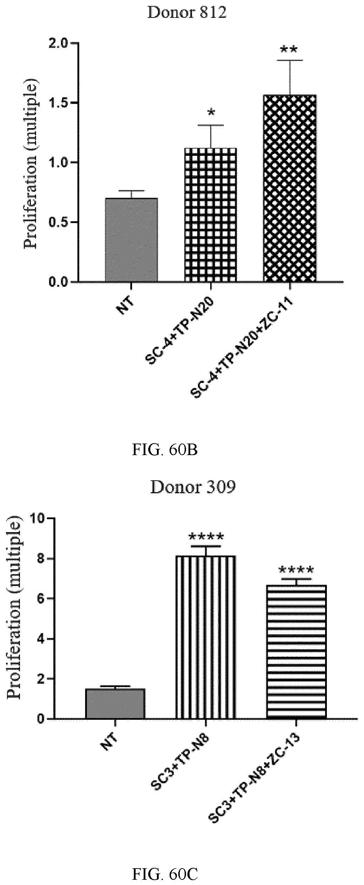

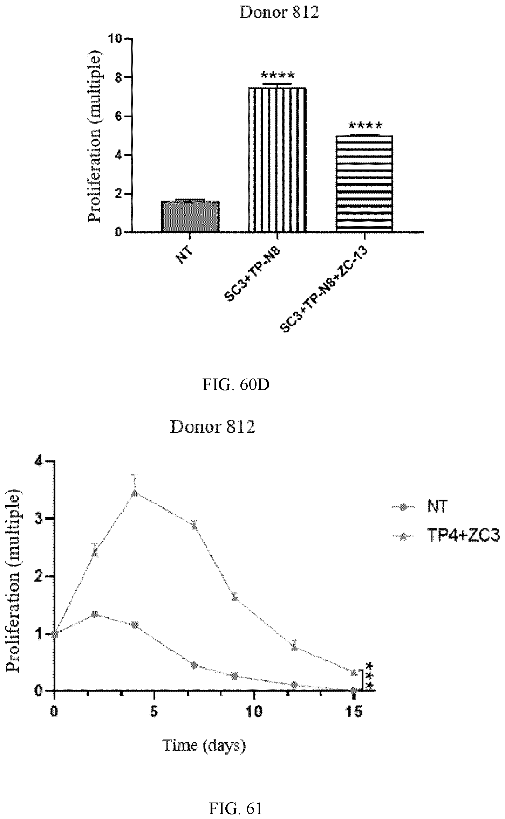

FIGs. 60A to 60D show that, after withdrawal of IL-2, under additional stimulation of transACT, TILs from multiple knockout combinations of TP+ZC, SC+TP, and SC+TP+ZC exhibited significantly enhanced proliferation ability compared with the NT group.

The results show that, the combined gene-edited TIL cells in the present application, after withdrawal of IL-2, may exhibit significantly enhanced expansion capacity.

**Example 23 Long-term persistence assay of TIL cells**

On day 10 after gene editing, the TILs from each knockout combination in the present application were re-plated on a 6-well suspension plate (withdrawl of IL-2) at the same total cell number (3,000,000 cells/well), and counted every 2-4 days.

FIG. 61 shows that, TIL cells with combined gene editing (TP4+ZC3) in the present application may have significantly enhanced long-term persistence *in vitro.*

**Example 24 Killing ability assay of TIL cells against tumor cells**

Starting from day 7 after gene editing, A375, Hcc827, and Caski tumor cell lines were plated in a 96-well flat-bottom plate. The next day, each group of TIL cells were co-cultured with the above tumor cells at an effector-target ratio (TIL cells: tumor cells, E:T) of 4:1, 3:1 or 1:1. Each 100 µL of tumor cells and TIL cells were added, and three replicate wells were set for each group. At the same time, a control group comprising only the tumor cells was set.

According to the instruction of apoptosis detection reagent (Incucyte Caspase-3/7 Green Dye for Apoptosis, Sartorius), the apoptosis detection reagent was added at 0.2 µL/well, and the culture medium was added at 25 µL/well to dilute the Caspase 3/7 Green Dye. The activity of Caspase 3/7 was recorded using an Incucyte recorder (Sartorius) to analyze the killing ability of TILs against tumor cells, with the results recorded once every 3 hours.

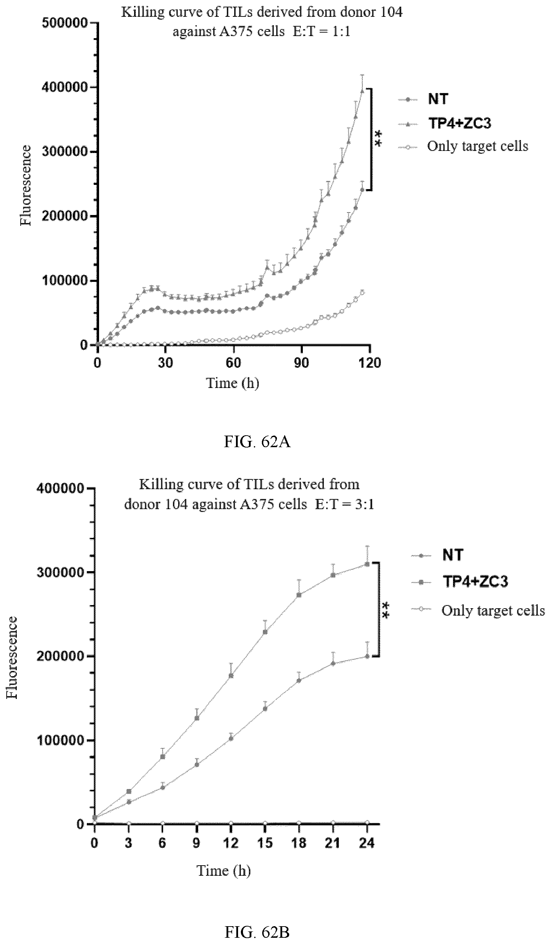

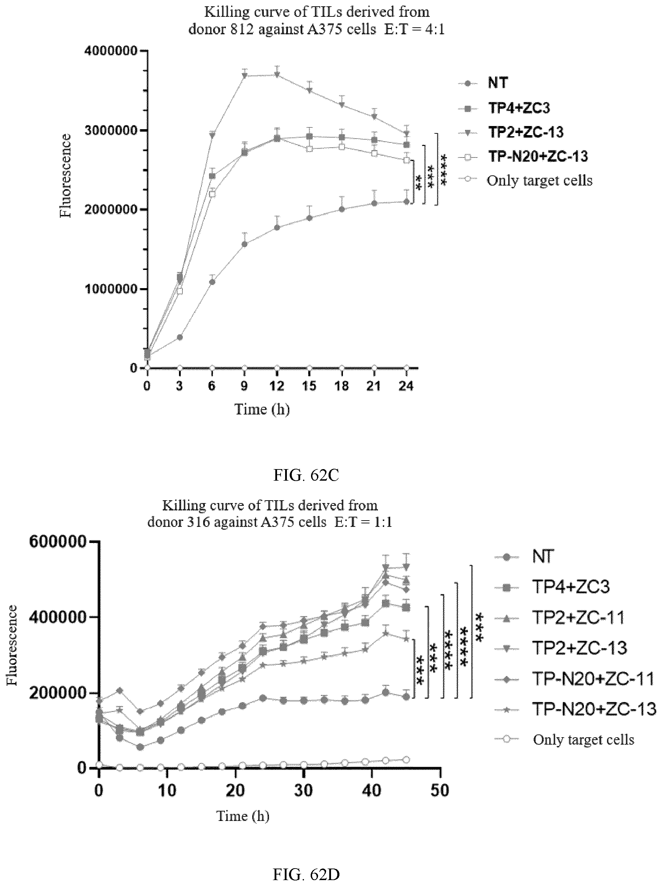

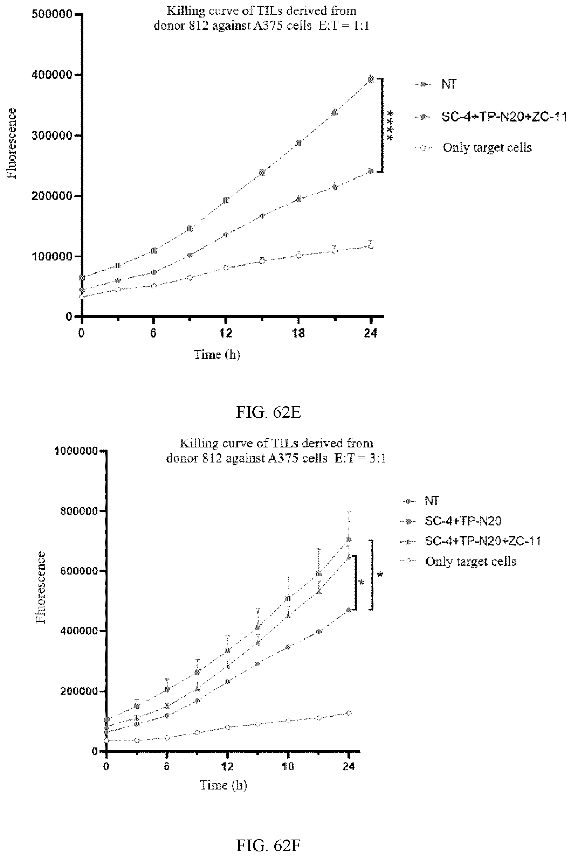

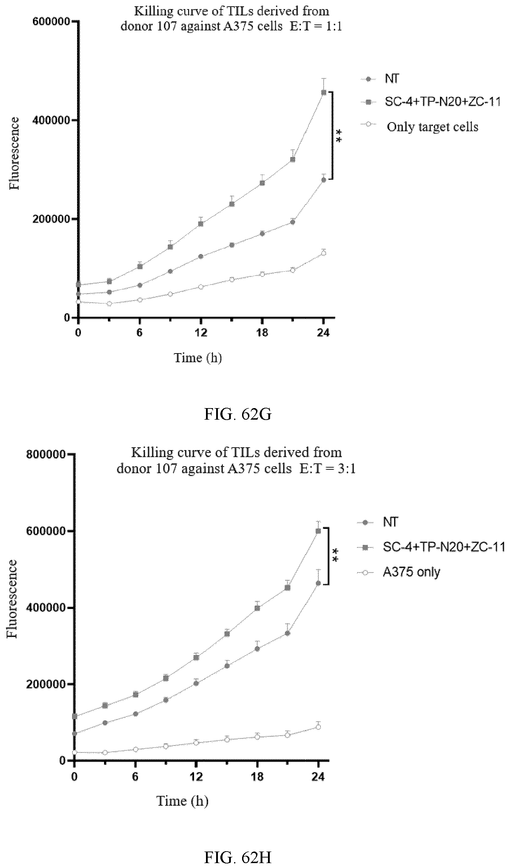

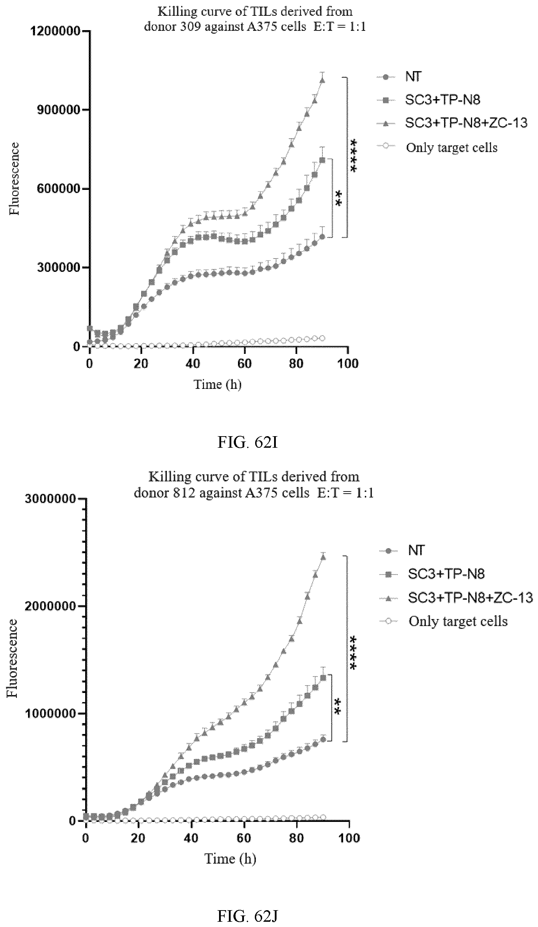

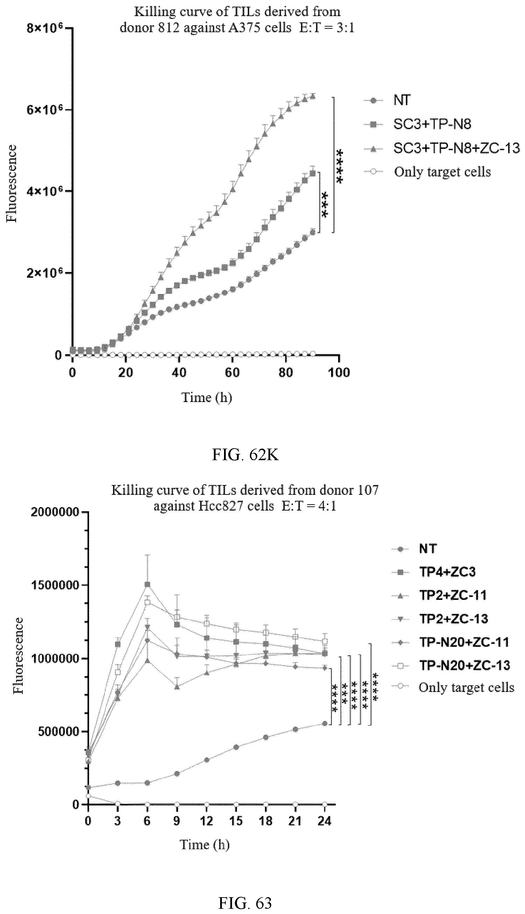

FIGs. 62A to 62K show the test results of the killing ability of TIL cells derived from donors 104, 812, 316, 107, and 309 which were co-cultured with A375 tumor cells at an effector-target ratio of 4:1, 3:1 or 1:1. TILs from multiple knockout combinations of TP+ZC, SC+TP, and SC+TP+ZC exhibited significantly enhanced tumor cell killing ability compared with the NT group.

FIG. 63 shows the test results of the killing ability of TIL cells derived from donor 107 which were co-cultured with Hcc827 tumor cells at an effector-target ratio of 4:1. TILs from multiple knockout combination of TP+ZC exhibited significantly enhanced tumor cell killing ability compared with the NT group.

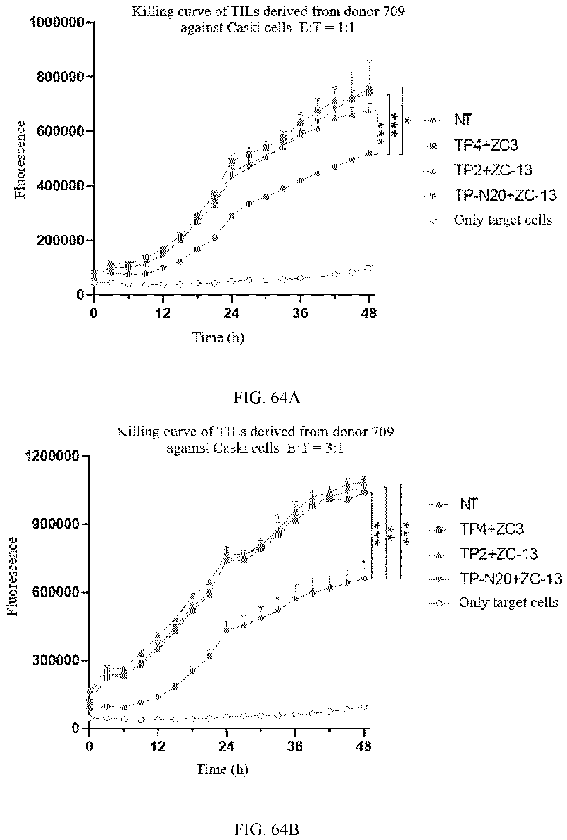

FIGs. 64A to 64B show the test results of the killing ability of TIL cells derived from donor 709 which were co-cultured with Caski tumor cells at an effector-target ratio of 3:1 or 1:1. TILs from multiple knockout combination of TP+ZC exhibited significantly enhanced tumor cell killing ability compared with the NT group.

The results show that, the combined gene-edited TIL cells in the present application may exhibit more significant tumor cell killing ability.

**Example 25 Flow cytometry for the expression of TIL cytokine**

The TIL populations obtained on day 7 or 8 after gene editing for each test group were tested by a flow cytometer for the expression of cytokine.

Test preparation

For the CD3 antibody group, a flat-bottom 96-well plate was coated with 30 ng/ml of CD3 antibody (Miltenyi Biotech, OKT3) 1 day in advance and overnight at 4°C.

Formulation of the culture medium required for the intracellular factor expression assay: the culture medium of T cells was taken, into which were added Golgistop at 0.7:1000, Golgiplug at 1: 1000, and CD107a antibody at 1:500 (i.e., 2 µL/mL) by volume ratio. No interleukin was added.

Assay steps

TILs from each test group were centrifuged, and then resuspended to $1 \times 10^6$ cells/mL using 600 μL of the culture medium required for the above intracellular factor expression assay, added to a 96-well plate at 200 μL/well, and for the CD28 antibody (for example, adding transACT comprising CD3 antibodies and CD28 antibodies) stimulation group, added at a volume ration of 1/1000 transACT, and incubated overnight in an incubator at 37°C.

At the end of the incubation, the cells were washed once with PBS at 200 μL/well, and centrifuged at 600 g for 3 minutes. The supernatant was discarded. An antibody mixed working solution of CD3/CD4/CD8 (BD) was formulated for cell surface staining, with an antibody concentration of 1:100, a viability of 1:10000, and a staining volume of 50 μL/well for 96-well plates and 100 μL/tube for flow tubes. Incubation was performed at 2-8°C in dark for 30 minutes. The reagents required for the staining of transcription factors was formulated during the staining process: Transcription Factor Buffer Set (BD) was used to dilute a $4 \times$ Fixation/Permeabilization Solution (BD) to produce $1 \times$ working solution A; double distilled water was used to dilute a $5 \times$ Perm/Wash Buffer (BD) to produce $1 \times$ working solution B. The working solutions were pre-cooled at 4 °C for later use. After staining, an appropriate amount of PBS was added to wash the cells twice (200 μL each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100 μL/well for 96-well plates, and 1 mL/tube for flow tubes) of $1 \times$ working solution A was added for fixation and permeabilization. Incubation was performed at 2-8°C in dark for 40-50 minutes. At the end of fixation and permeabilization, $1 \times$ working solution B was added to wash the cells (200 μL each time for 96-well plates, and 2 mL each time for flow tubes). Centrifugation was performed at 2-8°C and at 350 g for 6 minutes, and washing was carried out twice. $1 \times$ working solution B was used to formulate intracellular antibodies (CD107a, GZMB, TNF-$\alpha$, and IFN-$\gamma$, BD/BioLegend) at an antibody concentration of 1:100 to 1:200, with 50 μL/well for 96-well plates, and 100 μL/tube for flow tubes. Staining was performed at 2-8°C in dark for 30 minutes. At the end of staining, $1 \times$ working solution B was added to wash the cells (200 μL each time for 96-well plates, and 2 mL each time for flow tubes). Centrifugation was performed at 2-8°C and at 350 g for 6 minutes, and washing was carried out twice. The cells were resuspended in 100-500 μL of PBS for flow cytometry test on machine.

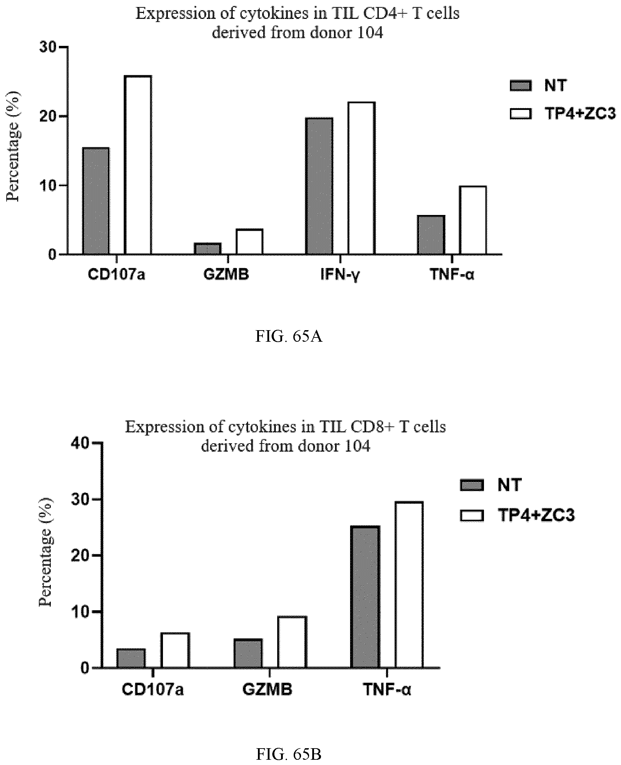

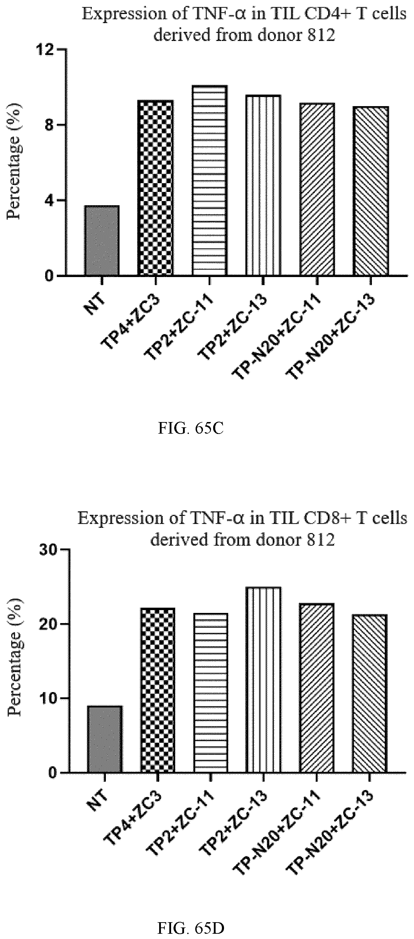

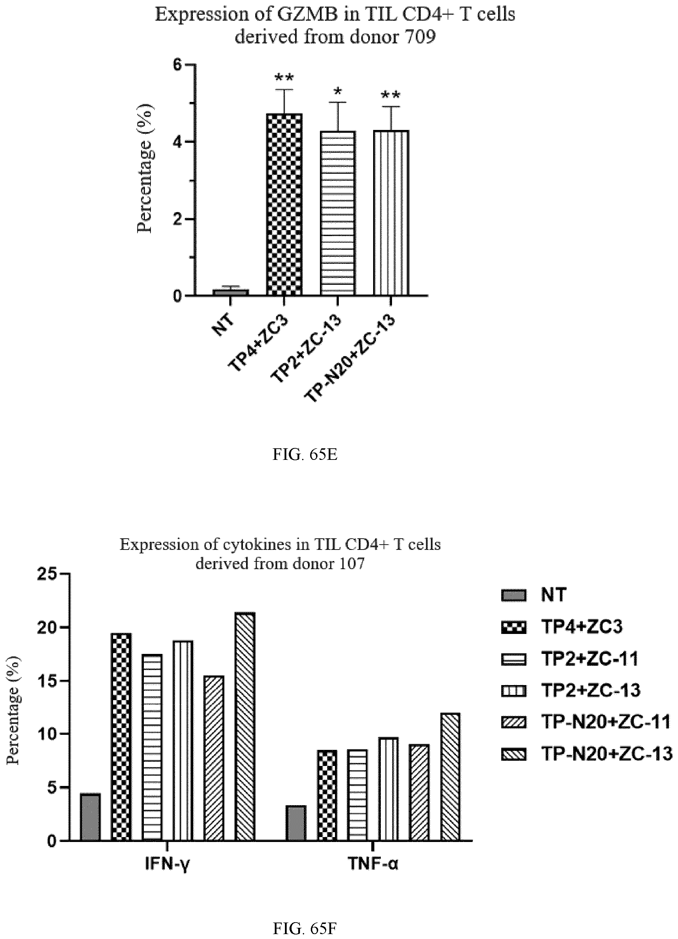

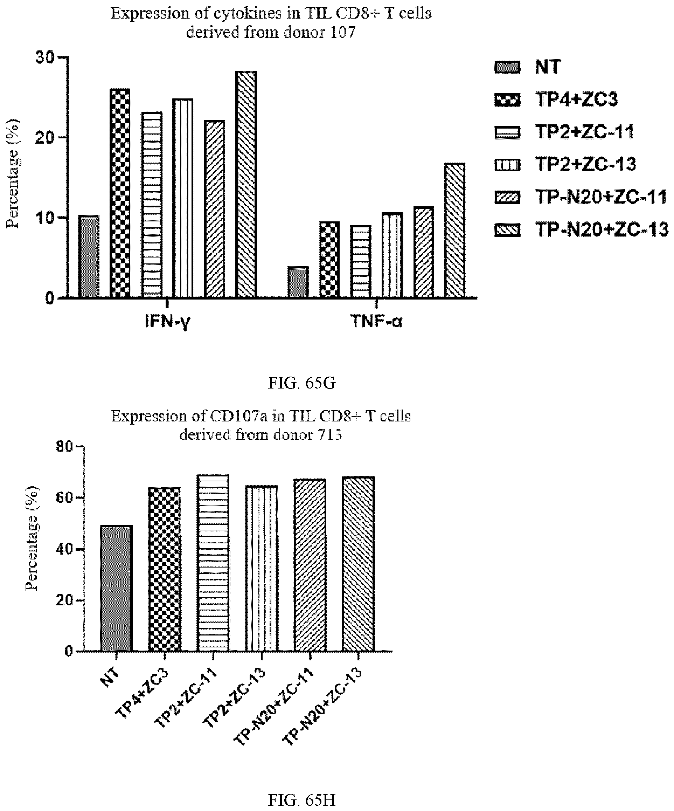

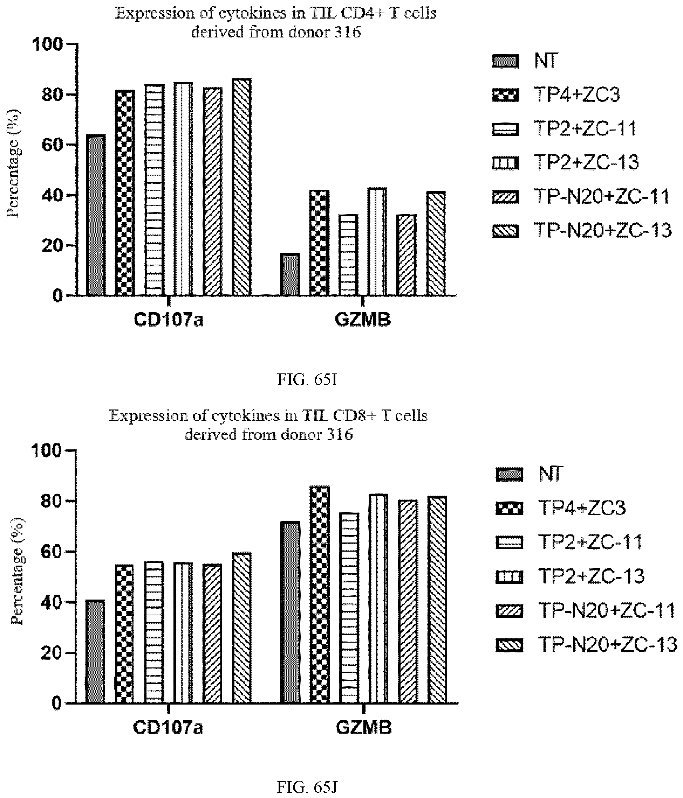

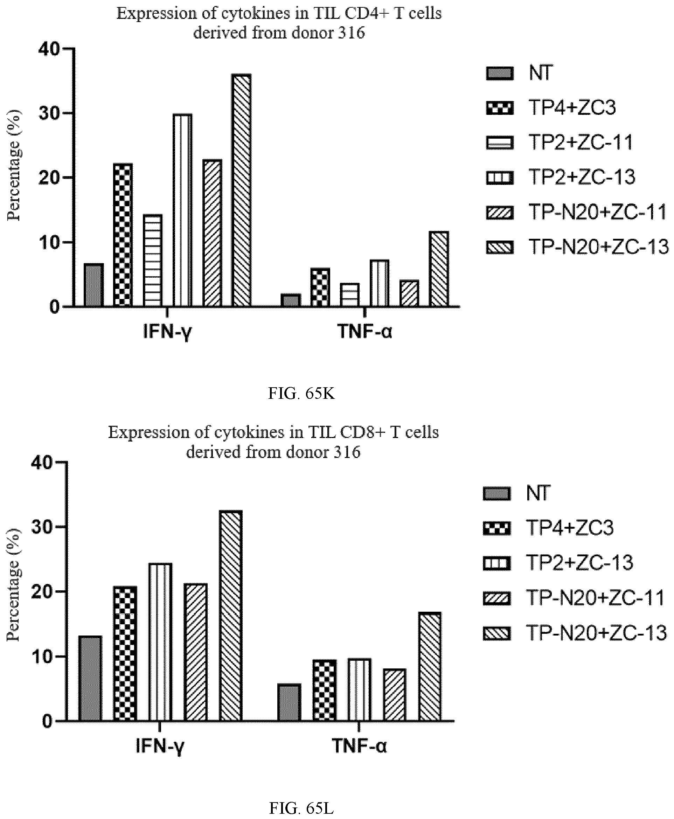

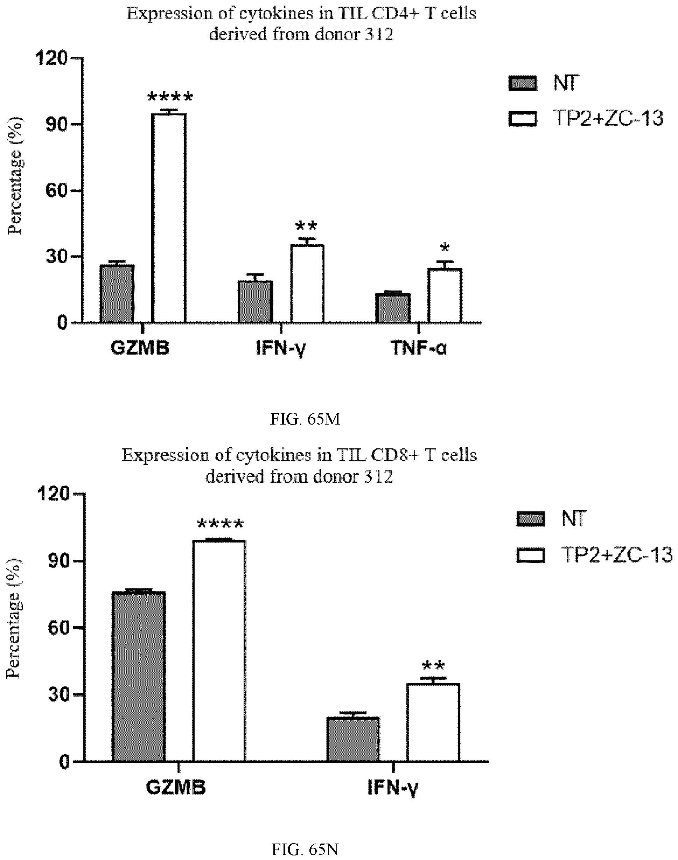

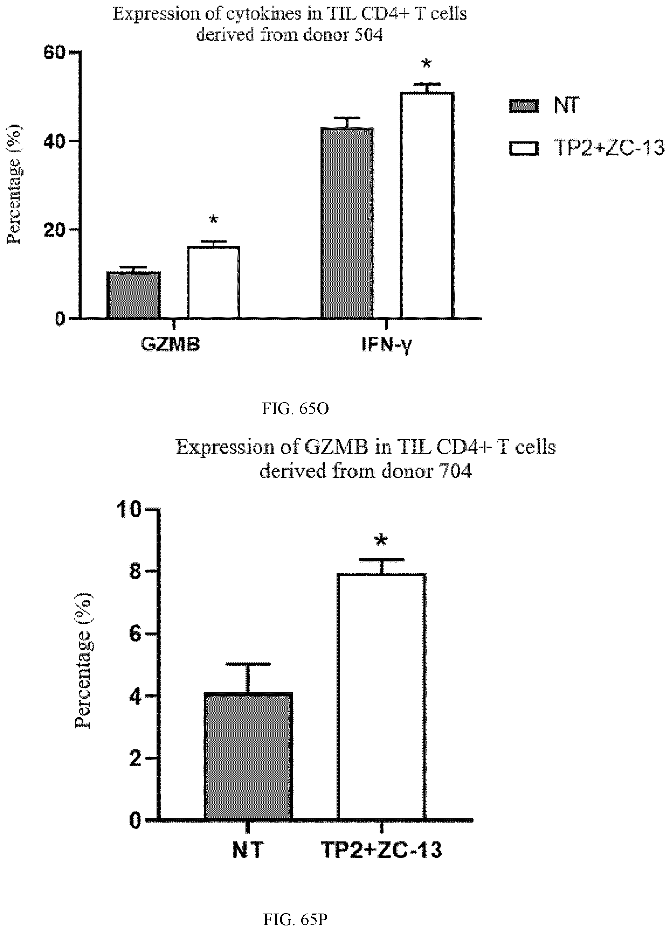

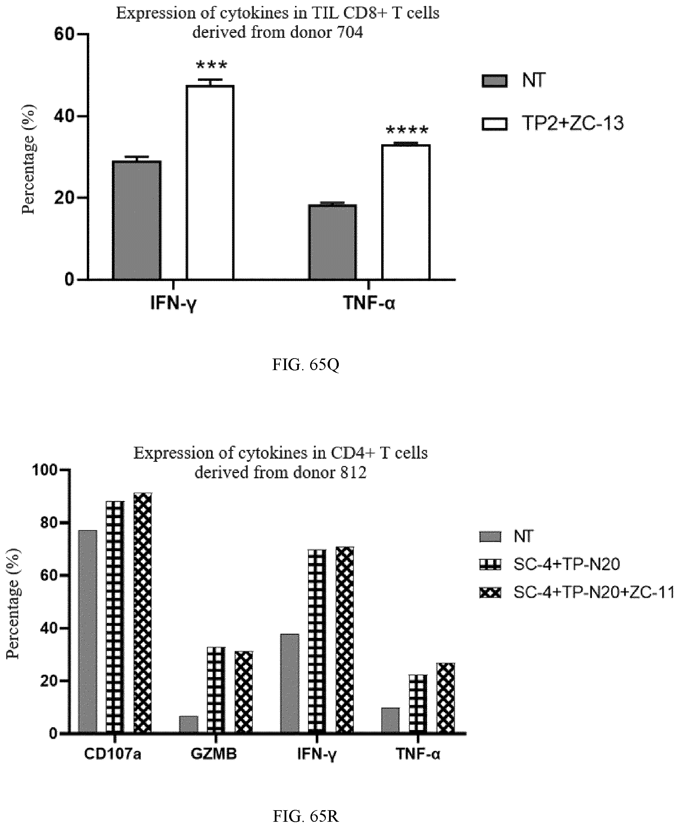

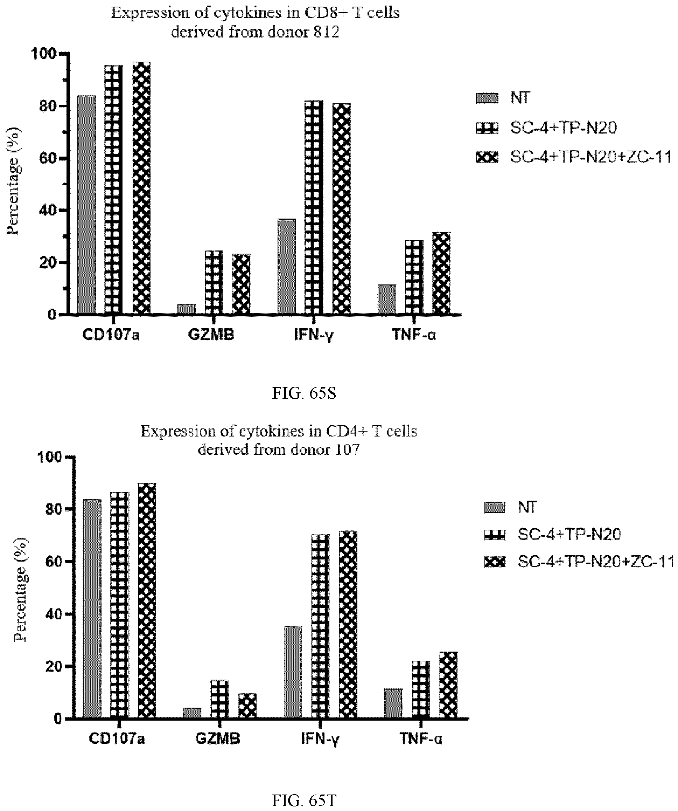

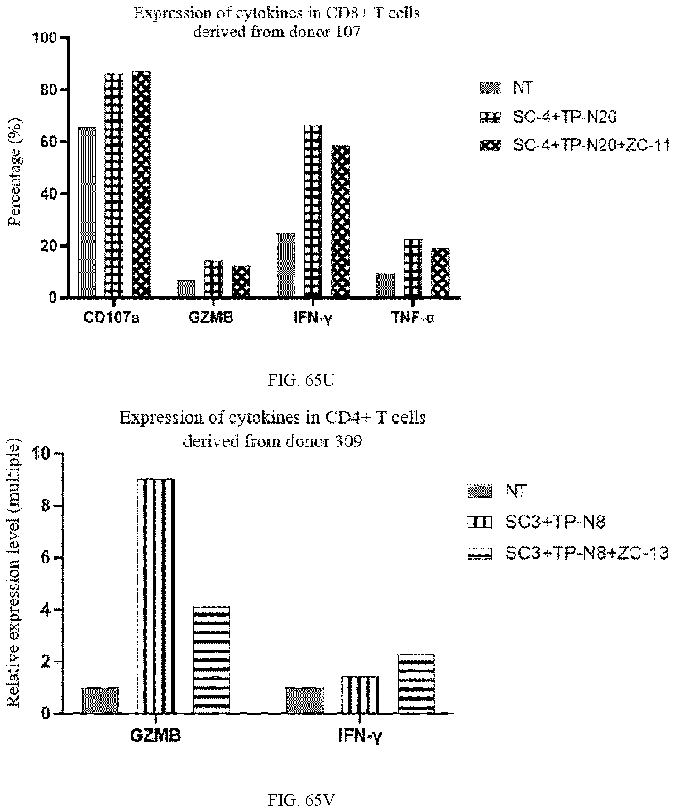

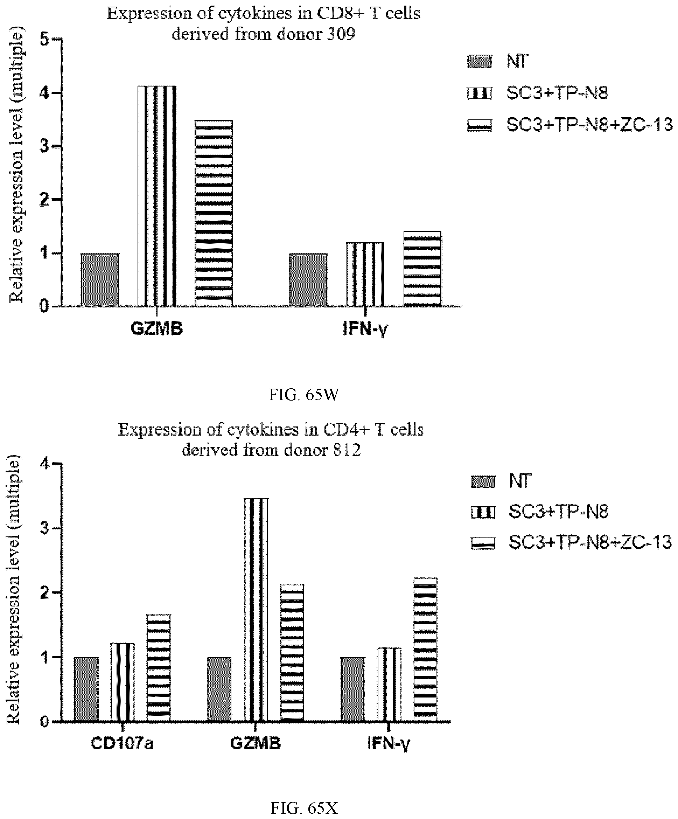

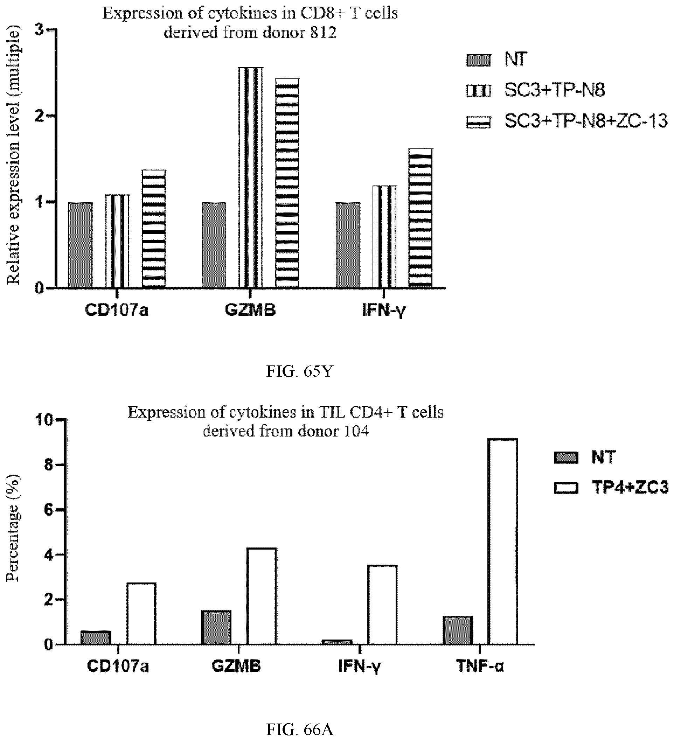

FIGs. 65A to 65Y show the relative expression level of CD107a, TNF-$\alpha$, GZMB, and IFN-$\gamma$ in each group of TIL cells in the absence of any stimulants, for TIL cells derived from donors 104, 812, 709, 107, 713, 316, 312, 504, 704, and 309. TILs from multiple knockout combinations of TP+ZC, SC+TP, and SC+TP+ZC exhibited significantly enhanced intracellular cytokine expression capacity compared with the NT group.

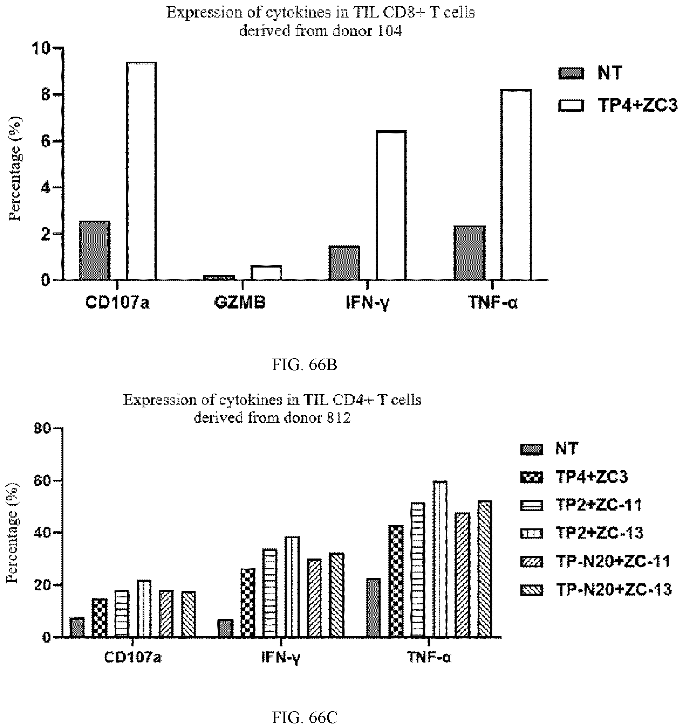

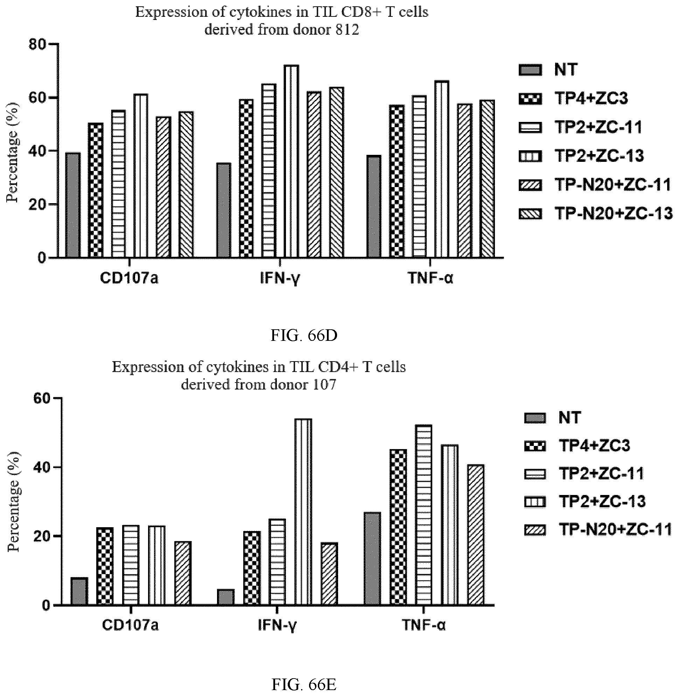

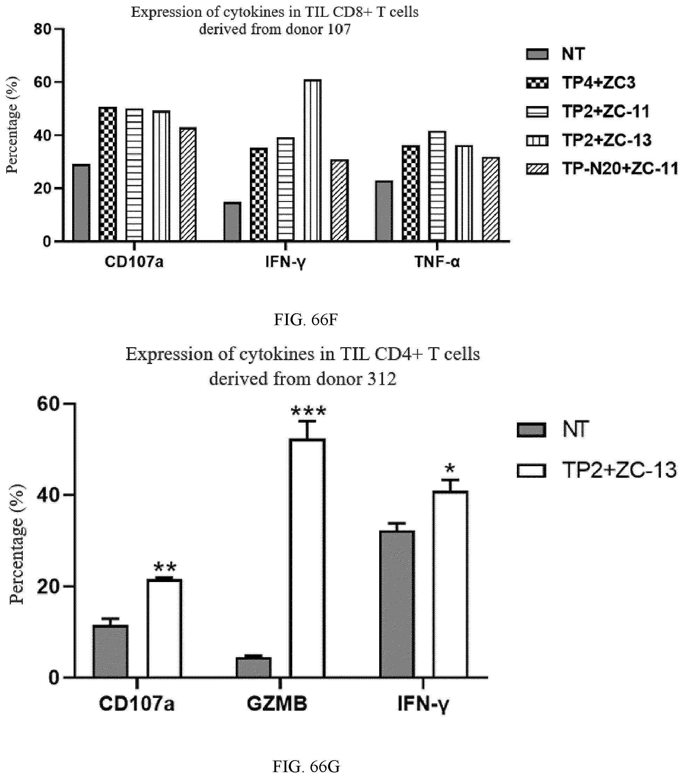

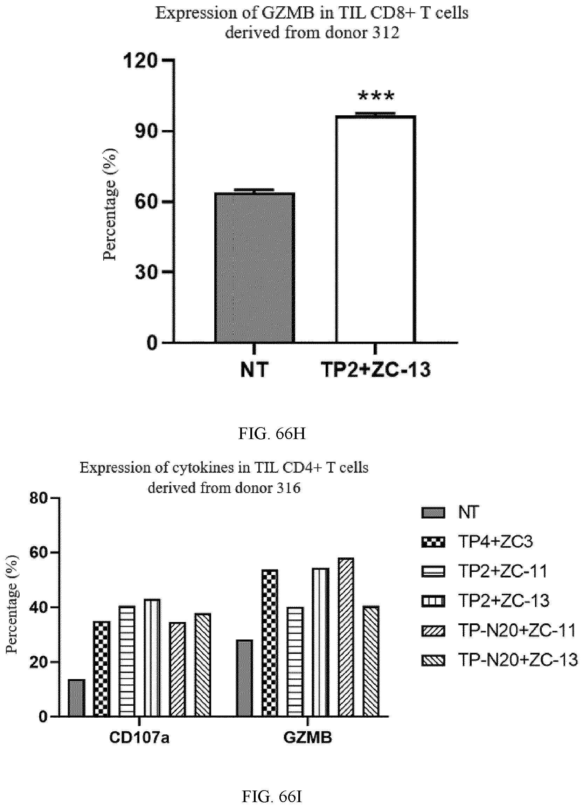

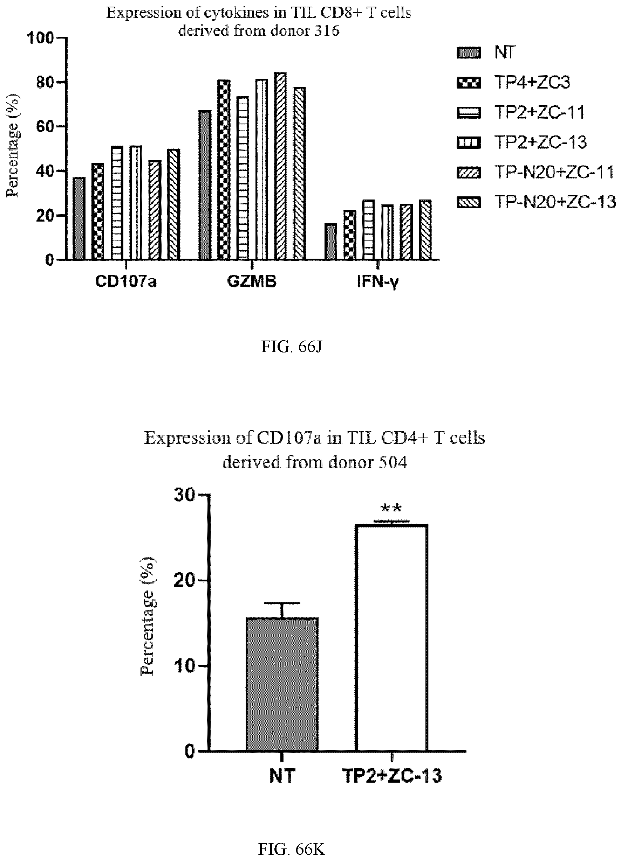

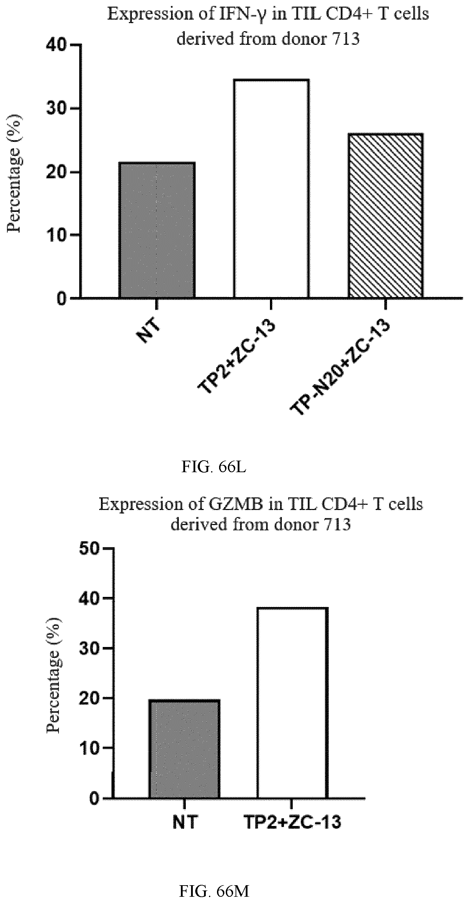

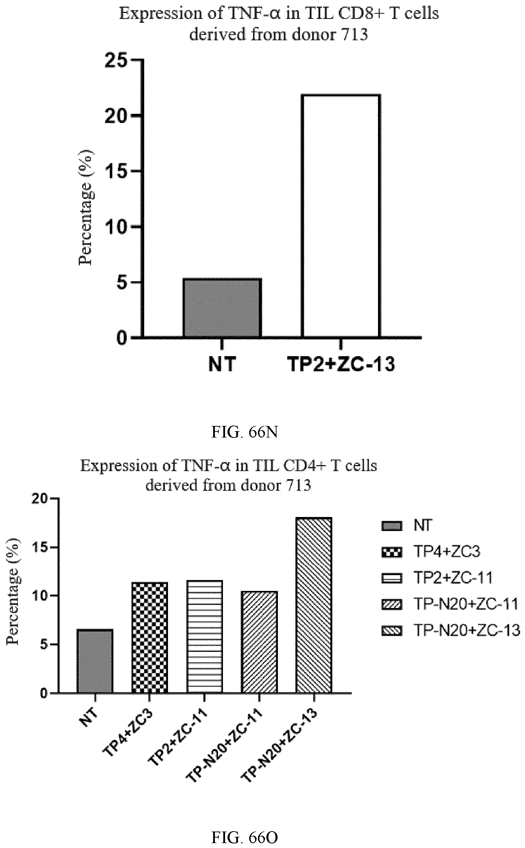

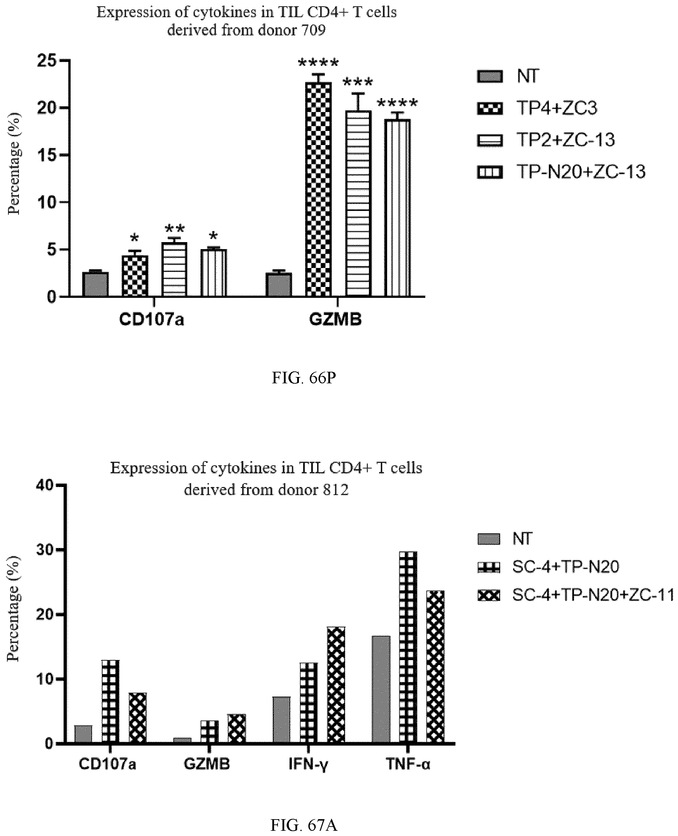

FIGs. 66A to 66P show the relative expression level of CD107a, TNF-$\alpha$, GZMB, and IFN-$\gamma$ in each group of TIL cells under the stimulation of OKT3, for TIL cells derived from donors 104, 812, 107, 312, 316, 504, 713, 709, and 309. TILs from multiple knockout combination of TP+ZC exhibited significantly enhanced intracellular cytokine expression capacity compared with the NT group.

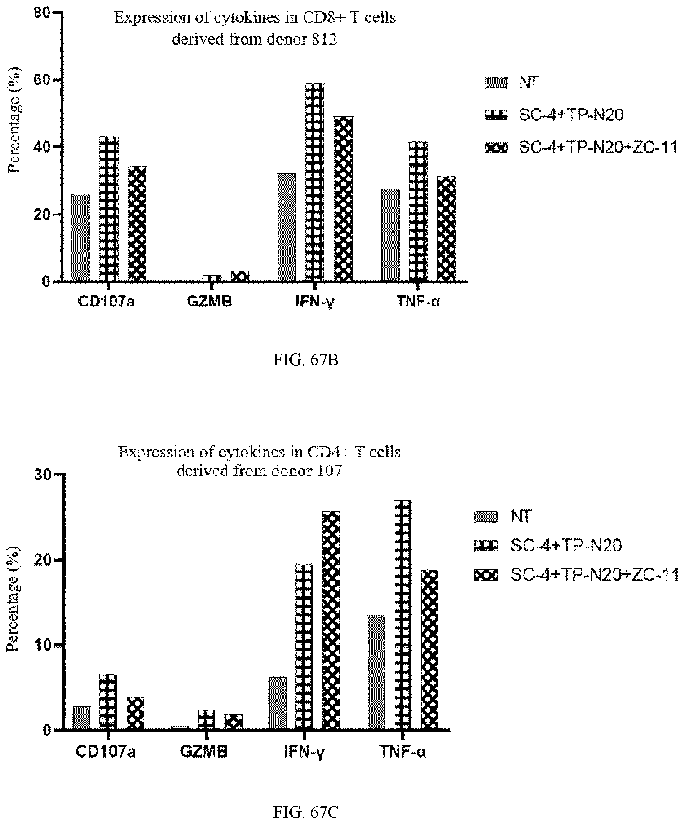

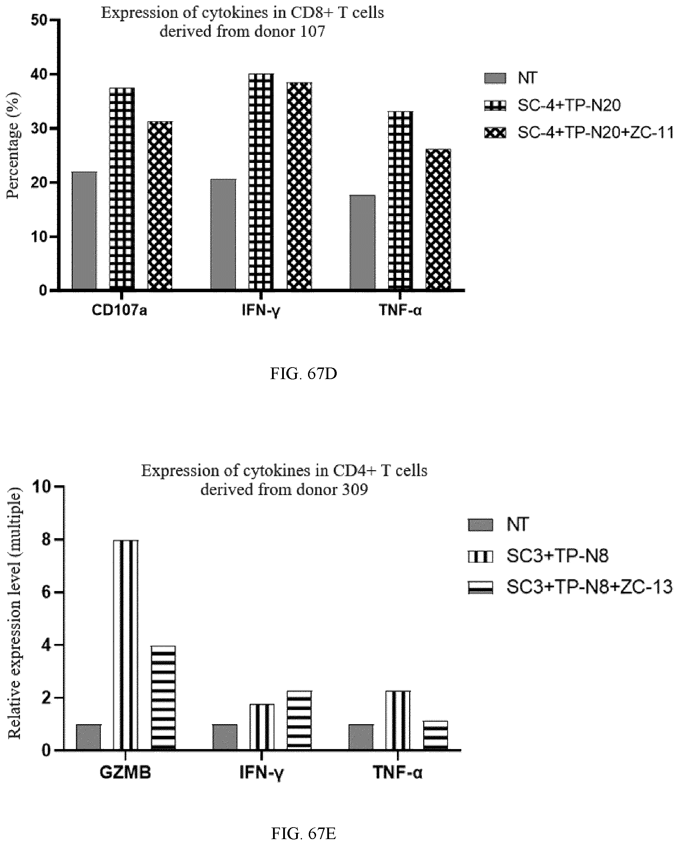

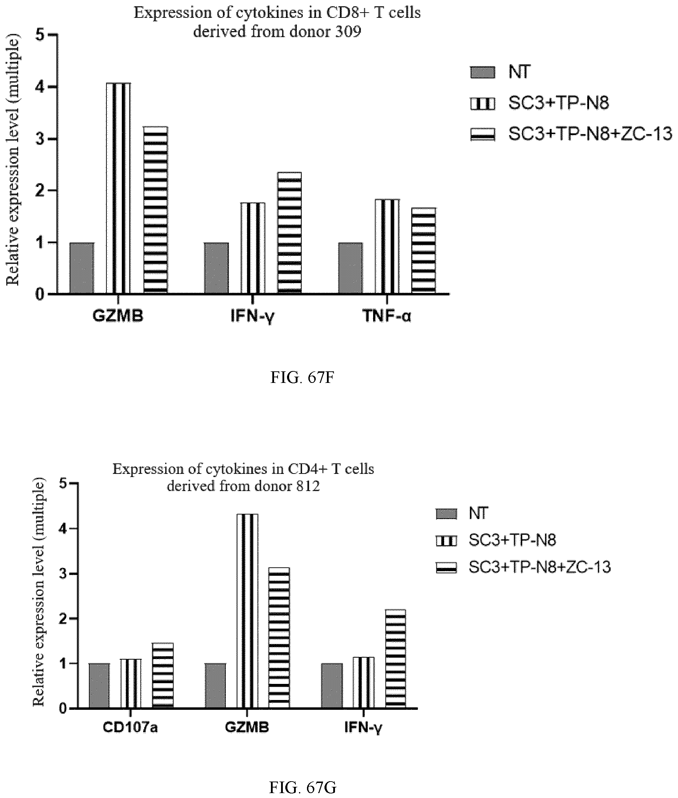

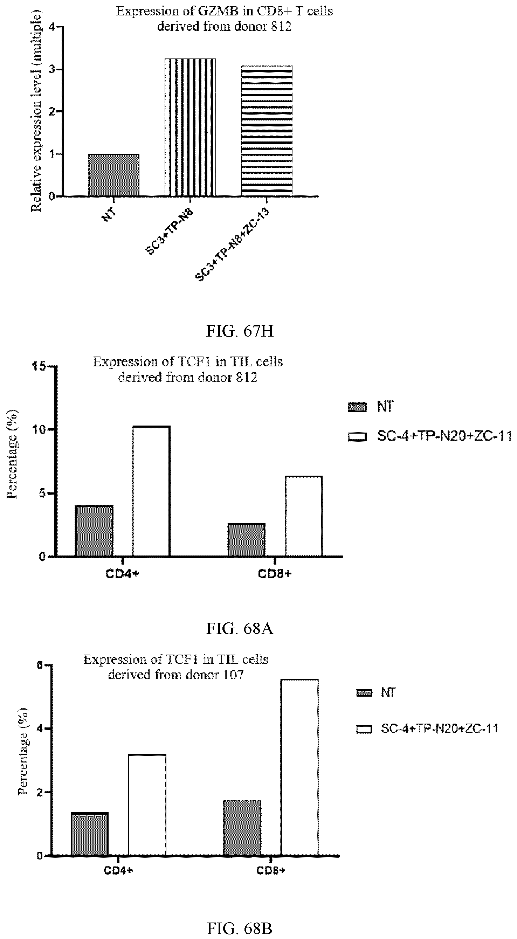

FIGs. 67A to 67H show the relative expression level of CD107a, TNF-$\alpha$, GZMB, and IFN-$\gamma$ in each group of TIL cells under the stimulation of transACT, for TIL cells derived from donors 812, 107, and 309. TILs from multiple knockout combinations of SC+TP and SC+TP+ZC exhibited significantly enhanced intracellular cytokine expression capacity compared with the NT group.

The results show that, gene-edited TIL cells have a higher intracellular factor expression capacity. For example, a higher CD107a expression capacity, a higher IFN-$\gamma$ expression capacity, a higher TNF-$\alpha$ expression capacity, or a higher GZMB expression capacity.

**Example 26 Flow cytometry assay of TIL cell subpopulations**

The TIL populations obtained on day 8 after gene editing for each test group were tested by a flow cytometer for the expression of T cell stem molecules and depletion molecules.

Sources of test materials by TIL flow cytometry

V-bottom 96-well plate, manufacturer Corning, product number 3894; flow tube, manufacturer Corning, product number 352052.

The flow antibodies used in this example were purchased from BD or Biolegend. $1 \times 10^5$ to $5 \times 10^5$ cell samples from each group were added into flow tubes or V-bottom 96-well plates, and centrifuged at 600 g for 3 minutes, the supernatant was discarded. Washing was carried out once using PBS, at 1 mL/tube for flow tubes, and 200 μL/well for 96-well plates, and the supernatant was discarded. A prepared antibody working solution was added for cell surface staining. The concentration of the antibodies (BD or Biolegend) was 1: 100 to 1:200, and the active detection dye was comprised at 1: 10000. Staining was carried out at 100 μL/tube for flow tubes, and 50 μL/well for 96-well plates, and incubation was performed at 2-8°C in dark for 30 minutes. After surface staining, the cells were washed once with PBS (200 μL each time

for 96-well plates, 1 mL each time for flow tubes), and centrifuged at 600 g at room temperature for 3 minutes. After the centrifugation, the supernatant was discarded. The cells were resuspended in 100-500 μL of PBS for flow cytometry test on machine.

For flow cytometry of intracellular factors, the reagents required for fixation and permeabilization was formulated: Transcription Factor Buffer Set (BD) was used to dilute a 4× Fixation/Permeabilization Solution (BD) to produce 1× working solution A; double distilled water was used to dilute a 5 × Perm/Wash Buffer (BD) to produce 1 × working solution B. The working solutions were pre-cooled at 4 °C for later use. After surface staining, an appropriate amount of PBS was added to wash the cells twice (200 μL each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100 μL/well for 96-well plates, and 1 mL/tube for flow tubes) of 1× working solution A was added for fixation and permeabilization. Incubation was performed at 2-8°C in dark for 40-50 minutes. At the end of fixation and permeabilization, 1 × working solution B was added to wash the cells (200 μL each time for 96-well plates, and 2 mL each time for flow tubes). Centrifugation was performed at 2-8°C and at 600 g for 3 minutes, and washing was carried out twice. 1× working solution B was used to formulate intracellular antibodies with 50 μL/well for 96-well plates, and 100 μL/tube for flow tubes, and the antibody concentration was 1:100 to 1:200. Staining was performed at 2-8°C in dark for 30 minutes. At the end of staining, 1× working solution B was added to wash the cells (200 μL each time for 96-well plates, and 2 mL each time for flow tubes). Centrifugation was performed at 2-8°C and at 600 g for 3 minutes, and washing was carried out twice. The cells were resuspended in 100-500 μL of PBS for flow cytometry test on machine.

Combined editing of multiple target genes in the present application

FIGs. 68A to 68B show the proportion of TCF1-positive cells in each group of TIL cells, for TIL cells derived from donors 812 and 107. The results show that, TILs from the knockout combination of SC+TP+ZC exhibited significantly enhanced expression capacity of TCF1 compared with the NT group. Therefore, gene-edited TIL cells have higher sternness, such as higher expression capacity of TCF1.

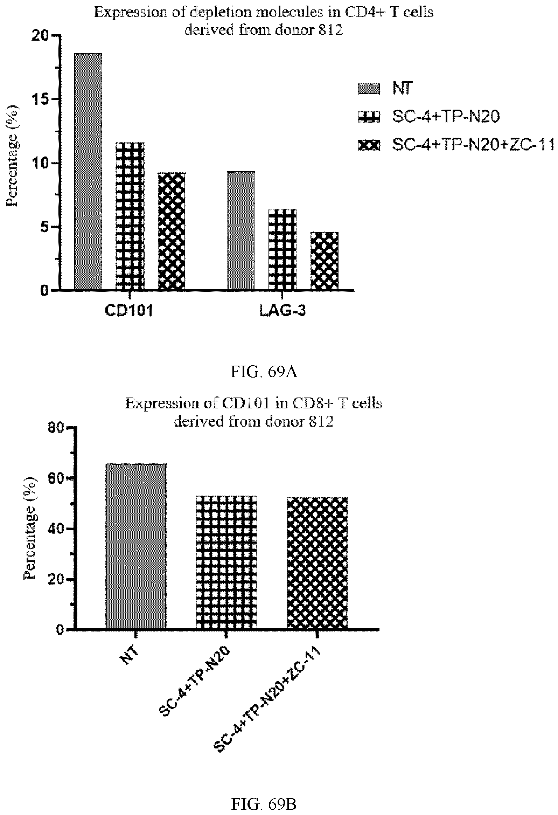

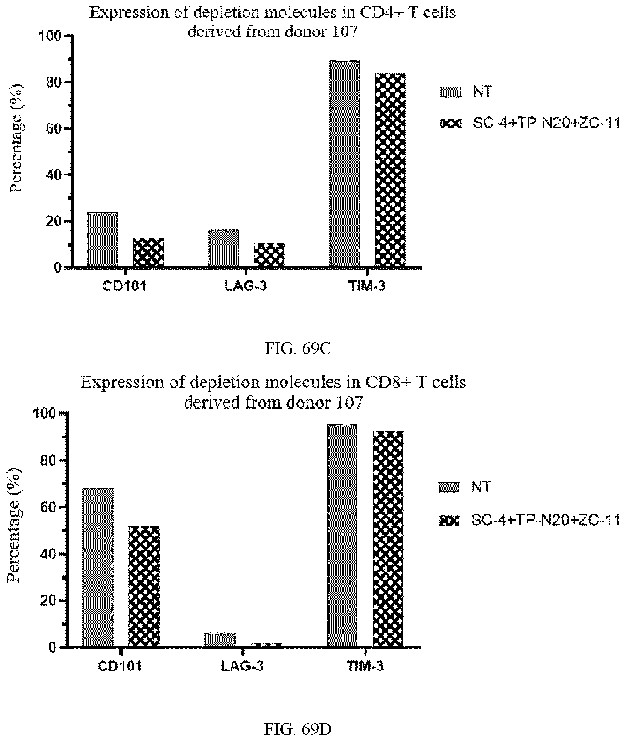

FIGs. 69A to 69D show the proportion of CD101-positive, LAG-3-positive, and TIM-3-positive cells in each group of TIL cells, for TIL cells derived from donors 812 and 107. The results show that, the TILs from the knockout combinations of SC+TP and SC+TP+ZC exhibited significantly reduced expression capacity of CD101, LAG-3, and TIM-3 compared with the NT group. Therefore, gene-edited TIL cells have reduced expression of T cell depletion molecules, such as lower expression capacity of CD101, LAG-3, and TIM-3.

## Example 27 Coculture assay of autologous tumor cells

Preparation of autologous tumor cells: The donor tumor tissue obtained in Example 1 was thoroughly minced using surgical scissors and collagenase was added. The mixture was then processed using a single-cell preparation instrument to obtain a single-cell suspension of the donor tumor tissue, i.e., autologous tumor cells.

The TIL populations (withdrawal of IL-2) obtained on day 8 after gene editing for each test group were cocultured with the above autologous tumor cells for 12 h (at an effector-target ratio of 10:1), and the supernatant was collected. The release level of cytokines was detected by the CBA method.

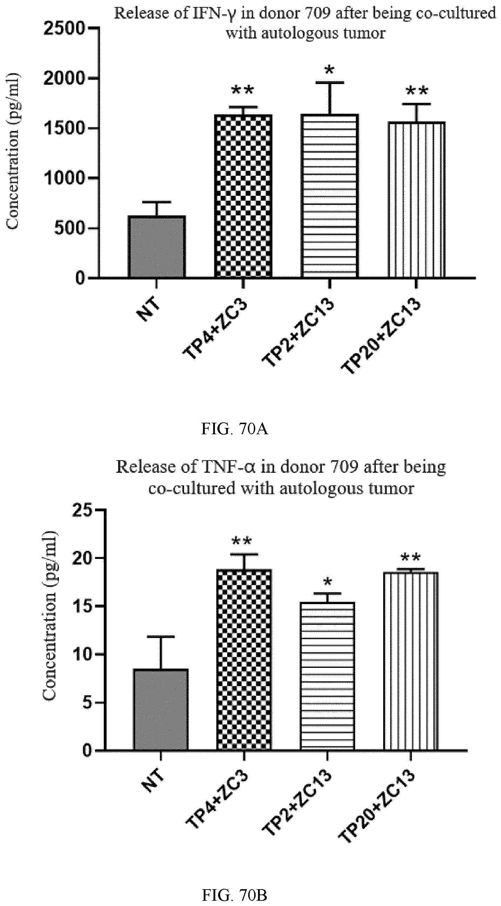

FIGs. 70A to 70B show the release level of cytokines for TIL cells derived from donor 709 after being co-cultured with autologous tumor cells. The results show that, TILs from multiple double-knockout combination of TP+ZC could significantly promote the release of IFN-γ and TNF-α after co-culture with autologous tumors compared with the NT group.

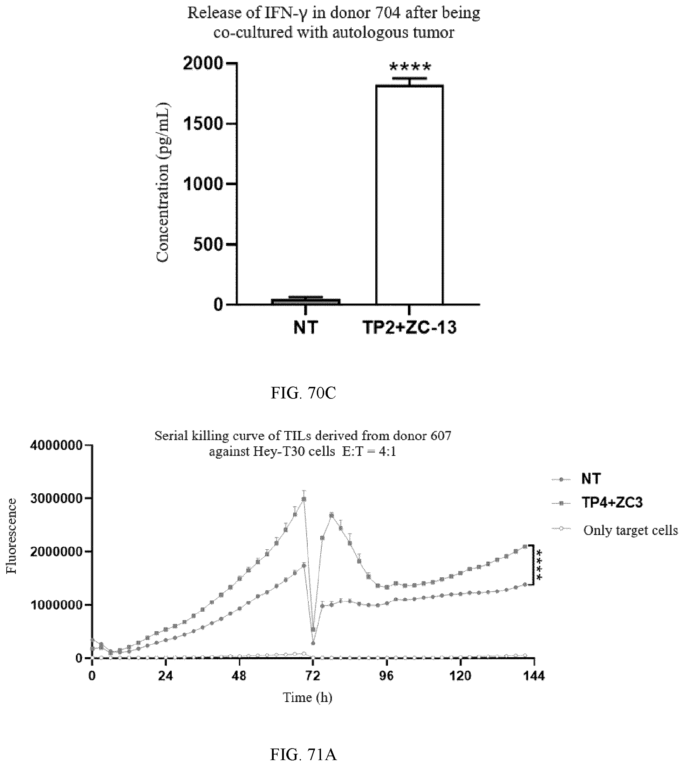

FIG. 70C shows the release level of cytokines for TIL cells derived from donor 704 after being co-cultured with autologous tumor cells. The results show that, TILs from the double-knockout combination of TP2+ZC-13 could significantly promote the release of IFN-γ after co-culture with autologous tumors compared with the NT group.

## Example 28 Serial killing ability assay of TIL cells against tumor cells

Starting from day 8 after gene editing, A375 and Hey-T30 tumor cell lines were plated in a 96-well flat-bottom plate (20,000 cells/well). 4 h later, each group of TIL cells were co-cultured with the above tumor cells at an effector-target ratio (TIL cells: tumor cells, E:T) of 4:1. Each 100 μL of tumor cells and TIL cells were added, and three replicate wells were set for each group. At the same time, a control group comprising only the tumor cells was set.

According to the instruction of apoptosis detection reagent (Incucyte Caspase-3/7 Green Dye for Apoptosis, Sartorius), the apoptosis detection reagent was added at 0.2 μL/well, and the culture medium was added at 25 μL/well to dilute the Caspase 3/7 Green Dye. The activity of Caspase 3/7 was recorded using an Incucyte recorder (Sartorius) to analyze the killing ability of TILs against tumor cells, with the results recorded once every 3 hours. Each round of killing was performed for 3 days. Half of the volume of co-culture supernatant was carefully aspirated and discarded at the beginning of each next round of killing, and tumor cells were added to each well at 20,000 cells/well (resuspended in 100 μL of T-cell culture medium). Each well was then supplemented with another 0.1 μL of apoptosis detection reagent, and the activity of Caspase 3/7 was continually recorded using the Incucyte recorder to analyze the killing ability of TILs against tumor cells, with the results recorded once every 3 hours. The ratio of the number of surviving tumor cells to the number of surviving TIL cells at the end of each round of killing was examined by flow cytometry. Two to five consecutive rounds of killing were performed.

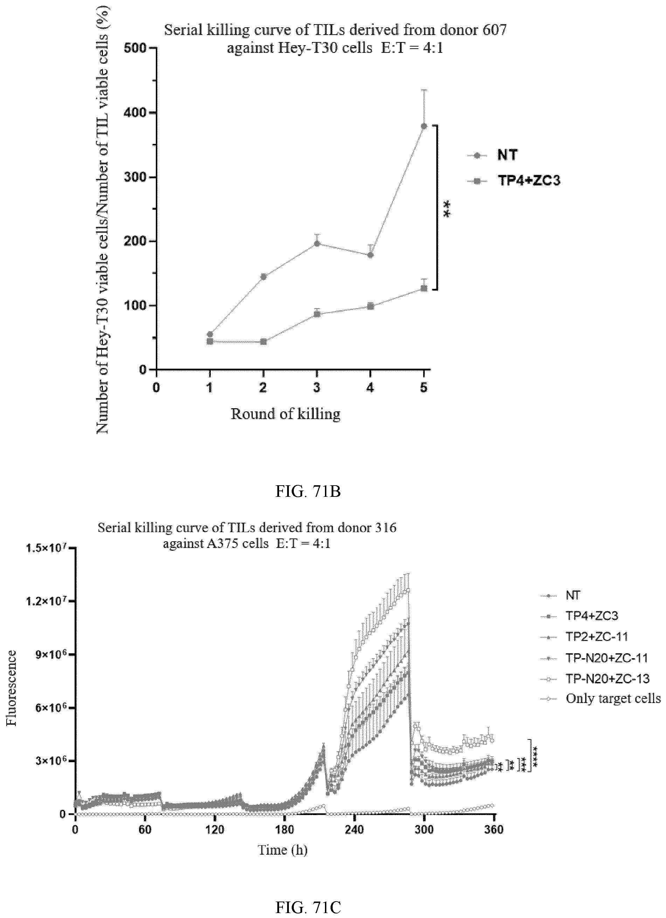

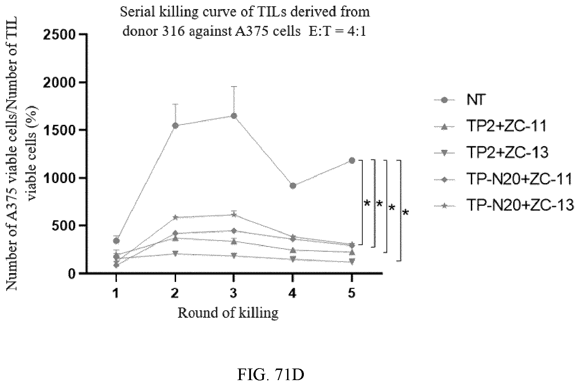

FIGs. 71A to 71B show the test results of the serial killing ability of TIL cells derived from donor 607 which were co-cultured with Hey-T30 tumor cells at an effector-target ratio of 4:1. TILs from the knockout combination of TP4+ZC3 exhibited significantly enhanced tumor cell serial killing ability compared with the NT group.

FIGs. 71C to 71D show the test results of the serial killing ability of TIL cells derived from donor 316 which were co-cultured with A375 tumor cells at an effector-target ratio of 4:1. TILs from multiple knockout combination of TP+ZC exhibited significantly enhanced tumor cell serial killing ability compared with the NT group.

Table 1A Coordinates for human CBLB genome

| No. | Chromosome No. | Coordinate starting point | Coordinate endpoint |
|---|---|---|---|
| A-1 | chr3 | 105869597 | 105869656 |
| A-2 | chr3 | 105867881 | 105869588 |
| A-3 | chr3 | 105867783 | 105867864 |
| A-4 | chr3 | 105867636 | 105867748 |
| A-5 | chr3 | 105867400 | 105867631 |
| A-6 | chr3 | 105867287 | 105867391 |
| A-7 | chr3 | 105867239 | 105867280 |
| A-8 | chr3 | 105866980 | 105867229 |
| A-9 | chr3 | 105866874 | 105866978 |
| A-10 | chr3 | 105866634 | 105866871 |
| A-11 | chr3 | 105866591 | 105866630 |
| A-12 | chr3 | 105866272 | 105866561 |
| A-13 | chr3 | 105865967 | 105866270 |
| A-14 | chr3 | 105865692 | 105865944 |
| A-15 | chr3 | 105865618 | 105865670 |
| A-16 | chr3 | 105865556 | 105865609 |
| A-17 | chr3 | 105865409 | 105865545 |
| A-18 | chr3 | 105865312 | 105865406 |
| A-19 | chr3 | 105865228 | 105865289 |
| A-20 | chr3 | 105865106 | 105865209 |
| A-21 | chr3 | 105865047 | 105865096 |
| A-22 | chr3 | 105864979 | 105865035 |
| A-23 | chr3 | 105864915 | 105864968 |
| A-24 | chr3 | 105864680 | 105864881 |
| A-25 | chr3 | 105864631 | 105864666 |
| A-26 | chr3 | 105864585 | 105864626 |
| A-27 | chr3 | 105864296 | 105864572 |
| A-28 | chr3 | 105863871 | 105864294 |
| A-29 | chr3 | 105863682 | 105863833 |
| A-30 | chr3 | 105863600 | 105863669 |
| A-31 | chr3 | 105863416 | 105863556 |

(continued)

| No. | Chromosome No. | Coordinate starting point | Coordinate endpoint |
|---|---|---|---|
| A-32 | chr3 | 105863315 | 105863409 |
| A-33 | chr3 | 105863113 | 105863286 |
| A-34 | chr3 | 105863054 | 105863095 |
| A-35 | chr3 | 105862999 | 105863040 |
| A-36 | chr3 | 105862737 | 105862995 |
| A-37 | chr3 | 105862648 | 105862735 |
| A-38 | chr3 | 105862254 | 105862605 |
| A-39 | chr3 | 105862200 | 105862241 |
| A-40 | chr3 | 105861977 | 105862187 |
| A-41 | chr3 | 105861811 | 105861956 |
| A-42 | chr3 | 105861745 | 105861809 |
| A-43 | chr3 | 105861387 | 105861697 |
| A-44 | chr3 | 105861340 | 105861375 |
| A-45 | chr3 | 105861268 | 105861334 |
| A-46 | chr3 | 105861109 | 105861248 |
| A-47 | chr3 | 105861040 | 105861089 |
| A-48 | chr3 | 105860788 | 105860970 |
| A-49 | chr3 | 105860605 | 105860757 |
| A-50 | chr3 | 105860086 | 105860590 |
| A-51 | chr3 | 105859948 | 105860074 |
| A-52 | chr3 | 105859887 | 105859937 |
| A-53 | chr3 | 105859329 | 105859832 |
| A-54 | chr3 | 105859091 | 105859311 |
| A-55 | chr3 | 105859032 | 105859067 |
| A-56 | chr3 | 105858905 | 105859021 |
| A-57 | chr3 | 105858544 | 105858881 |
| A-58 | chr3 | 105857906 | 105858234 |
| A-59 | chr3 | 105857787 | 105857899 |
| A-60 | chr3 | 105857644 | 105857784 |
| A-61 | chr3 | 105857604 | 105857639 |
| A-62 | chr3 | 105857478 | 105857599 |
| A-63 | chr3 | 105857437 | 105857473 |
| A-64 | chr3 | 105857375 | 105857416 |
| A-65 | chr3 | 105857255 | 105857366 |
| A-66 | chr3 | 105857051 | 105857209 |
| A-67 | chr3 | 105856858 | 105857043 |
| A-68 | chr3 | 105856811 | 105856855 |
| A-69 | chr3 | 105856768 | 105856803 |
| A-70 | chr3 | 105856616 | 105856764 |

(continued)

| No. | Chromosome No. | Coordinate starting point | Coordinate endpoint |
|---|---|---|---|
| A-71 | chr3 | 105856038 | 105856593 |
| A-72 | chr3 | 105855870 | 105856029 |
| A-73 | chr3 | 105855715 | 105855800 |
| A-74 | chr3 | 105855504 | 105855699 |
| A-75 | chr3 | 105855461 | 105855502 |
| A-76 | chr3 | 105855172 | 105855446 |
| A-77 | chr3 | 105854624 | 105855152 |
| A-78 | chr3 | 105854481 | 105854616 |
| A-79 | chr3 | 105854155 | 105854460 |
| A-80 | chr3 | 105853828 | 105854153 |
| A-81 | chr3 | 105853726 | 105853824 |
| A-82 | chr3 | 105853565 | 105853696 |
| A-83 | chr3 | 105853370 | 105853550 |
| A-84 | chr3 | 105853261 | 105853352 |
| A-85 | chr3 | 105852579 | 105853256 |
| A-86 | chr3 | 105852477 | 105852558 |
| A-87 | chr3 | 105852409 | 105852445 |
| A-88 | chr3 | 105852069 | 105852380 |
| A-89 | chr3 | 105851962 | 105852006 |
| A-90 | chr3 | 105851871 | 105851953 |
| A-91 | chr3 | 105851834 | 105851869 |
| A-92 | chr3 | 105851777 | 105851820 |
| A-93 | chr3 | 105851648 | 105851697 |
| A-94 | chr3 | 105851569 | 105851635 |
| A-95 | chr3 | 105850992 | 105851543 |
| A-96 | chr3 | 105850724 | 105850968 |
| A-97 | chr3 | 105850477 | 105850720 |
| A-98 | chr3 | 105850212 | 105850450 |
| A-99 | chr3 | 105849980 | 105850182 |
| A-100 | chr3 | 105849920 | 105849962 |
| A-101 | chr3 | 105849841 | 105849883 |
| A-102 | chr3 | 105849626 | 105849805 |
| A-103 | chr3 | 105849540 | 105849598 |
| A-104 | chr3 | 105849459 | 105849516 |
| A-105 | chr3 | 105849266 | 105849447 |
| A-106 | chr3 | 105849077 | 105849260 |
| A-107 | chr3 | 105848996 | 105849062 |
| A-108 | chr3 | 105848832 | 105848973 |
| A-109 | chr3 | 105848718 | 105848817 |

(continued)

| No. | Chromosome No. | Coordinate starting point | Coordinate endpoint |
|---|---|---|---|
| A-110 | chr3 | 105848652 | 105848693 |
| A-111 | chr3 | 105848545 | 105848627 |
| A-112 | chr3 | 105848370 | 105848494 |
| A-113 | chr3 | 105848324 | 105848366 |
| A-114 | chr3 | 105848180 | 105848316 |
| A-115 | chr3 | 105848065 | 105848156 |
| A-116 | chr3 | 105847832 | 105848044 |
| A-117 | chr3 | 105847591 | 105847826 |
| A-118 | chr3 | 105847376 | 105847587 |
| A-119 | chr3 | 105847243 | 105847340 |
| A-120 | chr3 | 105847097 | 105847236 |
| A-121 | chr3 | 105847044 | 105847079 |
| A-122 | chr3 | 105846986 | 105847033 |
| A-123 | chr3 | 105846780 | 105846954 |
| A-124 | chr3 | 105846738 | 105846773 |
| A-125 | chr3 | 105846576 | 105846735 |
| A-126 | chr3 | 105846533 | 105846568 |
| A-127 | chr3 | 105846469 | 105846504 |
| A-128 | chr3 | 105846324 | 105846437 |
| A-129 | chr3 | 105846161 | 105846292 |
| A-130 | chr3 | 105846046 | 105846120 |
| A-131 | chr3 | 105845999 | 105846040 |
| A-132 | chr3 | 105845927 | 105845984 |
| A-133 | chr3 | 105845597 | 105845864 |
| A-134 | chr3 | 105845510 | 105845566 |
| A-135 | chr3 | 105845439 | 105845477 |
| A-136 | chr3 | 105845311 | 105845376 |
| A-137 | chr3 | 105845042 | 105845292 |
| A-138 | chr3 | 105844879 | 105845032 |
| A-139 | chr3 | 105844715 | 105844855 |
| A-140 | chr3 | 105844636 | 105844676 |
| A-141 | chr3 | 105844563 | 105844624 |
| A-142 | chr3 | 105844460 | 105844522 |
| A-143 | chr3 | 105844119 | 105844379 |
| A-144 | chr3 | 105843801 | 105844079 |
| A-145 | chr3 | 105843663 | 105843789 |
| A-146 | chr3 | 105843623 | 105843659 |
| A-147 | chr3 | 105843536 | 105843611 |
| A-148 | chr3 | 105843436 | 105843528 |

(continued)

| No. | Chromosome No. | Coordinate starting point | Coordinate endpoint |
|---|---|---|---|
| A-149 | chr3 | 105843044 | 105843401 |
| A-150 | chr3 | 105842931 | 105843028 |
| A-151 | chr3 | 105842844 | 105842919 |
| A-152 | chr3 | 105842635 | 105842839 |
| A-153 | chr3 | 105841409 | 105842630 |
| A-154 | chr3 | 105841321 | 105841373 |
| A-155 | chr3 | 105840947 | 105841314 |
| A-156 | chr3 | 105840884 | 105840919 |
| A-157 | chr3 | 105840776 | 105840855 |
| A-158 | chr3 | 105840631 | 105840742 |
| A-159 | chr3 | 105840468 | 105840615 |
| A-160 | chr3 | 105840404 | 105840445 |
| A-161 | chr3 | 105840267 | 105840324 |
| A-162 | chr3 | 105840195 | 105840255 |
| A-163 | chr3 | 105840155 | 105840190 |
| A-164 | chr3 | 105840056 | 105840135 |
| A-165 | chr3 | 105839943 | 105840043 |
| A-166 | chr3 | 105839870 | 105839932 |
| A-167 | chr3 | 105839524 | 105839804 |
| A-168 | chr3 | 105839409 | 105839487 |
| A-169 | chr3 | 105838611 | 105839395 |
| A-170 | chr3 | 105838412 | 105838599 |
| A-171 | chr3 | 105837948 | 105838404 |
| A-172 | chr3 | 105837748 | 105837945 |
| A-173 | chr3 | 105837645 | 105837686 |
| A-174 | chr3 | 105837511 | 105837640 |
| A-175 | chr3 | 105837414 | 105837468 |
| A-176 | chr3 | 105837278 | 105837398 |
| A-177 | chr3 | 105837205 | 105837273 |
| A-178 | chr3 | 105837071 | 105837200 |
| A-179 | chr3 | 105836872 | 105836998 |
| A-180 | chr3 | 105836816 | 105836867 |
| A-181 | chr3 | 105836414 | 105836804 |
| A-182 | chr3 | 105836193 | 105836403 |
| A-183 | chr3 | 105835993 | 105836190 |
| A-184 | chr3 | 105835754 | 105835991 |
| A-185 | chr3 | 105835669 | 105835731 |
| A-186 | chr3 | 105835547 | 105835647 |
| A-187 | chr3 | 105835456 | 105835515 |

(continued)

| No. | Chromosome No. | Coordinate starting point | Coordinate endpoint |
|-----|----------------|---------------------------|---------------------|
| A-188 | chr3 | 105835314 | 105835441 |
| A-189 | chr3 | 105835274 | 105835309 |
| A-190 | chr3 | 105835167 | 105835241 |
| A-191 | chr3 | 105834713 | 105835155 |
| A-192 | chr3 | 105834285 | 105834681 |
| A-193 | chr3 | 105834222 | 105834272 |
| A-194 | chr3 | 105834044 | 105834213 |
| A-195 | chr3 | 105833798 | 105834031 |
| A-196 | chr3 | 105833746 | 105833781 |
| A-197 | chr3 | 105833538 | 105833698 |
| A-198 | chr3 | 105833436 | 105833502 |
| A-199 | chr3 | 105833320 | 105833401 |
| A-200 | chr3 | 105833144 | 105833250 |
| A-201 | chr3 | 105832886 | 105833126 |
| A-202 | chr3 | 105832843 | 105832878 |
| A-203 | chr3 | 105832757 | 105832839 |
| A-204 | chr3 | 105832616 | 105832741 |
| A-205 | chr3 | 105832421 | 105832613 |
| A-206 | chr3 | 105832027 | 105832388 |
| A-207 | chr3 | 105831890 | 105832022 |
| A-208 | chr3 | 105831813 | 105831854 |
| A-209 | chr3 | 105831654 | 105831807 |
| A-210 | chr3 | 105831593 | 105831631 |
| A-211 | chr3 | 105831166 | 105831532 |
| A-212 | chr3 | 105830988 | 105831144 |
| A-213 | chr3 | 105830867 | 105830984 |
| A-214 | chr3 | 105830672 | 105830838 |
| A-215 | chr3 | 105830486 | 105830623 |
| A-216 | chr3 | 105830094 | 105830479 |
| A-217 | chr3 | 105829938 | 105830077 |
| A-218 | chr3 | 105829811 | 105829926 |
| A-219 | chr3 | 105829349 | 105829776 |
| A-220 | chr3 | 105829302 | 105829343 |
| A-221 | chr3 | 105829245 | 105829280 |
| A-222 | chr3 | 105829086 | 105829237 |
| A-223 | chr3 | 105828884 | 105829075 |
| A-224 | chr3 | 105828778 | 105828880 |
| A-225 | chr3 | 105828648 | 105828730 |
| A-226 | chr3 | 105828593 | 105828634 |

(continued)

| No. | Chromosome No. | Coordinate starting point | Coordinate endpoint |
|---|---|---|---|
| A-227 | chr3 | 105828510 | 105828551 |
| A-228 | chr3 | 105828465 | 105828500 |
| A-229 | chr3 | 105828416 | 105828451 |
| A-230 | chr3 | 105828228 | 105828401 |
| A-231 | chr3 | 105828008 | 105828221 |
| A-232 | chr3 | 105827865 | 105827975 |
| A-233 | chr3 | 105827789 | 105827848 |
| A-234 | chr3 | 105827700 | 105827772 |
| A-235 | chr3 | 105827317 | 105827689 |
| A-236 | chr3 | 105826970 | 105827305 |
| A-237 | chr3 | 105826753 | 105826964 |
| A-238 | chr3 | 105826552 | 105826732 |
| A-239 | chr3 | 105826202 | 105826285 |
| A-240 | chr3 | 105826010 | 105826166 |
| A-241 | chr3 | 105825947 | 105825988 |
| A-242 | chr3 | 105825842 | 105825916 |
| A-243 | chr3 | 105825649 | 105825805 |
| A-244 | chr3 | 105825453 | 105825588 |
| A-245 | chr3 | 105825233 | 105825417 |
| A-246 | chr3 | 105823494 | 105825047 |
| A-247 | chr3 | 105822846 | 105823208 |
| A-248 | chr3 | 105822768 | 105822842 |
| A-249 | chr3 | 105822118 | 105822738 |
| A-250 | chr3 | 105822059 | 105822100 |
| A-251 | chr3 | 105821979 | 105822052 |
| A-252 | chr3 | 105821922 | 105821963 |
| A-253 | chr3 | 105821845 | 105821887 |
| A-254 | chr3 | 105821737 | 105821843 |
| A-255 | chr3 | 105821510 | 105821727 |
| A-256 | chr3 | 105821276 | 105821502 |
| A-257 | chr3 | 105821090 | 105821266 |
| A-258 | chr3 | 105820875 | 105821079 |
| A-259 | chr3 | 105820802 | 105820870 |
| A-260 | chr3 | 105820728 | 105820793 |
| A-261 | chr3 | 105820542 | 105820720 |
| A-262 | chr3 | 105820498 | 105820539 |
| A-263 | chr3 | 105820284 | 105820453 |
| A-264 | chr3 | 105820019 | 105820273 |
| A-265 | chr3 | 105819623 | 105819985 |

(continued)

| No. | Chromosome No. | Coordinate starting point | Coordinate endpoint |
|---|---|---|---|
| A-266 | chr3 | 105819552 | 105819593 |
| A-267 | chr3 | 105819173 | 105819548 |
| A-268 | chr3 | 105819135 | 105819170 |
| A-269 | chr3 | 105819091 | 105819126 |
| A-270 | chr3 | 105819031 | 105819072 |
| A-271 | chr3 | 105818956 | 105818998 |
| A-272 | chr3 | 105818880 | 105818925 |
| A-273 | chr3 | 105818804 | 105818865 |
| A-274 | chr3 | 105818688 | 105818729 |
| A-275 | chr3 | 105818637 | 105818678 |
| A-276 | chr3 | 105818570 | 105818611 |
| A-277 | chr3 | 105818514 | 105818549 |
| A-278 | chr3 | 105818431 | 105818486 |
| A-279 | chr3 | 105818225 | 105818424 |
| A-280 | chr3 | 105818094 | 105818214 |
| A-281 | chr3 | 105817926 | 105818090 |
| A-282 | chr3 | 105817868 | 105817909 |
| A-283 | chr3 | 105817711 | 105817863 |
| A-284 | chr3 | 105817591 | 105817703 |
| A-285 | chr3 | 105817091 | 105817524 |
| A-286 | chr3 | 105816921 | 105817078 |
| A-287 | chr3 | 105816728 | 105816907 |
| A-288 | chr3 | 105816515 | 105816622 |
| A-289 | chr3 | 105816434 | 105816505 |
| A-290 | chr3 | 105816253 | 105816407 |
| A-291 | chr3 | 105815952 | 105816216 |
| A-292 | chr3 | 105815742 | 105815949 |
| A-293 | chr3 | 105815416 | 105815737 |
| A-294 | chr3 | 105815116 | 105815283 |
| A-295 | chr3 | 105814603 | 105815096 |
| A-296 | chr3 | 105814467 | 105814574 |
| A-297 | chr3 | 105814394 | 105814435 |
| A-298 | chr3 | 105814226 | 105814379 |
| A-299 | chr3 | 105814151 | 105814224 |
| A-300 | chr3 | 105814066 | 105814141 |
| A-301 | chr3 | 105813983 | 105814035 |
| A-302 | chr3 | 105813708 | 105813976 |
| A-303 | chr3 | 105813583 | 105813679 |
| A-304 | chr3 | 105813475 | 105813581 |

(continued)

| No. | Chromosome No. | Coordinate starting point | Coordinate endpoint |
|---|---|---|---|
| A-305 | chr3 | 105813369 | 105813449 |
| A-306 | chr3 | 105813058 | 105813336 |
| A-307 | chr3 | 105812944 | 105813017 |
| A-308 | chr3 | 105812852 | 105812936 |
| A-309 | chr3 | 105812765 | 105812844 |
| A-310 | chr3 | 105812419 | 105812757 |
| A-311 | chr3 | 105812242 | 105812399 |
| A-312 | chr3 | 105811642 | 105812195 |
| A-313 | chr3 | 105811549 | 105811607 |
| A-314 | chr3 | 105811505 | 105811540 |
| A-315 | chr3 | 105811372 | 105811503 |
| A-316 | chr3 | 105811307 | 105811362 |
| A-317 | chr3 | 105811188 | 105811297 |
| A-318 | chr3 | 105811071 | 105811177 |
| A-319 | chr3 | 105810959 | 105811042 |
| A-320 | chr3 | 105810755 | 105810905 |
| A-321 | chr3 | 105810548 | 105810706 |
| A-322 | chr3 | 105810415 | 105810503 |
| A-323 | chr3 | 105810257 | 105810412 |
| A-324 | chr3 | 105810146 | 105810225 |
| A-325 | chr3 | 105810011 | 105810143 |
| A-326 | chr3 | 105809849 | 105810008 |
| A-327 | chr3 | 105809799 | 105809841 |
| A-328 | chr3 | 105809668 | 105809788 |
| A-329 | chr3 | 105809233 | 105809637 |
| A-330 | chr3 | 105809188 | 105809229 |
| A-331 | chr3 | 105809148 | 105809183 |
| A-332 | chr3 | 105809031 | 105809143 |
| A-333 | chr3 | 105808968 | 105809025 |
| A-334 | chr3 | 105808895 | 105808936 |
| A-335 | chr3 | 105808777 | 105808878 |
| A-336 | chr3 | 105808738 | 105808773 |
| A-337 | chr3 | 105808651 | 105808722 |
| A-338 | chr3 | 105808607 | 105808648 |
| A-339 | chr3 | 105808521 | 105808598 |
| A-340 | chr3 | 105808318 | 105808511 |
| A-341 | chr3 | 105808120 | 105808278 |
| A-342 | chr3 | 105807974 | 105808113 |
| A-343 | chr3 | 105807771 | 105807970 |

(continued)

| No. | Chromosome No. | Coordinate starting point | Coordinate endpoint |
|---|---|---|---|
| A-344 | chr3 | 105807707 | 105807760 |
| A-345 | chr3 | 105807664 | 105807705 |
| A-346 | chr3 | 105807606 | 105807641 |
| A-347 | chr3 | 105807518 | 105807586 |
| A-348 | chr3 | 105807243 | 105807516 |
| A-349 | chr3 | 105807176 | 105807232 |
| A-350 | chr3 | 105807103 | 105807155 |
| A-351 | chr3 | 105806881 | 105807090 |
| A-352 | chr3 | 105806825 | 105806866 |
| A-353 | chr3 | 105806566 | 105806807 |
| A-354 | chr3 | 105806405 | 105806552 |
| A-355 | chr3 | 105806301 | 105806403 |
| A-356 | chr3 | 105806158 | 105806194 |
| A-357 | chr3 | 105806064 | 105806125 |
| A-358 | chr3 | 105805937 | 105806053 |
| A-359 | chr3 | 105804677 | 105805932 |
| A-360 | chr3 | 105804565 | 105804606 |
| A-361 | chr3 | 105804211 | 105804525 |
| A-362 | chr3 | 105804095 | 105804203 |
| A-363 | chr3 | 105804035 | 105804088 |
| A-364 | chr3 | 105803883 | 105804021 |
| A-365 | chr3 | 105803827 | 105803880 |
| A-366 | chr3 | 105803364 | 105803799 |
| A-367 | chr3 | 105803197 | 105803360 |
| A-368 | chr3 | 105803044 | 105803166 |
| A-369 | chr3 | 105802910 | 105803038 |
| A-370 | chr3 | 105802771 | 105802849 |
| A-371 | chr3 | 105801732 | 105802726 |
| A-372 | chr3 | 105801552 | 105801721 |
| A-373 | chr3 | 105801431 | 105801537 |
| A-374 | chr3 | 105801393 | 105801428 |
| A-375 | chr3 | 105801075 | 105801301 |
| A-376 | chr3 | 105800912 | 105801025 |
| A-377 | chr3 | 105800671 | 105800883 |
| A-378 | chr3 | 105800626 | 105800667 |
| A-379 | chr3 | 105800567 | 105800623 |
| A-380 | chr3 | 105800285 | 105800560 |
| A-381 | chr3 | 105799898 | 105800277 |
| A-382 | chr3 | 105799610 | 105799895 |

(continued)

| No. | Chromosome No. | Coordinate starting point | Coordinate endpoint |
|---|---|---|---|
| A-383 | chr3 | 105799450 | 105799572 |
| A-384 | chr3 | 105799362 | 105799439 |
| A-385 | chr3 | 105799250 | 105799347 |
| A-386 | chr3 | 105799191 | 105799247 |
| A-387 | chr3 | 105799088 | 105799161 |
| A-388 | chr3 | 105799036 | 105799077 |
| A-389 | chr3 | 105798992 | 105799027 |
| A-390 | chr3 | 105798811 | 105798983 |
| A-391 | chr3 | 105798633 | 105798774 |
| A-392 | chr3 | 105798365 | 105798625 |
| A-393 | chr3 | 105798318 | 105798353 |
| A-394 | chr3 | 105798257 | 105798299 |
| A-395 | chr3 | 105798189 | 105798241 |
| A-396 | chr3 | 105798101 | 105798136 |
| A-397 | chr3 | 105797818 | 105798079 |
| A-398 | chr3 | 105797760 | 105797814 |
| A-399 | chr3 | 105797674 | 105797731 |
| A-400 | chr3 | 105797625 | 105797669 |
| A-401 | chr3 | 105797142 | 105797620 |
| A-402 | chr3 | 105797047 | 105797124 |
| A-403 | chr3 | 105796999 | 105797040 |
| A-404 | chr3 | 105796629 | 105796841 |
| A-405 | chr3 | 105796545 | 105796595 |
| A-406 | chr3 | 105796458 | 105796542 |
| A-407 | chr3 | 105796401 | 105796456 |
| A-408 | chr3 | 105796114 | 105796385 |
| A-409 | chr3 | 105795940 | 105796087 |
| A-410 | chr3 | 105795756 | 105795937 |
| A-411 | chr3 | 105795490 | 105795753 |
| A-412 | chr3 | 105795432 | 105795467 |
| A-413 | chr3 | 105794791 | 105795424 |
| A-414 | chr3 | 105794743 | 105794784 |
| A-415 | chr3 | 105794699 | 105794740 |
| A-416 | chr3 | 105794548 | 105794693 |
| A-417 | chr3 | 105794229 | 105794546 |
| A-418 | chr3 | 105793956 | 105794178 |
| A-419 | chr3 | 105793869 | 105793935 |
| A-420 | chr3 | 105793677 | 105793866 |
| A-421 | chr3 | 105793630 | 105793671 |

(continued)

| No. | Chromosome No. | Coordinate starting point | Coordinate endpoint |
|---|---|---|---|
| A-422 | chr3 | 105793543 | 105793627 |
| A-423 | chr3 | 105792566 | 105793527 |
| A-424 | chr3 | 105792389 | 105792552 |
| A-425 | chr3 | 105792295 | 105792385 |
| A-426 | chr3 | 105791995 | 105792265 |
| A-427 | chr3 | 105791880 | 105791986 |
| A-428 | chr3 | 105791497 | 105791858 |
| A-429 | chr3 | 105791429 | 105791480 |
| A-430 | chr3 | 105791360 | 105791422 |
| A-431 | chr3 | 105791225 | 105791331 |
| A-432 | chr3 | 105790627 | 105791074 |
| A-433 | chr3 | 105790404 | 105790609 |
| A-434 | chr3 | 105790215 | 105790321 |
| A-435 | chr3 | 105790165 | 105790200 |
| A-436 | chr3 | 105789981 | 105790127 |
| A-437 | chr3 | 105789851 | 105789963 |
| A-438 | chr3 | 105789763 | 105789829 |
| A-439 | chr3 | 105789681 | 105789751 |
| A-440 | chr3 | 105789388 | 105789671 |
| A-441 | chr3 | 105789269 | 105789305 |
| A-442 | chr3 | 105789201 | 105789264 |
| A-443 | chr3 | 105788676 | 105789133 |
| A-444 | chr3 | 105788490 | 105788672 |
| A-445 | chr3 | 105788103 | 105788429 |
| A-446 | chr3 | 105787955 | 105788089 |
| A-447 | chr3 | 105787826 | 105787911 |
| A-448 | chr3 | 105787643 | 105787824 |
| A-449 | chr3 | 105787575 | 105787624 |
| A-450 | chr3 | 105787004 | 105787546 |
| A-451 | chr3 | 105786965 | 105787000 |
| A-452 | chr3 | 105786845 | 105786956 |
| A-453 | chr3 | 105786285 | 105786798 |
| A-454 | chr3 | 105786125 | 105786274 |
| A-455 | chr3 | 105785835 | 105786112 |
| A-456 | chr3 | 105785751 | 105785812 |
| A-457 | chr3 | 105785555 | 105785721 |
| A-458 | chr3 | 105785395 | 105785549 |
| A-459 | chr3 | 105785240 | 105785360 |
| A-460 | chr3 | 105785202 | 105785237 |

(continued)

| No. | Chromosome No. | Coordinate starting point | Coordinate endpoint |
|---|---|---|---|
| A-461 | chr3 | 105785145 | 105785186 |
| A-462 | chr3 | 105784804 | 105785110 |
| A-463 | chr3 | 105784680 | 105784773 |
| A-464 | chr3 | 105784549 | 105784670 |
| A-465 | chr3 | 105784429 | 105784492 |
| A-466 | chr3 | 105784340 | 105784416 |
| A-467 | chr3 | 105784167 | 105784314 |
| A-468 | chr3 | 105783601 | 105784137 |
| A-469 | chr3 | 105783536 | 105783597 |
| A-470 | chr3 | 105783438 | 105783505 |
| A-471 | chr3 | 105783370 | 105783426 |
| A-472 | chr3 | 105783002 | 105783323 |
| A-473 | chr3 | 105782598 | 105782998 |
| A-474 | chr3 | 105782453 | 105782544 |
| A-475 | chr3 | 105782404 | 105782449 |
| A-476 | chr3 | 105782249 | 105782390 |
| A-477 | chr3 | 105781956 | 105782227 |
| A-478 | chr3 | 105781856 | 105781945 |
| A-479 | chr3 | 105781729 | 105781854 |
| A-480 | chr3 | 105781646 | 105781705 |
| A-481 | chr3 | 105781562 | 105781631 |
| A-482 | chr3 | 105781514 | 105781555 |
| A-483 | chr3 | 105781286 | 105781503 |
| A-484 | chr3 | 105781138 | 105781283 |
| A-485 | chr3 | 105780654 | 105781128 |
| A-486 | chr3 | 105780557 | 105780616 |
| A-487 | chr3 | 105780292 | 105780490 |
| A-488 | chr3 | 105780194 | 105780278 |
| A-489 | chr3 | 105779821 | 105780191 |
| A-490 | chr3 | 105779685 | 105779720 |
| A-491 | chr3 | 105779556 | 105779683 |
| A-492 | chr3 | 105779511 | 105779552 |
| A-493 | chr3 | 105779434 | 105779475 |
| A-494 | chr3 | 105779351 | 105779424 |
| A-495 | chr3 | 105779281 | 105779348 |
| A-496 | chr3 | 105779192 | 105779267 |
| A-497 | chr3 | 105779015 | 105779181 |
| A-498 | chr3 | 105778918 | 105779007 |
| A-499 | chr3 | 105778865 | 105778912 |

(continued)

| No. | Chromosome No. | Coordinate starting point | Coordinate endpoint |
|-----|----------------|---------------------------|---------------------|
| A-500 | chr3 | 105778808 | 105778856 |
| A-501 | chr3 | 105778718 | 105778784 |
| A-502 | chr3 | 105778538 | 105778695 |
| A-503 | chr3 | 105778432 | 105778504 |
| A-504 | chr3 | 105778331 | 105778429 |
| A-505 | chr3 | 105778235 | 105778327 |
| A-506 | chr3 | 105778081 | 105778203 |
| A-507 | chr3 | 105777957 | 105778050 |
| A-508 | chr3 | 105777683 | 105777906 |
| A-509 | chr3 | 105777622 | 105777677 |
| A-510 | chr3 | 105777219 | 105777611 |
| A-511 | chr3 | 105777139 | 105777191 |
| A-512 | chr3 | 105777054 | 105777136 |
| A-513 | chr3 | 105776797 | 105777030 |
| A-514 | chr3 | 105776667 | 105776793 |
| A-515 | chr3 | 105776626 | 105776664 |
| A-516 | chr3 | 105776575 | 105776616 |
| A-517 | chr3 | 105776301 | 105776559 |
| A-518 | chr3 | 105776194 | 105776286 |
| A-519 | chr3 | 105776123 | 105776164 |
| A-520 | chr3 | 105775821 | 105776089 |
| A-521 | chr3 | 105775712 | 105775788 |
| A-522 | chr3 | 105775527 | 105775677 |
| A-523 | chr3 | 105775447 | 105775524 |
| A-524 | chr3 | 105775362 | 105775432 |
| A-525 | chr3 | 105775143 | 105775350 |
| A-526 | chr3 | 105775051 | 105775086 |
| A-527 | chr3 | 105774843 | 105775030 |
| A-528 | chr3 | 105774639 | 105774824 |
| A-529 | chr3 | 105774591 | 105774633 |
| A-530 | chr3 | 105774530 | 105774574 |
| A-531 | chr3 | 105773836 | 105774515 |
| A-532 | chr3 | 105773745 | 105773806 |
| A-533 | chr3 | 105773662 | 105773703 |
| A-534 | chr3 | 105773621 | 105773656 |
| A-535 | chr3 | 105773537 | 105773607 |
| A-536 | chr3 | 105773094 | 105773517 |
| A-537 | chr3 | 105773000 | 105773071 |
| A-538 | chr3 | 105772797 | 105772990 |

(continued)

| No. | Chromosome No. | Coordinate starting point | Coordinate endpoint |
|---|---|---|---|
| A-539 | chr3 | 105772737 | 105772792 |
| A-540 | chr3 | 105772392 | 105772719 |
| A-541 | chr3 | 105771869 | 105772385 |
| A-542 | chr3 | 105771725 | 105771858 |
| A-543 | chr3 | 105771615 | 105771723 |
| A-544 | chr3 | 105771446 | 105771599 |
| A-545 | chr3 | 105771298 | 105771438 |
| A-546 | chr3 | 105770072 | 105770899 |
| A-547 | chr3 | 105770013 | 105770048 |
| A-548 | chr3 | 105769943 | 105769988 |
| A-549 | chr3 | 105769778 | 105769930 |
| A-550 | chr3 | 105769698 | 105769766 |
| A-551 | chr3 | 105769468 | 105769661 |
| A-552 | chr3 | 105769396 | 105769465 |
| A-553 | chr3 | 105768929 | 105769381 |
| A-554 | chr3 | 105768743 | 105768916 |
| A-555 | chr3 | 105768690 | 105768731 |
| A-556 | chr3 | 105768638 | 105768679 |
| A-557 | chr3 | 105768529 | 105768579 |
| A-558 | chr3 | 105768454 | 105768516 |
| A-559 | chr3 | 105768350 | 105768450 |
| A-560 | chr3 | 105768294 | 105768335 |
| A-561 | chr3 | 105768167 | 105768285 |
| A-562 | chr3 | 105768090 | 105768143 |
| A-563 | chr3 | 105767829 | 105768086 |
| A-564 | chr3 | 105767597 | 105767820 |
| A-565 | chr3 | 105767445 | 105767542 |
| A-566 | chr3 | 105767345 | 105767440 |
| A-567 | chr3 | 105767225 | 105767340 |
| A-568 | chr3 | 105767144 | 105767185 |
| A-569 | chr3 | 105766997 | 105767114 |
| A-570 | chr3 | 105766925 | 105766985 |
| A-571 | chr3 | 105766862 | 105766897 |
| A-572 | chr3 | 105766771 | 105766850 |
| A-573 | chr3 | 105766676 | 105766734 |
| A-574 | chr3 | 105766616 | 105766651 |
| A-575 | chr3 | 105766423 | 105766605 |
| A-576 | chr3 | 105766245 | 105766409 |
| A-577 | chr3 | 105766150 | 105766203 |

(continued)

| No. | Chromosome No. | Coordinate starting point | Coordinate endpoint |
|---|---|---|---|
| A-578 | chr3 | 105765840 | 105766119 |
| A-579 | chr3 | 105765161 | 105765513 |
| A-580 | chr3 | 105765026 | 105765133 |
| A-581 | chr3 | 105764985 | 105765022 |
| A-582 | chr3 | 105764815 | 105764975 |
| A-583 | chr3 | 105763712 | 105764516 |
| A-584 | chr3 | 105763527 | 105763647 |
| A-585 | chr3 | 105763206 | 105763514 |
| A-586 | chr3 | 105762217 | 105763197 |
| A-587 | chr3 | 105762136 | 105762201 |
| A-588 | chr3 | 105760975 | 105762123 |
| A-589 | chr3 | 105760912 | 105760964 |
| A-590 | chr3 | 105760802 | 105760889 |
| A-591 | chr3 | 105760757 | 105760799 |
| A-592 | chr3 | 105760642 | 105760738 |
| A-593 | chr3 | 105760529 | 105760622 |
| A-594 | chr3 | 105760481 | 105760522 |
| A-595 | chr3 | 105760414 | 105760479 |
| A-596 | chr3 | 105760277 | 105760365 |
| A-597 | chr3 | 105760216 | 105760266 |
| A-598 | chr3 | 105760109 | 105760188 |
| A-599 | chr3 | 105760011 | 105760065 |
| A-600 | chr3 | 105758457 | 105759995 |
| A-601 | chr3 | 105758188 | 105758371 |
| A-602 | chr3 | 105758040 | 105758183 |
| A-603 | chr3 | 105757991 | 105758026 |
| A-604 | chr3 | 105757903 | 105757986 |
| A-605 | chr3 | 105757656 | 105757900 |
| A-606 | chr3 | 105757596 | 105757639 |
| A-607 | chr3 | 105757312 | 105757551 |
| A-608 | chr3 | 105756756 | 105757285 |
| A-609 | chr3 | 105756679 | 105756743 |
| A-610 | chr3 | 105756522 | 105756669 |
| A-611 | chr3 | 105756440 | 105756503 |
| A-612 | chr3 | 105756381 | 105756432 |
| A-613 | chr3 | 105756296 | 105756331 |
| A-614 | chr3 | 105756226 | 105756282 |
| A-615 | chr3 | 105756177 | 105756218 |
| A-616 | chr3 | 105756051 | 105756086 |

(continued)

| No. | Chromosome No. | Coordinate starting point | Coordinate endpoint |
|---|---|---|---|
| A-617 | chr3 | 105755962 | 105756042 |
| A-618 | chr3 | 105755885 | 105755949 |
| A-619 | chr3 | 105755719 | 105755848 |
| A-620 | chr3 | 105755023 | 105755713 |
| A-621 | chr3 | 105754924 | 105755016 |
| A-622 | chr3 | 105754782 | 105754858 |
| A-623 | chr3 | 105754701 | 105754778 |
| A-624 | chr3 | 105754538 | 105754675 |
| A-625 | chr3 | 105754427 | 105754506 |
| A-626 | chr3 | 105754222 | 105754424 |
| A-627 | chr3 | 105754077 | 105754165 |
| A-628 | chr3 | 105753874 | 105753956 |
| A-629 | chr3 | 105753820 | 105753868 |
| A-630 | chr3 | 105753697 | 105753778 |
| A-631 | chr3 | 105753644 | 105753679 |
| A-632 | chr3 | 105753550 | 105753619 |
| A-633 | chr3 | 105753442 | 105753544 |
| A-634 | chr3 | 105753355 | 105753411 |
| A-635 | chr3 | 105753165 | 105753353 |
| A-636 | chr3 | 105753021 | 105753163 |
| A-637 | chr3 | 105752983 | 105753019 |
| A-638 | chr3 | 105752910 | 105752962 |
| A-639 | chr3 | 105752747 | 105752903 |
| A-640 | chr3 | 105752531 | 105752726 |
| A-641 | chr3 | 105752340 | 105752495 |
| A-642 | chr3 | 105752293 | 105752337 |
| A-643 | chr3 | 105752175 | 105752232 |
| A-644 | chr3 | 105751975 | 105752148 |
| A-645 | chr3 | 105751915 | 105751958 |
| A-646 | chr3 | 105751827 | 105751906 |
| A-647 | chr3 | 105751722 | 105751783 |
| A-648 | chr3 | 105751665 | 105751710 |
| A-649 | chr3 | 105751522 | 105751643 |
| A-650 | chr3 | 105751442 | 105751503 |
| A-651 | chr3 | 105751383 | 105751438 |
| A-652 | chr3 | 105751168 | 105751378 |
| A-653 | chr3 | 105750995 | 105751161 |
| A-654 | chr3 | 105750922 | 105750993 |
| A-655 | chr3 | 105750801 | 105750908 |

(continued)

| No. | Chromosome No. | Coordinate starting point | Coordinate endpoint |
|---|---|---|---|
| A-656 | chr3 | 105750650 | 105750775 |
| A-657 | chr3 | 105750590 | 105750632 |
| A-658 | chr3 | 105750489 | 105750574 |
| A-659 | chr3 | 105750005 | 105750380 |
| A-660 | chr3 | 105749940 | 105749993 |
| A-661 | chr3 | 105749854 | 105749914 |
| A-662 | chr3 | 105749745 | 105749832 |
| A-663 | chr3 | 105749564 | 105749706 |
| A-664 | chr3 | 105749469 | 105749561 |
| A-665 | chr3 | 105749364 | 105749449 |
| A-666 | chr3 | 105749268 | 105749345 |
| A-667 | chr3 | 105749190 | 105749251 |
| A-668 | chr3 | 105749118 | 105749174 |
| A-669 | chr3 | 105748191 | 105749049 |
| A-670 | chr3 | 105747992 | 105748111 |
| A-671 | chr3 | 105747837 | 105747978 |
| A-672 | chr3 | 105747691 | 105747824 |
| A-673 | chr3 | 105747602 | 105747651 |
| A-674 | chr3 | 105747470 | 105747582 |
| A-675 | chr3 | 105747278 | 105747460 |
| A-676 | chr3 | 105747016 | 105747268 |
| A-677 | chr3 | 105746732 | 105747003 |
| A-678 | chr3 | 105746276 | 105746722 |
| A-679 | chr3 | 105746098 | 105746258 |
| A-680 | chr3 | 105745957 | 105746065 |
| A-681 | chr3 | 105745643 | 105745890 |
| A-682 | chr3 | 105745547 | 105745612 |
| A-683 | chr3 | 105745091 | 105745459 |
| A-684 | chr3 | 105744978 | 105745085 |
| A-685 | chr3 | 105744410 | 105744954 |
| A-686 | chr3 | 105744274 | 105744379 |
| A-687 | chr3 | 105744095 | 105744240 |
| A-688 | chr3 | 105744019 | 105744082 |
| A-689 | chr3 | 105743885 | 105743986 |
| A-690 | chr3 | 105743717 | 105743868 |
| A-691 | chr3 | 105743496 | 105743714 |
| A-692 | chr3 | 105742966 | 105743460 |
| A-693 | chr3 | 105742880 | 105742955 |
| A-694 | chr3 | 105742801 | 105742842 |

(continued)

| No. | Chromosome No. | Coordinate starting point | Coordinate endpoint |
|---|---|---|---|
| A-695 | chr3 | 105742674 | 105742715 |
| A-696 | chr3 | 105742546 | 105742643 |
| A-697 | chr3 | 105742478 | 105742535 |
| A-698 | chr3 | 105742407 | 105742457 |
| A-699 | chr3 | 105742351 | 105742386 |
| A-700 | chr3 | 105742265 | 105742338 |
| A-701 | chr3 | 105742100 | 105742234 |
| A-702 | chr3 | 105741974 | 105742064 |
| A-703 | chr3 | 105741412 | 105741949 |
| A-704 | chr3 | 105741201 | 105741380 |
| A-705 | chr3 | 105741007 | 105741196 |
| A-706 | chr3 | 105740840 | 105740973 |
| A-707 | chr3 | 105740786 | 105740827 |
| A-708 | chr3 | 105740667 | 105740781 |
| A-709 | chr3 | 105740556 | 105740651 |
| A-710 | chr3 | 105740461 | 105740553 |
| A-711 | chr3 | 105740289 | 105740454 |
| A-712 | chr3 | 105740202 | 105740267 |
| A-713 | chr3 | 105739666 | 105740146 |
| A-714 | chr3 | 105739508 | 105739649 |
| A-715 | chr3 | 105739277 | 105739495 |
| A-716 | chr3 | 105738748 | 105739267 |
| A-717 | chr3 | 105738703 | 105738738 |
| A-718 | chr3 | 105738589 | 105738687 |
| A-719 | chr3 | 105738397 | 105738549 |
| A-720 | chr3 | 105738351 | 105738386 |
| A-721 | chr3 | 105738224 | 105738346 |
| A-722 | chr3 | 105738165 | 105738201 |
| A-723 | chr3 | 105738081 | 105738116 |
| A-724 | chr3 | 105738011 | 105738076 |
| A-725 | chr3 | 105737857 | 105737934 |
| A-726 | chr3 | 105737564 | 105737829 |
| A-727 | chr3 | 105737468 | 105737558 |
| A-728 | chr3 | 105737323 | 105737437 |
| A-729 | chr3 | 105737275 | 105737318 |
| A-730 | chr3 | 105737156 | 105737273 |
| A-731 | chr3 | 105737021 | 105737133 |
| A-732 | chr3 | 105736701 | 105736986 |
| A-733 | chr3 | 105736597 | 105736653 |

(continued)

| No. | Chromosome No. | Coordinate starting point | Coordinate endpoint |
|-----|----------------|---------------------------|---------------------|
| A-734 | chr3 | 105736450 | 105736520 |
| A-735 | chr3 | 105736346 | 105736437 |
| A-736 | chr3 | 105736215 | 105736315 |
| A-737 | chr3 | 105736116 | 105736158 |
| A-738 | chr3 | 105736013 | 105736074 |
| A-739 | chr3 | 105735473 | 105735965 |
| A-740 | chr3 | 105735361 | 105735443 |
| A-741 | chr3 | 105735247 | 105735321 |
| A-742 | chr3 | 105734928 | 105735221 |
| A-743 | chr3 | 105734759 | 105734877 |
| A-744 | chr3 | 105734582 | 105734752 |
| A-745 | chr3 | 105734492 | 105734553 |
| A-746 | chr3 | 105734353 | 105734470 |
| A-747 | chr3 | 105734174 | 105734333 |
| A-748 | chr3 | 105733979 | 105734172 |
| A-749 | chr3 | 105733909 | 105733972 |
| A-750 | chr3 | 105733677 | 105733801 |
| A-751 | chr3 | 105732979 | 105733674 |
| A-752 | chr3 | 105732909 | 105732974 |
| A-753 | chr3 | 105732860 | 105732901 |
| A-754 | chr3 | 105732588 | 105732850 |
| A-755 | chr3 | 105732473 | 105732565 |
| A-756 | chr3 | 105732371 | 105732452 |
| A-757 | chr3 | 105732318 | 105732353 |
| A-758 | chr3 | 105732259 | 105732306 |
| A-759 | chr3 | 105732210 | 105732254 |
| A-760 | chr3 | 105732123 | 105732208 |
| A-761 | chr3 | 105732047 | 105732105 |
| A-762 | chr3 | 105731895 | 105732035 |
| A-763 | chr3 | 105731832 | 105731885 |
| A-764 | chr3 | 105731557 | 105731785 |
| A-765 | chr3 | 105731341 | 105731489 |
| A-766 | chr3 | 105731177 | 105731320 |
| A-767 | chr3 | 105731011 | 105731175 |
| A-768 | chr3 | 105730933 | 105730974 |
| A-769 | chr3 | 105730812 | 105730853 |
| A-770 | chr3 | 105730407 | 105730505 |
| A-771 | chr3 | 105730161 | 105730362 |
| A-772 | chr3 | 105729971 | 105730146 |

(continued)

| No. | Chromosome No. | Coordinate starting point | Coordinate endpoint |
|-----|----------------|---------------------------|---------------------|
| A-773 | chr3 | 105729871 | 105729939 |
| A-774 | chr3 | 105729751 | 105729867 |
| A-775 | chr3 | 105729683 | 105729742 |
| A-776 | chr3 | 105729574 | 105729668 |
| A-777 | chr3 | 105729356 | 105729557 |
| A-778 | chr3 | 105729308 | 105729344 |
| A-779 | chr3 | 105729226 | 105729295 |
| A-780 | chr3 | 105729015 | 105729214 |
| A-781 | chr3 | 105728939 | 105728998 |
| A-782 | chr3 | 105728793 | 105728908 |
| A-783 | chr3 | 105728682 | 105728717 |
| A-784 | chr3 | 105728588 | 105728671 |
| A-785 | chr3 | 105728296 | 105728581 |
| A-786 | chr3 | 105727689 | 105728273 |
| A-787 | chr3 | 105727537 | 105727674 |
| A-788 | chr3 | 105727371 | 105727514 |
| A-789 | chr3 | 105727301 | 105727336 |
| A-790 | chr3 | 105727176 | 105727278 |
| A-791 | chr3 | 105727042 | 105727168 |
| A-792 | chr3 | 105726338 | 105727006 |
| A-793 | chr3 | 105725862 | 105726310 |
| A-794 | chr3 | 105725753 | 105725841 |
| A-795 | chr3 | 105725703 | 105725746 |
| A-796 | chr3 | 105725616 | 105725700 |
| A-797 | chr3 | 105725516 | 105725611 |
| A-798 | chr3 | 105725376 | 105725512 |
| A-799 | chr3 | 105725338 | 105725373 |
| A-800 | chr3 | 105725275 | 105725330 |
| A-801 | chr3 | 105725198 | 105725252 |
| A-802 | chr3 | 105725157 | 105725192 |
| A-803 | chr3 | 105724950 | 105725081 |
| A-804 | chr3 | 105724746 | 105724943 |
| A-805 | chr3 | 105724659 | 105724741 |
| A-806 | chr3 | 105724497 | 105724624 |
| A-807 | chr3 | 105724226 | 105724478 |
| A-808 | chr3 | 105723987 | 105724221 |
| A-809 | chr3 | 105723866 | 105723961 |
| A-810 | chr3 | 105723810 | 105723862 |
| A-811 | chr3 | 105723761 | 105723802 |

(continued)

| No. | Chromosome No. | Coordinate starting point | Coordinate endpoint |
|-----|----------------|---------------------------|---------------------|
| A-812 | chr3 | 105723617 | 105723753 |
| A-813 | chr3 | 105723522 | 105723579 |
| A-814 | chr3 | 105723265 | 105723464 |
| A-815 | chr3 | 105723137 | 105723213 |
| A-816 | chr3 | 105723082 | 105723128 |
| A-817 | chr3 | 105723011 | 105723074 |
| A-818 | chr3 | 105722915 | 105722956 |
| A-819 | chr3 | 105722628 | 105722801 |
| A-820 | chr3 | 105722538 | 105722624 |
| A-821 | chr3 | 105722388 | 105722527 |
| A-822 | chr3 | 105722341 | 105722377 |
| A-823 | chr3 | 105721970 | 105722297 |
| A-824 | chr3 | 105721811 | 105721953 |
| A-825 | chr3 | 105721614 | 105721794 |
| A-826 | chr3 | 105721533 | 105721608 |
| A-827 | chr3 | 105721456 | 105721506 |
| A-828 | chr3 | 105721375 | 105721446 |
| A-829 | chr3 | 105721247 | 105721359 |
| A-830 | chr3 | 105721045 | 105721241 |
| A-831 | chr3 | 105720973 | 105721035 |
| A-832 | chr3 | 105720869 | 105720965 |
| A-833 | chr3 | 105720793 | 105720861 |
| A-834 | chr3 | 105720726 | 105720770 |
| A-835 | chr3 | 105720291 | 105720716 |
| A-836 | chr3 | 105719722 | 105720282 |
| A-837 | chr3 | 105719648 | 105719683 |
| A-838 | chr3 | 105719392 | 105719623 |
| A-839 | chr3 | 105719314 | 105719389 |
| A-840 | chr3 | 105719213 | 105719312 |
| A-841 | chr3 | 105719107 | 105719180 |
| A-842 | chr3 | 105719054 | 105719089 |
| A-843 | chr3 | 105718287 | 105718738 |
| A-844 | chr3 | 105718116 | 105718239 |
| A-845 | chr3 | 105718008 | 105718102 |
| A-846 | chr3 | 105717732 | 105718006 |
| A-847 | chr3 | 105717686 | 105717727 |
| A-848 | chr3 | 105717562 | 105717652 |
| A-849 | chr3 | 105717503 | 105717556 |
| A-850 | chr3 | 105717344 | 105717499 |

(continued)

| No. | Chromosome No. | Coordinate starting point | Coordinate endpoint |
|-----|----------------|---------------------------|---------------------|
| A-851 | chr3 | 105717193 | 105717329 |
| A-852 | chr3 | 105716897 | 105717189 |
| A-853 | chr3 | 105716832 | 105716884 |
| A-854 | chr3 | 105716789 | 105716830 |
| A-855 | chr3 | 105716726 | 105716768 |
| A-856 | chr3 | 105716668 | 105716709 |
| A-857 | chr3 | 105716606 | 105716641 |
| A-858 | chr3 | 105716511 | 105716589 |
| A-859 | chr3 | 105716354 | 105716494 |
| A-860 | chr3 | 105716241 | 105716348 |
| A-861 | chr3 | 105716171 | 105716238 |
| A-862 | chr3 | 105716108 | 105716156 |
| A-863 | chr3 | 105715723 | 105716064 |
| A-864 | chr3 | 105715458 | 105715708 |
| A-865 | chr3 | 105715316 | 105715402 |
| A-866 | chr3 | 105715154 | 105715297 |
| A-867 | chr3 | 105715080 | 105715139 |
| A-868 | chr3 | 105715034 | 105715069 |
| A-869 | chr3 | 105714872 | 105714986 |
| A-870 | chr3 | 105714814 | 105714858 |
| A-871 | chr3 | 105714660 | 105714785 |
| A-872 | chr3 | 105714386 | 105714639 |
| A-873 | chr3 | 105714051 | 105714316 |
| A-874 | chr3 | 105713729 | 105714046 |
| A-875 | chr3 | 105713673 | 105713714 |
| A-876 | chr3 | 105713574 | 105713643 |
| A-877 | chr3 | 105713371 | 105713537 |
| A-878 | chr3 | 105712798 | 105713129 |
| A-879 | chr3 | 105712708 | 105712769 |
| A-880 | chr3 | 105712644 | 105712705 |
| A-881 | chr3 | 105712582 | 105712624 |
| A-882 | chr3 | 105712508 | 105712576 |
| A-883 | chr3 | 105712283 | 105712490 |
| A-884 | chr3 | 105712219 | 105712270 |
| A-885 | chr3 | 105712101 | 105712202 |
| A-886 | chr3 | 105711562 | 105712079 |
| A-887 | chr3 | 105711493 | 105711528 |
| A-888 | chr3 | 105711445 | 105711486 |
| A-889 | chr3 | 105711217 | 105711287 |

(continued)

| No. | Chromosome No. | Coordinate starting point | Coordinate endpoint |
|---|---|---|---|
| A-890 | chr3 | 105711004 | 105711207 |
| A-891 | chr3 | 105710831 | 105710997 |
| A-892 | chr3 | 105710681 | 105710817 |
| A-893 | chr3 | 105710574 | 105710678 |
| A-894 | chr3 | 105710456 | 105710536 |
| A-895 | chr3 | 105710400 | 105710453 |
| A-896 | chr3 | 105710243 | 105710342 |
| A-897 | chr3 | 105710157 | 105710234 |
| A-898 | chr3 | 105709774 | 105710154 |
| A-899 | chr3 | 105709704 | 105709746 |
| A-900 | chr3 | 105709593 | 105709674 |
| A-901 | chr3 | 105709475 | 105709585 |
| A-902 | chr3 | 105709413 | 105709467 |
| A-903 | chr3 | 105709324 | 105709405 |
| A-904 | chr3 | 105709082 | 105709313 |
| A-905 | chr3 | 105708921 | 105709078 |
| A-906 | chr3 | 105708843 | 105708905 |
| A-907 | chr3 | 105708576 | 105708761 |
| A-908 | chr3 | 105708497 | 105708555 |
| A-909 | chr3 | 105708436 | 105708471 |
| A-910 | chr3 | 105708318 | 105708424 |
| A-911 | chr3 | 105708238 | 105708299 |
| A-912 | chr3 | 105707980 | 105708232 |
| A-913 | chr3 | 105707885 | 105707940 |
| A-914 | chr3 | 105707840 | 105707881 |
| A-915 | chr3 | 105707741 | 105707797 |
| A-916 | chr3 | 105707685 | 105707732 |
| A-917 | chr3 | 105707560 | 105707656 |
| A-918 | chr3 | 105707516 | 105707557 |
| A-919 | chr3 | 105707410 | 105707498 |
| A-920 | chr3 | 105707280 | 105707362 |
| A-921 | chr3 | 105707241 | 105707276 |
| A-922 | chr3 | 105706958 | 105707234 |
| A-923 | chr3 | 105706873 | 105706953 |
| A-924 | chr3 | 105706812 | 105706848 |
| A-925 | chr3 | 105706764 | 105706806 |
| A-926 | chr3 | 105706682 | 105706757 |
| A-927 | chr3 | 105706436 | 105706678 |
| A-928 | chr3 | 105706330 | 105706402 |

(continued)

| No. | Chromosome No. | Coordinate starting point | Coordinate endpoint |
|---|---|---|---|
| A-929 | chr3 | 105706183 | 105706288 |
| A-930 | chr3 | 105705738 | 105706166 |
| A-931 | chr3 | 105705331 | 105705730 |
| A-932 | chr3 | 105705285 | 105705320 |
| A-933 | chr3 | 105705246 | 105705281 |
| A-934 | chr3 | 105705157 | 105705222 |
| A-935 | chr3 | 105704946 | 105705077 |
| A-936 | chr3 | 105704808 | 105704928 |
| A-937 | chr3 | 105704743 | 105704799 |
| A-938 | chr3 | 105704684 | 105704738 |
| A-939 | chr3 | 105704584 | 105704664 |
| A-940 | chr3 | 105704453 | 105704529 |
| A-941 | chr3 | 105703960 | 105704414 |
| A-942 | chr3 | 105703809 | 105703915 |
| A-943 | chr3 | 105703698 | 105703773 |
| A-944 | chr3 | 105703494 | 105703649 |
| A-945 | chr3 | 105703319 | 105703443 |
| A-946 | chr3 | 105703246 | 105703307 |
| A-947 | chr3 | 105703185 | 105703220 |
| A-948 | chr3 | 105703130 | 105703165 |
| A-949 | chr3 | 105703045 | 105703106 |
| A-950 | chr3 | 105702919 | 105703034 |
| A-951 | chr3 | 105702782 | 105702840 |
| A-952 | chr3 | 105702692 | 105702733 |
| A-953 | chr3 | 105702642 | 105702677 |
| A-954 | chr3 | 105702078 | 105702627 |
| A-955 | chr3 | 105701900 | 105702071 |
| A-956 | chr3 | 105701168 | 105701864 |
| A-957 | chr3 | 105700993 | 105701163 |
| A-958 | chr3 | 105700603 | 105700885 |
| A-959 | chr3 | 105700418 | 105700601 |
| A-960 | chr3 | 105700347 | 105700388 |
| A-961 | chr3 | 105700281 | 105700324 |
| A-962 | chr3 | 105700131 | 105700253 |
| A-963 | chr3 | 105700072 | 105700107 |
| A-964 | chr3 | 105699922 | 105699964 |
| A-965 | chr3 | 105699799 | 105699909 |
| A-966 | chr3 | 105699269 | 105699769 |
| A-967 | chr3 | 105698813 | 105699245 |

(continued)

| No. | Chromosome No. | Coordinate starting point | Coordinate endpoint |
|---|---|---|---|
| A-968 | chr3 | 105698548 | 105698757 |
| A-969 | chr3 | 105698242 | 105698532 |
| A-970 | chr3 | 105698109 | 105698189 |
| A-971 | chr3 | 105698065 | 105698106 |
| A-972 | chr3 | 105697946 | 105698061 |
| A-973 | chr3 | 105697890 | 105697925 |
| A-974 | chr3 | 105697818 | 105697859 |
| A-975 | chr3 | 105697710 | 105697811 |
| A-976 | chr3 | 105697639 | 105697704 |
| A-977 | chr3 | 105697464 | 105697634 |
| A-978 | chr3 | 105696565 | 105697422 |
| A-979 | chr3 | 105696467 | 105696557 |
| A-980 | chr3 | 105696361 | 105696451 |
| A-981 | chr3 | 105696090 | 105696330 |
| A-982 | chr3 | 105695764 | 105696001 |
| A-983 | chr3 | 105695650 | 105695762 |
| A-984 | chr3 | 105695505 | 105695637 |
| A-985 | chr3 | 105695459 | 105695494 |
| A-986 | chr3 | 105695346 | 105695412 |
| A-987 | chr3 | 105695297 | 105695338 |
| A-988 | chr3 | 105695103 | 105695289 |
| A-989 | chr3 | 105694749 | 105695078 |
| A-990 | chr3 | 105694709 | 105694744 |
| A-991 | chr3 | 105694608 | 105694660 |
| A-992 | chr3 | 105694512 | 105694597 |
| A-993 | chr3 | 105694447 | 105694498 |
| A-994 | chr3 | 105694402 | 105694443 |
| A-995 | chr3 | 105694304 | 105694362 |
| A-996 | chr3 | 105694179 | 105694288 |
| A-997 | chr3 | 105694060 | 105694171 |
| A-998 | chr3 | 105693971 | 105694046 |
| A-999 | chr3 | 105693883 | 105693941 |
| A-1000 | chr3 | 105693418 | 105693793 |
| A-1001 | chr3 | 105692644 | 105693377 |
| A-1002 | chr3 | 105692552 | 105692639 |
| A-1003 | chr3 | 105692457 | 105692533 |
| A-1004 | chr3 | 105692301 | 105692416 |
| A-1005 | chr3 | 105692251 | 105692287 |
| A-1006 | chr3 | 105692135 | 105692239 |

(continued)

| No. | Chromosome No. | Coordinate starting point | Coordinate endpoint |
|---|---|---|---|
| A-1007 | chr3 | 105691988 | 105692133 |
| A-1008 | chr3 | 105691686 | 105691980 |
| A-1009 | chr3 | 105691634 | 105691675 |
| A-1010 | chr3 | 105691572 | 105691607 |
| A-1011 | chr3 | 105691498 | 105691551 |
| A-1012 | chr3 | 105691387 | 105691470 |
| A-1013 | chr3 | 105691310 | 105691367 |
| A-1014 | chr3 | 105691250 | 105691285 |
| A-1015 | chr3 | 105691158 | 105691246 |
| A-1016 | chr3 | 105690951 | 105691070 |
| A-1017 | chr3 | 105690885 | 105690942 |
| A-1018 | chr3 | 105690467 | 105690872 |
| A-1019 | chr3 | 105690184 | 105690461 |
| A-1020 | chr3 | 105690106 | 105690178 |
| A-1021 | chr3 | 105690028 | 105690073 |
| A-1022 | chr3 | 105689990 | 105690025 |
| A-1023 | chr3 | 105689933 | 105689968 |
| A-1024 | chr3 | 105689847 | 105689888 |
| A-1025 | chr3 | 105689723 | 105689822 |
| A-1026 | chr3 | 105689521 | 105689673 |
| A-1027 | chr3 | 105689354 | 105689502 |
| A-1028 | chr3 | 105689153 | 105689322 |
| A-1029 | chr3 | 105688931 | 105689146 |
| A-1030 | chr3 | 105688868 | 105688903 |
| A-1031 | chr3 | 105688734 | 105688838 |
| A-1032 | chr3 | 105688599 | 105688719 |
| A-1033 | chr3 | 105688526 | 105688572 |
| A-1034 | chr3 | 105688435 | 105688519 |
| A-1035 | chr3 | 105688325 | 105688426 |
| A-1036 | chr3 | 105688039 | 105688313 |
| A-1037 | chr3 | 105687906 | 105688020 |
| A-1038 | chr3 | 105687797 | 105687864 |
| A-1039 | chr3 | 105687732 | 105687773 |
| A-1040 | chr3 | 105687695 | 105687730 |
| A-1041 | chr3 | 105687556 | 105687669 |
| A-1042 | chr3 | 105687369 | 105687502 |
| A-1043 | chr3 | 105687245 | 105687367 |
| A-1044 | chr3 | 105687027 | 105687201 |
| A-1045 | chr3 | 105686828 | 105687015 |

(continued)

| No. | Chromosome No. | Coordinate starting point | Coordinate endpoint |
|---|---|---|---|
| A-1046 | chr3 | 105686789 | 105686825 |
| A-1047 | chr3 | 105686732 | 105686773 |
| A-1048 | chr3 | 105686653 | 105686694 |
| A-1049 | chr3 | 105686475 | 105686648 |
| A-1050 | chr3 | 105686253 | 105686453 |
| A-1051 | chr3 | 105686069 | 105686250 |
| A-1052 | chr3 | 105685900 | 105686043 |
| A-1053 | chr3 | 105685772 | 105685896 |
| A-1054 | chr3 | 105685630 | 105685752 |
| A-1055 | chr3 | 105685554 | 105685590 |
| A-1056 | chr3 | 105685263 | 105685520 |
| A-1057 | chr3 | 105685118 | 105685224 |
| A-1058 | chr3 | 105685010 | 105685099 |
| A-1059 | chr3 | 105684898 | 105684993 |
| A-1060 | chr3 | 105684388 | 105684887 |
| A-1061 | chr3 | 105684140 | 105684375 |
| A-1062 | chr3 | 105684070 | 105684129 |
| A-1063 | chr3 | 105683962 | 105684055 |
| A-1064 | chr3 | 105683846 | 105683948 |
| A-1065 | chr3 | 105683787 | 105683838 |
| A-1066 | chr3 | 105683736 | 105683777 |
| A-1067 | chr3 | 105683687 | 105683722 |
| A-1068 | chr3 | 105683532 | 105683684 |
| A-1069 | chr3 | 105683327 | 105683528 |
| A-1070 | chr3 | 105683227 | 105683313 |
| A-1071 | chr3 | 105683178 | 105683214 |
| A-1072 | chr3 | 105683061 | 105683175 |
| A-1073 | chr3 | 105682994 | 105683038 |
| A-1074 | chr3 | 105682519 | 105682938 |
| A-1075 | chr3 | 105682300 | 105682515 |
| A-1076 | chr3 | 105682244 | 105682285 |
| A-1077 | chr3 | 105682023 | 105682196 |
| A-1078 | chr3 | 105681943 | 105682010 |
| A-1079 | chr3 | 105681714 | 105681934 |
| A-1080 | chr3 | 105681253 | 105681712 |
| A-1081 | chr3 | 105681208 | 105681243 |
| A-1082 | chr3 | 105681064 | 105681105 |
| A-1083 | chr3 | 105680766 | 105680984 |
| A-1084 | chr3 | 105680345 | 105680629 |

(continued)

| No. | Chromosome No. | Coordinate starting point | Coordinate endpoint |
|---|---|---|---|
| A-1085 | chr3 | 105680265 | 105680322 |
| A-1086 | chr3 | 105679894 | 105680235 |
| A-1087 | chr3 | 105679791 | 105679826 |
| A-1088 | chr3 | 105679443 | 105679780 |
| A-1089 | chr3 | 105679276 | 105679411 |
| A-1090 | chr3 | 105679169 | 105679265 |
| A-1091 | chr3 | 105679115 | 105679156 |
| A-1092 | chr3 | 105678952 | 105679107 |
| A-1093 | chr3 | 105678863 | 105678947 |
| A-1094 | chr3 | 105678791 | 105678857 |
| A-1095 | chr3 | 105678655 | 105678751 |
| A-1096 | chr3 | 105678342 | 105678603 |
| A-1097 | chr3 | 105678209 | 105678339 |
| A-1098 | chr3 | 105678098 | 105678187 |
| A-1099 | chr3 | 105678044 | 105678087 |
| A-1100 | chr3 | 105677984 | 105678033 |
| A-1101 | chr3 | 105677862 | 105677913 |
| A-1102 | chr3 | 105677735 | 105677776 |
| A-1103 | chr3 | 105677651 | 105677717 |
| A-1104 | chr3 | 105677553 | 105677636 |
| A-1105 | chr3 | 105677503 | 105677538 |
| A-1106 | chr3 | 105677336 | 105677487 |
| A-1107 | chr3 | 105677244 | 105677326 |
| A-1108 | chr3 | 105676685 | 105677202 |
| A-1109 | chr3 | 105676457 | 105676581 |
| A-1110 | chr3 | 105676405 | 105676440 |
| A-1111 | chr3 | 105676353 | 105676394 |
| A-1112 | chr3 | 105676264 | 105676329 |
| A-1113 | chr3 | 105676098 | 105676251 |
| A-1114 | chr3 | 105676048 | 105676094 |
| A-1115 | chr3 | 105675977 | 105676040 |
| A-1116 | chr3 | 105675568 | 105675973 |
| A-1117 | chr3 | 105675502 | 105675557 |
| A-1118 | chr3 | 105675459 | 105675500 |
| A-1119 | chr3 | 105675343 | 105675379 |
| A-1120 | chr3 | 105675282 | 105675335 |
| A-1121 | chr3 | 105675107 | 105675257 |
| A-1122 | chr3 | 105674982 | 105675087 |
| A-1123 | chr3 | 105674538 | 105674967 |

(continued)

| No. | Chromosome No. | Coordinate starting point | Coordinate endpoint |
|---|---|---|---|
| A-1124 | chr3 | 105674454 | 105674512 |
| A-1125 | chr3 | 105674347 | 105674430 |
| A-1126 | chr3 | 105674295 | 105674345 |
| A-1127 | chr3 | 105673773 | 105674277 |
| A-1128 | chr3 | 105673406 | 105673755 |
| A-1129 | chr3 | 105673081 | 105673397 |
| A-1130 | chr3 | 105672996 | 105673069 |
| A-1131 | chr3 | 105672951 | 105672986 |
| A-1132 | chr3 | 105672881 | 105672923 |
| A-1133 | chr3 | 105672761 | 105672840 |
| A-1134 | chr3 | 105672714 | 105672755 |
| A-1135 | chr3 | 105672578 | 105672678 |
| A-1136 | chr3 | 105672510 | 105672561 |
| A-1137 | chr3 | 105672379 | 105672474 |
| A-1138 | chr3 | 105672313 | 105672368 |
| A-1139 | chr3 | 105672220 | 105672302 |
| A-1140 | chr3 | 105672164 | 105672217 |
| A-1141 | chr3 | 105672104 | 105672151 |
| A-1142 | chr3 | 105672058 | 105672101 |
| A-1143 | chr3 | 105671996 | 105672049 |
| A-1144 | chr3 | 105671830 | 105671992 |
| A-1145 | chr3 | 105671656 | 105671789 |
| A-1146 | chr3 | 105671557 | 105671653 |
| A-1147 | chr3 | 105671458 | 105671553 |
| A-1148 | chr3 | 105671191 | 105671449 |
| A-1149 | chr3 | 105671118 | 105671178 |
| A-1150 | chr3 | 105670964 | 105671093 |
| A-1151 | chr3 | 105670707 | 105670940 |
| A-1152 | chr3 | 105670650 | 105670701 |
| A-1153 | chr3 | 105670550 | 105670591 |
| A-1154 | chr3 | 105670480 | 105670534 |
| A-1155 | chr3 | 105670411 | 105670446 |
| A-1156 | chr3 | 105670212 | 105670405 |
| A-1157 | chr3 | 105670169 | 105670204 |
| A-1158 | chr3 | 105669922 | 105670167 |
| A-1159 | chr3 | 105669179 | 105669916 |
| A-1160 | chr3 | 105669029 | 105669176 |
| A-1161 | chr3 | 105668918 | 105668953 |
| A-1162 | chr3 | 105668873 | 105668908 |

(continued)

| No. | Chromosome No. | Coordinate starting point | Coordinate endpoint |
|---|---|---|---|
| A-1163 | chr3 | 105668637 | 105668841 |
| A-1164 | chr3 | 105668581 | 105668635 |
| A-1165 | chr3 | 105668527 | 105668568 |
| A-1166 | chr3 | 105668301 | 105668472 |
| A-1167 | chr3 | 105668252 | 105668293 |
| A-1168 | chr3 | 105667742 | 105668243 |
| A-1169 | chr3 | 105667662 | 105667698 |
| A-1170 | chr3 | 105667580 | 105667636 |
| A-1171 | chr3 | 105667377 | 105667567 |
| A-1172 | chr3 | 105667218 | 105667333 |
| A-1173 | chr3 | 105667114 | 105667177 |
| A-1174 | chr3 | 105666850 | 105666941 |
| A-1175 | chr3 | 105666754 | 105666835 |
| A-1176 | chr3 | 105666305 | 105666736 |
| A-1177 | chr3 | 105666212 | 105666284 |
| A-1178 | chr3 | 105666158 | 105666207 |
| A-1179 | chr3 | 105666044 | 105666079 |
| A-1180 | chr3 | 105665717 | 105666030 |
| A-1181 | chr3 | 105665558 | 105665612 |
| A-1182 | chr3 | 105665465 | 105665505 |
| A-1183 | chr3 | 105665089 | 105665417 |
| A-1184 | chr3 | 105664997 | 105665032 |
| A-1185 | chr3 | 105664823 | 105664969 |
| A-1186 | chr3 | 105664569 | 105664793 |
| A-1187 | chr3 | 105664456 | 105664548 |
| A-1188 | chr3 | 105664351 | 105664435 |
| A-1189 | chr3 | 105664302 | 105664337 |
| A-1190 | chr3 | 105664148 | 105664279 |
| A-1191 | chr3 | 105664036 | 105664100 |
| A-1192 | chr3 | 105663979 | 105664031 |
| A-1193 | chr3 | 105663563 | 105663976 |
| A-1194 | chr3 | 105663212 | 105663543 |
| A-1195 | chr3 | 105662960 | 105663149 |
| A-1196 | chr3 | 105662712 | 105662954 |
| A-1197 | chr3 | 105662612 | 105662647 |
| A-1198 | chr3 | 105662535 | 105662609 |
| A-1199 | chr3 | 105662026 | 105662455 |
| A-1200 | chr3 | 105661934 | 105662004 |
| A-1201 | chr3 | 105661773 | 105661914 |

EP 4 471 128 A1
(continued)

| No. | Chromosome No. | Coordinate starting point | Coordinate endpoint |
|-----|----------------|---------------------------|---------------------|
| A-1202 | chr3 | 105661570 | 105661767 |
| A-1203 | chr3 | 105661491 | 105661560 |
| A-1204 | chr3 | 105661417 | 105661472 |
| A-1205 | chr3 | 105661313 | 105661366 |
| A-1206 | chr3 | 105661189 | 105661231 |
| A-1207 | chr3 | 105660981 | 105661182 |
| A-1208 | chr3 | 105660800 | 105660974 |
| A-1209 | chr3 | 105660565 | 105660749 |
| A-1210 | chr3 | 105659934 | 105660543 |
| A-1211 | chr3 | 105659892 | 105659927 |
| A-1212 | chr3 | 105659834 | 105659881 |
| A-1213 | chr3 | 105659760 | 105659832 |
| A-1214 | chr3 | 105659354 | 105659754 |
| A-1215 | chr3 | 105658884 | 105669345 |
| A-1216 | chr3 | 105658734 | 105658877 |
| A-1217 | chr3 | 105658425 | 105658715 |
| A-1218 | chr3 | 105658131 | 105658391 |
| A-1219 | chr3 | 105657838 | 105658121 |
| A-1220 | chr3 | 105657600 | 105657768 |
| A-1221 | chr3 | 105657537 | 105657572 |
| A-1222 | chr3 | 105657426 | 105657533 |
| A-1223 | chr3 | 105657351 | 105657404 |
| A-1224 | chr3 | 105657225 | 105657340 |
| A-1225 | chr3 | 105656945 | 105657222 |
| A-1226 | chr3 | 105656739 | 105656932 |
| A-1227 | chr3 | 105656602 | 105656698 |
| A-1228 | chr3 | 105656472 | 105656587 |
| A-1229 | chr3 | 105656285 | 105656451 |
| A-1230 | chr3 | 105656072 | 105656261 |
| A-1231 | chr3 | 105655893 | 105656057 |
| A-1232 | chr3 | 105655828 | 105655875 |
| A-1233 | chr3 | 105655575 | 105655822 |
| A-1234 | chr3 | 105655441 | 105655560 |
| A-1235 | chr3 | 105655356 | 105655397 |

Table 1B Coordinates for human ZC3H12A genome

| No. | Chromosome No. | Coordinate starting point | Coordinate endpoint |
|-----|----------------|---------------------------|---------------------|
| B-1 | chr1 | 37474388 | 37481808 |
| B-2 | chr1 | 37481811 | 37484114 |

100

(continued)

| No. | Chromosome No. | Coordinate starting point | Coordinate endpoint |
|-----|----------------|---------------------------|---------------------|
| B-3 | chr1 | 37484135 | 37484306 |
| B-4 | chr1 | 37484317 | 37484510 |

Table 1C Coordinates for human SOCS1 genome

| No. | Chromosome No. | Coordinate starting point | Coordinate endpoint |
|-----|----------------|---------------------------|---------------------|
| C-1 | chr16 | 11254294 | 11254414 |
| C-2 | chr16 | 11254439 | 11254480 |
| C-3 | chr16 | 11254494 | 11256330 |

Table 1D Coordinates for human TNFAIP3 genome

| No. | Chromosome No. | Coordinate starting point | Coordinate endpoint |
|-----|----------------|---------------------------|---------------------|
| D-1 | chr6 | 137866227 | 137866556 |
| D-2 | chr6 | 137866559 | 137866886 |
| D-3 | chr6 | 137866889 | 137868451 |
| D-4 | chr6 | 137868466 | 137868544 |
| D-5 | chr6 | 137868547 | 137868721 |
| D-6 | chr6 | 137868728 | 137868952 |
| D-7 | chr6 | 137868960 | 137868995 |
| D-8 | chr6 | 137869004 | 137869400 |
| D-9 | chr6 | 137869407 | 137870210 |
| D-10 | chr6 | 137870222 | 137870552 |
| D-11 | chr6 | 137870583 | 137871016 |
| D-12 | chr6 | 137871020 | 137871678 |
| D-13 | chr6 | 137871682 | 137871717 |
| D-14 | chr6 | 137871720 | 137871910 |
| D-15 | chr6 | 137871914 | 137872101 |
| D-16 | chr6 | 137872108 | 137872353 |
| D-17 | chr6 | 137872369 | 137872507 |
| D-18 | chr6 | 137872515 | 137872616 |
| D-19 | chr6 | 137872619 | 137872997 |
| D-20 | chr6 | 137873002 | 137873093 |
| D-21 | chr6 | 137873105 | 137873189 |
| D-22 | chr6 | 137873214 | 137873357 |
| D-23 | chr6 | 137873365 | 137873417 |
| D-24 | chr6 | 137873420 | 137873599 |
| D-25 | chr6 | 137873633 | 137873674 |
| D-26 | chr6 | 137873688 | 137873741 |
| D-27 | chr6 | 137873756 | 137873828 |
| D-28 | chr6 | 137873874 | 137873916 |
| D-29 | chr6 | 137873920 | 137874077 |

(continued)

| No. | Chromosome No. | Coordinate starting point | Coordinate endpoint |
|---|---|---|---|
| D-30 | chr6 | 137874079 | 137874487 |
| D-31 | chr6 | 137874492 | 137874646 |
| D-32 | chr6 | 137874682 | 137875576 |
| D-33 | chr6 | 137875608 | 137875652 |
| D-34 | chr6 | 137875660 | 137875779 |
| D-35 | chr6 | 137875783 | 137876255 |
| D-36 | chr6 | 137876267 | 137876302 |
| D-37 | chr6 | 137876327 | 137876655 |
| D-38 | chr6 | 137876668 | 137876968 |
| D-39 | chr6 | 137876993 | 137877059 |
| D-40 | chr6 | 137877061 | 137877443 |
| D-41 | chr6 | 137877451 | 137877550 |
| D-42 | chr6 | 137877555 | 137877681 |
| D-43 | chr6 | 137877691 | 137877876 |
| D-44 | chr6 | 137877903 | 137877963 |
| D-45 | chr6 | 137877978 | 137878103 |
| D-46 | chr6 | 137878115 | 137878201 |
| D-47 | chr6 | 137878213 | 137878249 |
| D-48 | chr6 | 137878263 | 137878369 |
| D-49 | chr6 | 137878391 | 137879329 |
| D-50 | chr6 | 137879331 | 137879657 |
| D-51 | chr6 | 137879694 | 137879920 |
| D-52 | chr6 | 137879932 | 137880488 |
| D-53 | chr6 | 137880490 | 137880565 |
| D-54 | chr6 | 137880584 | 137881948 |
| D-55 | chr6 | 137881987 | 137882047 |
| D-56 | chr6 | 137882055 | 137882090 |
| D-57 | chr6 | 137882102 | 137882232 |
| D-58 | chr6 | 137882245 | 137882321 |
| D-59 | chr6 | 137882333 | 137882844 |
| D-60 | chr6 | 137882847 | 137883093 |
| D-61 | chr6 | 137883125 | 137883166 |
| D-62 | chr6 | 137883199 | 137883256 |
| D-63 | chr6 | 137883324 | 137883364 |
| D-64 | chr6 | 137883368 | 137883438 |

The foregoing detailed description is provided in an illustrative and exemplary manner, and is not intended to limit the scope of the appended claims. Various modifications of the embodiments currently listed herein will be apparent to those of ordinary skills in the art, and are retained within the scope of the appended claims and equivalent embodiments thereof.

**Claims**

1. A method for culturing tumor infiltrating lymphocytes (TILs), wherein the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the TILs with feeder cells after contacting the TILs with T cell activators and/or T cell growth factors for a period of time.

2. The method according to claim 1, wherein the method comprises reducing the expression and/or decreasing the activity of two or more proteins of the TILs before co-culturing the TILs with the feeder cells.

3. The method according to any one of claims 1-2, wherein the method comprises reducing the expression and/or decreasing the activity of two or more proteins of the TILs after contacting the TILs with the T cell activators and/or the T cell growth factors and before co-culturing the TILs with the feeder cells.

4. A method for culturing tumor infiltrating lymphocytes (TILs), wherein the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein the TILs comprise TILs obtained by co-culturing the TILs with feeder cells after contacting the TILs with T cell activators and/or T cell growth factors for a period of time.

5. A method for culturing tumor infiltrating lymphocytes (TILs), wherein the method comprises co-culturing the TILs with feeder cells after contacting the TILs with T cell activators and/or T cell growth factors for a period of time, wherein the TILs comprise TILs obtained by reducing the expression and/or decreasing the activity of the two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

6. The method according to any one of claims 1-5, wherein compared to TILs with unchanged expression and/or activity of the two or more proteins, the TILs obtained by reducing the expression and/or decreasing the activity of the two or more proteins of the TILs show improved TIL properties.

7. The method according to claim 6, wherein the improved TIL properties comprise one or more properties selected from the group consisting of: increased number and expansion capacity of TIL cells, increased proportion of viable cells, enhanced persistence, improved proportion of T cell subpopulations, enhanced cytokine secretion capacity, enhanced tumor cell killing ability, enhanced anti-apoptotic ability, and enhanced T cell receptor (TCR) clonal diversity.

8. The method according to claim 7, wherein the improved proportion of T cell subpopulations comprises one or more selected from the group consisting of: increased proportion of central memory T cells and/or naive T cells, decreased proportion of regulatory T cells, increased proportion of activated T cells, increased proportion of tumor-specific T cells, and increased proportion of stem cell-like T cells.

9. The method according to any one of claims 1-8, wherein the step of reducing the expression and/or decreasing the activity of two or more proteins of the TILs comprises introducing a gene regulatory system into the TIL cells.

10. The method according to claim 9, wherein the gene regulatory system is capable of destroying the two or more proteins at the DNA level.

11. The method according to any one of claims 9-10, wherein the gene regulatory system comprises a guide nucleic acid molecule and a zymoprotein.

12. The method according to claim 11, wherein the step of reducing the expression and/or decreasing the activity of two or more proteins of the TILs comprises introducing a ribonucleoprotein complex (RNP) comprising the guide nucleic acid molecule and the zymoprotein into the TILs.

13. The method according to any one of claims 11-12, wherein the zymoprotein comprises a Cas protein, a Cas protein homolog, or functionally active fragments thereof.

14. The method according to any one of claims 11-13, wherein the guide nucleic acid molecule comprises a guide RNA (gRNA).

15. The method according to any one of claims 11-14, wherein the guide nucleic acid molecule is capable of binding to a sequence of the two or more proteins.

16. The method according to any one of claims 1-15, wherein the SOCS1 is a suppressor of Janus kinase-signal transducer and activator of transcription (JAK-STAT) pathway.

17. The method according to any one of claims 1-16, wherein the TNFAIP3 is a suppressor of NF-κB pathway.

18. The method according to any one of claims 1-17, wherein the CBLB is a E3 ubiquitin ligase.

19. The method according to any one of claims 1-18, wherein the ZC3H12A is a protein encoding a zinc finger domain.

20. The method according to any one of claims 11-19, wherein the guide nucleic acid molecule is capable of binding to a region or a fragment thereof defined by any one group of genomic coordinates shown in Tables 1A-1D.

21. The method according to any one of claims 11-20, wherein the guide nucleic acid molecule is capable of binding to a region or a fragment thereof selected from a group as shown below: SEQ ID NOs: 34 to 35, 38 to 39, 42 to 44, and 48 to 50.

22. The method according to any one of claims 11-21, wherein the guide nucleic acid molecule is capable of binding to a sequence consisting of about 15 to about 25 nucleotides upstream of 5' end of a protospacer adjacent motif (PAM) selected from a group as shown below: AGG, TGG, CGG, and GGG.

23. The method according to any one of claims 11-22, wherein the guide nucleic acid molecule comprises a sequence as shown in any one of SEQ ID NOs: 36 to 37, 40 to 41, 45 to 47, and 51 to 58.

24. The method according to any one of claims 1-23, wherein the proportion of cells expressing the two or more proteins is reduced and/or the expression of the two or more proteins in an individual cell is decreased in the TILs obtained by reducing the expression and/or decreasing the activity of the two or more proteins of the TILs, compared to that in TILs with unchanged expression and/or activity of the two or more proteins.

25. The method according to any one of claims 1-24, wherein the proportion of cells expressing the two or more proteins is about 95% or less in the TILs obtained by reducing the expression and/or decreasing the activity of the two or more proteins of the TILs.

26. The method according to any one of claims 1-25, wherein the method further comprises subjecting TILs derived from tumor tissues and not expanded *in vitro* to at least one stage *of in vitro* expansion, wherein the TILs are co-cultured with the feeder cells during the at least one stage *of in vitro* expansion.

27. The method according to claim 26, wherein the method comprises co-culturing the TILs with the feeder cells during a single stage *of in vitro* expansion.

28. The method according to any one of claims 26-27, wherein the method comprises reducing the expression and/or decreasing the activity of the two or more proteins of the TILs, and co-culturing the TILs with the feeder cells during the single stage *of in vitro* expansion.

29. The method according to any one of claims 26-28, wherein the method comprises subjecting the TILs derived from tumor tissues and not expanded *in vitro* to a first stage *of in vitro* expansion and a second stage of *in vitro* expansion, and during the second stage of *in vitro* expansion, co-culturing the TILs with the feeder cells.

30. The method according to claim 29, wherein the first stage *of in vitro* expansion is carried out for at least about 7 days.

31. The method according to any one of claims 29-30, wherein the first stage of *in vitro* expansion is carried out for about 7 days to about 14 days.

32. The method according to any one of claims 29-31, wherein the second stage *of in vitro* expansion is carried out for at least about 7 days.

33. The method according to any one of claims 29-32, wherein the second stage *of in vitro* expansion is carried out for about 7 days to about 14 days.

34. The method according to any one of claims 1-33, wherein the method comprises co-culturing the TILs with the feeder cells after contacting the TILs with the T cell activators and/or the T cell growth factors for at least about 2 hours.

35. The method according to any one of claims 1-34, wherein the method comprises co-culturing the TILs with the feeder cells after contacting the TILs with the T cell activators and/or the T cell growth factors for about 6 hours to about 72 hours.

36. The method according to any one of claims 1-35, wherein the method comprises co-culturing the TILs with the feeder cells after contacting the TILs with the T cell activators and/or the T cell growth factors for about 12 hours to about 48 hours.

37. The method according to any one of claims 1-36, wherein the method comprises co-culturing the TILs with the feeder cells after contacting the TILs with the T cell activators and/or the T cell growth factors for about 6 hours, about 12 hours, about 24 hours, about 48 hours, or about 72 hours.

38. The method according to any one of claims 1-37, wherein the feeder cells comprise antigen-presenting cells.

39. The method according to any one of claims 1-38, wherein the feeder cells comprise one or more cells selected from the group consisting of: peripheral mononuclear cells, dendritic cells, and artificial antigen-presenting cells.

40. The method according to any one of claims 1-39, wherein the feeder cells are peripheral mononuclear cells.

41. The method according to any one of claims 1-40, wherein the feeder cells are irradiated feeder cells.

42. The method according to any one of claims 1-41, wherein the step of co-culturing the TILs with the feeder cells comprises contacting the surface of the feeder cells with the surface of the TILs.

43. The method according to any one of claims 1-42, wherein the step of co-culturing the TILs with the feeder cells comprises adding the feeder cells into the cell culture medium of the TILs.

44. The method according to any one of claims 1-43, wherein the method comprises adding the feeder cells into the cell culture medium of the TILs at a ratio of the feeder cells to the TILs from about 40:1 to about 400:1.

45. The method according to any one of claims 1-44, wherein the method further comprises subjecting TILs derived from tumor tissues and not expanded *in vitro* to at least one stage *of in vitro* expansion, wherein the TILs are contacted with the T cell activators during the at least one stage *of in vitro* expansion.

46. The method according to claim 45, wherein the method comprises contacting the TILs with the T cell activators during the single stage of *in vitro* expansion.

47. The method according to any one of claims 45-46, wherein the method comprises reducing the expression and/or decreasing the activity of the two or more proteins of the TILs and contacting the TILs with the T cell activators during the single stage *of in vitro* expansion.

48. The method according to any one of claims 45-47, wherein the method comprises subjecting the TILs derived from tumor tissues and not expanded *in vitro* to a first stage *of in vitro* expansion and a second stage of *in vitro* expansion, and during the second stage of *in vitro* expansion, contacting the TILs with the T cell activators.

49. The method according to any one of claims 1-48, wherein the T cell activators comprise one or more T cell activators selected from the group consisting of: cluster of differentiation 80 (CD80), CD86, CD276, 4-1BB ligand (4-1BBL), CD27, CD30, CD134, CD275, CD40, CD258, and functionally active fragments thereof.

50. The method according to any one of claims 1-49, wherein the T cell activators comprise agonists of one or more targets selected from the group consisting of: CD3, CD28, herpes virus entry mediator (HVEM), CD40L, OX40, and 4-1BB.

51. The method according to any one of claims 1-50, wherein the T cell activators comprise a CD3 agonist and/or a CD28 agonist.

52. The method according to any one of claims 1-51, wherein the T cell activators comprise a CD3 agonist.

53. The method according to any one of claims 1-52, wherein the T cell activators comprise an anti-CD3 antibody and/or an antigen-binding fragment thereof.

54. The method according to any one of claims 1-53, wherein the T cell activators comprise a CD28 agonist.

55. The method according to any one of claims 1-54, wherein the T cell activators comprise an anti-CD28 antibody and/or an antigen-binding fragment thereof, CD80 and/or a functionally active fragment thereof, and/or CD86 and/or a functionally active fragment thereof.

56. The method according to any one of claims 1-55, wherein the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

57. The method according to claim 56, wherein the initial concentration of each of the T cell activators in the cell culture medium of the TILs is each independently at least about 30 ng/mL.

58. The method according to any one of claims 56-57, wherein the initial concentration of each of the T cell activators in the cell culture medium of the TILs is each independently about 30 ng/mL to about 300 ng/mL.

59. The method according to any one of claims 56-58, wherein the diameter of the solid medium is about 500 nm to about 10 μm.

60. The method according to any one of claims 56-59, wherein the diameter of the solid medium is about 1 nm to about 500 nm.

61. The method according to any one of claims 56-60, wherein the diameter of the solid medium is measured by transmission electron microscopy.

62. The method according to any one of claims 56-61, wherein the solid medium comprises a polymer.

63. The method according to any one of claims 56-62, wherein the amount of each of the T cell activators comprised in each mg of the solid medium is each independently at least about 25 μg.

64. The method according to any one of claims 56-63, wherein the method comprises adding the solid medium comprising the T cell activators into the cell culture medium of the TILs at a ratio of the solid medium to the TILs from about 2:1 to about 1:2.

65. The method according to any one of claims 56-64, wherein the method comprises adding the solid medium comprising the T cell activators into the cell culture medium of the TILs at a ratio of the solid medium to the TILs from about 1: 100 to about 1:2000.

66. The method according to any one of claims 1-65, wherein the method further comprises subjecting TILs derived from tumor tissues and not expanded *in vitro* to at least one stage *of in vitro* expansion, wherein the TILs are contacted with the T cell growth factors during the at least one stage *of in vitro* expansion.

67. The method according to claim 66, wherein the method comprises contacting the TILs with the T cell growth factors during the single stage of *in vitro* expansion.

68. The method according to any one of claims 66-67, wherein the method comprises contacting the TILs with the T cell activators and the T cell growth factors during the single stage *of in vitro* expansion.

69. The method according to any one of claims 66-68, wherein the method comprises subjecting TILs derived from tumor tissues and not expanded *in vitro* to a first stage of *in vitro* expansion and a second stage of *in vitro* expansion, and during the second stage of *in vitro* expansion, contacting the TILs with the T cell growth factors.

70. The method according to any one of claims 1-69, wherein the method comprises contacting the TILs with the T cell activators and the T cell growth factors substantially simultaneously.

71. The method according to any one of claims 1-70, wherein the T cell growth factors are one or more T cell growth factors selected from the group consisting of: IL-2, IL-7, IL-12, IL-15, IL-21, interferon-γ, and functionally active fragments thereof.

72. The method according to any one of claims 1-71, wherein the T cell growth factors comprise IL-2 and/or a functionally active fragment thereof.

73. The method according to any one of claims 1-72, wherein the step of contacting the TILs with the T cell growth factors comprises adding the T cell growth factors into the cell culture medium of the TILs.

74. The method according to any one of claims 1-73, wherein the initial concentration of each of the T cell growth factors in the cell culture medium of the TILs is each independently at least about 300 IU/mL.

75. The method according to any one of claims 1-74, wherein the TILs are selected from the group consisting of: TILs derived from tumor tissue debris, TILs derived from lymphatic metastasis debris, TILs derived from pleural effusion, TILs derived from peritoneal effusion, and TILs resuscitated after cryopreservation.

76. The method according to claim 75, wherein the debris has a volume of about 1 mm$^3$ to about 27 mm$^3$.

77. A method for culturing tumor infiltrating lymphocytes (TILs), which comprises:

    (A) contacting a first TIL population derived from tumor tissues and not expanded *in vitro* with T cell growth factors, wherein a second TIL population is obtained via the step (A);
    (B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the second TIL population with feeder cells after contacting the second TIL population with T cell activators and/or T cell growth factors for a period of time, wherein a third TIL population is obtained via the step (B).

78. A method for culturing tumor infiltrating lymphocytes (TILs), which comprises:

    (A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro;*
    (B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and co-culturing the second TIL population with feeder cells after contacting the second TIL population with T cell activators and/or T cell growth factors for a period of time, wherein a third TIL population is obtained via the step (B).

79. The method according to claim 78, wherein the *in vitro* TIL population comprises a TIL population obtained by contacting the first TIL population with T cell growth factors.

80. The method according to any one of claims 78-79, wherein the *in vitro* TIL population comprises a TIL population obtained by cryopreserving the first TIL population.

81. The method according to any one of claims 77-80, wherein the step (A) is carried out for about 7 days to about 14 days.

**82.** The method according to any one of claims 77-81, wherein the step (B) is carried out for about 7 days to about 14 days.

**83.** A method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the second TIL population with T cell activators and/or T cell growth factors, wherein a third TIL population is obtained via the step (B);
(C) co-culturing the third TIL population with feeder cells, wherein a fourth TIL population is obtained via the step (C).

**84.** A method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro;*
(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the second TIL population with T cell activators and/or T cell growth factors, wherein a third TIL population is obtained via the step (B);
(C) co-culturing the third TIL population with feeder cells, wherein a fourth TIL population is obtained via the step (C).

**85.** The method according to claim 84, wherein the *in vitro* TIL population comprises a TIL population obtained by contacting the first TIL population with T cell growth factors.

**86.** The method according to any one of claims 84-85, wherein the *in vitro* TIL population comprises a TIL population obtained by cryopreserving the first TIL population.

**87.** The method according to any one of claims 83-86, wherein the step (A) is carried out for about 7 days to about 14 days.

**88.** The method according to any one of claims 83-87, wherein the step (B) is carried out for about 0 days to about 8 days.

**89.** The method according to any one of claims 83-88, wherein the step (C) is carried out for about 5 days to about 14 days.

**90.** A method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);
(B) contacting the second TIL population with T cell activators and/or T cell growth factors, wherein a third TIL population is obtained via the step (B);
(C) reducing the expression and/or decreasing the activity of two or more proteins of the third TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein a fourth TIL population is obtained via the step (C);
(D) co-culturing the fourth TIL population with feeder cells, wherein a fifth TIL population is obtained via the step (D).

**91.** A method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of a first TIL population derived from tumor tissues and not expanded *in vitro;*
(B) contacting the second TIL population with T cell activators and/or T cell growth factors, wherein a third TIL population is obtained via the step (B);

(C) reducing the expression and/or decreasing the activity of two or more proteins of the third TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein a fourth TIL population is obtained via the step (C);

(D) co-culturing the fourth TIL population with feeder cells, wherein a fifth TIL population is obtained via the step (D).

92. The method according to claim 91, wherein the *in vitro* TIL population comprises a TIL population obtained by contacting the first TIL population with T cell growth factors.

93. The method according to any one of claims 91-92, wherein the *in vitro* TIL population comprises a TIL population obtained by cryopreserving the first TIL population.

94. The method according to any one of claims 90-93, wherein the step (A) is carried out for about 7 days to about 14 days.

95. The method according to any one of claims 90-94, wherein the step (B) is carried out for about 0 days to about 4 days.

96. The method according to any one of claims 90-95, wherein the step (C) is carried out for about 0 days to about 4 days.

97. The method according to any one of claims 90-96, wherein the step (D) is carried out for about 5 days to about 14 days.

98. The method according to any one of claims 77-97, wherein compared to TILs with unchanged expression and/or activity of the two or more proteins, TILs obtained by reducing the expression and/or decreasing the activity of the two or more proteins of the TILs show improved TIL properties.

99. The method according to claim 98, wherein the improved TIL properties comprise one or more properties selected from the group consisting of: increased number and expansion capacity of TIL cells, increased proportion of viable cells, enhanced persistence, improved proportion of T cell subpopulations, enhanced cytokine secretion capacity, enhanced tumor cell killing ability, enhanced anti-apoptotic ability, and enhanced T cell receptor (TCR) clonal diversity.

100.
The method according to claim 99, wherein the improved proportion of T cell subpopulations comprises one or more selected from the group consisting of: increased proportion of central memory T cells and/or naive T cells, decreased proportion of regulatory T cells, increased proportion of activated T cells, increased proportion of tumor-specific T cells, and increased proportion of stem cell-like T cells.

101.
The method according to any one of claims 77-100, wherein the step of reducing the expression and/or decreasing the activity of two or more proteins of the TILs comprises introducing a gene regulatory system into the TIL cells.

102.
The method according to claim 101, wherein the gene regulatory system is capable of destroying the two or more proteins at the DNA level.

103.
The method according to any one of claims 101-102, wherein the gene regulatory system comprises a guide nucleic acid molecule and a zymoprotein.

104.
The method according to claim 103, wherein the step of reducing the expression and/or decreasing the activity of two or more proteins of the TILs comprises introducing a ribonucleoprotein complex (RNP) comprising the guide nucleic acid molecule and the zymoprotein into the TILs.

105.
The method according to any one of claims 103-104, wherein the zymoprotein comprises a Cas protein, a Cas protein homolog, or functionally active fragments thereof.

**106.**
The method according to any one of claims 103-105, wherein the guide nucleic acid molecule comprises a guide RNA (gRNA).

**107.**
The method according to any one of claims 103-106, wherein the guide nucleic acid molecule is capable of binding to a sequence of the two or more proteins.

**108.**
The method according to any one of claims 77-107, wherein the SOCS1 is a suppressor of Janus kinase-signal transducer and activator of transcription (JAK-STAT) pathway.

**109.**
The method according to any one of claims 77-108, wherein the TNFAIP3 is a suppressor of NF-κB pathway.

**110.**
The method according to any one of claims 77-109, wherein the CBLB is an E3 ubiquitin ligase.

**111.** The method according to any one of claims 77-110, wherein the ZC3H12A is a protein encoding a zinc finger domain.

**112.**
The method according to any one of claims 103-111, wherein the guide nucleic acid molecule is capable of binding to a region or a fragment thereof defined by any one group of genomic coordinates shown in Tables 1A-1D.

**113.**
The method according to any one of claims 103-112, wherein the guide nucleic acid molecule is capable of binding to a region or a fragment thereof selected from a group as shown below: SEQ ID NOs: 34 to 35, 38 to 39, 42 to 44, and 48 to 50.

**114.**
The method according to any one of claims 103-113, wherein the guide nucleic acid molecule is capable of binding to a sequence consisting of about 15 to about 25 nucleotides upstream of 5' end of a protospacer adjacent motif (PAM) selected from the group consisting of: AGG, TGG, CGG, and GGG.

**115.**
The method according to any one of claims 103-114, wherein the guide nucleic acid molecule comprises a sequence as shown in any one of SEQ ID NOs: 36 to 37, 40 to 41, 45 to 47, and 51 to 58.

**116.**
The method according to any one of claims 77-115, wherein the proportion of cells expressing the two or more proteins is reduced and/or the expression of the two or more proteins in an individual cell is decreased in the TILs obtained by reducing the expression and/or decreasing the activity of the two or more proteins of the TILs, compared to that in TILs with unchanged expression and/or activity of the two or more proteins.

**117.**
The method according to any one of claims 77-116, wherein the proportion of cells expressing the two or more proteins is about 95% or less in the TILs obtained by reducing the expression and/or decreasing the activity of the two or more proteins of the TILs.

**118.**
The method according to any one of claims 77-117, wherein the method comprises co-culturing the TILs with the feeder cells after contacting the TILs with the T cell activators and/or the T cell growth factors for at least about 2 hours.

**119.**
The method according to any one of claims 77-118, wherein the method comprises co-culturing the TILs with the feeder cells after contacting the TILs with the T cell activators and/or the T cell growth factors for about 6 hours to about 72 hours.

**120.**
The method according to any one of claims 77-119, wherein the method comprises co-culturing the TILs with the feeder cells after contacting the TILs with the T cell activators and/or the T cell growth factors for about 12 hours to about 48 hours.

**121.**

The method according to any one of claims 77-120, wherein the method comprises co-culturing the TILs with the feeder cells after contacting the TILs with the T cell activators and/or the T cell growth factors for about 6 hours, about 12 hours, about 24 hours, about 48 hours, or about 72 hours.

**122.**

The method according to any one of claims 77-121, wherein the feeder cells comprise antigen-presenting cells.

**123.**

The method according to any one of claims 77-122, wherein the feeder cells comprise one or more cells selected from the group consisting of: peripheral mononuclear cells, dendritic cells, and artificial antigen-presenting cells.

**124.**

The method according to any one of claims 77-123, wherein the feeder cells are peripheral mononuclear cells.

**125.**

The method according to any one of claims 77-124, wherein the feeder cells are irradiated feeder cells.

**126.**

The method according to any one of claims 77-125, wherein the step of co-culturing the TILs with the feeder cells comprises contacting the surface of the feeder cells with the surface of the TILs.

**127.**

The method according to any one of claims 77-126, wherein the step of co-culturing the TILs with the feeder cells comprises adding the feeder cells into the cell culture medium of the TILs.

**128.**

The method according to any one of claims 77-127, wherein the method comprises adding the feeder cells into the cell culture medium of the TILs at a ratio of the feeder cells to the TILs from about 40:1 to about 400:1.

**129.**

The method according to any one of claims 77-128, wherein the T cell activators comprise one or more T cell activators selected from the group consisting of: cluster of differentiation 80 (CD80), CD86, CD276, 4-1BB ligand (4-1BBL), CD27, CD30, CD134, CD275, CD40, CD258, and the functionally active fragments thereof.

**130.**

The method according to any one of claims 77-129, wherein the T cell activators comprise agonists of one or more targets selected from the group consisting of: CD3, CD28, herpes virus entry mediator (HVEM), CD40L, OX40, and 4-1BB.

**131.**

The method according to any one of claims 77-130, wherein the T cell activators comprise a CD3 agonist and/or a CD28 agonist.

**132.**

The method according to any one of claims 77-131, wherein the T cell activators comprise a CD3 agonist.

**133.**

The method according to any one of claims 77-132, wherein the T cell activators comprise an anti-CD3 antibody and/or an antigen-binding fragment thereof.

**134.**

The method according to any one of claims 77-133, wherein the T cell activators comprise a CD28 agonist.

**135.**

The method according to any one of claims 77-134, wherein the T cell activators comprise an anti-CD28 antibody and/or an antigen-binding fragment thereof, CD80 and/or a functionally active fragment thereof, and/or CD86 and/or a functionally active fragment thereof.

**136.**

The method according to any one of claims 77-135, wherein the step of contacting the TILs with the T cell activators comprises one or more ways selected from the group consisting of: (1) adding the T cell activators into the cell culture medium of the TILs; (2) adding engineered cells expressing the T cell activators into the cell culture medium of the TILs; and (3) adding a solid medium comprising the T cell activators into the cell culture medium of the TILs.

**137.**

The method according to claim 136, wherein the initial concentration of each of the T cell activators in the cell culture medium of the TILs is each independently at least about 30 ng/mL.

**138.**

The method according to any one of claims 136-137, wherein the initial concentration of each of the T cell activators in the cell culture medium of the TILs is each independently about 30 ng/mL to about 300 ng/mL.

**139.**

The method according to any one of claims 136-138, wherein the diameter of the solid medium is about 500 nm to about 10 $\mu$m.

**140.**

The method according to any one of claims 136-139, wherein the diameter of the solid medium is about 1 nm to about 500 nm.

**141.**

The method according to any one of claims 139-140, wherein the diameter of the solid medium is measured by transmission electron microscopy.

**142.**

The method according to any one of claims 136-141, wherein the solid medium comprises a polymer.

**143.**

The method according to any one of claims 136-142, wherein the amount of each of the T cell activators comprised in each mg of the solid medium is each independently at least about 25 $\mu$g.

**144.**

The method according to any one of claims 136-143, wherein the method comprises adding the solid medium comprising the T cell activators into the cell culture medium of the TILs at a ratio of the solid medium to the TILs from about 2:1 to about 1:2.

**145.**

The method according to any one of claims 136-144, wherein the method comprises adding the solid medium comprising the T cell activators into the cell culture medium of the TILs at a ratio of the solid medium to the TILs from about 1:100 to about 1 :2000.

**146.**

The method according to any one of claims 77-145, wherein the method comprises contacting the TILs with the T cell activators and the T cell growth factors substantially simultaneously.

**147.**

The method according to any one of claims 77-146, wherein the T cell growth factors are one or more T cell growth factors selected from the group consisting of: IL-2, IL-7, IL-12, IL-15, IL-21, interferon-$\gamma$, and functionally active fragments thereof.

**148.**

The method according to any one of claims 77-147, wherein the T cell growth factors comprise IL-2 and/or a functionally active fragment thereof.

**149.**

The method according to any one of claims 77-148, wherein the step of contacting the TILs with the T cell growth factors comprises adding the T cell growth factors into the cell culture medium of the TILs.

**150.**

The method according to any one of claims 77-149, wherein the initial concentration of each of the T cell growth factors in the cell culture medium of the TILs is each independently at least about 300 IU/mL.

**151.**

The method according to any one of claims 77-150, wherein the TILs are selected from the group consisting of: TILs derived from tumor tissue debris, TILs derived from lymphatic metastasis debris, TILs derived from pleural effusion, TILs derived from peritoneal effusion, and TILs resuscitated after cryopreservation.

**152.**

The method according to claim 151, wherein the debris has a volume of about 1 mm$^3$ to about 27 mm$^3$.

**153.**

A method for culturing tumor infiltrating lymphocytes (TILs), wherein the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist.

**154.**

The method according to claim 153, wherein the method comprises reducing the expression and/or decreasing the activity of the two or more proteins of the TILs after contacting the TILs with the CD28 agonist.

**155.**

A method for culturing tumor infiltrating lymphocytes (TILs), wherein the method comprises reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), wherein the TILs comprise TILs obtained by contacting the TILs with a CD28 agonist.

**156.**

A method for culturing tumor infiltrating lymphocytes (TILs), wherein the method comprises contacting the TILs with a CD28 agonist, wherein the TILs comprise TILs obtained by reducing the expression and/or decreasing the activity of two or more proteins of the TILs selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A).

**157.**

The method according to any one of claims 153-156, wherein compared to TILs with unchanged expression and/or activity of the two or more proteins, TILs obtained by reducing the expression and/or decreasing the activity of the two or more proteins of the TILs show improved TIL properties.

**158.**

The method according to claim 157, wherein the improved TIL properties comprise one or more properties selected from the group consisting of: increased number and expansion capacity of TIL cells, increased proportion of viable cells, enhanced persistence, improved proportion of T cell subpopulations, enhanced cytokine secretion capacity, enhanced tumor cell killing ability, and enhanced T cell receptor (TCR) clonal diversity.

**159.**

The method according to claim 158, wherein the improved proportion of T cell subpopulations comprises one or more selected from the group consisting of: increased proportion of central memory T cells and/or naive T cells, decreased proportion of regulatory T cells, increased proportion of activated T cells, increased proportion of tumor-specific T cells, and increased proportion of stem cell-like T cells.

**160.**

The method according to any one of claims 153-159, wherein compared to corresponding TILs that have not been contacted with the CD28 agonist during the stage *of in vitro* expansion, the TILs that have been contacted with the CD28 agonist during at least one stage of *in vitro* expansion show an improved gene editing effect.

**161.**

The method according to claim 160, wherein the improved gene editing effect comprises an enhanced gene knockout efficiency.

**162.**

The method according to any one of claims 153-161, wherein the step of reducing the expression and/or decreasing the activity of the two or more proteins of the TILs comprises introducing a gene regulatory system into the TIL cells.

**163.**

The method according to claim 162, wherein the gene regulatory system is capable of destroying the two or more proteins at the DNA level.

**164.**

The method according to any one of claims 162-163, wherein the gene regulatory system comprises a guide nucleic acid molecule and a zymoprotein.

**165.**

The method according to claim 164, wherein the step of reducing the expression and/or decreasing the activity of the two or more proteins of the TILs comprises introducing a ribonucleoprotein complex (RNP) comprising the guide nucleic acid molecule and the zymoprotein into the TILs.

**166.**

The method according to any one of claims 164-165, wherein the zymoprotein comprises a Cas protein, a Cas protein homolog, or functionally active fragments thereof.

**167.**

The method according to any one of claims 164-166, wherein the guide nucleic acid molecule comprises a guide RNA (gRNA).

**168.**

The method according to any one of claims 164-167, wherein the guide nucleic acid molecule is capable of binding to a sequence of the two or more proteins.

**169.**

The method according to any one of claims 153-168, wherein the SOCS1 is a suppressor of Janus kinase-signal transducer and activator of transcription (JAK-STAT) pathway.

**170.**

The method according to any one of claims 153-169, wherein the TNFAIP3 is a suppressor of NF-κB pathway.

**171.**

The method according to any one of claims 153-170, wherein the CBLB is an E3 ubiquitin ligase.

**172.**

The method according to any one of claims 153-171, wherein the ZC3H12A is a protein encoding a zinc finger domain.

**173.**

The method according to any one of claims 164-172, wherein the guide nucleic acid molecule is capable of binding to a region or a fragment thereof defined by any one group of genomic coordinates shown in Tables 1A-1D.

**174.**

The method according to any one of claims 164-173, wherein the guide nucleic acid molecule is capable of binding to a region or a fragment thereof selected from a group as shown below: SEQ ID NOs: 34 to 35, 38 to 39, 42 to 44, and 48 to 50.

**175.**

The method according to any one of claims 164-174, wherein the guide nucleic acid molecule is capable of binding to a sequence consisting of about 15 to about 25 nucleotides upstream of 5' end of a protospacer adjacent motif (PAM) selected from the group consisting of: AGG, TGG, CGG, and GGG.

**176.**
The method according to any one of claims 164-175, wherein the guide nucleic acid molecule comprises a sequence as shown in any one of SEQ ID NOs: 36 to 37, 40 to 41, 45 to 47, and 51 to 58.

**177.**
The method according to any one of claims 153-176, wherein the proportion of cells expressing the two or more proteins is reduced and/or the expression of the two or more proteins in an individual cell is decreased in the TILs obtained by reducing the expression and/or decreasing the activity of the two or more proteins of the TILs, compared to that in TILs with unchanged expression and/or activity of the two or more proteins.

**178.**
The method according to any one of claims 153-177, wherein the proportion of cells expressing the two or more proteins is about 95% or less in the TILs obtained by reducing the expression and/or decreasing the activity of the two or more proteins of the TILs.

**179.**
The method according to any one of claims 153-178, wherein the method comprises subjecting TILs derived from tumor tissues and not expanded *in vitro* to at least one stage *of in vitro* expansion, wherein the TILs are contacted with the CD28 agonist during the at least one stage *of in vitro* expansion.

**180.**
The method according to claim 179, wherein the method comprises subjecting the TILs derived from tumor tissues and not expanded *in vitro* to a first stage *of in vitro* expansion and a second stage *of in vitro* expansion, and during the second stage *of in vitro* expansion, contacting the TILs that have been expanded *in vitro* in the first stage with the CD28 agonist.

**181.**
The method according to claim 180, wherein the first stage *of in vitro* expansion is carried out for at least about 7 days.

**182.**
The method according to any one of claims 180-181, wherein the first stage of *in vitro* expansion is carried out for about 7 days to about 14 days.

**183.**
The method according to any one of claims 180-182, wherein the second stage *of in vitro* expansion is carried out for at least about 7 days.

**184.**
The method according to any one of claims 180-183, wherein the second stage *of in vitro* expansion is carried out for about 7 days to about 14 days.

**185.**
The method according to any one of claims 153-184, wherein the CD28 agonist comprises an anti-CD28 antibody and/or an antigen-binding fragment thereof, CD80 and/or a functionally active fragment thereof, and/or CD86 and/or a functionally active fragment thereof.

**186.**
The method according to any one of claims 153-185, wherein the method further comprises subjecting the TILs derived from tumor tissues and not expanded *in vitro* to at least one stage of *in vitro* expansion, wherein the TILs are contacted with other T cell activators other than the CD28 agonist during the at least one stage *of in vitro* expansion.

**187.**
The method according to claim 186, wherein the method comprises contacting the TILs with the other T cell activators during a single stage *of in vitro* expansion.

**188.**

The method according to any one of claims 186-187, wherein the method comprises reducing the expression and/or decreasing the activity of the two or more proteins of the TILs and contacting the TILs with the other T cell activators during the single stage *of in vitro* expansion.

**189.**

The method according to any one of claims 186-188, wherein the method comprises subjecting the TILs derived from tumor tissues and not expanded *in vitro* to a first stage *of in vitro* expansion and a second stage of *in vitro* expansion, and during the second stage of *in vitro* expansion, contacting the TILs with the other T cell activators.

**190.**

The method according to any one of claims 186-189, wherein the method comprises contacting the TILs with the CD28 agonist and the other T cell activators substantially simultaneously.

**191.**

The method according to any one of claims 186-190, wherein the other T cell activators comprise agonists of one or more targets selected from the group consisting of: CD3, HVEM, CD40L, OX40, and 4-1BB.

**192.**

The method according to any one of claims 186-191, wherein the other T cell activators comprise a CD3 agonist.

**193.**

The method according to any one of claims 186-192, wherein the other T cell activators comprise an anti-CD3 antibody and/or an antigen-binding fragment thereof.

**194.**

The method according to any one of claims 186-193, wherein the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; and (3) adding a solid medium comprising the CD28 agonist and the other T cell activators into the cell culture medium of the TILs.

**195.**

The method according to claim 194, wherein the initial concentration of the other T cell activators in the cell culture medium of the TILs is at least about 30 ng/mL.

**196.**

The method according to any one of claims 194-195, wherein the initial concentration of the other T cell activators in the cell culture medium of the TILs is about 30 ng/mL to about 300 ng/mL.

**197.**

The method according to any one of claims 194-196, wherein the diameter of the solid medium is about 500 nm to about 10 $\mu$m.

**198.**

The method according to any one of claims 194-197, wherein the diameter of the solid medium is about 1 nm to about 500 nm.

**199.**

The method according to any one of claims 197-198, wherein the diameter of the solid medium is measured by transmission electron microscopy.

**200.**

The method according to any one of claims 194-199, wherein the solid medium comprises a polymer.

**201.**

The method according to any one of claims 194-200, wherein each mg of the solid medium comprises at least about 25 $\mu$g of the CD28 agonist and the other T cell activators.

**202.**

The method according to any one of claims 194-201, wherein the solid medium comprising the CD28 agonist and the other T cell activators is added into the cell culture medium of the TILs at a ratio of the solid medium to the TILs from about 2:1 to about 1:2.

**203.**

The method according to any one of claims 194-202, wherein the solid medium comprising the CD28 agonist and the other T cell activators is added into the cell culture medium of the TILs at a ratio of the solid medium to the TILs from about 1:100 to about 1 :2000.

**204.**

The method according to any one of claims 153-203, wherein the method further comprises subjecting the TILs derived from tumor tissues and not expanded *in vitro* to at least one stage *of in vitro* expansion, wherein the TILs are co-cultured with the feeder cells after contacting the TILs with the CD28 agonist for a period of time during the at least one stage of *in vitro* expansion.

**205.**

The method according to claim 204, wherein the method comprises co-culturing the TILs with the feeder cells during the single stage *of in vitro* expansion.

**206.**

The method according to any one of claims 204-205, wherein the method comprises contacting the TILs with the CD28 agonist and co-culturing the TILs with the feeder cells during the single stage *of in vitro* expansion.

**207.**

The method according to any one of claims 204-206, wherein the method comprises subjecting the TILs derived from tumor tissues and not expanded *in vitro* to a first stage *of in vitro* expansion and a second stage of *in vitro* expansion, and during the second stage of *in vitro* expansion, co-culturing the TILs with the feeder cells.

**208.**

The method according to any one of claims 204-207, wherein the method comprises co-culturing the TILs with the feeder cells after contacting the TILs with the CD28 agonist for at least about 2 hours.

**209.**

The method according to any one of claims 204-208, wherein the method comprises co-culturing the TILs with the feeder cells after contacting the TILs with the CD28 agonist for about 6 hours to about 72 hours.

**210.**

The method according to any one of claims 204-209, wherein the method comprises co-culturing the TILs with the feeder cells after contacting the TILs with the CD28 agonist for about 12 hours to about 48 hours.

**211.**

The method according to any one of claims 204-210, wherein the method comprises co-culturing the TILs with the feeder cells after contacting the TILs with the CD28 agonist for about 6 hours, about 12 hours, about 24 hours, about 48 hours, or about 72 hours.

**212.**

The method according to any one of claims 204-211, wherein the feeder cells comprise antigen-presenting cells.

**213.**

The method according to any one of claims 204-212, wherein the feeder cells comprise one or more cells selected from the group consisting of: peripheral mononuclear cells, dendritic cells, and artificial antigen-presenting cells.

**214.**

The method according to any one of claims 204-213, wherein the feeder cells are peripheral mononuclear cells.

**215.**

The method according to any one of claims 204-214, wherein the feeder cells are irradiated feeder cells.

**216.**

The method according to any one of claims 204-215, wherein the step of co-culturing the TILs with the feeder cells comprises contacting the surface of the feeder cells with the surface of the TILs.

**217.**

The method according to any one of claims 204-216, wherein the step of co-culturing the TILs with the feeder cells comprises adding the feeder cells into the cell culture medium of the TILs.

**218.**

The method according to any one of claims 204-217, wherein the method comprises adding the feeder cells into the cell culture medium of the TILs at a ratio of the feeder cells to the TILs from about 40:1 to about 400:1.

**219.**

The method according to any one of claims 153-218, wherein the method further comprises subjecting the TILs derived from tumor tissues and not expanded *in vitro* to at least one stage of *in vitro* expansion, wherein the TILs are contacted with the T cell growth factors during the at least one stage of *in vitro* expansion.

**220.**

The method according to claim 219, wherein the method comprises contacting the TILs with the T cell growth factors during the single stage *of in vitro* expansion.

**221.**

The method according to any one of claims 219-220, wherein the method comprises contacting the TILs with the CD28 agonist and the T cell growth factors during the single stage of *in vitro* expansion.

**222.**

The method according to any one of claims 219-221, wherein the method comprises subjecting the TILs derived from tumor tissues and not expanded *in vitro* to a first stage *of in vitro* expansion and a second stage of *in vitro* expansion, and during the second stage of *in vitro* expansion, contacting the TILs with the T cell growth factors.

**223.**

The method according to any one of claims 219-222, wherein the method comprises contacting the TILs with the CD28 agonist and the T cell growth factors substantially simultaneously.

**224.**

The method according to any one of claims 219-223, wherein the T cell growth factors are one or more T cell growth factors selected from the group consisting of: IL-2, IL-7, IL-12, IL-15, IL-21, interferon-$\gamma$, and functionally active fragments thereof.

**225.**

The method according to any one of claims 219-224, wherein the T cell growth factors comprise IL-2 and/or a functionally active fragment thereof.

**226.**

The method according to any one of claims 219-225, wherein the step of contacting the TILs with the T cell growth factors comprises adding the T cell growth factors into the cell culture medium of the TILs.

**227.**

The method according to any one of claims 219-226, wherein the initial concentration of each of the T cell growth factors in the cell culture medium of the TILs is each independently at least about 300 IU/mL.

**228.**

The method according to any one of claims 153-227, wherein the TILs are selected from the group consisting of: TILs derived from tumor tissue debris, TILs derived from lymphatic metastasis debris, TILs derived from pleural effusion, TILs derived from peritoneal effusion, and TILs resuscitated after cryopreservation.

**229.**

The method according to claim 228, wherein the debris has a volume of about 1 mm$^3$ to about 27 mm$^3$.

**230.**

A method for culturing tumor infiltrating lymphocytes (TILs), which comprises:

(A) contacting a first TIL population derived from tumor tissues and not expanded *in vitro* with T cell growth factors, wherein a second TIL population is obtained via the step (A);

(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist, wherein a third TIL population is obtained via the step (B).

**231.**

A method for culturing tumor infiltrating lymphocytes (TILs), which comprises:

(A) resuscitating and/or continuing culturing an *in vitro* TIL population to obtain a second TIL population, wherein the *in vitro* TIL population comprises a TIL population obtained by *in vitro* expansion of the first TIL population derived from tumor tissues and not expanded *in vitro;*

(B) reducing the expression and/or decreasing the activity of two or more proteins of the second TIL population selected from the group consisting of: SOCS1 (suppressor of cytokine signaling 1), TNFAIP3 (tumor necrosis factor, alpha-induced protein 3), CBLB (Cbl Proto-Oncogene B), and ZC3H12A (Zinc Finger CCCH-Type Containing 12A), and contacting the TILs with a CD28 agonist, wherein a third TIL population is obtained via the step (B).

**232.**

The method according to claim 231, wherein the *in vitro* TIL population comprises a TIL population obtained by contacting the first TIL population with T cell growth factors.

**233.**

The method according to any one of claims 231-232, wherein the *in vitro* TIL population comprises a TIL population obtained by cryopreserving the first TIL population.

**234.**

The method according to any one of claims 230-233, wherein the step (A) is carried out for about 7 days to about 14 days.

**235.**

The method according to any one of claims 230-234, wherein the step (B) is carried out for about 7 days to about 14 days.

**236.**

The method according to any one of claims 230-235, wherein compared to TILs with unchanged expression and/or activity of the two or more proteins, TILs obtained by reducing the expression and/or decreasing the activity of the two or more proteins of the TILs show improved TIL properties.

**237.**

The method according to claim 236, wherein the improved TIL properties comprise one or more properties selected from the group consisting of: increased number and expansion capacity of TIL cells, increased proportion of viable cells, enhanced persistence, improved proportion of T cell subpopulations, enhanced cytokine secretion capacity, enhanced tumor cell killing ability, and enhanced T cell receptor (TCR) clonal diversity.

**238.**

The method according to claim 237, wherein the improved proportion of T cell subpopulations comprises one or more selected from the group consisting of: increased proportion of central memory T cells and/or naive T cells, decreased proportion of regulatory T cells, increased proportion of activated T cells, increased proportion of tumor-specific T cells, and increased proportion of stem cell-like T cells.

**239.**

The method according to any one of claims 230-238, wherein compared to corresponding TILs that have not been contacted with the CD28 agonist during the stage *of in vitro* expansion, the TILs that have been contacted with the CD28 agonist during at least one stage of *in vitro* expansion show an improved gene editing effect.

**240.**

The method according to claim 239, wherein the improved gene editing effect comprises an enhanced gene knockout efficiency.

**241.**

The method according to any one of claims 230-240, wherein the step of reducing the expression and/or decreasing the activity of two or more proteins of the TILs comprises introducing a gene regulatory system into the TIL cells.

**242.**

The method according to claim 241, wherein the gene regulatory system is capable of destroying the two or more proteins at the DNA level.

**243.**

The method according to any one of claims 241-242, wherein the gene regulatory system comprises a guide nucleic acid molecule and a zymoprotein.

**244.**

The method according to claim 243, wherein the step of reducing the expression and/or decreasing the activity of two or more proteins of the TILs comprises introducing a ribonucleoprotein complex (RNP) comprising the guide nucleic acid molecule and the zymoprotein into the TILs.

**245.**

The method according to any one of claims 243-244, wherein the zymoprotein comprises a Cas protein, a Cas protein homolog, or functionally active fragments thereof.

**246.**

The method according to any one of claims 243-244, wherein the guide nucleic acid molecule comprises a guide RNA (gRNA).

**247.**

The method according to any one of claims 243-246, wherein the guide nucleic acid molecule is capable of binding to a sequence of the two or more proteins.

**248.**

The method according to any one of claims 230-247, wherein the SOCS1 is a suppressor of Janus kinase-signal transducer and activator of transcription (JAK-STAT) pathway.

**249.**

The method according to any one of claims 230-248, wherein the TNFAIP3 is a suppressor of NF-κB pathway.

**250.**

The method according to any one of claims 230-249, wherein the CBLB is an E3 ubiquitin ligase.

**251.**

The method according to any one of claims 230-250, wherein the ZC3H12A is a protein encoding a zinc finger domain.

**252.**

The method according to any one of claims 243-251, wherein the guide nucleic acid molecule is capable of binding to a region or a fragment thereof defined by any one group of genomic coordinates shown in Tables 1A-1D.

**253.**

The method according to any one of claims 243-252, wherein the guide nucleic acid molecule is capable of binding to a region or a fragment thereof selected from a group as shown below: SEQ ID NOs: 34 to 35, 38 to 39, 42 to 44, and 48 to 50.

**254.**

The method according to any one of claims 243-253, wherein the guide nucleic acid molecule is capable of binding to a sequence consisting of about 15 to about 25 nucleotides upstream of 5' end of a protospacer adjacent motif (PAM) selected from the group consisting of: AGG, TGG, CGG, and GGG.

**255.**

The method according to any one of claims 243-254, wherein the guide nucleic acid molecule comprises a sequence as shown in any one of SEQ ID NOs: 36 to 37, 40 to 41, 45 to 47, and 51 to 58.

**256.**

The method according to any one of claims 230-255, wherein the proportion of cells expressing the two or more proteins is reduced and/or the expression of the two or more proteins in an individual cell is decreased in the TILs obtained by reducing the expression and/or decreasing the activity of the two or more proteins of the TILs, compared to that in TILs with unchanged expression and/or activity of the two or more proteins.

**257.**

The method according to any one of claims 230-256, wherein the proportion of cells expressing the two or more proteins is about 95% or less in the TILs obtained by reducing the expression and/or decreasing the activity of the two or more proteins of the TILs.

**258.**

The method according to any one of claims 230-257, wherein the CD28 agonist comprises an anti-CD28 antibody and/or an antigen-binding fragment thereof, CD80 and/or a functionally active fragment thereof, and/or CD86 and/or a functionally active fragment thereof.

**259.**

The method according to any one of claims 230-258, wherein the method comprises contacting the TILs with the CD28 agonist and the other T cell activators substantially simultaneously.

**260.**

The method according to claim 259, wherein the other T cell activators comprise agonists of one or more targets selected from the group consisting of: CD3, HVEM, CD40L, OX40, and 4-1BB.

**261.**

The method according to any one of claims 259-260, wherein the other T cell activators comprise a CD3 agonist.

**262.**

The method according to any one of claims 259-261, wherein the other T cell activators comprise an anti-CD3 antibody and/or an antigen-binding fragment thereof.

**263.**

The method according to any one of claims 259-262, wherein the step of contacting the TILs with the CD28 agonist and the other T cell activators comprises one or more ways selected from the group consisting of: (1) adding the CD28 agonist and the other T cell activators into the cell culture medium of the TILs; (2) adding engineered cells expressing the CD28 agonist and the other T cell activators into the cell culture medium of the TILs; and (3) adding a solid medium comprising the CD28 agonist and the other T cell activators into the cell culture medium of the TILs.

**264.**

The method according to claim 263, wherein the initial concentration of the other T cell activators in the cell culture medium of the TILs is at least about 30 ng/mL.

**265.**

The method according to any one of claims 263-264, wherein the initial concentration of the other T cell activators in the cell culture medium of the TILs is about 30 ng/mL to about 300 ng/mL.

**266.**

The method according to any one of claims 263-265, wherein the diameter of the solid medium is about 500 nm to about 10 μm.

**267.**

The method according to any one of claims 263-266, wherein the diameter of the solid medium is about 1 nm to about 500 nm.

**268.**

The method according to any one of claims 266-267, wherein the diameter of the solid medium is measured by transmission electron microscopy.

**269.**

The method according to any one of claims 263-268, wherein the solid medium comprises a polymer.

**270.**

The method according to any one of claims 263-269, wherein each mg of the solid medium comprises at least about 25 μg of the CD28 agonist and the other T cell activators.

**271.**

The method according to any one of claims 263-270, wherein the solid medium comprising the CD28 agonist and the other T cell activators is added into the cell culture medium of the TILs at a ratio of the solid medium to the TILs from about 2:1 to about 1:2.

**272.**

The method according to any one of claims 263-271, wherein the solid medium comprising the CD28 agonist and the other T cell activators is added into the cell culture medium of the TILs at a ratio of the solid medium to the TILs from about 1:100 to about 1 :2000.

**273.**

The method according to any one of claims 230-272, wherein the method comprises co-culturing the TILs with the feeder cells after contacting the TILs with the CD28 agonist for at least about 2 hours.

**274.**

The method according to claim 273, wherein the method comprises co-culturing the TILs with the feeder cells after contacting the TILs with the CD28 agonist for about 6 hours to about 72 hours.

**275.**

The method according to any one of claims 273-274, wherein the method comprises co-culturing the TILs with the feeder cells after contacting the TILs with the CD28 agonist for about 12 hours to about 48 hours.

**276.**

The method according to any one of claims 273-275, wherein the method comprises co-culturing the TILs with the feeder cells after contacting the TILs with the CD28 agonist for about 6 hours, about 12 hours, about 24 hours, about 48 hours, or about 72 hours.

**277.**

The method according to any one of claims 273-276, wherein the feeder cells comprise antigen-presenting cells.

**278.**

The method according to any one of claims 273-277, wherein the feeder cells comprise one or more cells selected from the group consisting of: peripheral mononuclear cells, dendritic cells, and artificial antigen-presenting cells.

**279.**

The method according to any one of claims 273-278, wherein the feeder cells are peripheral mononuclear cells.

**280.**

The method according to any one of claims 273-279, wherein the feeder cells are irradiated feeder cells.

**281.**

The method according to any one of claims 273-280, wherein the step of co-culturing the TILs with the feeder cells comprises contacting the surface of the feeder cells with the surface of the TILs.

**282.**

The method according to any one of claims 273-281, wherein the step of co-culturing the TILs with the feeder cells comprises adding the feeder cells into the cell culture medium of the TILs.

**283.**

The method according to any one of claims 273-282, wherein the method comprises adding the feeder cells into the cell culture medium of the TILs at a ratio of the feeder cells to the TILs from about 40:1 to about 400:1.

**284.**

The method according to any one of claims 230-283, wherein the method comprises contacting the TILs with the CD28 agonist and the T cell growth factors substantially simultaneously.

**285.**

The method according to claim 284, wherein the T cell growth factors are one or more T cell growth factors selected from the group consisting of: IL-2, IL-7, IL-12, IL-15, IL-21, interferon-$\gamma$, and functionally active fragments thereof.

**286.**

The method according to any one of claims 284-285, wherein the T cell growth factors comprise IL-2 and/or a functionally active fragment thereof.

**287.**

The method according to any one of claims 284-286, wherein the step of contacting the TILs with the T cell growth factors comprises adding the T cell growth factors into the cell culture medium of the TILs.

**288.**

The method according to any one of claims 284-287, wherein the initial concentration of each of the T cell growth factors in the cell culture medium of the TILs is each independently at least about 300 IU/mL.

**289.**

The method according to any one of claims 230-288, wherein the TILs are selected from the group consisting of: TILs derived from tumor tissue debris, TILs derived from lymphatic metastasis debris, TILs derived from pleural effusion, TILs derived from peritoneal effusion, and TILs resuscitated after cryopreservation.

**290.**

The method according to claim 289, wherein the debris has a volume of about 1 mm$^3$ to about 27 mm$^3$.

**291.**

A tumor infiltrating lymphocyte (TIL) obtained by the method according to any one of claims 1-290.

**292.**

A composition comprising the TIL of claim 291.

**293.**

A pharmaceutical composition, comprising the TIL of claim 291 and/or the composition of claim 292, and optionally a pharmaceutically acceptable carrier.

**294.**

A method for affecting the tumor cell growth, comprising administering to a subject the TIL of claim 291, the composition of claim 292 and/or the pharmaceutical composition of claim 293.

**295.**

Use of the TIL of claim 291, the composition of claim 292 and/or the pharmaceutical composition of claim 293 in the manufacture of drugs for preventing and/or treating a tumor.

**296.**

The use according to claim 295, wherein the tumor is a solid tumor.

**297.**

The use according to any one of claims 295-296, wherein the tumor is one or more tumors selected from the group consisting of: melanoma, ovarian cancer, cervical cancer, lung cancer, bladder cancer, breast cancer, head and neck cancer, pancreatic cancer, liver cancer, gastric cancer, colorectal cancer, and kidney cancer.

## Donor I-A-1

## Donor I-A-2

## Donor I-A-3

## Donor I-A-4

FIG. 1

## Donor I-B-1

## Donor I-B-1

## Donor I-B-2

## Donor I-B-2

FIG. 2

FIG. 3

FIG. 4

FIG. 5

FIG. 6

FIG. 7

FIG. 8

# Donor II-A

FIG. 9

FIG. 10

Donor II-B-2

Donor II-B-2

Donor II-B-2

FIG. 11

FIG. 12

FIG. 13

FIG. 14

Donor II-E

FIG. 15

Donor II-F

FIG. 16

Donor II-G-1

FIG. 17

## Donor II-G-2

FIG. 18

## Donor II-G-3

FIG. 19

## Donor II-G-4

FIG. 20

## Donor II-H

## Donor II-H

## Donor II-H

FIG. 21

FIG. 22

Donor II-I-2

Donor II-I-2

Donor II-I-2

FIG. 23

Donor II-J-1

FIG. 24

FIG. 25

FIG. 26

Donor II-K-2

FIG. 27

Donor II-K-3

FIG. 28

Donor 504

FIG. 29A

Donor 713

FIG. 29B

Donor 504

FIG. 30A

Donor 713

FIG. 30B

Donor 316

FIG. 30C

Donor 713

FIG. 30D

Donor 504

FIG. 31A

Donor 316

FIG. 31B

Donor 713

FIG. 31C

Donor 316

FIG. 31D

Donor 713

FIG. 31E

Donor 504

FIG. 32A

Donor 713

FIG. 32B

Donor 713

FIG. 32C

Donor 316

FIG. 32D

Donor 316

FIG. 32E

Donor 504

FIG. 33A

Donor 713

FIG. 33B

Donor 504

FIG. 34A

Donor 713

FIG. 34B

Donor 316

FIG. 34C

Donor 713

FIG. 34D

Donor 316

FIG. 34E

Donor 713

FIG. 34F

Donor 504  E:T=1:1

FIG. 35A

Donor 504  E:T=1:3

FIG. 35B

Donor 713 E:T=1:1

FIG. 35C

Donor 504 E:T=1:1

FIG. 36A

Donor 504  E : T = 1 : 3

FIG. 36B

Donor 713  E : T = 1 : 1

FIG. 36C

Donor 316    E : T=1 : 1

FIG. 36D

Donor 504    E : T=1 : 1

FIG. 37A

Donor 504   E : T = 1 : 3

FIG. 37B

Donor 713   E : T = 1 : 1

FIG. 37C

Donor 713   E : T = 1 : 3

FIG. 37D

Donor 607   E : T = 1 : 1

FIG. 37E

Donor 316   E : T = 1 : 1

FIG. 37F

Donor 504   E : T = 1 : 1

FIG. 38A

Donor 504    E : T=1 : 3

FIG. 38B

Donor 713   E : T=1 : 1

FIG. 38C

Donor 713  E : T＝1 : 3

FIG. 38D

Donor 316  E : T＝1 : 1

FIG. 38E

Donor 504   E : T = 1 : 1

FIG. 39A

Donor 504   E : T = 1 : 3

FIG. 39B

Donor 713　E:T=1:1

FIG. 39C

Donor 607　E:T=1:1

FIG. 39D

Donor 504 E:T=1:1

FIG. 40A

Donor 504 E:T=1:3

FIG. 40B

Donor 713   E : T = 1 : 1

FIG. 40C

Donor 713   E : T = 1 : 3

FIG. 40D

Donor 607  E:T=1:1

FIG. 40E

Donor 316  E:T=1:3

FIG. 40F

Donor 316 E : T=1 : 1

FIG. 40G

Donor 504 CD4+ T cell apoptosis

FIG. 41A

Donor 504   CD8+ T cell apoptosis

FIG. 41B

Proportion of CD38+ in CD8+ T cells
from Donor 504

FIG. 42A

Expression of PD-1+ in CD4+ T cells
from Donor 713

FIG. 42B

Proportion of LAG-3+ in CD8+ T cells
from Donor 713

FIG. 42C

Donor 316CM CD8+ central memory T cells

FIG. 43A

Donor 713 CD4+ central memory T cells

FIG. 43B

Donor 713 CD8+ central memory T cells

FIG. 43C

Proportion of PD-1+ in CD4+ T cells from Donor 504

FIG. 43D

Expression of depletion markers in
CD8+ T cells from Donor 504

FIG. 43E

Proportion of PD-1+ in CD4+ T cells
from Donor 713

FIG. 43F

Proportion of CD101+ in CD8+ T cells
from Donor 713

FIG. 43G

Expression of depletion markers in CD4+ T cells
from Donor 713

FIG. 43H

Proportion of CD101+ in CD8+ T cells
from Donor 713

FIG. 43I

Donor 713 CD4+ central memory T cells

FIG. 44A

Donor 713 CD8+ central memory T cells

FIG. 44B

Expression of depletion markers in CD4+ T cells
from Donor 504

FIG. 44C

Expression of depletion markers in CD8+ T cells
from Donor 504

FIG. 44D

Proportion of PD-1+ in CD4+ T cells from Donor 713

FIG. 44E

FIG. 44F

FIG. 44G

Proportion of PD-1+ in CD8+ T cells
from Donor 607

FIG. 44H

Donor 316 CD8+ central memory T cells

FIG. 45A

Donor 713 CD4+ central memory T cells

FIG. 45B

Donor 713 CD8+ central memory T cells

FIG. 45C

Expression of depletion markers in CD4+ T cells from Donor 504

FIG. 45D

Expression of depletion markers in CD8+ T cells from Donor 504

FIG. 45E

Proportion of PD-1+ in CD4+ T cells
from Donor 713

FIG. 45F

Proportion of CD101+ in CD8+ T cells
from Donor 713

FIG. 45G

Expression of depletion markers in CD4+ T cells from Donor 713

FIG. 45H

Proportion of CD101+ in CD8+ T cells from Donor 713

FIG. 45I

FIG. 46A

FIG. 46B

Expression of depletion markers in CD4+ T cells
from Donor 713

FIG. 46C

Expression of depletion markers in CD8+ T cells
from Donor 713

FIG. 46D

Donor 504 CD8+ Naive T cells

FIG. 47A

Donor 713 CD8+ Central memory T cells

FIG. 47B

Proportion of PD-1+ in CD4+ T cells
from Donor 504

FIG. 47C

Expression of depletion markers in CD8+ T cells
from Donor 504

FIG. 47D

Proportion of TIM-3+ in CD4+ T cells
from Donor 713

FIG. 47E

Proportion of CD101+ in CD8+ T cells
from Donor 713

FIG. 47F

Proportion of CD101+ in CD8+ T cells
from Donor 607

FIG. 47G

Expression of depletion markers in CD4+ T cells
from Donor 713

FIG. 47H

Expression of depletion markers in CD8+ T cells
from Donor 713

FIG. 47I

Expression of cytokines in CD4+ T cells
from Donor 713

FIG. 48A

Expression of cytokines in CD8+ T cells
from Donor 713

FIG. 48B

Expression of cytokines in CD4+ T cells
from Donor 713

FIG. 49A

Expression of cytokines in CD8+ T cells
from Donor 713

FIG. 49B

Expression of cytokines in CD4+ T cells
from Donor 316

FIG. 49C

Expression of cytokines in CD8+ T cells
from Donor 316

FIG. 49D

Expression of cytokines in CD4+ T cells
from Donor 713

FIG. 49E

Expression of cytokines in CD8+ T cells
from Donor 713

FIG. 49F

Expression of cytokines in CD4+ T cells
from Donor 713

FIG. 50A

Expression of cytokines in CD8+ T cells
from Donor 713

NT

SC3 + TP4

FIG. 50B

Expression of cytokines in CD4+ T cells
from Donor 316

NT

SC3 + TP4

FIG. 50C

Expression of cytokines in CD8+ T cells
from Donor 316

FIG. 50D

Expression of cytokines in CD4+ T cells
from Donor 713

FIG. 50E

Expression of cytokines in CD8+ T cells
from Donor 713

FIG. 50F

Expression of cytokines in CD4+ T cells
from Donor 713

FIG. 51A

FIG. 51B

FIG. 51C

Expression of cytokines in CD8+ T cells
from Donor 316

FIG. 51D

Expression of cytokines in CD4+ T cells
from Donor 713

FIG. 51E

FIG. 51F

FIG. 52A

FIG. 52B

FIG. 52C

Expression of cytokines in CD8+ T cells from Donor 607

FIG. 52D

Expression of cytokines in CD4+ T cells from Donor 607

FIG. 53A

Expression of cytokines in CD8+ T cells
from Donor 607

FIG. 53B

Expression of cytokines in CD4+ T cells
from Donor 713

FIG. 53C

FIG. 53D

FIG. 53E

FIG. 53F

FIG. 53G

Expression of cytokines in CD8+ T cells from Donor 607

Legend: NT, TP4+ZC3

FIG. 53H

Legend: 0h, 24h, 48h

FIG. 54A

FIG. 54B

FIG. 54C

FIG. 54D

FIG. 54E

FIG. 54F

FIG. 54G

CD8+41BB+

CD4+41BB+

FIG. 54H

FIG. 54I

FIG. 54J

FIG. 54K

FIG. 54L

CD8+

FIG. 54M

Tcm

FIG. 54N

NKT

FIG. 54O

FIG. 54P

FIG. 54Q

FIG. 55A

FIG. 55B

Body weight change curve of mice

FIG. 55C

Change curve of CD3+ T cells in mouse plasma

FIG. 55D

FIG. 55E

FIG. 55F

FIG. 56A

FIG. 56B

FIG. 56C

Change curve of CD3+ T cells in mouse plasma

FIG. 56D

T cell memory phenotype

FIG. 56E

Donor 713

FIG. 57A

Donor 309

FIG. 57B

FIG. 57C

FIG. 58A

Donor 107

FIG. 58B

Donor 713

FIG. 58C

FIG. 58D

FIG. 58E

Donor 812

FIG. 58F

Donor 107

FIG. 58G

Donor 309

FIG. 58H

Donor 812

FIG. 58I

Donor 104

FIG. 59A

Donor 107

FIG. 59B

Donor 316

FIG. 59C

Donor 704

FIG. 59D

Donor 812

FIG. 59E

Donor 504

FIG. 60A

Donor 812

FIG. 60B

Donor 309

FIG. 60C

Donor 812

FIG. 60D

Donor 812

FIG. 61

FIG. 62A

FIG. 62B

FIG. 62C

FIG. 62D

FIG. 62E

FIG. 62F

FIG. 62G

FIG. 62H

FIG. 62I

FIG. 62J

FIG. 62K

FIG. 63

FIG. 64A

FIG. 64B

Expression of cytokines in TIL CD4+ T cells derived from donor 104

FIG. 65A

Expression of cytokines in TIL CD8+ T cells derived from donor 104

FIG. 65B

Expression of TNF-α in TIL CD4+ T cells derived from donor 812

FIG. 65C

Expression of TNF-α in TIL CD8+ T cells derived from donor 812

FIG. 65D

FIG. 65E

FIG. 65F

Expression of cytokines in TIL CD8+ T cells
derived from donor 107

FIG. 65G

Expression of CD107a in TIL CD8+ T cells
derived from donor 713

FIG. 65H

FIG. 65I

FIG. 65J

Expression of cytokines in TIL CD4+ T cells derived from donor 316

FIG. 65K

Expression of cytokines in TIL CD8+ T cells derived from donor 316

FIG. 65L

Expression of cytokines in TIL CD4+ T cells
derived from donor 312

FIG. 65M

Expression of cytokines in TIL CD8+ T cells
derived from donor 312

FIG. 65N

FIG. 65O

FIG. 65P

FIG. 65Q

FIG. 65R

Expression of cytokines in CD8+ T cells
derived from donor 812

FIG. 65S

Expression of cytokines in CD4+ T cells
derived from donor 107

FIG. 65T

FIG. 65U

FIG. 65V

FIG. 65W

FIG. 65X

Expression of cytokines in CD8+ T cells
derived from donor 812

FIG. 65Y

Expression of cytokines in TIL CD4+ T cells
derived from donor 104

FIG. 66A

Expression of cytokines in TIL CD8+ T cells
derived from donor 104

FIG. 66B

Expression of cytokines in TIL CD4+ T cells
derived from donor 812

FIG. 66C

Expression of cytokines in TIL CD8+ T cells
derived from donor 812

Legend:
- NT
- TP4+ZC3
- TP2+ZC-11
- TP2+ZC-13
- TP-N20+ZC-11
- TP-N20+ZC-13

FIG. 66D

Expression of cytokines in TIL CD4+ T cells
derived from donor 107

Legend:
- NT
- TP4+ZC3
- TP2+ZC-11
- TP2+ZC-13
- TP-N20+ZC-11

FIG. 66E

FIG. 66F

FIG. 66G

Expression of GZMB in TIL CD8+ T cells
derived from donor 312

FIG. 66H

Expression of cytokines in TIL CD4+ T cells
derived from donor 316

FIG. 66I

Expression of cytokines in TIL CD8+ T cells
derived from donor 316

FIG. 66J

Expression of CD107a in TIL CD4+ T cells
derived from donor 504

FIG. 66K

Expression of IFN-γ in TIL CD4+ T cells
derived from donor 713

FIG. 66L

Expression of GZMB in TIL CD4+ T cells
derived from donor 713

FIG. 66M

Expression of TNF-α in TIL CD8+ T cells
derived from donor 713

FIG. 66N

Expression of TNF-α in TIL CD4+ T cells
derived from donor 713

FIG. 66O

Expression of cytokines in TIL CD4+ T cells derived from donor 709

FIG. 66P

Expression of cytokines in TIL CD4+ T cells derived from donor 812

FIG. 67A

FIG. 67B

FIG. 67C

FIG. 67D

FIG. 67E

Expression of cytokines in CD8+ T cells
derived from donor 309

FIG. 67F

Expression of cytokines in CD4+ T cells
derived from donor 812

FIG. 67G

FIG. 67H

FIG. 68A

FIG. 68B

Expression of depletion molecules in CD4+ T cells
derived from donor 812

FIG. 69A

Expression of CD101 in CD8+ T cells
derived from donor 812

FIG. 69B

Expression of depletion molecules in CD4+ T cells
derived from donor 107

FIG. 69C

Expression of depletion molecules in CD8+ T cells
derived from donor 107

FIG. 69D

Release of IFN-γ in donor 709 after being co-cultured with autologous tumor

FIG. 70A

Release of TNF-α in donor 709 after being co-cultured with autologous tumor

FIG. 70B

Release of IFN-γ in donor 704 after being
co-cultured with autologous tumor

FIG. 70C

FIG. 71A

FIG. 71B

FIG. 71C

FIG. 71D

## INTERNATIONAL SEARCH REPORT

| International application No. |
| --- |
| **PCT/CN2023/073559** |

**A. CLASSIFICATION OF SUBJECT MATTER**

C12N 5/0781(2010.01)i;C12N 5/0783(2010.01)i;C12N 5/078(2010.01)i;A61K 35/17(2015.01)i;A61P 35/00(2006.01)i

According to International Patent Classification (IPC) or to both national classification and IPC

**B. FIELDS SEARCHED**

Minimum documentation searched (classification system followed by classification symbols)

C12N,A61K,A61P

Documentation searched other than minimum documentation to the extent that such documents are included in the fields searched

Electronic data base consulted during the international search (name of data base and, where practicable, search terms used)

CNABS, DWPI, WPABS, CNTXT, WOTXT, EPTXT, USTXT, CNKI, ISI WEB OF SCIENCE, PUBMED, 万方数据库, WANFANG DATABASE: 肿瘤浸润细胞, T细胞激活剂, T细胞生长因子, 细胞因子信号抑制物1, 肿瘤坏死因子a诱导蛋白3, 敲除, 饲养细胞, 外周单个核细胞, 树突状细胞, 人工抗原呈递细胞, TIL, CD3, CD28, CD82, CD86, CD276, 4-1BBL, CD27, CD30, CD134, CD275, CD40, CD258, SOCS1, TNFAIP3, CBLB, ZC3H12A, gRNA, crispr, feeder, PBMC, DC, APC; GenBank+EBI+中国专利生物序列检索平台: 对SEQ ID NOs.34-58进行序列检索, GenBank+EBI+China Patent Biological Sequence Search System: search for SEQ ID NOs.34-58.

**C. DOCUMENTS CONSIDERED TO BE RELEVANT**

| Category* | Citation of document, with indication, where appropriate, of the relevant passages | Relevant to claim No. |
| --- | --- | --- |
| X | WO 2019136459 A1 (IOVANCE BIOTHERAPEUTICS, INC.) 11 July 2019 (2019-07-11) see description, paragraphs 5-130 | 1-9, 16, 18, 24-101, 108, 110, 116-162, 169, 171, 177-241, 248, 250, 256-293, 295-297 |
| Y | WO 2019136459 A1 (IOVANCE BIOTHERAPEUTICS, INC.) 11 July 2019 (2019-07-11) see description, paragraphs 5-130 | 10-15, 17, 19-23, 102-107, 109, 111-115, 163-168, 170, 172-176, 242-247, 249, 251-255 |
| Y | CN 112040987 A (KSQ THERAPEUTICS, INC.) 04 December 2020 (2020-12-04) see claims 1-3, 41, 52, 59, 148 | 10-15, 17, 19-23, 102-107, 109, 111-115, 163-168, 170, 172-176, 242-247, 249, 251-255 |

☑ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **04 April 2023** | **26 April 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2023/073559** |

**C.     DOCUMENTS CONSIDERED TO BE RELEVANT**

| Category* | Citation of document, with indication, where appropriate, of the relevant passages | Relevant to claim No. |
| --- | --- | --- |
| Y | CN 112041433 A (KSQ THERAPEUTICS, INC.) 04 December 2020 (2020-12-04) see claims 1-3, 52, 54, 55 | 10-15, 17, 19-23, 102-107, 109, 111-115, 163-168, 170, 172-176, 242-247, 249, 251-255 |
| Y | US 2019284553 A1 (KSQ THERAPEUTICS, INC.) 19 September 2019 (2019-09-19) see claims 289-293, 314, 321, 323 | 10-15, 17, 19-23, 102-107, 109, 111-115, 163-168, 170, 172-176, 242-247, 249, 251-255 |
| Y | CN 113396216 A (KSQ THERAPEUTICS, INC.) 14 September 2021 (2021-09-14) see claims 1-5, 10-27 | 10-15, 17, 19-23, 102-107, 109, 111-115, 163-168, 170, 172-176, 242-247, 249, 251-255 |
| Y | CN 112040986 A (KSQ THERAPEUTICS, INC.) 04 December 2020 (2020-12-04) see claims 1-15, 59-60 | 10-15, 17, 19-23, 102-107, 109, 111-115, 163-168, 170, 172-176, 242-247, 249, 251-255 |
| A | CN 111836887 A (IOVANCE BIOTHERAPEUTICS, INC.) 27 October 2020 (2020-10-27) see claims 1-70 | 1-293, 295-297 |
| A | CN 112368003 A (IOVANCE BIOTHERAPEUTICS, INC.) 12 February 2021 (2021-02-12) see claims 1-214 | 1-293, 295-297 |
| A | QIN, Y. et al. "Immune Profiling of Uveal Melanoma Identifies a Potential Signature Associated with Response to Immunotherapy" *Journal for ImmunoTherapy of Cancer,* Vol. 8, No. (2), 17 November 2020 (2020-11-17), pages 1-12, see abstract | 1-293, 295-297 |
| A | 孙黎飞等 (SUN, Lifei et al.). "T细胞生长因子活化癌性腹水中TIL对树突状细胞抗原提呈的应答能力 (Capacity of Specific Response of The TIL Activated Using T Cell Growth Factor in Malignant Ascites to Dendritic Ceil Presenting Antigen)" 肿瘤防治杂志 *(Chinese Journal of Cancer Prevention and Treatment),* Vol. 9, No. (1), 25 March 2002 (2002-03-25), pages 35-38, see abstract | 1-293, 295-297 |

Form PCT/ISA/210 (second sheet) (July 2022)

**INTERNATIONAL SEARCH REPORT**

| International application No. |
| --- |
| **PCT/CN2023/073559** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
|---|
| **PCT/CN2023/073559** |

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **294**
   because they relate to subject matter not required to be searched by this Authority, namely:

   The subject matter of claim 294 relates to a method for treatment of diseases, which belongs to the subject matter for which a search is not required by the International Searching Authority as defined in PCT Rule 39.1.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/073559**

| Patent document cited in search report | | | Publication date (day/month/year) | Patent family member(s) | | | Publication date (day/month/year) |
|---|---|---|---|---|---|---|---|
| WO | 2019136459 | A1 | 11 July 2019 | None | | | |
| CN | 112040987 | A | 04 December 2020 | WO | 2019178421 | A1 | 19 September 2019 |
| | | | | JP | 2021518161 | A | 02 August 2021 |
| | | | | AU | 2019236204 | A1 | 08 October 2020 |
| | | | | KR | 20200130826 | A | 20 November 2020 |
| | | | | CA | 3093919 | A1 | 19 September 2019 |
| CN | 112041433 | A | 04 December 2020 | EP | 3765608 | A1 | 20 January 2021 |
| | | | | EP | 3765608 | A4 | 12 January 2022 |
| | | | | JP | 2021518162 | A | 02 August 2021 |
| | | | | AU | 2019236205 | A1 | 08 October 2020 |
| | | | | KR | 20200133218 | A | 26 November 2020 |
| | | | | WO | 2019178422 | A1 | 19 September 2019 |
| | | | | CA | 3093968 | A1 | 19 September 2019 |
| | | | | US | 2019284529 | A1 | 19 September 2019 |
| | | | | BR | 112020018573 | A2 | 29 December 2020 |
| US | 2019284553 | A1 | 19 September 2019 | BR | 112020018658 | A2 | 29 December 2020 |
| | | | | US | 2020181610 | A1 | 11 June 2020 |
| | | | | US | 11111493 | B2 | 07 September 2021 |
| | | | | EP | 3765094 | A1 | 20 January 2021 |
| | | | | EP | 3765094 | A4 | 22 December 2021 |
| | | | | US | 2022315921 | A1 | 06 October 2022 |
| | | | | US | 2021388349 | A1 | 16 December 2021 |
| | | | | US | 11421228 | B2 | 23 August 2022 |
| CN | 113396216 | A | 14 September 2021 | AU | 2020217715 | A1 | 23 September 2021 |
| | | | | KR | 20210138587 | A | 19 November 2021 |
| | | | | JP | 2022519595 | A | 24 March 2022 |
| | | | | WO | 2020163365 | A2 | 13 August 2020 |
| | | | | WO | 2020163365 | A3 | 24 September 2020 |
| | | | | SG | 11202108452 | WA | 29 September 2021 |
| | | | | EP | 3920942 | A2 | 15 December 2021 |
| | | | | EP | 3920942 | A4 | 18 January 2023 |
| | | | | IL | 285307 | A | 30 September 2021 |
| | | | | CA | 3128823 | A1 | 13 August 2020 |
| | | | | US | 2020347386 | A1 | 05 November 2020 |
| CN | 112040986 | A | 04 December 2020 | BR | 112020018620 | A2 | 29 December 2020 |
| | | | | JP | 2021518160 | A | 02 August 2021 |
| | | | | AU | 2019234926 | A1 | 08 October 2020 |
| | | | | KR | 20200133219 | A | 26 November 2020 |
| | | | | US | 2022267727 | A1 | 25 August 2022 |
| | | | | US | 11459544 | B2 | 04 October 2022 |
| | | | | WO | 2019178420 | A1 | 19 September 2019 |
| | | | | EP | 3765092 | A1 | 20 January 2021 |
| | | | | EP | 3765092 | A4 | 12 January 2022 |
| | | | | US | 2019284530 | A1 | 19 September 2019 |
| | | | | US | 11261428 | B2 | 01 March 2022 |
| | | | | CA | 3093915 | A1 | 19 September 2019 |
| | | | | US | 2022220442 | A1 | 14 July 2022 |
| CN | 111836887 | A | 27 October 2020 | KR | 20200119242 | A | 19 October 2020 |
| | | | | MX | 2020007046 | A | 07 September 2020 |

Form PCT/ISA/210 (patent family annex) (July 2022)

**INTERNATIONAL SEARCH REPORT**
Information on patent family members

International application No.

**PCT/CN2023/073559**

| Patent document cited in search report | | | Publication date (day/month/year) | Patent family member(s) | | | Publication date (day/month/year) |
|---|---|---|---|---|---|---|---|
| | | | | WO | 2019136456 | A1 | 11 July 2019 |
| | | | | TW | 201938177 | A | 01 October 2019 |
| | | | | CA | 3087771 | A1 | 11 July 2019 |
| | | | | IL | 275724 | A | 31 August 2020 |
| | | | | AU | 2019205823 | A1 | 30 July 2020 |
| | | | | JP | 2021509586 | A | 01 April 2021 |
| | | | | MA | 51636 | A | 18 November 2020 |
| | | | | EP | 3737743 | A1 | 18 November 2020 |
| | | | | BR | 112020013848 | A2 | 01 December 2020 |
| | | | | SG | 11202006541 | UA | 28 August 2020 |
| | | | | US | 2022204932 | A1 | 30 June 2022 |
| CN | 112368003 | A | 12 February 2021 | US | 2022403334 | A1 | 22 December 2022 |
| | | | | AU | 2019257749 | A1 | 22 October 2020 |
| | | | | CA | 3098303 | A1 | 31 October 2019 |
| | | | | US | 2022010278 | A1 | 13 January 2022 |
| | | | | US | 11384337 | B2 | 12 July 2022 |
| | | | | KR | 20210005138 | A | 13 January 2021 |
| | | | | US | 2022396769 | A1 | 15 December 2022 |
| | | | | MX | 2020011134 | A | 11 November 2020 |
| | | | | US | 2022396768 | A1 | 15 December 2022 |
| | | | | IL | 278231 | A | 30 November 2020 |
| | | | | TW | 202014195 | A | 16 April 2020 |
| | | | | JP | 2021521846 | A | 30 August 2021 |
| | | | | SG | 11202010319 | RA | 27 November 2020 |
| | | | | MA | 52533 | A | 03 March 2021 |
| | | | | BR | 112020021660 | A2 | 02 February 2021 |
| | | | | US | 2021130779 | A1 | 06 May 2021 |
| | | | | US | 2022403333 | A1 | 22 December 2022 |
| | | | | EP | 3784254 | A1 | 03 March 2021 |
| | | | | WO | 2019210131 | A1 | 31 October 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TAY, R. E** ; **RICHARDSON, E. K**. *Cancer Gene Therapy*, 2020, 1-13
- **CHENG-PRUSOFF**. *Biochem. Pharmacol.*, 1973, vol. 22, 3099
- **BRINKMAN et al.** *Nucl. Acids Res.*, 2014